(19) Europäisches Patentamt
European Patent Office
Office européen des brevets

(11) EP 2 804 870 B1

(12) **EUROPEAN PATENT SPECIFICATION**

(45) Date of publication and mention of the grant of the patent:
**30.11.2016 Bulletin 2016/48**

(21) Application number: **13701416.3**

(22) Date of filing: **18.01.2013**

(51) Int Cl.:
***C07D 513/04*** $^{(2006.01)}$ ***A61K 31/5517*** $^{(2006.01)}$
***A61P 25/28*** $^{(2006.01)}$

(86) International application number:
**PCT/EP2013/050945**

(87) International publication number:
**WO 2013/107862 (25.07.2013 Gazette 2013/30)**

(54) **2-AMINO-4,5,6,8-TETRAHYDROPYRAZOLO[3,4-b]THIAZOLO [4,5-d]AZEPINE DERIVATIVES AND THEIR USE AS ALLOSTERIC MODULATORS OF METABOTROPIC GLUTAMATE RECEPTORS**

2-AMINO-4,5,6,8-TETRAHYDROPYRAZOLO-[3,4-B-]THIAZOLO-[4,5-D-]AZEPIN-DERIVATE UND DEREN VERWENDUNG ALS ALLOSTERISCHE MODULATOREN VON METABOTROPEN GLUTAMATREZEPTOREN

2-AMINO-4,5,6,8-TÉTRAHYDROPYRAZOLO[3,4-B]THIAZOLO[4,5-D]AZÉPINE ET LEUR UTILISATION COMME MODULATEURS ALLOSTÉRIQUES DES RÉCEPTEURS MÉTABOTROPIQUES AU GLUTAMATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.01.2012 GB 201200800**
**18.09.2012 GB 201216657**

(43) Date of publication of application:
**26.11.2014 Bulletin 2014/48**

(73) Proprietor: **ADDEX Pharma S.A.**
**1228 Geneva (CH)**

(72) Inventors:
- **BOLÉA, Christelle**
**CH-1228 Geneva (CH)**
- **BOUDOU, Cédric**
**CH-1228 Geneva (CH)**
- **CELANIRE, Sylvain**
**CH-1228 Geneva (CH)**
- **DARMENCY, Vincent**
**CH-1228 Geneva (CH)**
- **MORDANT, Céline**
**CH-1228 Geneva (CH)**
- **PERICOLLE, Vincent**
**CH-1228 Geneva (CH)**
- **REGEREAU, Yannick**
**CH-1228 Geneva (CH)**
- **ROCHER, Jean-Philippe**
**CH-1228 Geneva (CH)**
- **SOUISSI, Radouane**
**CH-1228 Geneva (CH)**
- **TANG, Lam**
**CH-1228 Geneva (CH)**

(74) Representative: **Davies, Jonathan Mark**
**Reddie & Grose LLP**
**16 Theobalds Road**
**London WC1X 8PL (GB)**

(56) References cited:
**WO-A1-2010/079238** **WO-A1-2010/079239**
**WO-A2-2012/009001**

- **WERMUTH C G: "MOLECULAR VARIATIONS BASED ON ISOSTERIC REPLACEMENTS", PRACTICE OF MEDICINAL CHEMISTRY, XX, XX, 1 January 1996 (1996-01-01), pages 203-237, XP002190259,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 804 870 B1

## Description

FIELD OF THE INVENTION

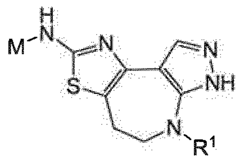

(I)

The present invention relates to novel compounds of Formula (I), wherein M and $R^1$ are defined as in Formula (I); invention compounds are modulators of metabotropic glutamate receptors - subtype 4 ("$mGluR_4$") which are useful for the treatment or prevention of central nervous system disorders as well as other disorders modulated by $mGluR_4$ receptors. The invention is also directed to pharmaceutical compositions and the use of such compounds for the prevention and treatment of such diseases in which $mGluR_4$ is involved.

BACKGROUND OF THE INVENTION

Glutamate is the major amino-acid transmitter in the mammalian central nervous system (CNS). Glutamate plays a major role in numerous physiological functions, such as learning and memory but also sensory perception, development of synaptic plasticity, motor control, respiration and regulation of cardiovascular function. Furthermore, glutamate is at the center of several different neurological and psychiatric diseases, where there is an imbalance in glutamatergic neurotransmission.

Glutamate mediates synaptic neurotransmission through the activation of ionotropic glutamate receptor channels (iGluRs), namely the NMDA, AMPA and kainate receptors which are responsible for fast excitatory transmission (Nakanishi S. et al., (1998) Brain Res. Rev., 26(2-3):230-235).

In addition, glutamate activates metabotropic glutamate receptors (mGluRs) which have a more modulatory role that contributes to the fine-tuning of synaptic efficacy.

The mGluRs are G protein-coupled receptors (GPCRs) with seven-transmembrane spanning domains and belong to GPCR family 3 along with the calcium-sensing, GABAb and pheromone receptors.

The mGluR family is composed of eight members. They are classified into three groups (group I comprising $mGluR_1$ and $mGluR_5$; group II comprising $mGluR_2$ and $mGluR_3$; group III comprising $mGluR_4$, $mGluR_6$, $mGluR_7$ and $mGluR_8$) according to sequence homology, pharmacological profile and nature of intracellular signalling cascades activated (Schoepp D.D. et al., (1999) Neuropharmacology, 38(10):1431-1476).

Glutamate activates the mGluRs through binding to the large extracellular amino-terminal domain of the receptor, herein called the orthosteric binding site. This activation induces a conformational change of the receptor which results in the activation of the G-protein and intracellular signalling pathways.

In the central nervous system, $mGluR_4$ receptors are expressed most intensely in the cerebellar cortex, basal ganglia, sensory relay nuclei of the thalamus and hippocampus (Bradley et al., (1999) Journal of Comparative Neurology, 407:33-46; Corti et al., (2002) Neuroscience, 110:403-420). The $mGluR_4$ subtype is negatively coupled to adenylate cyclase via activation of the $G\alpha i/o$ protein, is expressed primarily on presynaptic terminals, functioning as an autoreceptor or heteroceptor and activation of $mGluR_4$ leads to decreases in transmitter release from presynaptic terminals (Corti et al., (2002) Neuroscience, 110:403-420; Millan et al., (2002) Journal of Biological Chemistry, 277:47796-47803; Valenti et al., (2003) Journal of Neuroscience, 23:7218-7226).

Orthosteric agonists of $mGluR_4$ are not selective and activate the other Group III mGluRs (Schoepp et al., (1999) Neuropharmacology, 38:1431-1476). The Group III orthosteric agonist L-AP4 (L-2-amino-4-phosphonobutyrate) was able to reduce motor deficits in animal models of Parkinson's disease (Valenti et al., (2003) J. Neurosci., 23:7218-7226) and decrease excitotoxicity (Bruno et al., (2000) J. Neurosci., 20:6413-6420) and these effects appear to be mediated through $mGluR_4$ (Marino et al., (2005) Curr. Topics Med. Chem., 5:885-895). In addition to L-AP4, ACPT-1, another selective group III mGluR agonist has been shown to caused a dose and structure-dependent decrease in haloperidol-induced catalepsy and attenuated haloperidol-increased Proenkephalin mRNA expression in the striatum (Konieczny et al., (2007) Neuroscience, 145:611-620). Furthemore, Lopez et al. (2007, J. Neuroscience, 27:6701-6711) have shown that bilateral infusions of ACPT-I or L-AP4 into the globus pallidus fully reversed the severe akinetic deficits produced by 6-hydroxydopamine lesions of nigrostriatal dopamine neurons in a reaction-time task without affecting the performance of controls. In addition, the reversal of haloperidol-induced catalepsy by intrapallidal ACPT-1 was prevented

by concomitant administration of a selective group III receptor antagonist (*RS*)-alpha-cyclopropyl-4-phosphonophenylglycine. The opposite effects produced by group III mGluR activation in the SNr strongly suggest a role of $mGluR_4$ rather than other mGluR receptor sub-types in normalizing basal ganglia activity (Lopez et al. 2007).

These results suggest that, among mGluR subtypes, $mGluR_4$ is believed to be the most interesting novel drug target for the treatment of Parkinson's disease (for a review, see Conn et al., (2005) Nature Review Neuroscience, 6:787-798).

Symptoms of Parkinson's disease appear to be due to an imbalance in the direct and indirect output pathways of the basal ganglia, and reduction of transmission at the inhibitory GABAergic striato-pallidal synapse in the indirect pathway may result in alleviation of these symptoms (Marino et al., (2002) Amino Acids, 23:185-191).

$mGluR_4$ is more abundant in striato-pallidal synapses than in striato-nigral synapses, and its localization suggests function as a presynaptic heteroreceptor on GABAergic neurons (Bradley et al., (1999) Journal of Comparative Neurology, 407:33-46) suggesting that selective activation or positive modulation of $mGluR_4$ would decrease GABA release in this synapse thereby decreasing output of the indirect pathway and reducing or eliminating the Parkinson's disease symptoms. Classical treatment of Parkinsonism typically involves the use of levodopa combined with carbidopa (SINEMET™) or benserazide (MADOPAR™). Dopamine agonists such as bromocriptine (PARLODEL™), lisuride and pergolide (CELANCE™) act directly on dopamine receptors and are also used for the treatment of Parkinsonism. These molecules have the same side-effect profile as levodopa.

A new avenue for developing selective compounds acting at mGluRs is to identify molecules that act through allosteric mechanisms, modulating the receptor by binding to a site different from the highly conserved orthosteric binding site.

Positive allosteric modulators of mGluRs have emerged recently as novel pharmacological entities offering this attractive alternative. This type of molecule has been discovered for $mGluR_1$, $mGluR_2$, $mGluR_4$, $mGluR_5$, $mGluR_7$ and $mGluR_8$ (Knoflach F. et al. (2001) Proc. Natl. Acad. Sci. USA, 98:13402-13407; Johnson M.P. et al., (2002) Neuropharmacology, 43:799-808; O'Brien J.A. et al., (2003) Mol. Pharmacol., 64:731-740; Johnson M.P. et al., (2003) J. Med. Chem., 46:3189-3192; Marino M.J. et al., (2003) Proc. Natl. Acad. Sci. USA, 100:13668-13673; Mitsukawa K. et al., (2005) Proc. Natl. Acad. Sci. USA, 102(51):18712-18717; Wilson J. et al., (2005) Neuropharmacology, 49:278; for a review see Mutel V., (2002) Expert Opin. Ther. Patents, 12:1-8; Kew J.N., (2004) Pharmacol. Ther., 104(3):233-244; Johnson M.P. et al., (2004) Biochem. Soc. Trans., 32:881-887; recently Ritzen A., Mathiesen, J.M. and Thomsen C., (2005) Basic Clin. Pharmacol. Toxicol., 97:202-213).

In particular molecules have been described as $mGluR_4$ positive allosteric modulators (Maj et al., (2003) Neuropharmacology, 45:895-906; Mathiesen et al., (2003) British Journal of Pharmacology, 138:1026-1030). It has been demonstrated that such molecules have been characterized in *in vitro* systems as well as in rat brain slices where they potentiated the effect of L-AP4 in inhibiting transmission at the striatopallidal synapse. These compounds do not activate the receptor by themselves (Marino et al., (2003) Proc. Nat. Acad. Sci. USA, 100:13668-13673). Rather, they enable the receptor to produce a maximal response to a concentration of glutamate or the Group III orthosteric agonist L-AP4 which by itself induces a minimal response.

PHCCC (*N*-phenyl-7-(hydroxyimino)cyclopropa[*b*]chromen-l*a*-carboxamide), a positive allosteric modulator of $mGluR_4$ not active on other mGluRs (Maj et al., (2003) Neuropharmacology, 45:895-906), has been shown to be efficacious in animal models of Parkinson's disease thus representing a potential novel therapeutic approach for Parkinson's disease as well as for other motor disorders and disturbances (Marino et al., (2003) Proc. Nat. Acad. Sci. USA, 100:13668-13673), neurodegeneration in Parkinson's disease (Marino et al., (2005) Curr. Topics Med. Chem., 5:885-895; Valenti et al., (2005) J. Pharmacol. Exp. Ther., 313:1296-1304; Vernon et al., (2005) Eur. J. Neurosci., 22:1799-1806, Battaglia et al., (2006) J. Neurosci., 26:7222-7229), and neurodegeneration in Alzheimer's disease or due to ischemic or traumatic insult (Maj et al., (2003) Neuropharmacology, 45:895-906).

PHCCC also has been shown to be active in an animal model of anxiety (Stachowicz et al., (2004) Eur. J. Pharmacol., 498:153-156). Previously, ACPT-1 has been shown to produce a dose-dependent anti-conflict effect after intrahippocampal administration and anti-depressant-like effects in rats after intracerebroventricular administration (Tatarczynska et al., (2002) Pol. J. Pharmacol., 54(6):707-710). More recently, ACPT-1 has also been shown to have anxiolytic-like effects in the stress-induced hyperthermia, in the elevated-plus maze in mice and in the Vogel conflict test in rats when injected intraperitoneally (Stachowicz et al., (2009) Neuropharmacology, 57(3):227-234).

Activation of $mGluR_4$ receptors which are expressed in α- and F-cells in the islets of Langerhans inhibits glucagon secretion. Molecules which activate or potentiate the agonist activity of these receptors may be an effective treatment for hyperglycemia, one of the symptoms of type 2 diabetes (Uehara et al., (2004) Diabetes, 53:998-1006).

The β-chemokine RANTES is importantly involved in neuronal inflammation and has been implicated in the pathophysiology of multiple sclerosis (MS). Activation of Group III mGluRs with L-AP4 reduced the synthesis and release of RANTES in wild-type cultured astrocytes, whereas the ability of L-AP4 to inhibit RANTES was greatly decreased in astrocyte cultures from $mGluR_4$ knockout mice (Besong et al., (2002) Journal of Neuroscience, 22:5403-5411). A high amount of glutamate is found in the brain of patients with MS and glutamate, in addition to inflammatory cytokines, is a

major contributor to neurodegeneration in MS (Stover J.F. et al. (1997) Eur. J. Clin. Invest. 27:1038-1043; Srinivasan R. et al. (2005) Brain, 128:1016-1025; Frigo M. et al. (2012) Current Med. Chem. 19:1295-1299). Experimental autoimmune encephalomyelitis (EAE) is an animal model of brain inflammation and is an animal model of the human CNS demyelinating diseases, including the diseases multiple sclerosis and acute disseminated encephalomyelitis (ADEM). Repeated administration of PHCCC in the myelin oligodendrocyte glycoprotein (MOG) induced EAE model showed attenuated disease in wildtype mice (Fallarino et al., (2010) Nature Medicine, 16:897-902). PHCCC also reduced the number of relapses and their severity when administrated after the first attack in a mice model of relapsing-remitting-EAE (RR-EAE) model of MS. These data suggest that positive allosteric modulators of $mGluR_4$ may be an effective treatment for neuroinflammatory disorders of the central nervous system, including multiple sclerosis and related disorders.

Glutamate receptors play a critical role in the pain pathway and the role of mGluR4 has been demonstrated in several studies (Goudet C. et al. (2009) Brain Res. Rev., 60:43-56; (2008) Pain, 137:112-24). Goudet et al. demonstrated that both the agonist ACPT-1 and mGluR4 PAM (-)-PHCCC were able to dose dependently inhibit the mechanical hypersensitivity associated with different pathological pain states when administered intrathecally. ACPT-1 demonstrated efficacy in the rat formalin test and against the mechanical hyperalgesia elicited in inflammatory pain models and neuropathic pain models. (-)-PHCCC showed efficacy in both the chronic constriction injury model (CCI) and vincristine-induced peripheral neuropathy rat models. Similar results were obtained using the allosteric agonist VU0155041, which dose dependently attenuated hyperalgesia in rat neuropathic pain model (Wang H. et al. (2011) NeuroReport, 22:244-248). Thus, positive allosteric modulators of $mGluR_4$ may be useful as new agents for the treatment of pain or related disorders.

Recent studies using Group III mGluR orthosteric agonists showed evidence of their involvement in schizophrenia as observed in rodent model of psychosis. Orthosteric $mGluR_4$ agonist LSP1-2111 showed antipsychotic-like activity in relevant models for positive symptoms of psychosis, producing a dose-dependent reversal of both MK-801- and amphetamine-induced hyperactivities in rodents. $mGluR_{4/8}$ preferred agonists ACPT-1 (Palucha-Poniewiera et al., (2008) Neuropharmacology, 55:517-24) and LSP1-2111 dose dependently inhibited 5-$HT_{2A}$ agonist DOI-induced head twitches in mice, a serotonergic relevant model for psychosis and hallucination (Wieronska et al., (2012) Psychopharmacology, 220(3):481-494). These results represent strong evidence for additional therapeutic use of $mGluR_4$ activators as potential antipsychotic drugs.

Two different variants of the $mGluR_4$ receptor are expressed in taste tissues and may function as receptors for the umami taste sensation (Monastyrskaia et al., (1999) Br. J Pharmacol., 128:1027-1034; Toyono et al., (2002) Arch. Histol. Cytol., 65:91-96). Thus positive allosteric modulators of $mGluR_4$ may be useful as taste agents, flavour agents, flavour enhancing agents or food additives.

There is anatomical evidence that the majority of vagal afferents innervating gastric muscle express group III mGluRs ($mGluR_4$, $mGluR_6$, $mGluR_7$ and $mGluR_8$) and actively transport receptors to their peripheral endings (Page et al., (2005) Gastroenterology, 128:402-10). Recently, it was shown that the activation of peripheral group III mGluRs inhibited vagal afferent mechanosensitivity *in vitro* which translates into reduced triggering of transient lower esophageal sphincter relaxations and gastroesophageal reflux *in vivo* (Young et al., (2008) Neuropharmacol, 54:965-975). Labelling for $mGluR_4$ and $mGluR_8$ was abundant in gastric vagal afferents in the nodose ganglion, at their termination sites in the nucleus tractus solitarius and in gastric vagal motorneurons. These data suggest that positive allosteric modulators of $mGluR_4$ may be an effective treatment for gastroesophageal reflux disease (GERD) and lower esophageal disorders and gastro-intestinal disorders.

International patent publication WO2005/007096 has described $mGluR_4$ receptor positive allosteric modulators useful, alone or in combination with a neuroleptic agent, for treating or preventing movement disorders. However, none of the specifically disclosed compounds are structurally related to the compounds of the invention.

Recently, new $mGluR_4$ receptor positive allosteric modulators have been described: pyrazolo[3,4-d]pyrimidine derivatives (Niswender et al., (2008) Bioorganic & Medicinal Chemistry Letters, 18(20):5626-5630), functionalized benzylidene hydrazinyl-3-methylquinazoline and *bis*-2,3-dihydroquinazolin-4(1*H*)-one (Williams et al., (2009) Bioorganic & Medicinal Chemistry Letters, 19:962-966) and heterobiarylamides (Engers et al., (2009) Journal of Medicinal Chemistry, 52 (14):4115-4118). Niswender et al. (2008) Molecular Pharmacology, 74(5):1345-1358), described (±)-*cis*-2-(3,5-dichlorophenylcarbamoyl)cyclohexane carboxylic acid as a positive allosteric modulator of $mGluR_4$ also having agonist activity. This moderately active molecule has demonstrated evidence of efficacy following icv injection in rat models of Parkinson’s disease. International patent publications WO2009/010454 and WO2009/010455 have mentioned amido derivatives and novel heteroaromatic derivatives, respectively, as positive allosteric modulators of metabotropic glutamate receptors. The subject of the latter case has been examined in the following article: East Stephen P. et al., (2010) Expert Opin. Ther. Patents, 20(3):441-445. Finally, Williams R. et al., described in (2010) ACS Chemical Neuroscience, 1(6):411-419, the "Re-exploration of the PHCCC scaffold".

International patent publication WO2010/079238 and WO2012/009001 have described novel tricyclic heteroaromatic derivatives and their use as positive allosteric modulators of mGluRs. More recently, a review on recent

progress on the identification of metabotropic glutamate 4 receptor ligands and their potential utility as CNS therapeutics (Robichaud A. et al., (2011) ACS Chem. Neuroscience, 2:433-49) has cited some of the examples described in the WO2010/079238 patent application; Hong S.-P et al., (2011) J. Med. Chem., 54(14):5070-5081, have described tricyclic thiazolopyrazole derivatives as metabotropic glutamate receptor 4 positive allosteric modulators.

The present inventors have discovered novel thiazole compounds of general Formula (I) which, surprisingly, show potent activity and selectivity on the $mGluR_4$ receptor. The compounds of the invention demonstrate advantageous properties over compounds of the prior art. Improvements have been observed in one or more of the following characteristics of the compounds of the invention: the potency on the target, the selectivity for the target, the pharmacokinetic, the brain penetration, and the activity in behavioural models.

Such aminothiazole derivatives are useful for treating or preventing a condition in a mammal, including a human, the treatment or prevention of which is affected or facilitated by the neuromodulatory effect of $mGluR_4$ modulators.

In the case of the treatment of movement disorders such as Parkinson's Disease, the compounds of the invention can be used alone or in combination with an agent selected from the group consisting of: a dopamine precursor such as levodopa, melevodopa and etilevodopa, levodopa with a selective extracerebral decarboxylase inhibitor such as carbidopa and benserazide, a catechol-O-methyl transferase (COMT) inhibitor such as entacapone and tolcapone, a dopamine agonist such as pramipexole, ropinorole, apomorphine, rotigotine, bromocriptine cabergoline and pergolide, a monoamine oxidase B (MAO-B) inhibitor such as selegiline and rasagiline, an anticholinergic agent such as benztropine, trihexyphenidyl, procyclidine and biperiden, a glutamate (NMDA) blocking drug such as amantadine, an adenosine $A_{2a}$ antagonist such as istradefylline and preladenant, an alpha-2 adrenergic antagonist such as atipamezole or fipamezole, a $5\text{-}HT_{1A}$ agonist such as piclozotan or a $5\text{-}HT_{1N}/_{1B}$ partial agonist such as eltoprazine. Finally, in the case of the treatment of iatrogenic movement disorders, the compounds of the invention can be used in combination wih an agent selected from the group of: a butyrophenone neuroleptic agent, a diphenylbutylpiperidine neuroleptic agent, a heterocyclic dibenzazepine neuroleptic agent, an indolone neuroleptic agent, a phenothiazine neuroleptic agent or a thioxanthene neuroleptic agent

## SUMMARY OF THE INVENTION

The invention provides a compound according to claim 1 and a pharmaceutical composition according to claim 2. The invention also provides a compound according to claim 1 or a composition according to claim 2 for use according to claims 3, 4, 5 or 6. The invention also provides the use of a compound according to claim 7.

## DETAILED DESCRIPTION OF THE INVENTION

The invention relates to compounds having metabotropic glutamate receptor 4 modulator activity. In its most general compound aspect, a compound according to Formula (I) is disclosed,

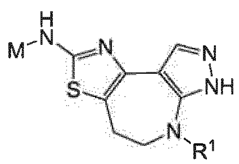

(I)

a pharmaceutically acceptable acid or base addition salt thereof, a stereochemically isomeric form thereof and an *N*-oxide form thereof, wherein:

M is an optionally substituted heteroaryl;

$R^1$ is hydrogen or an optionally substituted radical selected from the group of $-(C_3\text{-}C_7)$cycloalkyl, $-(C_1\text{-}C_6)$alkylene-$(C_3\text{-}C_7)$cycloalkyl, $-(C_3\text{-}C_7)$cycloalkylene-$(C_1\text{-}C_6)$alkyl, $-(C_0\text{-}C_6)$alkylene-$(C_3\text{-}C_7)$spiroalkylene-$(C_0\text{-}C_6)$alkyl, $-(C_1\text{-}C_6)$alkylene-aryl, aryl, $-(C_1\text{-}C_6)$alkylene-heteroaryl, heteroaryl, $-(C_1\text{-}C_6)$alkylene-heterocycle, heterocycle, $-(C_2\text{-}C_6)$alkylene-$OR^2$, $-(C_2\text{-}C_6)$alkylene-$NR^2R^3$, $-(C_0\text{-}C_6)$alkylene-C(=O)-$NR^2R^3$, $-(C_0\text{-}C_6)$alkylene-C(=O)-$R^2$;

$R^2$ and $R^3$ are each independently hydrogen or an optionally substituted radical selected from the group of $-(C_1\text{-}C_6)$haloalkyl, $-(C_1\text{-}C_6)$alkyl, $-(C_1\text{-}C_6)$cyanoalkyl, $-(C_3\text{-}C_7)$cycloalkyl, $-(C_1\text{-}C_6)$alkylene-$(C_3\text{-}C_7)$cycloalkyl, $-(C_3\text{-}C_7)$cycloalkylene-$(C_1\text{-}C_6)$alkyl, $-(C_0\text{-}C_6)$alkylene-$(C_3\text{-}C_7)$spiroalkylene-$(C_0\text{-}C_6)$alkyl, heteroaryl, $-(C_1\text{-}C_6)$alkylene-heteroaryl, aryl, $-(C_1\text{-}C_6)$alkylene-aryl, $-(C_1\text{-}C_6)$alkylene-heterocycle, heterocycle,

-($C_2$-$C_6$)alkylene-O-($C_o$-$C_6$)alkyl and -($C_2$-$C_6$)alkylene-N-(($C_0$-$C_6$)alkyl)$_2$;

$R^2$ and $R^3$ may be taken together to form an optionally substituted 3 to 10 membered carbocyclic or heterocyclic ring; and

provided that the compound is not:

6-(Cyclopropylmethyl)-*N*-(5-fluoropyrimidin-2-yl)-4,5,6,8-tetrahydropyrazolo[3,4-b] thiazolo[4,5-d]azepin-2-amine
6-(2-Methoxyethyl)-N-(4-methylpyrimidin-2-yl)-4,5,6,8-tetrahydropyrazolo[3,4-b] thiazolo[4,5-d]azepin-2-amine
*N*-(4-Methylpyrimidin-2-yl)-6-((tetrahydrofuran-2-yl)methyl)-4,5,6,8-tetrahydro pyrazolo[3,4-*b*]thiazolo[4,5-*d*] azepin-2-amine
6-(1-Methoxypropan-2-yl)-*N*-(4-methylpyrimidin-2-yl)-4,5,6,8-tetrahydropyrazolo [3,4-*b*]thiazolo[4,5-*d*]azepin-2-amine
*N*-(5-Fluoropyrimidin-2-yl)-6-(2-methoxyethyl)-4,5,6,8-tetrahydropyrazolo[3,4-*b*] thiazolo[4,5-*d*]azepin-2-amine
6-(2-Methoxyethyl)-*N*-(6-methylpyridin-2-yl)-4,5,6,8-tetrahydropyrazolo[3,4-*b*] thiazolo[4,5-*d*]azepin-2-amine
*N*-(6-Fluoropyridin-2-yl)-6-(2-methoxyethyl)-4,5,6,8-tetrahydropyrazolo[3,4-*b*] thiazolo[4,5-*d*]azepin-2-amine
*N*-(5-Fluoropyridin-2-yl)-6-(2-methoxyethyl)-4,5,6,8-tetrahydropyrazolo[3,4-*b*] thiazolo[4,5-*d*]azepin-2-amine
6-((1-Methyl-1*H*-pyrazol-3-yl)methyl)-*N*-(4-methylpyrimidin-2-yl)-4,5,6,8-tetrahydro pyrazolo[3,4-*b*]thiazolo[4,5-*d*] azepin-2-amine
N,N-Dimethyl-2-(2-(4-methylpyrimidin-2-ylamino)-4,5-dihydropyrazolo[3,4-*b*] thiazolo[4,5-*d*]azepin-6(8*H*)-yl)acetamide
6-(2-Methoxyethyl)-*N*-(4-methoxypyrimidin-2-yl)-4,5,6,8-tetrahydropyrazolo[3,4-*b*] thiazolo[4,5-*d*]azepin-2-amine
6-(2-Methoxyethyl)-*N*-(5-methyl-1,2,4-thiadiazol-3-yl)-4,5,6,8-tetrahydropyrazolo[3,4-*b*]thiazolo[4,5-*d*]azepin-2-amine
2-(2-(5-Fluoropyrimidin-2-ylamino)-4,5-dihydropyrazolo[3,4-*b*]thiazolo[4,5-*d*]azepin-6(8*H*)-yl)ethanol
*N*$^2$-(6-(2-Methoxyethyl)-4,5,6, 8-tetrahydropyrazolo[3,4-*b*]thiazolo[4,5-*d*]azepin-2-yl) pyridine-2,6-diamine
*N*-(5-Fluoropyrimidin-2-yl)-6-((5-methylisoxazol-3-yl)methyl)-4,5,6,8-tetrahydro pyrazolo[3,4-*b*]thiazolo[4,5-*d*] azepin-2-amine
6-((3,5-Dimethylisoxazol-4-yl)methyl)-*N*-(5-fluoropyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((2-isopropyloxazol-4-yl)methyl)-4,5,6,8-tetrahydro pyrazolo[3,4-*b*]thiazolo[4,5-*d*] azepin-2-amine
*N*-(5-Fluoropyrimidin-2-yl)-4,5,6,8-tetrahydropyrazolo[3,4-*b*]thiazolo[4,5-*d*]azepin-2-amine
*N*-(5-Fluoropyrimidin-2-yl)-6-(pyridin-2-ylmethyl)-4,5,6,8-tetrahydropyrazolo[3,4-*b*] thiazolo[4,5-*d*]azepin-2-amine
*N*-(5-Fluoropyrimidin-2-yl)-6-(pyridin-4-ylmethyl)-4,5,6,8-tetrahydropyrazolo[3,4-*b*] thiazolo[4,5-*d*]azepin-2-amine
*N*-(5-Fluoropyrimidin-2-yl)-6-(tetrahydrofuran-3-yl)-4,5,6,8-tetrahydropyrazolo[3,4-b] thiazolo[4,5-*d*]azepin-2-amine
*N*-(5-Fluoropyrimidin-2-yl)-6-(2-(methylamino)ethyl)-4,5,6,8-tetrahydropyrazolo[3,4-*b*]thiazolo[4,5-*d*]azepin-2-amine
*N*-(5-Fluoropyrimidin-2-yl)-6-(3-methoxypropyl)-4,5,6,8-tetrahydropyrazolo[3,4-*b*] thiazolo[4,5-*d*]azepin-2-amine
*N*-(5-Fluoropyrimidin-2-yl)-6-((3-methylisoxazol-5-yl)methyl)-4,5,6,8-tetrahydro pyrazolo[3,4-*b*]thiazolo[4,5-*d*] azepin-2-amine
6-(Cyclopcntylmcthyl)-*N*-(5-fluoropyrimidin-2-yl)-4,5,6,8-tctrahydropyrazolo[3,4-*b*] thiazolo[4,5-*d*]azepin-2-amine
6-(Cyclopropylmethyl)-N-(4-methylpyrimidin-2-yl)-4,5,6,8-tetrahydropyrazolo[3,4-*b*] thiazolo[4,5-*d*]azepin-2-amine
6-(Cyclohexylmethyl)-*N*-(5-fluoropyrimidin-2-yl)-4,5,6,8-tetrahydropyrazolo[3,4-*b*] thiazolo[4,5-*d*]azepin-2-amine
6-(Cyclopropylmethyl)-*N*-(6-methylpyridin-2-yl)-4,5,6,8-tetrahydropyrazolo[3,4-*b*] thiazolo[4,5-*d*]azepin-2-amine
6-(Cyclopropylmethyl)-*N*-(6-fluoropyridin-2-yl)-4,5,6,8-tetrahydropyrazolo[3,4-*b*] thiazolo[4,5-*d*]azepin-2-amine
6-((5-Chloropyridin-2-yl)methyl)-*N*-(5-fluoropyrimidin-2-yl)-4,5,6,8-tetrahydro pyrazolo[3,4-*b*]thiazolo[4,5-*d*]azepin-2-amine
*N*-(5-Fluoropyrimidin-2-yl)-6-((5-isopropylisoxazol-3-yl)methyl)-4,5,6,8-tetrahydro pyrazolo[3,4-*b*]thiazolo[4,5-*d*] azepin-2-amine
*N*-(5-Fluoropyrimidin-2-yl)-6-(2-isopropoxyethyl)-4,5,6,8-tetrahydropyrazolo[3,4-*b*] thiazolo[4,5-*d*]azepin-2-amine
6-(Cyclobutylmethyl)-*N*-(5-fluoropyrimidin-2-yl)-4,5,6,8-tetrahydropyrazolo[3,4-*b*] thiazolo[4,5-*d*]azepin-2-amine
6-Benzyl-*N*-(5-fluoropyrimidin-2-yl)-4,5,6,7-tetrahydropyrazolo[3,4-*b*]thiazolo[4,5-*d*] azepin-2-amine
6-((3-Methylisoxazol-5-yl)methyl)-*N*-(4-methylpyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((tetrahydrofuran-2-yl)methyl)-4,5,6,7-tetrahydro pyrazolo[3,4-*b*]thiazolo[4,5-*d*] azepin-2-amine

*N*-(5-Fluoropyrimidin-2-yl)-6-(pyrimidin-2-ylmethyl)-4,5,6,7-tetrahydropyrazolo[3,4-*b*]thiazolo[4,5-*d*]azepin-2-amine
2-(6-(2-Methoxyethyl)-4,5,6,7-tetrahydropyrazolo[3,4-*b*]thiazolo[4,5-*d*]azepin-2-ylamino)pyrimidin-5-ol
*N*-(5-Fluoropyrimidin-2-yl)-6-(((*R*)-tetrahydrofuran-2-yl)methyl)-4,5,6,7-tetrahydro pyrazolo[3,4-*b*]thiazolo[4,5-*d*]azepin-2-amine
*N*-(5-Fluoropyrimidin-2-yl)-6-((2-methyl-2*H*-1,2,3-triazol-4-yl)methyl)-4,5,6,7-tetrahydropyrazolo[3,4-*b*]thiazolo[4,5-*d*]azepin-2-amine
(6-(6-(2-Methoxyethyl)-4,5,6,8-tetrahydropyrazolo[3,4-*b*]thiazolo[4,5-*d*]azepin-2-ylamino)pyridin-2-yl)methanol
6-(2-Methoxyethyl)-*N*-(2-methylpyrimidin-4-yl)-4,5,6,8-tetrahydropyrazolo[3,4-*b*] thiazolo[4,5-*d*]azepin-2-amine
6-(2-Methoxyethyl)-*N*-(pyrimidin-4-yl)-4,5,6,8-tetrahydropyrazolo[3,4-*b*]thiazolo[4,5-*d*]azepin-2-amine
6-((1*H*-Pyrazol-5-yl)methyl)-N(5-fluoropyrimidin-2-yl)-4,5,6,8-tetrahydropyrazolo [3,4-*b*]thiazolo[4,5-*d*]azepin-2-amine
6-((4-Bromo-1*H*-pyrazol-5-yl)methyl)-*N*-(5-fluoropyrimidin-2-yl)-4,5,6,8-tetrahydro pyrazolo[3,4-*b*]thiazolo[4,5-*d*]azepin-2-amine
6-(4-Chlorobcnzyl)-*N*-(5-fluoropyrimidin-2-yl)-4,5,6,7-tctrahydropyrazolo[3,4-*b*] thiazolo[4,5-*d*]azepin-2-amine
*N*-(5-Fluoropyrimidin-2-yl)-6-(2-methylbenzyl)-4,5,6,7-tetrahydropyrazolo[3,4-*b*] thiazolo[4,5-*d*]azepin-2-amine
*N*-(5-Fluoropyrimidin-2-yl)-6-(3-methoxybenzyl)-4,5,6,7-tetrahydropyrazolo[3,4-*b*] thiazolo[4,5-*d*]azepin-2-amine
6-((5-Fluoropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-((5-(trifluoromethyl)pyridin-2-yl)methyl)-4,5,6,7-tetrahydropyrazolo[3,4-*b*]thiazolo[4,5-*d*]azepin-2-amine
*N*-(5-Fluoropyrimidin-2-yl)-6-((4-methylpyridin-2-yl)methyl)-4,5,6,7-tetrahydro pyrazolo[3,4-*b*]thiazolo[4,5-*d*]azepin-2-amine
6-((3-Chloropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-phenethyl-4,5,6,7-tetrahydropyrazolo[3,4-*b*]thiazolo[4,5-*d*]azepin-2-amine
*N*-(3-Fluoro-6-methylpyridin-2-yl)-6-(2-methoxyethyl)-4,5,6,7-tetrahydropyrazolo[3,4-*b*]thiazolo[4,5-*d*]azepin-2-amine
6-(3-Chlorobenzyl)-*N*-(5-fluoropyrimidin-2-yl)-4,5,6,7-tetrahydropyrazolo[3,4-*b*] thiazolo[4,5-*d*]azepin-2-amine
4-((2-(5-Fluoropyrimidin-2-ylamino)-4,5-dihydropyrazolo[3,4-*b*]thiazolo[4,5-*d*]azepin-6(7*H*)-yl)methyl)benzonitrile
*N*-(5-Fluoropyrimidin-2-yl)-6-(4-(trifluoromethyl)benzyl)-4,5,6,7-tetrahydropyrazolo [3,4-*b*]thiazolo[4,5-*d*]azepin-2-amine
*N*-(5-Fluoropyrimidin-2-yl)-6-((6-methylpyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-(2-(pyridin-2-yl)ethyl)-4,5,6,7-tetrahydropyrazolo[3,4-*b*] thiazolo[4,5-*d*]azepin-2-amine
*N*-(5-Fluoropyrimidin-2-yl)-6-((1-methyl-1*H*-1,2,4-triazol-5-yl)methyl)-4,5,6,7-tetrahydropyrazolo[3,4-*b*]thiazolo[4,5-id]azepin-2-amine
*N*-(5-Fluoropyrimidin-2-yl)-6-((1-methyl-1*H*-1,2,4-triazol-3-yl)methyl)-4,5,6,7-tetrahydropyrazolo[3,4-*b*]thiazolo[4,5-*d*]azepin-2-amine
6-(2-Chlorobenzyl)-*N*-(5-fluoropyrimidin-2-yl)-4,5,6,7-tetrahydropyrazolo[3,4-*b*] thiazolo[4,5-*d*]azepin-2-amine
6-(4-Fluorobenzyl)-*N*-(5-fluoropyrimidin-2-yl)-4,5,6,7-tetrahydropyrazolo[3,4-*b*] thiazolo[4,5-*d*]azepin-2-amine
6-((2-(5-Fluoropyrimidin-2-ylamino)-4,5-dihydropyrazolo[3,4-*b*]thiazolo[4,5-*d*]azepin-6(7*H*)-yl)methyl)nicotinonitrile
*N*-(5-Fluoropyrimidin-2-yl)-6-((5-methoxypyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-(piperidin-4-ylmethyl)-4,5,6,7-tetrahydropyrazolo[3,4-*b*] thiazolo[4,5-*d*]azepin-2-amine
6-((5-Chlorothiazol-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-((1-methyl-1*H*-imidazol-4-yl)methyl)-4,5,6,7-tetrahydro pyrazolo[3,4-*b*]thiazolo[4,5-*d*]azepin-2-amine
6-(1-(5-Chloropyridin-2-yl)ethyl)-*N*-(5-fluoropyrimidin-2-yl)-4,5,6,7-tetrahydro pyrazolo[3,4-*b*]thiazolo[4,5-*d*]azepin-2-amine
6-Cyclobutyl-*N*-(5-fluoropyrimidin-2-yl)-4,5,6,7-tetrahydropyrazolo[3,4-*b*]thiazolo [4,5-*d*]azepin-2-amine and
*N*-(6-(Fluoromethyl)pyridin-2-yl)-6-(2-methoxyethyl)-4,5,6,7-tetrahydropyrazolo[3,4-*b*]thiazolo[4,5-*d*]azepin-2-amine.

A compound according to Formula (II) is also disclosed herein:

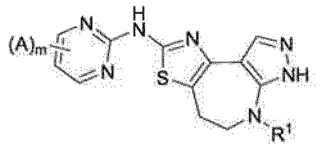

(II)

a pharmaceutically acceptable acid or base addition salt thereof, a stereochemically isomeric form thereof and an *N*-oxide form thereof, wherein:

$(A)_m$ are each independently selected from the group of hydrogen, halogen, -CN,-OH, -$CF_3$, -$OCF_3$, -$NH_2$ and an optionally substituted radical selected from the group of -($C_1$-$C_6$)alkyl, -($C_1$-$C_6$)haloalkyl, -($C_3$-C7)cycloalkyl, -($C_1$-$C_6$)alkylene-($C_3$-$C_7$)cycloalkyl, -($C_3$-$C_7$)cycloalkylene-($C_1$-$C_6$)alkyl, -($C_0$-$C_6$)alkylene-($C_3$-$C_7$)spiroalkylene-($C_0$-$C_6$)alkyl, -($C_0$-$C_6$)alkylene-$OR^4$, -($C_0$-$C_6$)alkylene-$NR^4R^5$ and - ($C_0$-$C_6$)alkylene-C(=O)-$R^4$;

m is an integer ranging from 1 to 2;

$R^4$ and $R^5$ are each independently hydrogen or an optionally substituted radical selected from the group of -($C_1$-$C_6$)haloalkyl, -($C_1$-$C_6$)alkyl, -($C_1$-$C_6$)cyanoalkyl, -($C_3$-C7)cycloalkyl, -($C_1$-$C_6$)alkylene-($C_3$-$C_7$)cycloalkyl, -($C_3$-$C_7$)cycloalkylene-($C_1$-$C_6$)alkyl, -($C_0$-$C_6$)alkylene-($C_3$-$C_7$)spiroalkylene-($C_0$-$C_6$)alkyl, heteroaryl, -($C_1$-$C_6$)alkylene-heteroaryl, aryl, -($C_1$-$C_6$)alkylene-aryl, -($C_1$-$C_6$)alkylene-heterocycle, heterocycle, -($C_2$-$C_6$)alkylene-O-($C_0$-$C_6$)alkyl and -($C_2$-$C_6$)alkylene-N-(($C_0$-$C_6$)alkyl$)_2$; and

provided that the compound is not:

6-(Cyclopropylmethyl)-*N*-(5-fluoropyrimidin-2-yl)-4,5,6,8-tetrahydropyrazolo[3,4-*b*] thiazolo[4,5-*d*]azepin-2-amine
6-(2-Methoxyethyl)-*N*-(4-methylpyrimidin-2-yl)-4,5,6,8-tetrahydropyrazolo[3,4-*b*] thiazolo[4,5-*d*]azepin-2-amine
*N*-(4-Methylpyrimidin-2-yl)-6-((tetrahydrofuran-2-yl)methyl)-4,5,6,8-tetrahydro pyrazolo[3,4-*b*]thiazolo[4,5-*d*] azepin-2-amine
6-(1-Methoxypropan-2-yl)-*N*-(4-methylpyrimidin-2-yl)-4,5,6,8-tetrahydropyrazolo [3,4-*b*]thiazolo[4,5-*d*]azepin-2-amine
*N*-(5-Fluoropyrimidin-2-yl)-6-(2-methoxyethyl)-4,5,6,8-tetrahydropyrazolo[3,4-*b*] thiazolo[4,5-*d*]azepin-2-amine
6-((1-Methyl-1*H*-pyrazol-3-yl)methyl)-*N*-(4-methylpyrimidin-2-yl)-4,5,6,8-tetrahydro pyrazolo[3,4-*b*]thiazolo[4,5-*d*] azepin-2-amine
*N*,*N*-Dimethyl-2-(2-(4-methylpyrimidin-2-ylamino)-4,5-dihydropyrazolo[3,4-*b*] thiazolo[4,5-*d*]azepin-6(8*H*)-yl)acetamide
6-(2-Methoxyethyl)-*N*-(4-methoxypyrimidin-2-yl)-4,5,6,8-tetrahydropyrazolo[3,4-*b*] thiazolo[4,5-*d*]azepin-2-amine
2-(2-(5-Fluoropyrimidin-2-ylamino)-4,5-dihydropyrazolo[3,4-*b*]thiazolo[4,5-*d*]azepin-6(8*H*)-yl)ethanol
*N*-(5-Fluoropyrimidin-2-yl)-6-((5-methylisoxazol-3-yl)methyl)-4,5,6,8-tetrahydro pyrazolo[3,4-*b*]thiazolo[4,5-*d*] azepin-2-amine
6-((3,5-Dimethylisoxazol-4-yl)methyl)-*N*-(5-fluoropyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((2-isopropyloxazol-4-yl)methyl)-4,5,6,8-tetrahydro pyrazolo[3,4-*b*]thiazolo[4,5-*d*] azepin-2-amine
*N*-(5-Fluoropyrimidin-2-yl)-4,5,6,8-tetrahydropyrazolo[3,4-*b*]thiazolo[4,5-*d*]azepin-2-amine
*N*-(5-Fluoropyrimidin-2-yl)-6-(pyridin-2-ylmethyl)-4,5,6,8-tetrahydropyrazolo[3,4-*b*] thiazolo[4,5-*d*]azepin-2-amine
*N*-(5-Fluoropyrimidin-2-yl)-6-(pyridin-4-ylmethyl)-4,5,6,8-tetrahydropyrazolo[3,4-*b*] thiazolo[4,5-*d*]azepin-2-amine
*N*-(5-Fluoropyrimidin-2-yl)-6-(tetrahydrofuran-3-yl)-4,5,6,8-tetrahydropyrazolo[3,4-*b*] thiazolo[4,5-*d*]azepin-2-amine
*N*-(5-Fluoropyrimidin-2-yl)-6-(2-(methylamino)ethyl)-4,5,6,8-tetrahydropyrazolo[3,4-*b*]thiazolo[4,5-*d*]azepin-2-amine
*N*-(5-Fluoropyrimidin-2-yl)-6-(3-methoxypropyl)-4,5,6,8-tetrahydropyrazolo[3,4-*b*] thiazolo[4,5-*d*]azepin-2-amine
*N*-(5-Fluoropyrimidin-2-yl)-6-((3-methylisoxazol-5-yl)methyl)-4,5,6,8-tetrahydro pyrazolo[3,4-*b*]thiazolo[4,5-*d*] azepin-2-amine
6-(Cyclopcntylmcthyl)-*N*-(5-fluoropyrimidin-2-yl)-4,5,6,8-tctrahydropyrazolo[3,4-*b*] thiazolo[4,5-*d*]azepin-2-amine
6-(Cyclopropylmethyl)-N-(4-methylpyrimidin-2-yl)-4,5,6,8-tetrahydropyrazolo[3,4-*b*] thiazolo[4,5-*d*]azepin-2-amine
6-(Cyclohexylmethyl)-*N*-(5-fluoropyrimidin-2-yl)-4,5,6,8-tetrahydropyrazolo[3,4-*b*] thiazolo[4,5-*d*]azepin-2-amine
6-((5-Chloropyridin-2-yl)methyl)-*N*-(5-fluoropyrimidin-2-yl)-4,5,6,8-tetrahydro pyrazolo[3,4-*b*]thiazolo[4,5-*d*]azepin-2-amine

*N*-(5-Fluoropyrimidin-2-yl)-6-((5-isopropylisoxazol-3-yl)methyl)-4,5,6,8-tetrahydro pyrazolo[3,4-*b*]thiazolo[4,5-*d*]azepin-2-amine
*N*-(5-Fluoropyrimidin-2-yl)-6-(2-isopropoxyethyl)-4,5,6,8-tetrahydropyrazolo[3,4-*b*] thiazolo[4,5-*d*]azepin-2-amine
6-(Cyclobutylmethyl)-*N*-(5-fluoropyrimidin-2-yl)-4,5,6,8-tetrahydropyrazolo[3,4-*b*] thiazolo[4,5-*d*]azepin-2-amine
6-Benzyl-*N*-(5-fluoropyrimidin-2-yl)-4,5,6,7-tetrahydropyrazolo[3,4-b]thiazolo[4,5-d] azepin-2-amine
6-((3-Methylisoxazol-5-yl)methyl)-*N*-(4-methylpyrimidin-2-yl)-4,5,6,8-tetrahydro pyrazolo[3,4-*b*]thiazolo[4,5-*d*]azepin-2-amine
*N*-(5-Fluoropyrimidin-2-yl)-6-((tetrahydrofuran-2-yl)methyl)-4,5,6,7-tetrahydro pyrazolo[3,4-*b*]thiazolo[4,5-*d*]azepin-2-amine
*N*-(5-Fluoropyrimidin-2-yl)-6-(pyrimidin-2-ylmethyl)-4,5,6,7-tetrahydropyrazolo[3,4-*b*]thiazolo[4,5-*d*]azepin-2-amine
2-(6-(2-Methoxyethyl)-4,5,6,7-tetrahydropyrazolo[3,4-*b*]thiazolo[4,5-*d*]azepin-2-ylamino)pyrimidin-5-ol
*N*-(5-Fluoropyrimidin-2-yl)-6-(((iR)-tetrahydrofuran-2-yl)methyl)-4,5,6,7-tetrahydro pyrazolo[3,4-*b*]thiazolo[4,5-*d*]azepin-2-amine
*N*-(5-Fluoropyrimidin-2-yl)-6-((2-methyl-2*H*-1,2,3-triazol-4-yl)methyl)-4,5,6,7-tetrahydropyrazolo[3,4-*b*]thiazolo[4,5-*d*]azepin-2-amine
6-((1*H*-Pyrazol-5-yl)methyl)-N(5-fluoropyrimidin-2-yl)-4,5,6,8-tetrahydropyrazolo [3,4-*b*]thiazolo[4,5-*d*]azepin-2-amine
6-((4-Bromo-1*H*-pyrazol-5-yl)methyl)-*N*-(5-fluoropyrimidin-2-yl)-4,5,6,8-tetrahydro pyrazolo[3,4-*b*]thiazolo[4,5-*d*]azepin-2-amine
6-(4-Chlorobcnzyl)-*N*-(5-fluoropyrimidin-2-yl)-4,5,6,7-tctrahydropyrazolo[3,4-*b*] thiazolo[4,5-*d*]azepin-2-amine
*N*-(5-Fluoropyrimidin-2-yl)-6-(2-methylbenzyl)-4,5,6,7-tetrahydropyrazolo[3,4-*b*] thiazolo[4,5-*d*]azepin-2-amine
*N*-(5-Fluoropyrimidin-2-yl)-6-(3-methoxybenzyl)-4,5,6,7-tetrahydropyrazolo[3,4-*b*] thiazolo[4,5-*d*]azepin-2-amine
6-((5-Fluoropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-((5-(trifluoromethyl)pyridin-2-yl)methyl)-4,5,6,7-tetrahydropyrazolo[3,4-*b*]thiazolo[4,5-*d*]azepin-2-amine
*N*-(5-Fluoropyrimidin-2-yl)-6-((4-methylpyridin-2-yl)methyl)-4,5,6,7-tetrahydro pyrazolo[3,4-*b*]thiazolo[4,5-*d*]azepin-2-amine
6-((3-Chloropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-phenethyl-4,5,6,7-tetrahydropyrazolo[3,4-*b*]thiazolo[4,5-*d*]azepin-2-amine
6-(3-Chlorobenzyl)-*N*-(5-fluoropyrimidin-2-yl)-4,5,6,7-tetrahydropyrazolo[3,4-*b*] thiazolo[4,5-*d*]azepin-2-amine
4-((2-(5-Fluoropyrimidin-2-ylamino)-4,5-dihydropyrazolo[3,4-*b*]thiazolo[4,5-*d*]azepin-6(7*H*)-yl)methyl)benzonitrile
*N*-(5-Fluoropyrimidin-2-yl)-6-(4-(trifluoromethyl)benzyl)-4,5,6,7-tetrahydropyrazolo [3,4-*b*]thiazolo[4,5-*d*]azepin-2-amine
*N*-(5-Fluoropyrimidin-2-yl)-6-((6-methylpyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-(2-(pyridin-2-yl)ethyl)-4,5,6,7-tetrahydropyrazolo[3,4-*b*] thiazolo[4,5-*d*]azepin-2-amine
*N*-(5-Fluoropyrimidin-2-yl)-6-((1-methyl-1*H*-1,2,4-triazol-5-yl)methyl)-4,5,6,7-tetrahydropyrazolo[3,4-*b*]thiazolo[4,5-*d*]azepin-2-amine
*N*-(5-Fluoropyrimidin-2-yl)-6-((1-methyl-1*H*-1,2,4-triazol-3-yl)methyl)-4,5,6,7-tetrahydropyrazolo[3,4-*b*]thiazolo[4,5-*d*]azepin-2-amine
6-(2-Chlorobenzyl)-*N*-(5-fluoropyrimidin-2-yl)-4,5,6,7-tetrahydropyrazolo[3,4-*b*] thiazolo[4,5-*d*]azepin-2-amine
6-(4-Fluorobenzyl)-*N*-(5-fluoropyrimidin-2-yl)-4,5,6,7-tetrahydropyrazolo[3,4-*b*] thiazolo[4,5-*d*]azepin-2-amine
6-((2-(5-Fluoropyrimidin-2-ylamino)-4,5-dihydropyrazolo[3,4-*b*]thiazolo[4,5-*d*]azepin-6(7*H*)-yl)methyl)nicotinonitrile
*N*-(5-Fluoropyrimidin-2-yl)-6-((5-methoxypyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-(piperidin-4-ylmethyl)-4,5,6,7-tetrahydropyrazolo[3,4-*b*] thiazolo[4,5-*d*]azepin-2-amine
6-((5-Chlorothiazol-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-((1-methyl-1H-imidazol-4-yl)methyl)-4,5,6,7-tetrahydro pyrazolo[3,4-*b*]thiazolo[4,5-*d*]azepin-2-amine
6-(1-(5-Chloropyridin-2-yl)ethyl)-*N*-(5-fluoropyrimidin-2-yl)-4,5,6,7-tetrahydro pyrazolo[3,4-*b*]thiazolo[4,5-*d*]azepin-2-amine and
6-Cyclobutyl-*N*-(5-fluoropyrimidin-2-yl)-4,5,6,7-tetrahydropyrazolo[3,4-*b*]thiazolo [4,5-*d*]azepin-2-amine.

In another aspect of Formula (I), a compound according to Formula (III) is disclosed:

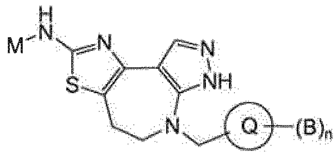

(III)

a pharmaceutically acceptable acid or base addition salt thereof, a stereochemically isomeric form thereof and an *N*-oxide form thereof, wherein:

Q is an optionally substituted aryl, heteroaryl, heterocycle or cycloalkyl;

$(B)_n$ are each independently selected from the group of hydrogen, halogen, -CN,-OH, $-CF_3$, $-OCF_3$, $-NH_2$ and an optionally substituted radical selected from the group of $-(C_1-C_6)$alkyl, $-(C_1-C_6)$haloalkyl, $-(C_3-C_7)$cycloalkyl, $-(C_1-C_6)$alkylene-$(C_3-C_7)$cycloalkyl, $-(C_3-C_7)$cycloalkylene-$(C_1-C_6)$alkyl, $-(C_0-C_6)$alkylene-$(C_3-C_7)$spiroalkylene-$(C_0-C_6)$alkyl, $-(C_0-C_6)$alkylene-$OR^6$, $-(C_0-C_6)$alkylene-$NR^6R^7$ and - $(C_0-C_6)$alkylene-C(=O)-$R^6$;

n is an integer ranging from 1 to 2;

$R^6$ and $R^7$ are each independently hydrogen or an optionally substituted radical selected from the group of $-(C_1-C_6)$haloalkyl, $-(C_1-C_6)$alkyl, $-(C_1-C_6)$cyanoalkyl, $-(C_3-C_7)$cycloalkyl, $-(C_1-C_6)$alkylene-$(C_3-C_7)$cycloalkyl, $-(C_3-C_7)$cycloalkylene-$(C_1-C_6)$alkyl, $-(C_0-C_6)$alkylene-$(C_3-C_7)$spiroalkylene-$(C_0-C_6)$alkyl, heteroaryl, $-(C_1-C_6)$alkylene-heteroaryl, aryl, $-(C_1-C_6)$alkylene-aryl, $-(C_1-C_6)$alkylene-heterocycle, heterocycle, $-(C_2-C_6)$alkylene-O-$(C_0-C_6)$alkyl and $-(C_2-C_6)$alkylene-N-$((C_0-C_6)alkyl)_2$; and

provided that the compound is not:

6-(Cyclopropylmethyl)-*N*-(5-fluoropyrimidin-2-yl)-4,5,6,8-tetrahydropyrazolo[3,4-*b*] thiazolo[4,5-*d*]azepin-2-amine
*N*-(4-Methylpyrimidin-2-yl)-6-((tetrahydrofuran-2-yl)methyl)-4,5,6,8-tetrahydro pyrazolo[3,4-*b*]thiazolo[4,5-*d*] azepin-2-amine
6-((1-Methyl-1*H*-pyrazol-3-yl)methyl)-*N*-(4-methylpyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((5-methylisoxazol-3-yl)methyl)-4,5,6,8-tetrahydro pyrazolo[3,4-*b*]thiazolo[4,5-*d*] azepin-2-amine
6-((3,5-Dimethylisoxazol-4-yl)methyl)-*N*-(5-fluoropyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((2-isopropyloxazol-4-yl)methyl)-4,5,6,8-tetrahydro pyrazolo[3,4-*b*]thiazolo[4,5-*d*] azepin-2-amine
*N*-(5-Fluoropyrimidin-2-yl)-6-(pyridin-2-ylmethyl)-4,5,6,8-tetrahydropyrazolo[3,4-*b*] thiazolo[4,5-*d*]azepin-2-amine
*N*-(5-Fluoropyrimidin-2-yl)-6-(pyridin-4-ylmethyl)-4,5,6,8-tetrahydropyrazolo[3,4-*b*] thiazolo[4,5-*d*]azepin-2-amine
*N*-(5-Fluoropyrimidin-2-yl)-6-((3-methylisoxazol-5-yl)methyl)-4,5,6,8-tetrahydro pyrazolo[3,4-*b*]thiazolo[4,5-*d*] azepin-2-amine
6-(Cyclopcntylmcthyl)-*N*-(5-fluoropyrimidin-2-yl)-4,5,6,8-tetrahydropyrazolo[3,4-*b*] thiazolo[4,5-*d*]azepin-2-amine
6-(Cyclopropylmethyl)-*N*-(4-methylpyrimidin-2-yl)-4,5,6,8-tetrahydropyrazolo[3,4-*b*] thiazolo[4,5-*d*]azepin-2-amine
6-(Cyclohexylmethyl)-*N*-(5-fluoropyrimidin-2-yl)-4,5,6,8-tetrahydropyrazolo[3,4-*b*] thiazolo[4,5-*d*]azepin-2-amine
6-(Cyclopropylmethyl)-*N*-(6-methylpyridin-2-yl)-4,5,6,8-tetrahydropyrazolo[3,4-*b*] thiazolo[4,5-*d*]azepin-2-amine
6-(Cyclopropylmethyl)-*N*-(6-fluoropyridin-2-yl)-4,5,6,8-tctrahydropyrazolo[3,4-*b*] thiazolo[4,5-*d*]azepin-2-amine
6-((5-Chloropyridin-2-yl)methyl)-*N*-(5-fluoropyrimidin-2-yl)-4,5,6,8-tetrahydro pyrazolo[3,4-*b*]thiazolo[4,5-*d*]azepin-2-amine
*N*-(5-Fluoropyrimidin-2-yl)-6-((5-isopropylisoxazol-3-yl)methyl)-4,5,6,8-tetrahydro pyrazolo[3,4-*b*]thiazolo[4,5-*d*] azepin-2-amine
6-(Cyclobutylmethyl)-*N*-(5-fluoropyrimidin-2-yl)-4,5,6,8-tetrahydropyrazolo[3,4-*b*] thiazolo[4,5-*d*]azepin-2-amine
6-Benzyl-*N*-(5-fluoropyrimidin-2-yl)-4,5,6,7-tetrahydropyrazolo[3,4-b]thiazolo[4,5-*d*] azepin-2-amine
6-((3-Methylisoxazol-5-yl)methyl)-*N*-(4-methylpyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((tetrahydrofuran-2-yl)methyl)-4,5,6,7-tetrahydro pyrazolo[3,4-*b*]thiazolo[4,5-*d*] azepin-2-amine
*N*-(5-Fluoropyrimidin-2-yl)-6-(pyrimidin-2-ylmethyl)-4,5,6,7-tetrahydropyrazolo[3,4-*b*]thiazolo[4,5-d]azepin-2-amine

*N*-(5-Fluoropyrimidin-2-yl)-6-(((*R*)-tetrahydrofuran-2-yl)methyl)-4,5,6,7-tetrahydro pyrazolo[3,4-*b*]thiazolo[4,5-*d*]azepin-2-amine
*N*-(5-Fluoropyrimidin-2-yl)-6-((2-methyl-2*H*-1,2,3-triazol-4-yl)methyl)-4,5,6,7-tetrahydropyrazolo[3,4-*b*]thiazolo[4,5-*d*]azepin-2-amine
6-((1*H*-Pyrazol-5-yl)methyl)-*N*-(5-fluoropyrimidin-2-yl)-4,5,6,8-tetrahydropyrazolo [3,4-*b*]thiazolo[4,5-*d*]azepin-2-amine
6-((4-Bromo-1*H*-pyrazol-5-yl)methyl)-*N*-(5-fluoropyrimidin-2-yl)-4,5,6,8-tetrahydro pyrazolo[3,4-*b*]thiazolo[4,5-*d*]azepin-2-amine
6-(4-Chlorobenzyl)-*N*-(5-fluoropyrimidin-2-yl)-4,5,6,7-tetrahydropyrazolo[3,4-*b*] thiazolo[4,5-*d*]azepin-2-amine
*N*-(5-Fluoropyrimidin-2-yl)-6-(2-methylbenzyl)-4,5,6,7-tetrahydropyrazolo[3,4-*b*] thiazolo[4,5-*d*]azepin-2-amine
*N*-(5-Fluoropyrimidin-2-yl)-6-(3-methoxybenzyl)-4,5,6,7-tetrahydropyrazolo[3,4-*b*] thiazolo[4,5-*d*]azepin-2-amine
6-((5-Fluoropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-((5-(trifluoromethyl)pyridin-2-yl)methyl)-4,5,6,7-tetrahydropyrazolo[3,4-*b*]thiazolo[4,5-*d*]azepin-2-amine
*N*-(5-Fluoropyrimidin-2-yl)-6-((4-methylpyridin-2-yl)methyl)-4,5,6,7-tetrahydro pyrazolo[3,4-*b*]thiazolo[4,5-*d*]azepin-2-amine
6-((3-Chloropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
6-(3-Chlorobenzyl)-*N*-(5-fluoropyrimidin-2-yl)-4,5,6,7-tetrahydropyrazolo[3,4-*b*] thiazolo[4,5-*d*]azepin-2-amine
4-((2-(5-Fluoropyrimidin-2-ylamino)-4,5-dihydropyrazolo[3,4-*b*]thiazolo[4,5-*d*]azepin-6(7*H*)-yl)methyl)benzonitrile
*N*-(5-Fluoropyrimidin-2-yl)-6-(4-(trifluoromethyl)benzyl)-4,5,6,7-tetrahydropyrazolo [3,4-*b*]thiazolo[4,5-*d*]azepin-2-amine
*N*-(5-Fluoropyrimidin-2-yl)-6-((6-methylpyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-((1-methyl-1*H*-1,2,4-triazol-5-yl)methyl)-4,5,6,7-tetrahydropyrazolo[3,4-*b*]thiazolo[4,5-*d*]azepin-2-amine
*N*-(5-Fluoropyrimidin-2-yl)-6-((1-methyl-1*H*-1,2,4-triazol-3-yl)methyl)-4,5,6,7-tetrahydropyrazolo[3,4-*b*]thiazolo[4,5-*d*]azepin-2-amine
6-(2-Chlorobenzyl)-*N*-(5-fluoropyrimidin-2-yl)-4,5,6,7-tetrahydropyrazolo[3,4-*b*] thiazolo[4,5-*d*]azepin-2-amine
6-(4-Fluorobenzyl)-*N*-(5-fluoropyrimidin-2-yl)-4,5,6,7-tetrahydropyrazolo[3,4-*b*] thiazolo[4,5-*d*]azepin-2-amine
6-((2-(5-Fluoropyrimidin-2-ylamino)-4,5-dihydropyrazolo[3,4-*b*]thiazolo[4,5-*d*]azepin-6(7*H*)-yl)methyl)nicotinonitrile
*N*-(5-Fluoropyrimidin-2-yl)-6-((5-methoxypyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-(piperidin-4-ylmethyl)-4,5,6,7-tetrahydropyrazolo[3,4-*b*] thiazolo[4,5-*d*]azepin-2-amine
6-((5-Chlorothiazol-2-yl)methyl)-*N*-(5-fluoropyrimidin-2-yl)-4,5,6,7-tetrahydro pyrazolo[3,4-*b*]thiazolo[4,5-*d*]azepin-2-amine and
*N*-(5-Fluoropyrimidin-2-yl)-6-((1-methyl-1*H*-imidazol-4-yl)methyl)-4,5,6,7-tetrahydro pyrazolo[3,4-*b*]thiazolo[4,5-d]azepin-2-amine.

In another aspect of Formula (III), a compound according to Formula (IV) is disclosed:

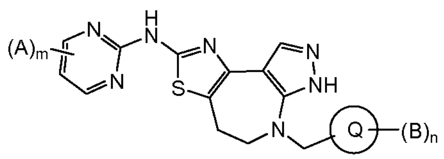

(IV)

a pharmaceutically acceptable acid or base addition salt thereof, a stereochemically isomeric form thereof and an *N*-oxide form thereof, wherein:

$(A)_m$ are each independently selected from the group of hydrogen, halogen, -CN,-OH, $-CF_3$, $-OCF_3$, $-NH_2$ and an optionally substituted radical selected from the group of $-(C_1-C_6)$alkyl, $-(C_1-C_6)$haloalkyl, $-(C_3$-C7)cycloalkyl, $-(C_1-C_6)$alkylene-$(C_3-C_7)$cycloalkyl, $-(C_3-C_7)$cycloalkylene-$(C_1-C_6)$alkyl, -(Co-$C_6$)alkylene- $(C_3-C_7)$spiroalkylene-$(C_0-C_6)$alkyl, $-(C_0-C_6)$alkylene-$OR^4$, $-(C_0-C_6)$alkylene-$NR^4R^5$ and - $(C_0-C_6)$alkylene-C(=O)-$R^4$;

m is an integer ranging from 1 to 2; and

$R^4$ and $R^5$ are each independently hydrogen or an optionally substituted radical selected from the group of $-(C_1-C_6)$haloalkyl, $-(C_1-C_6)$alkyl, $-(C_1-C_6)$cyanoalkyl, $-(C_3-C_7)$cycloalkyl, $-(C_1-C_6)$alkylene-$(C_3-C_7)$cycloalkyl, $-(C_3-C_7)$cycloalkylene-$(C_1-C_6)$alkyl, $-(C_0-C_6)$alkylene-$(C_3-C_7)$spiroalkylene-$(C_0-C_6)$alkyl, heteroaryl, $-(C_1-C_6)$alkylene-heteroaryl, aryl, $-(C_1-C_6)$alkylene-aryl, $-(C_1-C_6)$alkylene-heterocycle, heterocycle, $-(C_2-C_6)$alkylene-O-$(C_0-C_6)$alkyl and $-(C_2-C_6)$alkylene-N-$((C_0-C_6)$alkyl$)_2$; and

provided that the compound is not:

6-(Cyclopropylmethyl)-*N*-(5-fluoropyrimidin-2-yl)-4,5,6,8-tetrahydropyrazolo[3,4-*b*] thiazolo[4,5-*d*]azepin-2-amine
*N*-(4-Methylpyrimidin-2-yl)-6-((tetrahydrofuran-2-yl)methyl)-4,5,6,8-tetrahydro pyrazolo[3,4-*b*]thiazolo[4,5-*d*] azepin-2-amine
6-((1-Methyl-1*H*-pyrazol-3-yl)methyl)-*N*-(4-methylpyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((5-methylisoxazol-3-yl)methyl)-4,5,6,8-tetrahydro pyrazolo[3,4-*b*]thiazolo[4,5-*d*] azepin-2-amine
6-((3,5-Dimethylisoxazol-4-yl)methyl)-*N*-(5-fluoropyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((2-isopropyloxazol-4-yl)methyl)-4,5,6,8-tetrahydro pyrazolo[3,4-*b*]thiazolo[4,5-*d*] azepin-2-amine
*N*-(5-Fluoropyrimidin-2-yl)-6-(pyridin-2-ylmethyl)-4,5,6,8-tetrahydropyrazolo[3,4-*b*] thiazolo[4,5-*d*]azepin-2-amine
*N*-(5-Fluoropyrimidin-2-yl)-6-(pyridin-4-ylmethyl)-4,5,6,8-tetrahydropyrazolo[3,4-*b*] thiazolo[4,5-*d*]azepin-2-amine
*N*-(5-Fluoropyrimidin-2-yl)-6-((3-methylisoxazol-5-yl)methyl)-4,5,6,8-tetrahydro pyrazolo[3,4-*b*]thiazolo[4,5-*d*] azepin-2-amine
6-(Cyclopcntylmcthyl)-*N*-(5-fluoropyrimidin-2-yl)-4,5,6,8-tctrahydropyrazolo[3,4-*b*] thiazolo[4,5-*d*]azepin-2-amine
6-(Cyclopropylmethyl)-N-(4-methylpyrimidin-2-yl)-4,5,6,8-tetrahydropyrazolo[3,4-*b*] thiazolo[4,5-*d*]azepin-2-amine
6-(Cyclohexylmethyl)-*N*-(5-fluoropyrimidin-2-yl)-4,5,6,8-tetrahydropyrazolo[3,4-*b*] thiazolo[4,5-*d*]azepin-2-amine
6-((5-Chloropyridin-2-yl)methyl)-*N*-(5-fluoropyrimidin-2-yl)-4,5,6,8-tetrahydro pyrazolo[3,4-*b*]thiazolo[4,5-*d*]azepin-2-amine
*N*-(5-Fluoropyrimidin-2-yl)-6-((5-isopropylisoxazol-3-yl)methyl)-4,5,6,8-tetrahydro pyrazolo[3,4-*b*]thiazolo[4,5-*d*] azepin-2-amine
6-(Cyclobutylmethyl)-*N*-(5-fluoropyrimidin-2-yl)-4,5,6,8-tetrahydropyrazolo[3,4-*b*] thiazolo[4,5-*d*]azepin-2-amine
6-Benzyl-*N*-(5-fluoropyrimidin-2-yl)-4,5,6,7-tetrahydropyrazolo[3,4-*b*]thiazolo[4,5-*d*] azepin-2-amine
6-((3-Methylisoxazol-5-yl)methyl)-*N*-(4-methylpyrimidin-2-yl)-4,5,6,8-tetrahydro pyrazolo[3,4-*b*]thiazolo[4,5-*d*] azepin-2-amine
*N*-(5-Fluoropyrimidin-2-yl)-6-((tetrahydrofuran-2-yl)methyl)-4,5,6,7-tetrahydro pyrazolo[3,4-*b*]thiazolo[4,5-*d*] azepin-2-amine
*N*-(5-Fluoropyrimidin-2-yl)-6-(pyrimidin-2-ylmethyl)-4,5,6,7-tetrahydropyrazolo[3,4-*b*]thiazolo[4,5-*d*]azepin-2-amine
*N*-(5-Fluoropyrimidin-2-yl)-6-(((*R*)-tetrahydrofuran-2-yl)methyl)-4,5,6,7-tetrahydro pyrazolo[3,4-*b*]thiazolo[4,5-*d*] azepin-2-amine
*N*-(5-Fluoropyrimidin-2-yl)-6-((2-methyl-2*H*-1,2,3-triazol-4-yl)methyl)-4,5,6,7-tetrahydropyrazolo[3,4-*b*]thiazolo[4,5-*d*]azepin-2-amine
6-((1*H*-Pyrazol-5-yl)methyl)-N(5-fluoropyrimidin-2-yl)-4,5,6,8-tetrahydropyrazolo [3,4-*b*]thiazolo[4,5-*d*]azepin-2-amine
6-((4-Bromo-1*H*-pyrazol-5-yl)methyl)-*N*-(5-fluoropyrimidin-2-yl)-4,5,6,8-tetrahydro pyrazolo[3,4-*b*]thiazolo[4,5-*d*] azepin-2-amine
6-(4-Chlorobenzyl)-*N*-(5-fluoropyrimidin-2-yl)-4,5,6,7-tetrahydropyrazolo[3,4-*b*] thiazolo[4,5-*d*]azepin-2-amine
*N*-(5-Fluoropyrimidin-2-yl)-6-(2-methylbenzyl)-4,5,6,7-tetrahydropyrazolo[3,4-*b*] thiazolo[4,5-*d*]azepin-2-amine
*N*-(5-Fluoropyrimidin-2-yl)-6-(3-methoxybenzyl)-4,5,6,7-tetrahydropyrazolo[3,4-*b*] thiazolo[4,5-*d*]azepin-2-amine
6-((5-Fluoropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-((5-(trifluoromethyl)pyridin-2-yl)methyl)-4,5,6,7-tetrahydropyrazolo[3,4-*b*]thiazolo[4,5-*d*]azepin-2-amine
*N*-(5-Fluoropyrimidin-2-yl)-6-((4-methylpyridin-2-yl)methyl)-4,5,6,7-tetrahydro pyrazolo[3,4-*b*]thiazolo[4,5-*d*] azepin-2-amine
6-((3-Chloropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
6-(3-Chlorobenzyl)-*N*-(5-fluoropyrimidin-2-yl)-4,5,6,7-tetrahydropyrazolo[3,4-*b*] thiazolo[4,5-*d*]azepin-2-amine

4-((2-(5-Fluoropyrimidin-2-ylamino)-4,5-dihydropyrazolo[3,4-*b*]thiazolo[4,5-*d*]azepin-6(7*H*)-yl)methyl)benzonitrile
*N*-(5-Fluoropyrimidin-2-yl)-6-(4-(trifluoromethyl)benzyl)-4,5,6,7-tetrahydropyrazolo [3,4-*b*]thiazolo[4,5-*d*]azepin-2-amine
*N*-(5-Fluoropyrimidin-2-yl)-6-((6-methylpyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-((1-methyl-1*H*-1,2,4-triazol-5-yl)methyl)-4,5,6,7-tetrahydropyrazolo[3,4-*b*]thiazolo[4,5-*d*]azepin-2-amine
*N*-(5-Fluoropyrimidin-2-yl)-6-((1-methyl-1*H*-1,2,4-triazol-3-yl)methyl)-4,5,6,7-tetrahydropyrazolo[3,4-*b*]thiazolo[4,5-*d*]azepin-2-amine
6-(2-Chlorobenzyl)-*N*-(5-fluoropyrimidin-2-yl)-4,5,6,7-tetrahydropyrazolo[3,4-*b*] thiazolo[4,5-*d*]azepin-2-amine
6-(4-Fluorobenzyl)-*N*-(5-fluoropyrimidin-2-yl)-4,5,6,7-tetrahydropyrazolo[3,4-*b*] thiazolo[4,5-*d*]azepin-2-amine
6-((2-(5-Fluoropyrimidin-2-ylamino)-4,5-dihydropyrazolo[3,4-*b*]thiazolo[4,5-*d*]azepin-6(7*H*)-yl)methyl)nicotinonitrile
*N*-(5-Fluoropyrimidin-2-yl)-6-((5-methoxypyridin-2-yl)methyl)-4,5,6,7-tetrahydro pyrazolo[3,4-*b*]thiazolo[4,5-*d*]azepin-2-amine
*N*-(5-Fluoropyrimidin-2-yl)-6-(piperidin-4-ylmethyl)-4,5,6,7-tetrahydropyrazolo[3,4-*b*] thiazolo[4,5-*d*]azepin-2-amine
6-((5-Chlorothiazol-2-yl)methyl)-*N*-(5-fluoropyrimidin-2-yl)-4,5,6,7-tetrahydro pyrazolo[3,4-*b*]thiazolo[4,5-*d*]azepin-2-amine and
*N*-(5-Fluoropyrimidin-2-yl)-6-((1-methyl-1*H*-imidazol-4-yl)methyl)-4,5,6,7-tetrahydro pyrazolo[3,4-*b*]thiazolo[4,5-*d*]azepin-2-amine.

In another aspect of Formula (III), a compound is disclosed wherein:

M is selected from the group of formula:

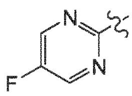 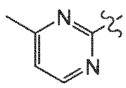 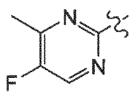 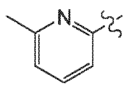 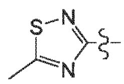 ;

and

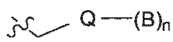

is selected from the group of formula:

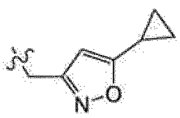 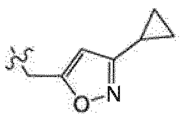 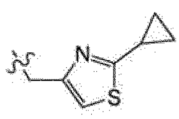 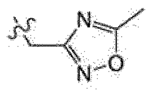 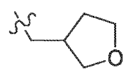 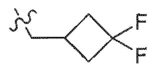

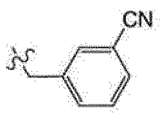 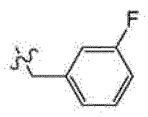 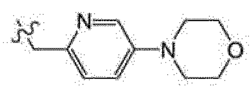 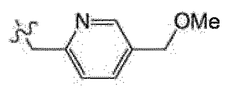 .

This aspect of the invention does not encompass any of the compounds disclaimed in Formula (III).
In another aspect of Formula (III), a compound is disclosed wherein:

M is:

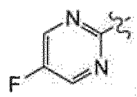 ;

and

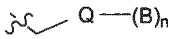

is selected from the group of formula:

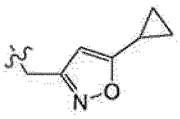 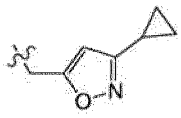 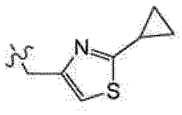 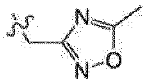 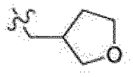 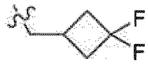

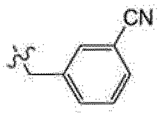 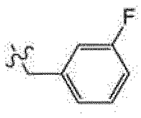 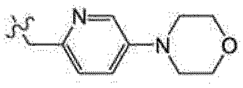 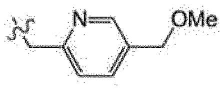 .

This aspect of the invention does not encompass any of the compounds disclaimed in Formula (III).

In another aspect of Formula (I), a compound is disclosed wherein:

M is selected from the group of formula:

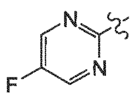 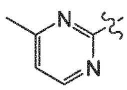 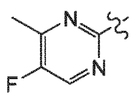 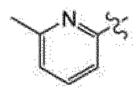 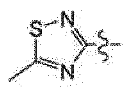 ;

and $R^1$ is selected from the group of formula:

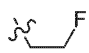 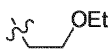 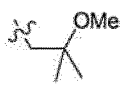 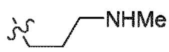 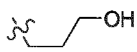

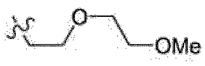 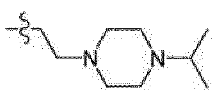 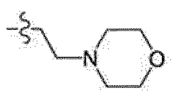 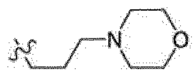 .

This aspect of the invention does not encompass any of the compounds disclaimed in Formula (I).

In another aspect of Formula (I), a compound is disclosed wherein:

M is:

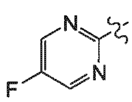 ;

and $R^1$ is selected from the group of formula:

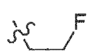 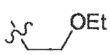 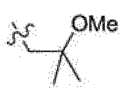 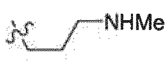 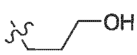

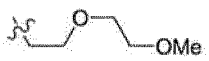 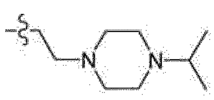 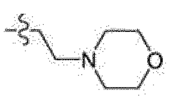 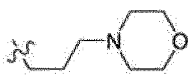 .

This aspect of the invention does not encompass any of the compounds disclaimed in Formula (I).

In another aspect of Formula (I), a compound is disclosed wherein:

M is:

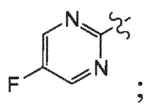 ;

and $R^1$ is selected from the group of formula:

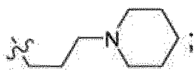 ; 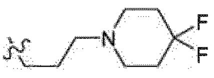 ; 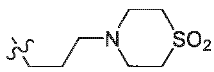 ; 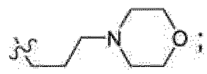 ; 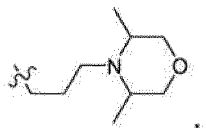 .

This aspect of the invention does not encompass any of the compounds disclaimed in Formula (I).

In another aspect of Formula (III), a compound is disclosed wherein:

M is:

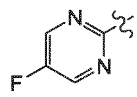 ;

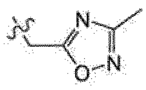 ; 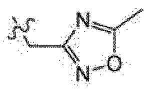 ; 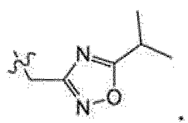 .

This aspect of the invention does not encompass any of the compounds disclaimed in Formula (III).

The compounds of the invention are compounds as mentioned in the following list (List of Particular Preferred Compounds), as well as a pharmaceutically acceptable acid or base addition salt thereof, a stereochemically isomeric form thereof and an *N*-oxide form thereof:

3-((2-(5-Fluoropyrimidin-2-ylamino)-4,5-dihydropyrazolo[3,4-*b*]thiazolo[4,5-*d*]azepin-6(7*H*)-yl)methyl)benzonitrile

6-(3-Fluorobenzyl)-*N*-(5-fluoropyrimidin-2-yl)-4,5,6,7-tetrahydropyrazolo[3,4-*b*] thiazolo[4,5-*d*]azepin-2-amine

6-(Cyclopropylmethyl)-N-(5-methyl-1,2,4-thiadiazol-3-yl)-4,5,6,7-tetrahydropyrazolo [3,4-*b*]thiazolo[4,5-*d*]azepin-2-amine

6-((5-Cyclopropylisoxazol-3-yl)methyl)-*N*-(5-fluoropyrimidin-2-yl)-4,5,6,7-tetrahydro pyrazolo[3,4-*b*]thiazolo[4,5-*d*] azepin-2-amine

6-((2,3-Dihydrobenzofuran-5-yl)methyl)-*N*-(5-fluoropyrimidin-2-yl)-4,5,6,7-tetrahydro pyrazolo[3,4-*b*]thiazolo[4,5-*d*] azepin-2-amine

*N*-(5-Fluoropyrimidin-2-yl)-6-(3-(trifluoromethyl)benzyl)-4,5,6,7-tetrahydropyrazolo [3,4-*b*]thiazolo[4,5-*d*]azepin-2-amine

6-((3-Cyclopropylisoxazol-5-yl)methyl)-*N*-(5-fluoropyrimidin-2-yl)-4,5,6,7-tetrahydro pyrazolo[3,4-*b*]thiazolo[4,5-*d*] azepin-2-amine

6-((2-Cyclopropylthiazol-4-yl)methyl)-*N*-(5-fluoropyrimidin-2-yl)-4,5,6,7-tetrahydro pyrazolo[3,4-*b*]thiazolo[4,5-*d*] azepin-2-amine

6-(1-Methoxypropan-2-yl)-*N*-(5-methyl-1,2,4-thiadiazol-3-yl)-4,5,6,7-tetrahydro pyrazolo[3,4-*b*]thiazolo[4,5-*d*] azepin-2-amine

6-((5-Chloropyridin-2-yl)methyl)-*N*-(4-methylpyrimidin-2-yl)-4,5,6,7-tetrahydro pyrazolo[3,4-*b*]thiazolo[4,5-*d*] azepin-2-amine

*N*-(5-Fluoropyrimidin-2-yl)-6-(3-methoxycyclobutyl)-4,5,6,7-tetrahydropyrazolo[3,4-*b*]thiazolo[4,5-*d*]azepin-2-amine
*N*-(5-Fluoropyrimidin-2-yl)-6-(oxetan-3-yl)-4,5,6,7-tetrahydropyrazolo[3,4-*b*]thiazolo [4,5-*d*]azepin-2-amine
6-(3,3-Difluorocyclobutyl)-*N*-(5-fluoropyrimidin-2-yl)-4,5,6,7-tetrahydropyrazolo[3,4-*b*]thiazolo[4,5-*d*]azepin-2-amine
6-((3,3-Difluorocyclobutyl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-(oxetan-3-ylmethyl)-4,5,6,7-tetrahydropyrazolo[3,4-*b*] thiazolo[4,5-*d*]azepin-2-amine
*N*-(5-Methyl-1,2,4-thiadiazol-3-yl)-4,5,6,7-tetrahydropyrazolo[3,4-*b*]thiazolo[4,5-*d*] azepin-2-amine
*N*-(5-Methyl-1,2,4-thiadiazol-3-yl)-6-(oxetan-3-ylmethyl)-4,5,6,7-tetrahydropyrazolo [3,4-*b*]thiazolo[4,5-*d*]azepin-2-amine
*N*-(5-Methyl-1,2,4-thiadiazol-3-yl)-6-((3-methyloxetan-3-yl)methyl)-4,5,6,7-tetrahydro pyrazolo[3,4-*b*]thiazolo[4,5-*d*]azepin-2-amine
6-(Cyclobutylmethyl)-*N*-(5-methyl-1,2,4-thiadiazol-3-yl)-4,5,6,7-tetrahydropyrazolo [3,4-*b*]thiazolo[4,5-*d*]azepin-2-amine
*N*-(5-Methyl-1,2,4-thiadiazol-3-yl)-6-(oxetan-3-yl)-4,5,6,7-tetrahydropyrazolo[3,4-*b*]thiazolo[4,5-*d*]azepin-2-amine
6-Cyclopropyl-*N*-(5-methyl-1,2,4-thiadiazol-3-yl)-4,5,6,7-tetrahydropyrazolo[3,4-*b*] thiazolo[4,5-*d*]azepin-2-amine
*N*-(5-Methyl-1,2,4-thiadiazol-3-yl)-6-((tetrahydrofuran-2-yl)methyl)-4,5,6,7-tetrahydro pyrazolo[3,4-*b*]thiazolo[4,5-*d*]azepin-2-amine
*N*-(5-Methyl-1,2,4-thiadiazol-3-yl)-6-((tetrahydrofuran-3-yl)methyl)-4,5,6,7-tetrahydro pyrazolo[3,4-*b*]thiazolo[4,5-*d*]azepin-2-amine
6-((1-(Methoxymethyl)cyclopropyl)methyl)-*N*-(5-methyl-1,2,4-thiadiazol-3-yl)-4,5,6,7 -tetrahydropyrazolo[3,4-*b*]thiazolo[4,5-*d*]azepin-2-amine
6-((3,3-Difluorocyclobutyl)methyl)-*N*-(5-methyl-1,2,4-thiadiazol-3-yl)-4,5,6,7-tetrahydropyrazolo[3,4-*b*]thiazolo[4,5-*d*]azepin-2-amine
6-(3,3-Difluorocyclobutyl)-*N*-(5-methyl-1,2,4-thiadiazol-3-yl)-4,5,6,7-tetrahydro pyrazolo[3,4-*b*]thiazolo[4,5-*d*]azepin-2-amine
*N*-(5-Methyl-1,2,4-thiadiazol-3-yl)-6-(tetrahydro-2*H*-pyran-4-yl)-4,5,6,7-tetrahydro pyrazolo[3,4-*b*]thiazolo[4,5-*d*]azepin-2-amine
6-(2-Cyclopropoxyethyl)-*N*-(5-methyl-1,2,4-thiadiazol-3-yl)-4,5,6,7-tetrahydro pyrazolo[3,4-*b*]thiazolo[4,5-*d*]azepin-2-amine
6-(2-Ethoxyethyl)-*N*-(5-methyl-1,2,4-thiadiazol-3-yl)-4,5,6,7-tetrahydropyrazolo[3,4-*b*]thiazolo[4,5-*d*]azepin-2-amine
6-(1-Methoxypropan-2-yl)-*N*-(5-methyl-1,2,4-thiadiazol-3-yl)-4,5,6,7-tetrahydro pyrazolo[3,4-*b*]thiazolo[4,5-*d*]azepin-2-amine
*N*-(4-Methoxypyrimidin-2-yl)-4,5,6,7-tetrahydropyrazolo[3,4-*b*]thiazolo[4,5-*d*]azepin-2-amine
*N*-(4-Methoxypyrimidin-2-yl)-6-(oxetan-3-ylmethyl)-4,5,6,7-tetrahydropyrazolo[3,4-*b*] thiazolo[4,5-*d*]azepin-2-amine
*N*-(4-Methoxypyrimidin-2-yl)-6-((3-methyloxetan-3-yl)methyl)-4,5,6,7-tetrahydro pyrazolo[3,4-*b*]thiazolo[4,5-*d*]azepin-2-amine
6-(Cyclobutylmethyl)-*N*-(4-methoxypyrimidin-2-yl)-4,5,6,7-tetrahydropyrazolo[3,4-*b*] thiazolo[4,5-*d*]azepin-2-amine
*N*-(4-Methoxypyrimidin-2-yl)-6-(oxetan-3-yl)-4,5,6,7-tetrahydropyrazolo[3,4-*b*] thiazolo[4,5-*d*]azepin-2-amine
6-Cyclopropyl-*N*-(4-methoxypyrimidin-2-yl)-4,5,6,7-tetrahydropyrazolo[3,4-*b*]thiazolo [4,5-*d*]azepin-2-amine
*N*-(4-Methoxypyrimidin-2-yl)-6-((tetrahydrofuran-2-yl)methyl)-4,5,6,7-tetrahydro pyrazolo[3,4-*b*]thiazolo[4,5-*d*]azepin-2-amine
*N*-(4-Methoxypyrimidin-2-yl)-6-((tetrahydrofuran-3-yl)methyl)-4,5,6,7-tetrahydro pyrazolo[3,4-*b*]thiazolo[4,5-*d*]azepin-2-amine
6-((1-(Methoxymethyl)cyclopropyl)methyl)-*N*-(4-methoxypyrimidin-2-yl)-4,5,6,7-tetrahydropyrazolo[3,4-*b*]thiazolo[4,5-*d*]azepin-2-amine
6-((3,3-Difluorocyclobutyl)methyl)-*N*-(4-methoxypyrimidin-2-yl)-4,5,6,7-tetrahydro pyrazolo[3,4-*b*]thiazolo[4,5-*d*]azepin-2-amine
6-(3,3-Difluorocyclobutyl)-*N*-(4-methoxypyrimidin-2-yl)-4,5,6,7-tetrahydropyrazolo [3,4-*b*]thiazolo[4,5-*d*]azepin-2-amine
*N*-(4-Methoxypyrimidin-2-yl)-6-(tetrahydro-2*H*-pyran-4-yl)-4,5,6,7-tetrahydropyrazolo [3,4-*b*]thiazolo[4,5-*d*]azepin-2-amine
6-(2-Cyclopropoxyethyl)-*N*-(4-methoxypyrimidin-2-yl)-4,5,6,7-tetrahydropyrazolo [3,4-*b*]thiazolo[4,5-*d*]azepin-2-amine
6-(2-Ethoxyethyl)-*N*-(4-methoxypyrimidin-2-yl)-4,5,6,7-tetrahydropyrazolo[3,4-*b*] thiazolo[4,5-*d*]azepin-2-amine

6-(1-Methoxypropan-2-yl)-*N*-(4-methoxypyrimidin-2-yl)-4,5,6,7-tetrahydropyrazolo [3,4-*b*]thiazolo[4,5-*d*]azepin-2-amine
*N*-(5-Fluoropyrimidin-2-yl)-6-((1-(methoxymethyl)cyclopropyl)methyl)-4,5,6,7-tetrahydropyrazolo[3,4-*b*]thiazolo[4,5-*d*]azepin-2-amine
*N*-(5-Fluoropyrimidin-2-yl)-6-(tetrahydro-2Hpyran-4-yl)-4,5,6,7-tetrahydropyrazolo [3,4-*b*]thiazolo[4,5-*d*]azepin-2-amine
6-Cyclopropyl-*N*-(5-fluoropyrimidin-2-yl)-4,5,6,7-tetrahydropyrazolo[3,4-*b*]thiazolo [4,5-*d*]azepin-2-amine
6-(2-Ethoxyethyl)-*N*-(5-fluoropyrimidin-2-yl)-4,5,6,7-tetrahydropyrazolo[3,4-*b*] thiazolo[4,5-*d*]azepin-2-amine
*N*-(5-Fluoropyrimidin-2-yl)-6-(2-methoxypropyl)-4,5,6,7-tetrahydropyrazolo[3,4-*b*] thiazolo[4,5-*d*]azepin-2-amine
*N*-(4-Methylpyrimidin-2-yl)-4,5,6,7-tetrahydropyrazolo[3,4-*b*]thiazolo[4,5-*d*]azepin-2-amine
*N*-(4-Methylpyrimidin-2-yl)-6-(oxetan-3-yl)-4,5,6,7-tetrahydropyrazolo[3,4-*b*]thiazolo [4,5-*d*]azepin-2-amine
6-Cyclopropyl-*N*-(4-methylpyrimidin-2-yl)-4,5,6,7-tetrahydropyrazolo[3,4-*b*]thiazolo [4,5-*d*]azepin-2-amine
*N*-(4-Methylpyrimidin-2-yl)-6-(oxetan-3-ylmethyl)-4,5,6,7-tetrahydropyrazolo[3,4-*b*]thiazolo[4,5-*d*]azepin-2-amine
*N*-(4-Methylpyrimidin-2-yl)-6-((tetrahydrofuran-3-yl)methyl)-4,5,6,7-tetrahydro pyrazolo[3,4-*b*]thiazolo[4,5-*d*]azepin-2-amine
*N*-(4-Methylpyrimidin-2-yl)-6-(tetrahydro-2*H*-pyran-4-yl)-4,5,6,7-tetrahydropyrazolo [3,4-*b*]thiazolo[4,5-*d*]azepin-2-amine
$N^2$-(6-(Cyclopropylmethyl)-4,5,6,7-tetrahydropyrazolo[3,4-*b*]thiazolo[4,5-*d*]azepin-2-yl)pyrimidine-2,4-diamine
$N^2$-(6-(2-Methoxyethyl)-4,5,6,7-tetrahydropyrazolo[3,4-*b*]thiazolo[4,5-*d*]azepin-2-yl) pyrimidine-2,4-diamine
4-(2-(5-Fluoropyrimidin-2-ylamino)-4,5-dihydropyrazolo[3,4-*b*]thiazolo[4,5-*d*]azepin-6(7*H*)-yl)butan-2-one
6-(2-(Azetidin-1-yl)ethyl)-*N*-(5-fluoropyrimidin-2-yl)-4,5,6,7-tetrahydropyrazolo[3,4-*b*]thiazolo[4,5-*d*]azepin-2-amine
6-((4-Cyclopropyl-1-methyl-1*H*-pyrrol-2-yl)methyl)-*N*-(5-fluoropyrimidin-2-yl)-4,5,6,7-tetrahydropyrazolo[3,4-*b*]thiazolo[4,5-*d*]azepin-2-amine
6-((1-Cyclopropyl-1*H*-pyrrol-3-yl)methyl)-*N*-(5-fluoropyrimidin-2-yl)-4,5,6,7-tetrahydropyrazolo[3,4-*b*]thiazolo[4,5-*d*]azepin-2-amine
*N*-(5-Fluoropyrimidin-2-yl)-6-((1-methyl-1H-imidazol-2-yl)methyl)-4,5,6,7-tetrahydro pyrazolo[3,4-*b*]thiazolo[4,5-*d*]azepin-2-amine
6-((1-Cyclopropyl-1*H*-pyrazol-4-yl)methyl)-*N*-(5-fluoropyrimidin-2-yl)-4,5,6,7-tetrahydropyrazolo[3,4-*b*]thiazolo[4,5-*d*]azepin-2-amine
6-((1-Cyclopropyl-1*H*-pyrazol-3-yl)methyl)-*N*-(5-fluoropyrimidin-2-yl)-4,5,6,7-tetrahydropyrazolo[3,4-*b*]thiazolo[4,5-*d*]azepin-2-amine
6-((3-Fluoropyridin-2-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-((2-methylpyridin-4-yl)methyl)-4,5,6,7-tetrahydro pyrazolo[3,4-*b*]thiazolo[4,5-*d*]azepin-2-amine
6-((2-Cyclopropylpyridin-4-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-(pyrimidin-4-ylmethyl)-4,5,6,7-tetrahydropyrazolo[3,4-*b*]thiazolo[4,5-*d*]azepin-2-amine
*N*-(5-Fluoropyrimidin-2-yl)-6-((2-methylpyrimidin-4-yl)methyl)-4,5,6,7-tetrahydro pyrazolo[3,4-*b*]thiazolo[4,5-*d*]azepin-2-amine
6-((2,5-Dimethylthiazol-4-yl)methyl)-*N*-(5-fluoropyrimidin-2-yl)-4,5,6,7-tetrahydro pyrazolo[3,4-*b*]thiazolo[4,5-*d*]azepin-2-amine
6-((2,4-Dimethylthiazol-5-yl)methyl)-*N*-(5-fluoropyrimidin-2-yl)-4,5,6,7-tetrahydro pyrazolo[3,4-*b*]thiazolo[4,5-*d*]azepin-2-amine
6-((2-Cyclopropyloxazol-4-yl)methyl)-*N*-(5-fluoropyrimidin-2-yl)-4,5,6,7-tetrahydro pyrazolo[3,4-*b*]thiazolo[4,5-*d*]azepin-2-amine
6-((2-Cyclopropyl-5-methyloxazol-4-yl)methyl)-*N*-(5-fluoropyrimidin-2-yl)-4,5,6,7-tetrahydropyrazolo[3,4-*b*]thiazolo[4,5-*d*]azepin-2-amine
4-(2-(4-Methylpyrimidin-2-ylamino)-4,5-dihydropyrazolo[3,4-*b*]thiazolo[4,5-*d*]azepin-6(7*H*)-yl)butan-2-one
6-(2-(Azetidin-1-yl)ethyl)-*N*-(4-methylpyrimidin-2-yl)-4,5,6,7-tetrahydropyrazolo[3,4-*b*]thiazolo[4,5-*d*]azepin-2-amine
6-((4-Cyclopropyl-1-methyl-1*H*-pyrrol-2-yl)methyl)-*N*-(4-methylpyrimidin-2-yl)-4,5,6,7-tetrahydropyrazolo[3,4-*b*]thiazolo[4,5-*d*]azepin-2-amine
6-((1-Cyclopropyl-1*H*-pyrrol-3-yl)methyl)-*N*-(4-methylpyrimidin-2-yl)-4,5,6,7-tetrahydropyrazolo[3,4-*b*]thiazolo[4,5-*d*]azepin-2-amine
6-((1-Methyl-1*H*-imidazol-2-yl)methyl)-*N*-(4-methylpyrimidin-2-yl)-4,5,6,7-tetrahydro pyrazolo[3,4-*b*]thiazolo[4,5-*d*]azepin-2-amine

6-((1-Cyclopropyl-1*H*-pyrazol-4-yl)methyl)-*N*-(4-methylpyrimidin-2-yl)-4,5,6,7-tetrahydropyrazolo[3,4-*b*]thiazolo[4,5-*d*]azepin-2-amine
6-((1-Cyclopropyl-1*H*-pyrazol-3-yl)methyl)-*N*-(4-methylpyrimidin-2-yl)-4,5,6,7-tetrahydropyrazolo[3,4-*b*]thiazolo[4,5-*d*]azepin-2-amine
6-((3-Fluoropyridin-2-yl)methyl)-*N*-(4-methylpyrimidin-2-yl)-4,5,6,7-tetrahydro pyrazolo[3,4-*b*]thiazolo[4,5-*d*] azepin-2-amine
6-((2-Methylpyridin-4-yl)methyl)-*N*-(4-methylpyrimidin-2-yl)-4,5,6,7-tetrahydro pyrazolo[3,4-*b*]thiazolo[4,5-*d*] azepin-2-amine
6-((2-Cyclopropylpyridin-4-yl)methyl)-*N*-(4-methylpyrimidin-2-yl)-4,5,6,7-tetrahydro pyrazolo[3,4-*b*]thiazolo[4,5-*d*] azepin-2-amine
*N*-(4-Methylpyrimidin-2-yl)-6-(pyrimidin-4-ylmethyl)-4,5,6,7-tetrahydropyrazolo[3,4-*b*]thiazolo[4,5-*d*]azepin-2-amine
*N*-(4-Methylpyrimidin-2-yl)-6-((2-methylpyrimidin-4-yl)methyl)-4,5,6,7-tetrahydro pyrazolo[3,4-*b*]thiazolo[4,5-*d*] azepin-2-amine
6-((2,5-Dimethylthiazol-4-yl)methyl)-*N*-(4-methylpyrimidin-2-yl)-4,5,6,7-tetrahydro pyrazolo[3,4-*b*]thiazolo[4,5-*d*] azepin-2-amine
6-((2,4-Dimethylthiazol-5-yl)methyl)-*N*-(4-methylpyrimidin-2-yl)-4,5,6,7-tetrahydro pyrazolo[3,4-*b*]thiazolo[4,5-*d*] azepin-2-amine
6-((2-Cyclopropyloxazol-4-yl)methyl)-*N*-(4-methylpyrimidin-2-yl)-4,5,6,7-tetrahydro pyrazolo[3,4-*b*]thiazolo[4,5-*d*] azepin-2-amine
6-((2-Cyclopropyl-5-methyloxazol-4-yl)methyl)-*N*-(4-methylpyrimidin-2-yl)-4,5,6,7-tetrahydropyrazolo[3,4-*b*]thiazolo[4,5-*d*]azepin-2-amine
4-(2-(4-Methoxypyrimidin-2-ylamino)-4,5-dihydropyrazolo[3,4-*b*]thiazolo[4,5-*d*] azepin-6(7*H*)-yl)butan-2-one
6-(2-(Azetidin-1-yl)ethyl)-*N*-(4-methoxypyrimidin-2-yl)-4,5,6,7-tetrahydropyrazolo [3,4-*b*]thiazolo[4,5-*d*]azepin-2-amine
6-((4-Cyclopropyl-1-methyl-1*H*-pyrrol-2-yl)methyl)-*N*-(4-methoxypyrimidin-2-yl)-4,5,6,7-tetrahydropyrazolo[3,4-*b*] thiazolo[4,5-*d*]azepin-2-amine
6-((1-Cyclopropyl-1*H*-pyrrol-3-yl)methyl)-*N*-(4-methoxypyrimidin-2-yl)-4,5,6,7-tetrahydropyrazolo[3,4-*b*]thiazolo[4,5-*d*]azepin-2-amine
*N*-(4-Methoxypyrimidin-2-yl)-6-((1-methyl-1*H*-imidazol-2-yl)methyl)-4,5,6,7-tetrahydropyrazolo[3,4-*b*]thiazolo[4,5-*d*]azepin-2-amine
6-((1-Cyclopropyl-1*H*-pyrazol-4-yl)methyl)-*N*-(4-methoxypyrimidin-2-yl)-4,5,6,7-tetrahydropyrazolo[3,4-*b*]thiazolo[4,5-*d*]azepin-2-amine
6-((1-Cyclopropyl-1*H*-pyrazol-3-yl)methyl)-*N*-(4-methoxypyrimidin-2-yl)-4,5,6,7-tetrahydropyrazolo[3,4-*b*]thiazolo[4,5-*d*]azepin-2-amine
6-((3-Fluoropyridin-2-yl)methyl)-*N*-(4-methoxypyrimidin-2-yl)-4,5,6,7-tetrahydro pyrazolo[3,4-*b*]thiazolo[4,5-*d*] azepin-2-amine
*N*-(4-Methoxypyrimidin-2-yl)-6-((2-methylpyridin-4-yl)methyl)-4,5,6,7-tetrahydro pyrazolo[3,4-*b*]thiazolo[4,5-*d*] azepin-2-amine
6-((2-Cyclopropylpyridin-4-yl)methyl)-*N*-(4-methoxypyrimidin-2-yl)-4,5,6,7-tetrahydropyrazolo[3,4-*b*]thiazolo[4,5-*d*] azepin-2-amine
*N*-(4-Methoxypyrimidin-2-yl)-6-(pyrimidin-4-ylmethyl)-4,5,6,7-tetrahydropyrazolo [3,4-*b*]thiazolo[4,5-*d*]azepin-2-amine
*N*-(4-Methoxypyrimidin-2-yl)-6-((2-methylpyrimidin-4-yl)methyl)-4,5,6,7-tetrahydro pyrazolo[3,4-*b*]thiazolo[4,5-*d*] azepin-2-amine
6-((2,5-Dimethylthiazol-4-yl)methyl)-*N*-(4-methoxypyrimidin-2-yl)-4,5,6,7-tetrahydro pyrazolo[3,4-*b*]thiazolo[4,5-*d*] azepin-2-amine
6-((2,4-Dimethylthiazol-5-yl)methyl)-*N*-(4-methoxypyrimidin-2-yl)-4,5,6,7-tetrahydro pyrazolo[3,4-*b*]thiazolo[4,5-*d*] azepin-2-amine
6-((2-Cyclopropyloxazol-4-yl)methyl)-*N*-(4-methoxypyrimidin-2-yl)-4,5,6,7-tetrahydropyrazolo[3,4-*b*]thiazolo[4,5-*d*] azepin-2-amine
6-((2-Cyclopropyl-5-methyloxazol-4-yl)methyl)-*N*-(4-methoxypyrimidin-2-yl)-4,5,6,7-tetrahydropyrazolo[3,4-*b*]thiazolo[4,5-*d*]azepin-2-amine
4-(2-(6-Methylpyridin-2-ylamino)-4,5-dihydropyrazolo[3,4-*b*]thiazolo[4,5-*d*]azepin-6(7*H*)-yl)butan-2-one
6-(2-(Azetidin-1-yl)ethyl)-*N*--(6-methylpyridin-2-yl)-4,5,6,7-tetrahydropyrazolo[3,4-*b*] thiazolo[4,5-*d*]azepin-2-amine
6-((4-Cyclopropyl-1-methyl-1H-pyrrol-2-yl)methyl)-*N*-(6-methylpyridin-2-yl)-4,5,6,7-tetrahydropyrazolo[3,4-*b*]thiazolo[4,5-*d*]azepin-2-amine
6-((1-Cyclopropyl-1*H*-pyrrol-3-yl)methyl)-*N*-(6-methylpyridin-2-yl)-4,5,6,7-tetrahydro pyrazolo[3,4-*b*]thiazolo[4,5-*d*]

azepin-2-amine
6-((1-Methyl-1*H*-imidazol-2-yl)methyl)-*N*-(6-methylpyridin-2-yl)-4,5,6,7-tetrahydro pyrazolo[3,4-*b*]thiazolo[4,5-*d*]azepin-2-amine
6-((1-Cyclopropyl-1*H*-pyrazol-4-yl)methyl)-*N*-(6-methylpyridin-2-yl)-4,5,6,7-tetrahydropyrazolo[3,4-*b*]thiazolo[4,5-*d*]azepin-2-amine
6-((1-Cyclopropyl-1*H*-pyrazol-3-yl)methyl)-*N*-(6-methylpyridin-2-yl)-4,5,6,7-tetrahydropyrazolo[3,4-*b*]thiazolo[4,5-*d*]azepin-2-amine
6-((3-Fluoropyridin-2-yl)methyl)-*N*-(6-methylpyridin-2-yl)-4,5,6,7-tetrahydropyrazolo [3,4-*b*]thiazolo[4,5-*d*]azepin-2-amine
*N*-(6-Methylpyridin-2-yl)-6-((2-methylpyridin-4-yl)methyl)-4,5,6,7-tetrahydropyrazolo [3,4-*b*]thiazolo[4,5-*d*]azepin-2-amine
6-((2-Cyclopropylpyridin-4-yl)methyl)-*N*-(6-methylpyridin-2-yl)-4,5,6,7-tetrahydro pyrazolo[3,4-*b*]thiazolo[4,5-*d*]azepin-2-amine
*N*-(6-Methylpyridin-2-yl)-6-(pyrimidin-4-ylmethyl)-4,5,6,7-tetrahydropyrazolo[3,4-*b*] thiazolo[4,5-*d*]azepin-2-amine
*N*-(6-Methylpyridin-2-yl)-6-((2-methylpyrimidin-4-yl)methyl)-4,5,6,7-tetrahydro pyrazolo[3,4-*b*]thiazolo[4,5-*d*]azepin-2-amine
6-((2,5-Dimethylthiazol-4-yl)methyl)-*N*-(6-methylpyridin-2-yl)-4,5,6,7-tetrahydro pyrazolo[3,4-*b*]thiazolo[4,5-*d*]azepin-2-amine
6-((2,4-Dimethylthiazol-5-yl)methyl)-*N*-(6-methylpyridin-2-yl)-4,5,6,7-tetrahydro pyrazolo[3,4-*b*]thiazolo[4,5-*d*]azepin-2-amine
6-((2-Cyclopropyloxazol-4-yl)methyl)-*N*-(6-methylpyridin-2-yl)-4,5,6,7-tetrahydro pyrazolo[3,4-*b*]thiazolo[4,5-*d*]azepin-2-amine
6-((2-Cyclopropyl-5-methyloxazol-4-yl)methyl)-*N*-(6-methylpyridin-2-yl)-4,5,6,7-tetrahydropyrazolo[3,4-*b*]thiazolo[4,5-*d*]azepin-2-amine
.Also disclosed are:
*N*-(5-Fluoropyrimidin-2-yl)-6-(2-methoxy-2-methylpropyl)-4,5,6,8-tetrahydropyrazolo [3,4-*b*]thiazolo[4,5-*d*]azepin-2-amine
*N*-(5-Fluoropyrimidin-2-yl)-6-(1-methoxypropan-2-yl)-4,5,6,8-tetrahydropyrazolo[3,4-*b*]thiazolo[4,5-*d*]azepin-2-amine
*N*-(5-Fluoropyrimidin-2-yl)-6-(((*S*)-tetrahydrofuran-2-yl)methyl)-4,5,6,8-tetrahydro pyrazolo[3,4-*b*]thiazolo[4,5-*d*]azepin-2-amine
*N*-(6-Methylpyridin-2-yl)-6-(((*S*)-tetrahydrofuran-2-yl)methyl)-4,5,6,8-tetrahydro pyrazolo[3,4-*b*]thiazolo[4,5-*d*]azepin-2-amine
6-((5-Methylisoxazol-3-yl)methyl)-*N*-(6-methylpyridin-2-yl)-4,5,6,8-tetrahydro pyrazolo[3,4-*b*]thiazolo[4,5-*d*]azepin-2-amine
*N*-(5-Fluoro-4-methylpyrimidin-2-yl)-6-(2-methoxyethyl)-4,5,6,8-tetrahydropyrazolo [3,4-*b*]thiazolo[4,5-*d*]azepin-2-amine
*N*-(5-Fluoropyrimidin-2-yl)-6-(3-(methylamino)propyl)-4,5,6,8-tetrahydropyrazolo [3,4-*b*]thiazolo[4,5-*d*]azepin-2-amine
*N*-(5-Fluoropyrimidin-2-yl)-6-(2-methoxy-l(*S*)-methyl-ethyl)-4,5,6,8-tetrahydro pyrazolo[3,4-*b*]thiazolo[4,5-*d*]azepin-2-amine
3-(2-(5-Fluoropyrimidin-2-ylamino)-4,5-dihydropyrazolo[3,4-*b*]thiazolo[4,5-*d*]azepin-6(8*H*)-yl)propan-1-ol
*N*-(5-Fluoropyrimidin-2-yl)-6-(2-(2-methoxyethoxy)ethyl)-4,5,6,8-tetrahydropyrazolo [3,4-*b*]thiazolo[4,5-*d*]azepin-2-amine
6-(2-Ethoxyethyl)-*N*-(5-fluoropyrimidin-2-yl)-4,5,6,8-tetrahydropyrazolo[3,4-*b*] thiazolo[4,5-*d*]azepin-2-amine
*N*-(5-Fluoropyrimidin-2-yl)-6-((5-morpholinopyridin-2-yl)methyl)-4,5,6,8-tetrahydro pyrazolo[3,4-*b*]thiazolo[4,5-*d*]azepin-2-amine
*N*-(5-Fluoropyrimidin-2-yl)-6-(2-morpholinoethyl)-4,5,6,8-tetrahydropyrazolo[3,4-*b*] thiazolo[4,5-*d*]azepin-2-amine
6-(2-Fluoroethyl)-*N*-(5-fluoropyrimidin-2-yl)-4,5,6,8-tetrahydropyrazolo[3,4-*b*] thiazolo[4,5-*d*]azepin-2-amine
*N*-(5-Fluoropyrimidin-2-yl)-6-((5-methyl-1,2,4-oxadiazol-3-yl)methyl)-4,5,6,8-tetrahydropyrazolo[3,4-*b*]thiazolo[4,5-*d*]azepin-2-amine
*N*-(5-Fluoropyrimidin-2-yl)-6-((1-methyl-1*H*-pyrazol-3-yl)methyl)-4,5,6,8-tetrahydro pyrazolo[3,4-*b*]thiazolo[4,5-*d*]azepin-2-amine
*N*-(5-Fluoropyrimidin-2-yl)-6-(3-morpholinopropyl)-4,5,6,8-tetrahydropyrazolo[3,4-*b*] thiazolo[4,5-*d*]azepin-2-amine
*N*-(5-Fluoropyrimidin-2-yl)-6-(2-(4-isopropylpiperazin-1-yl)ethyl)-4,5,6,8-tetrahydro pyrazolo[3,4-*b*]thiazolo[4,5-*d*]azepin-2-amine
*N*-(5-Fluoropyrimidin-2-yl)-6-((tetrahydrofuran-3-yl)methyl)-4,5,6,8-tetrahydro pyrazolo[3,4-*b*]thiazolo[4,5-*d*]azepin-2-amine

*N*-(5-Fluoropyrimidin-2-yl)-6-((5-(methoxymethyl)pyridin-2-yl)methyl)-4,5,6,8-tetrahydropyrazolo[3,4-*b*]thiazolo[4,5-*d*]azepin-2-amine

*N*-(5-Fluoropyrimidin-2-yl)-6-(3-(piperidin-1-yl)propyl)-4,5,6,8-tetrahydropyrazolo [3,4-*b*]thiazolo[4,5-*d*]azepin-2-amine

*N*-(4-Methylpyrimidin-2-yl)-6-(3-morpholinopropyl)-4,5,6,8-tetrahydropyrazolo[3,4-*b*] thiazolo[4,5-*d*]azepin-2-amine

6-((5-Methyl-1,2,4-oxadiazol-3-yl)methyl)-*N*-(4-methylpyrimidin-2-yl)-4,5,6,8-tetrahydropyrazolo[3,4-*b*]thiazolo[[4,5-*d*]azepin-2-amine

*N*-(5-Fluoropyrimidin-2-yl)-6-((5-isopropyl-1,2,4-oxadiazol-3-yl)methyl)-4,5,6,8-tetrahydropyrazolo[3,4-*b*]thiazolo[[4,5-*d*]azepin-2-amine

6-(3-(4,4-Difluoropiperidin-1-yl)propyl)-*N*-(5-fluoropyrimidin-2-yl)-4,5,6,8-tetrahydro pyrazolo[3,4-*b*]thiazolo[4,5-*d*] azepin-2-amine

6-(3-Fluoropropyl)-*N*-(5-fluoropyrimidin-2-yl)-4,5,6,8-tetrahydropyrazolo[3,4-*b*] thiazolo[4,5-*d*]azepin-2-amine

*N*-(5-Fluoropyrimidin-2-yl)-6-((3-methyl-1,2,4-oxadiazol-5-yl)methyl)-4,5,6,8-tetrahydropyrazolo[3,4-*b*]thiazolo[[4,5-*d*]azepin-2-amine

4-(3-(2-((5-Fluoropyrimidin-2-yl)amino)-4,5-dihydropyrazolo[3,4-*b*]thiazolo[4,5-*d*] azepin-6(8*H*)-yl)propyl)thiomorpholine 1,1-dioxide

*N*-(4-Methylpyrimidin-2-yl)-6-(pyrimidin-2-ylmethyl)-4,5,6,8-tetrahydropyrazolo[3,4-*b*]thiazolo[4,5-*d*]azepin-2-amine

6-(3-(3,5-Dimethylmorpholino)propyl)-*N*-(5-fluoropyrimidin-2-yl)-4,5,6,8-tetrahydro pyrazolo[3,4-*b*]thiazolo[4,5-*d*] azepin-2-amine

*N*-(5-Fluoropyrimidin-2-yl)-6-((5-morpholinopyridin-2-yl)methyl)-4,5,6,8-tetrahydro pyrazolo[3,4-*b*]thiazolo[4,5-*d*] azepin-2-amine trihydrochloride

*N*-(5-Fluoropyrimidin-2-yl)-6-((5-methyl-1,2,4-oxadiazol-3-yl)methyl)-4,5,6,8-tetrahydropyrazolo[3,4-*b*]thiazolo[4,5-d]azepin-2-amine monohydrochloride and

*N*-(5-Fluoropyrimidin-2-yl)-6-(3-morpholinopropyl)-4,5,6,8-tetrahydropyrazolo[3,4-*b*] thiazolo[4,5-*d*]azepin-2-amine dihydrochloride dihydrate.

Particularly relevant to the present invention is the tautomeric pair that exists for the pyrazole ring, illustrated below:

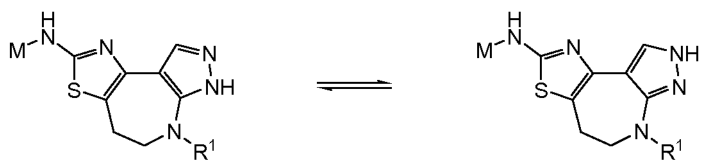

In this specification, reference to a generic formula or a compound as such indicating one tautomer is to be understood to refer to the tautomeric pair and the other tautomer thereof.

The disclosed compounds also include all pharmaceutically acceptable isotopic variations, in which at least one atom is replaced by an atom having the same atomic number, but an atomic mass different from the atomic mass usually found in nature. Examples of isotopes suitable for inclusion in the disclosed compounds include, without limitation, isotopes of hydrogen, such as $^{2}H$ and $^{3}H$; isotopes of carbon, such as $^{13}C$ and $^{14}C$; isotopes of nitrogen, such as $^{15}N$; isotopes of oxygen, such as $^{17}O$ and $^{18}O$; isotopes of phosphorus, such as $^{32}P$ and $^{33}P$; isotopes of sulfur, such as $^{35}S$; isotopes of fluorine, such as $^{18}F$; and isotopes of chlorine, such as $^{36}Cl$. Use of isotopic variations (e.g., deuterium, $^{2}H$) may afford certain therapeutic advantages resulting from greater metabolic stability, for example, *increased in vivo* half-life or reduced dosage requirements. Additionally, certain isotopic variations of the disclosed compounds may incorporate a radioactive isotope (e.g., tritium, $^{3}H$, or $^{14}C$), which may be useful in drug and/or substrate tissue distribution studies. Substitution with positron emitting isotopes, such as $^{11}C$, $^{18}F$, $^{15}O$ and $^{13}N$, can be useful in Positron Emission Topography (PET) studies for examining substrate receptor occupancy. Isotopically-labelled compounds of Formula (I) to (III) can generally be prepared by conventional techniques known to those skilled in the art or by processes analogous to those described in the accompanying Examples using appropriate isotopically-labeled reagents in place of the non-labeled reagents previously employed.

DEFINITION OF TERMS

Listed below are definitions of various terms used in the specification and claims to describe the present invention.

For the avoidance of doubt it is to be understood that in this specification "($C_1$-$C_6$)" means a carbon radical having 1, 2, 3, 4, 5 or 6 carbon atoms. "($C_0$-$C_6$)" means a carbon radical having 0, 1, 2, 3, 4, 5 or 6 carbon atoms. In this specification "C" means a carbon atom, "N" means a nitrogen atom, "O" means an oxygen atom and "S" means a sulphur atom.

In the case where a subscript is the integer 0 (zero) the radical to which the subscript refers, indicates that the radical is absent, i.e. there is a direct bond between the radicals.

In the case where a subscript is the integer 0 (zero) and the radical to which the subscript refers is alkyl, this indicates the radical is a hydrogen atom.

In this specification, unless stated otherwise, the term "bond" refers to a saturated covalent bond. When two or more bonds are adjacent to one another, they are assumed to be equal to one bond. For example, a radical -A-B-, wherein both A and B may be a bond, the radical is depicting a single bond.

In this specification, unless stated otherwise, the term "alkyl" includes both straight and branched chain alkyl radicals and may be methyl, ethyl, *n*-propyl, *i*-propyl, *n*-butyl, *i*-butyl, *s*-butyl, *t*-butyl, *n*-pentyl, *i*-pentyl, *t*-pentyl, *neo*-pentyl, *n*-hexyl, *i*-hexyl or *t*-hexyl. The term "($C_0$-$C_3$)alkyl" refers to an alkyl radical having 0, 1, 2 or 3 carbon atoms and may be methyl, ethyl, *n*-propyl and *i*-propyl.

In this specification, unless stated otherwise, the term "alkylene" includes both straight and branched difunctional saturated hydrocarbon radicals and may be methylene ($-CH_2-$), ethylene ($-CH_2-CH_2-$), *n*-propylene ($-CH_2-CH_2-CH_2-$), *i*-propylene ($-CH-(CH_3)-CH_2-$), *n*-butylene ($-CH_2-CH_2-CH_2-CH_2-$), *i*-butylene ($-CH_2-CH-(CH_3)-CH_2-$), *t*-butylene ($-CH_2-C-(CH_3)-CH_2-$), *n*-pentylene ($-CH_2-CH_2-CH_2-CH_2-CH_2-$), *i*-pentylene ($-CH_2-CH(CH_3)-CH_2-CH_2-$), *neo*-pentylene ($-CH_2-C(CH_3)_2-CH_2-$), *n*-hexylene ($-CH_2-CH_2-CH_2-CH_2-CH_2-CH_2-$) or *i*-hexylene ($-CH_2-CH-(CH_3)-CH_2-CH_2-CH_2-$).

In this specification, unless stated otherwise, the term "cycloalkyl" refers to an optionally substituted carbocycle containing no heteroatoms, including mono-, bi-, and tricyclic saturated carbocycles, as well as fused ring systems. Such fused ring systems can include one ring that is partially or fully unsaturated such as a benzene ring to form fused ring systems such as benzo- fused carbocycles. Cycloalkyl includes such fused ring systems as spirofused ring systems. Examples of cycloalkyl include cyclopropyl, cyclobutyl, cyclopentyl, cyclohexyl, decahydronaphthalene, adamantane, indanyl, fluorenyl and 1,2,3,4-tetrahydronaphthalene and the like. The term "($C_3$-$C_7$)cycloalkyl" may be cyclopropyl, cyclobutyl, cyclopentyl, cyclohexyl, cycloheptyl and the like.

The term "aryl" refers to an optionally substituted monocyclic or bicyclic hydrocarbon ring system containing at least one unsaturated aromatic ring. Examples and suitable values of the term "aryl" are phenyl, naphthyl, 1,2,3,4-tetrahydronaphthyl, indyl, indenyl and the like.

In this specification, unless stated otherwise, the term "heteroaryl" refers to an optionally substituted monocyclic or bicyclic unsaturated, aromatic ring system containing at least one heteroatom selected independently from N, O or S. Examples of "heteroaryl" may be, but are not limited to thienyl, pyridinyl, thiazolyl, isothiazolyl, furyl, pyrrolyl, triazolyl, imidazolyl, triazinyl, oxadiazolyl, oxazolyl, isoxazolyl, pyrazolyl, imidazolonyl, oxazolonyl, thiazolonyl, tetrazolyl, thiadiazolyl, benzoimidazolyl, benzoxazolyl, benzothiazolyl, tetrahydrotriazolopyridinyl, tetrahydrotriazolopyrimidinyl, benzofuryl, benzothiophenyl, thionaphthyl, indolyl, isoindolyl, pyridonyl, pyridazinyl, pyrazinyl, pyrimidinyl, quinolyl, phtalazinyl, naphthyridinyl, quinoxalinyl, quinazolyl, imidazopyridinyl, oxazolopyridinyl, thiazolopyridinyl, imidazopyridazinyl, oxazolopyridazinyl, thiazolopyridazinyl, cynnolyl, pteridinyl, furazanyl, benzotriazolyl, pyrazolopyridinyl and purinyl.

In this specification, unless stated otherwise, the term "heterocycle" refers to an optionally substituted, monocyclic or bicyclic saturated, partially saturated or unsaturated ring system containing at least one heteroatom selected independently from N, O and S. Examples of such rings may be, but are not limited to, morpholinyl, piperazinyl, piperidyl and dioxothiomorpholinyl.

In this specification, unless stated otherwise, the term "alkylene-aryl", "alkylene-heteroaryl", "alkylene-heterocycle" and "alkylene-cycloalkyl" refers respectively to a substituent that is attached via the alkyl radical to an aryl, heteroaryl, heterocycle or cycloalkyl radical, respectively. The term "($C_1$-$C_6$)alkylene-aryl" includes aryl-$C_1$-$C_6$-alkyl radicals such as benzyl, 1-phenylethyl, 2-phenylethyl, 1-phenylpropyl, 2-phenylpropyl, 3-phenylpropyl, 1-naphthylmethyl and 2-naphthylmethyl. The term "($C_1$-$C_6$)alkylene-heteroaryl" includes heteroaryl-$C_1$-$C_6$-alkyl radicals, wherein examples of heteroaryl are the same as those illustrated in the above definition, such as 2-furylmethyl, 3-furylmethyl, 2-thienylmethyl, 3-thienylmethyl, 1-imidazolylmethyl, 2-imidazolylmethyl, 3-imidazolylmethyl, 2-oxazolylmethyl, 3-oxazolylmethyl, 2-thiazolylmethyl, 3-thiazolylmethyl, 2-pyridinylmethyl, 3-pyridinylmethyl, 4-pyridinylmethyl, 1-quinolylmethyl or the like.

In this specification, unless stated otherwise, a 5- or 6-membered ring containing one or more atoms independently selected from C, N, O and S, includes aromatic and heteroaromatic rings as well as carbocyclic and heterocyclic rings which may be saturated or unsaturated. Examples of such rings may be, but are not limited to, furyl, isoxazolyl, isothiazolyl, oxazolyl, pyrazinyl, pyrazolyl, pyridazinyl, pyridinyl, pyrimidinyl, pyrrolyl, thiazolyl, thienyl, imidazolyl, imidazolidinyl, imidazolinyl, triazolyl, morpholinyl, piperazinyl, piperidyl, piperidonyl, pyrazolidinyl, pyrazolinyl, pyrrolidinyl, pyrrolinyl, tetrahydropyranyl, tetrahydrothiopyranyl, oxazolidinonyl, thiomorpholinyl, oxadiazolyl, thiadiazolyl, tetrazolyl, phenyl, cyclohexyl, cyclopentyl, cyclohexenyl and cyclopentenyl.

In this specification, unless stated otherwise, a 3- to 10-membered ring containing one or more atoms independently selected from C, N, O and S, includes aromatic and heteroaromatic rings as well as carbocyclic and heterocyclic rings which may be saturated or unsaturated. Examples of such rings may be, but are not limited to imidazolidinyl, imidazolinyl, morpholinyl, piperazinyl, piperidyl, piperidonyl, pyrazolidinyl, pyrazolinyl, pyrrolidinyl, pyrrolinyl, tetrahydropyranyl, thiomorpholinyl, tetrahydrothiopyranyl, furyl, pyrrolyl, dihydropyrrolyl isoxazolyl, isothiazolyl, isoindolinonyl, dihydropyrrolo[1,2-*b*]pyrazolyl, oxazolyl, oxazolidinonyl, pyrazinyl, pyrazolyl, pyridazinyl, pyridinyl, tetrahydropyridinyl, pyrimidinyl, pyrrolyl, thiazolyl, thienyl, imidazolyl, triazolyl, phenyl, cyclopropyl, aziridinyl, cyclobutyl, azetidinyl, oxadiazolyl, thiadiazolyl, tetrazolyl, cyclopentyl, cyclopentenyl, cyclohexyl, cyclohexenyl, cycloheptyl, cycloheptenyl, cyclooctyl and cyclooctenyl.

In this specification, unless stated otherwise, the term "halo" or "halogen" may be fluoro, chloro, bromo or iodo.

In this specification, unless stated otherwise, the term "haloalkyl" means an alkyl radical as defined above, substituted with one or more halo radicals. The term "($C_1$-$C_6$)haloalkyl" may include, but is not limited to, fluoromethyl, difluoromethyl, trifluoromethyl, fluoroethyl and difluoroethyl. The term "O-$C_1$-$C_6$-haloalkyl" may include, but is not limited to, fluoromethoxy, difluoromethoxy, trifluoromethoxy and fluoro ethoxy.

In this specification, unless stated otherwise, the term "cyanoalkyl" means an alkyl radical as defined above, substituted with one or more cyano.

In this specification, unless stated otherwise, the term "optionally substituted" refers to radicals further bearing one or more substituents which may be, ($C_1$-$C_6$)alkyl, -($C_1$-$C_6$)haloalkyl, -($C_3$-$C_7$)cycloalkyl, -($C_1$-$C_6$)alkylene-($C_3$-$C_7$)cycloalkyl, -($C_3$-$C_7$)cycloalkyl-($C_1$-$C_6$)alkylene, -($C_0$-$C_6$)alkylene-($C_3$-$C_7$)spiroalkylene-($C_0$-$C_6$)alkylene, hydroxy, ($C_1$-$C_6$)alkylene-oxy, mercapto, aryl, heterocycle, heteroaryl, ($C_1$-$C_6$)alkylene-aryl, ($C_1$-$C_6$)alkylene-heterocycle, ($C_1$-$C_6$)alkylene-heteroaryl, halogen, trifluoromethyl, pentafluoroethyl, haloalkoxy, cyano, cyanomethyl, nitro, amino, amido, amidinyl, carboxyl, carboxamide, ($C_1$-$C_6$)alkylene-oxycarbonyl, carbamate, sulfonamide, ester and sulfonyl.

In this specification, unless stated otherwise, the term "independently" means that where more than one substituent is selected from a number of possible substituents, those substituents may be the same or different.

In this specification, unless stated otherwise, the term "solvate" refers to a complex of variable stoichiometry formed by a solute (e.g. a compound of Formula (I)) and a solvent. The solvent is a pharmaceutically acceptable solvent as preferably water; such solvent may not interfere with the biological activity of the solute.

In this specification, unless stated otherwise, the term "salt" refers to a complex of variable stoichiometry formed by an ionic form of the solute (e.g. a compound of Formula (I)) and its counter-ion. For example, a reference to carboxylic acid also includes the anionic (carboxylate) form, a salt or a solvate thereof, as well as conventional protected forms. Similarly, a reference to a basic (for example amino) group also includes the protonated (for example ammonium) form, a salt or solvate of the basic (for example amino) group, for example, a hydrochloride salt, as well as conventional protected forms of such group. Similarly, a reference to a hydroxyl group also includes the anionic form, a salt or solvate thereof, as well as conventional protected form of a hydroxyl group. The salt is a pharmaceutically acceptable salt; such salt may not interfere with the biological activity of the solute.

Pharmaceutically acceptable acid addition salts can be formed with inorganic and organic acids, e.g. acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulfate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

Pharmaceutically acceptable base addition salts can be formed with inorganic and organic bases. Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium, and magnesium salts. Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine, and tromethamine.

The pharmaceutically acceptable salts of the invention can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate, and the like), or by reacting free base forms of these compounds with a stoichiometric amount of the appropriate acid. Such reactions are typically carried out in water or in an organic solvent, or in a mixture of the two. Generally, use of non-aqueous media like diethylether, ethyl acetate, ethanol, isopropanol, or acetonitrile is desirable, where practicable. Lists of additional suitable salts can be found, e.g., in "Remington's Pharmaceutical Sciences", 20th ed., Mack Publishing company, Easton, Pa. (1985); and in "Handbook of Pharmaceutical Salts: Properties, Selection, and Use" by Stahl and Wermuth, Wiley-VCH, Weinheim, Germany, (2002).

When both a basic and an acid group are present in the same molecule, the compounds of the invention may also form internal salts, e.g., zwitterionic molecules.

In this specification, unless stated otherwise, certain compounds may exist in one or more particular geometric, optical, enantiomeric, diastereoisomeric, epimeric, stereoisomeric, tautomeric, conformational, or anomeric forms, including, but not limited to, *cis*- and *trans*-forms; *E*- and *Z*-forms; *endo*- and *exo*-forms, *R*-, *S*-, and *meso*-forms; *D*- and *L*-forms; *d*- and *l*-forms; (+) and (-) forms; keto-, enol-, and enolate-forms; $\alpha$- and $\beta$-forms; axial and equatorial forms; and combinations thereof, collectively referred to as "isomers" or "isomeric forms".

Note that specifically included in the term "isomer" are compounds with one or more isotopic substitutions. For example, H may be in any isotopic form, including, but not limited to, $^{1}H$, $^{2}H$ (D), and $^{3}H$ (T); C may be in any isotopic form, including, but not limited to, $^{12}C$, $^{13}C$, $^{14}C$; O may be in any isotopic form, including, but not limited to, $^{16}O$ and $^{18}O$; and the like. F may be in any isotopic form, including, but not limited to, $^{19}F$ and $^{18}F$; and the like.

In this specification, unless stated otherwise, the term "positive allosteric modulator of $mGluR_4$" or "allosteric modulator of $mGluR_4$" refers also to a pharmaceutically acceptable acid or base addition salt thereof, a stereochemically isomeric form thereof and an *N*-oxide form thereof.

As used herein, the term multiple sclerosis (MS) encompasses the different forms of the disease, including benign, relapsing remitting, primary progressive, secondary progressive and progressive relapsing multiple sclerosis.

PHARMACEUTICAL COMPOSITIONS

Allosteric modulators of $mGluR_4$ described herein, and the pharmaceutically acceptable salts, solvates and hydrates thereof can be used in pharmaceutical preparations in combination with a pharmaceutically acceptable carrier or diluent. Suitable pharmaceutically acceptable carriers include inert solid fillers or diluents and sterile aqueous or organic solutions. The allosteric modulators of $mGluR_4$ will be present in such pharmaceutical compositions in amounts sufficient to provide the desired dosage amount in the range described herein. Techniques for formulation and administration of the compounds of the instant invention can be found in Remington: the Science and Practice of Pharmacy, 19th edition, Mack Publishing Co., Easton, PA (1995).

The amount of allosteric modulators of $mGluR_4$, administered to the subject will depend on the type and severity of the disease or condition and on the characteristics of the subject, such as general health, age, sex, body weight and tolerance to drugs. The skilled artisan will be able to determine appropriate dosages depending on these and other factors. Effective dosages for commonly used CNS drugs are well known to the skilled person. The total daily dose usually ranges from about 0.05 - 2000 mg.

The present invention relates to pharmaceutical compositions which provide from about 0.01 to 1000 mg of the active ingredient per unit dose. The compositions may be administered by any suitable route. For example, orally in the form of capsules and the like, parenterally in the form of solutions for injection, topically in the form of onguents or lotions, ocularly in the form of eye-drops, rectally in the form of suppositories, intranasally or transcutaneously in the form of a delivery system like patches.

For oral administration, the allosteric modulators of $mGluR_4$ thereof can be combined with a suitable solid or liquid carrier or diluent to form capsules, tablets, pills, powders, syrups, solutions, suspensions and the like.

The tablets, pills, capsules, and the like contain from about 0.01 to about 99 weight percent of the active ingredient and a binder such as gum tragacanth, acacias, corn starch or gelatin; excipients such as dicalcium phosphate; a disintegrating agent such as corn starch, potato starch, alginic acid, a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

For parenteral administration the disclosed allosteric modulators of $mGluR_4$ can be combined with sterile aqueous or organic media to form injectable solutions or suspensions. For example, solutions in sesame or peanut oil, aqueous propylene glycol and the like can be used, as well as aqueous solutions of water-soluble pharmaceutically-acceptable salts of the compounds. Dispersions can also be prepared in glycerol, liquid polyethylene glycols and mixtures thereof in oils. Under ordinary conditions of storage and use, these preparations contain a preservative to prevent the growth of microorganisms.

In addition, to the formulations described previously, the compounds may also be formulated as a depot preparation. Such long acting formulations may be administered for example, by subcutaneously implantation or by intramuscular injection. Thus, for example, as an emulsion in an acceptable oil, or ion exchange resins, or as sparingly soluble derivatives, for example, as sparingly soluble salts.

Preferably disclosed allosteric modulators of $mGluR_4$ or pharmaceutical formulations containing these compounds are in unit dosage form for administration to a mammal. The unit dosage form can be any unit dosage form known in the art including, for example, a capsule, an IV bag, a tablet, or a vial. The quantity of active ingredient in a unit dose of composition is an effective amount and may be varied according to the particular treatment involved. It may be appreciated that it may be necessary to make routine variations to the dosage depending on the age and condition of the patient. The dosage will also depend on the route of administration which may be by a variety of routes including oral, aerosol, rectal, transdermal, subcutaneous, intravenous, intramuscular, intraperitoneal and intranasal.

In the case of the treatment of movement disorders such as Parkinson's disease, the compounds of the invention can be used alone or in combination with an agent selected from the group consisting of: a dopamine precursor such as levodopa, melevodopa and etilevodopa, levodopa with a selective extracerebral decarboxylase inhibitor, such as carbidopa (SINEMET™), benserazide (MADOPAR™), a catechol-O-methyl transferase (COMT) inhibitor such as entacapone and tolcapone, a COMT inhibitor, a dopamine agonist such as bromocriptine (PARLODEL™), pramipexole, ropinorole, apomorphine, rotigotine, cabergoline and pergolide, lisuride or pergolide (CELANCE™), an anticholinergic such as benztropine, trihexyphenidyl, procyclidine and biperiden, a cholinergic agonist, an NMDA receptor antagonist such as amantadine, an MAO-B inhibitor such as selegiline and rasagiline, an $mGluR_5$ antagonist or an $A_{2A}$ antagonist such as istradefylline and preladenant an alpha-2 adrenergic antagonist such as atipamezole or fipamezole, a 5-$HT_{1A}$ agonist such as piclozotan or a 5-$HT_{1A}/_{1B}$ partial agonist such as eltoprazine. Finally, in the case of the treatment of iatrogenic movement disorders, the compounds of the invention can be used in combination wih an agent selected from the group of: a butyrophenone neuroleptic agent, a diphenylbutylpiperidine neuroleptic agent, a heterocyclic dibenzazepine neuroleptic agent, an indolone neuroleptic agent, a phenothiazine neuroleptic agent or a thioxanthene neuroleptic agent

METHODS OF SYNTHESIS

The compounds according to the invention, and also the the compounds according to the Formula (I) to (III), may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (Green T.W. and Wuts P.G.M., (1991) Protecting Groups in Organic Synthesis, John Wiley & Sons). These groups are removed at a convenient stage of the compound synthesis using methods that are readily apparent to those skilled in the art. The selection of process as well as the reaction conditions and order of their execution shall be consistent with the preparation of compounds of Formula (I) to (III).

The compounds according to the invention may be represented as a mixture of enantiomers, which may be resolved into the individual pure *R*- or *S*-enantiomers. If for instance, a particular enantiomer is required, it may be prepared by asymmetric synthesis or by derivation with a chiral auxiliary, where the resulting diastereomeric mixture is separated and the auxiliary group cleaved to provide the pure desired enantiomers. Alternatively, where the molecule contains a basic functional group such as an amino or an acidic functional group such as carboxyl, this resolution may be conveniently performed by fractional crystallization from various solvents as the salts of an optical active acid or by other methods known in the literature (e.g. chiral column chromatography).

Resolution of the final product, an intermediate or a starting material may be performed by any suitable method known in the art (Eliel E.L., Wilen S.H. and Mander L.N., (1984) Stereochemistiy of Organic Compounds, Wiley-Interscience).

Many of the heterocyclic compounds of the invention can be prepared using synthetic routes well known in the art (Katrizky A.R. and. Rees C.W., (1984) Comprehensive Heterocyclic Chemistry, Pergamon Press).

The product from the reaction can be isolated and purified by employing standard techniques, such as extraction, chromatography, crystallization and distillation.

The compounds of the invention may be prepared by general route of synthesis as disclosed in the following methods.

In one embodiment of the present invention, compounds of Formula (I) may be prepared according to the synthetic sequences illustrated in Scheme 1.Pyrazole **g1** can be protected by *p*-methoxybenzyl, for example, using standard conditions. Then compound **g2** may be hydrolyzed and the resulting carboxylic acid **g3** can be transformed into the corresponding Weinreb amide **g4.** Functionalized pyrazole **g5** can be obtained from deprotonation of pyrazole **g4** using LDA as a base in THF at -78°C followed by the addition of 1,2-dibromo-1,1,2,2-tetrachloroethane. The aminopyrazole **g6** may be obtained by methods such as Buchwald coupling reactions, known in the art of organic synthesis, mediated by palladium-complex catalysts such as $Pd(OAc)_2$, in the presence of a base such as $Cs_2CO_3$, in a solvent

such as dioxane, at an appropriate temperature. **g6** can then be reacted with allylbromide in the presence of anhydride acetic to give the tertiary amine **g7.** Vinyl Grignard reagent can be added on the Weinreb amide **g7** to generate the compound **g8** which can undergo metathesis using Grubbs catalysts. The resulting $\alpha,\beta$-unsaturated ketone **g9** can be reduced in the presence of $H_2$ and $Pd(OH)_2$ to afford the ketone **g10**. The intermediate compound **g10** can be functionalized by $R_1X$ **(gII)** in the presence of a base such as NaH, KOtBu or NaOtBu and in the presence of a crown ether, in a solvent such as DMF or THF, to yield the intermediate compound **g12.** Subsequently, ketone **g12** can be transformed into bromoketone **g13** by using a brominating agent such as $CuBr_2$ or pyridinium tribromium, in a solvent such as MeOH, at an appropriate temperature. Intermediate compound **g13** can then be cyclized into an aminothiazole **g14** by reaction with a suitable substituted thiourea **g12,** in a solvent such as the mixture EtOH/Acetone, at an appropriate temperature. Finally, the expected compound **g15** can be obtained via deprotection of **g14** in the presence of TFA or a mixture of TFA/TfOH, at an appropriate temperature when p-methoxybenzyl is used as the protecting group.

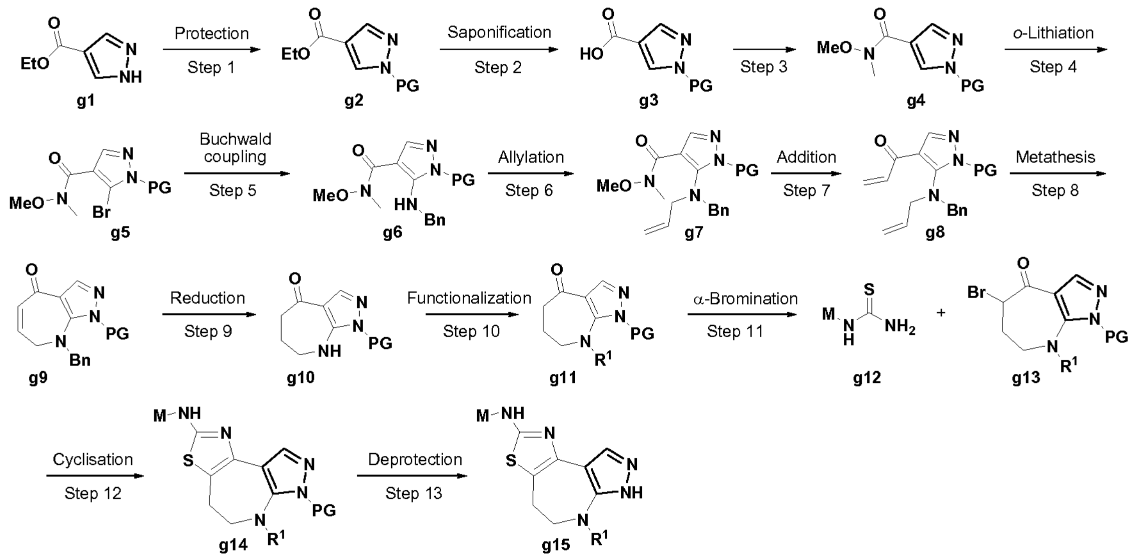


**Scheme 1**

In general, substituted thiourea M-NH-C(=S)-$NH_2$ **g12,** used in Scheme 1, are prepared according to methods known by persons skilled in the art. For example, 5-fluoropyrimidin-2-amine can be reacted with ethyl carbonisothiocyanatidate in acetonitrile, then the resulting product can be treated with ammonium formate in ammonia affording the thiourea 5-fluoropyrimidin-2-yl-NHC(=S)-$NH_2$.

In one embodiment of the present invention, compounds of Formula (I) may be prepared according to the synthetic sequences illustrated in Scheme 2. The aminopyrazole **g16** may be obtained by methods such as Buchwald coupling reactions, known in the art of organic synthesis, mediated by palladium-complex catalysts such as $Pd(OAc)_2$ or $Pd_2(dba)_3$, in the presence of a base such as $Cs_2CO_3$, in a solvent such as dioxane, at an appropriate temperature. **g16** can then be reacted with allylbromide in the presence of anhydride acetic to give the tertiary amine **g17**. Vinyl Grignard reagent can be added on the Weinreb amide **g17** to generate the compound **g18** which can undergo metathesis using Grubbs catalysts. The resulting $\alpha,\varepsilon$-unsaturated ketone **g19** can be reduced in the presence of $H_2$ or ammonium formate, and $Pd(OH)_2$ to afford the ketone **g11**. Intermediate compound **g11** can then be cyclized into the aminothiazole **g14** by reaction with a suitable substituted thiourea **g12** in the presence of diiodine in a solvent such as pyridine, at an appropriate temperature. Finally, the expected compound **g15** can be obtained via deprotection of **g14** in the presence of TFA or a mixture of TFA/TfOH, at an appropriate temperature when p-methoxybenzyl is used as the protecting group.

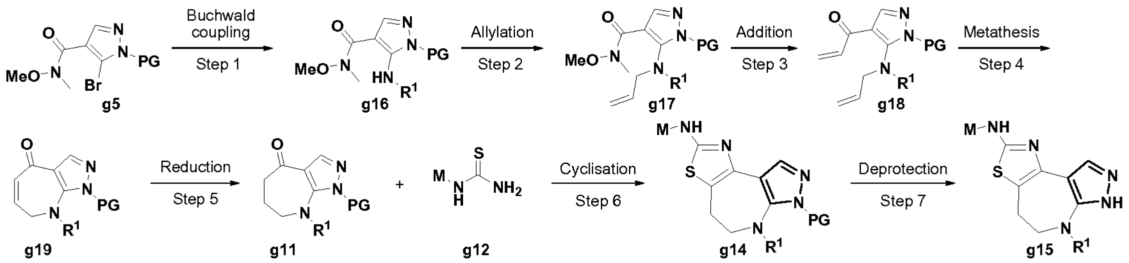


**Scheme 2**

In one embodiment of the present invention, compounds of Formula (I) may be prepared according to the synthetic sequences illustrated in Scheme 3. The ketone **g10** can be functionalized by $R_1X$ in the presence of a base such as NaH, KOtBu or NaOtBu, in the presence of a crown ether, in a solvent such as DMF or THF, to yield the intermediate compound **g11** which can be cyclized into the aminothiazole **g14** by reaction with a suitable substituted thiourea **g12** in the presence of diiodine, in a solvent such as pyridine, at an appropriate temperature. Finally, the expected compound **g15** can be obtained via deprotection of **g14** in the presence of TFA or a mixture of TFA/TfOH, at an appropriate temperature when p-methoxybenzyl is used as the protecting group.

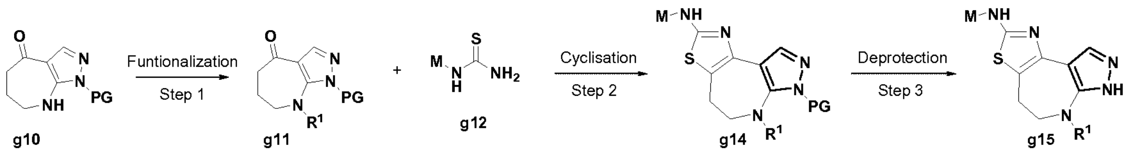


**Scheme 3**

In one embodiment of the present invention, compounds of Formula (I) may be prepared according to the synthetic sequences illustrated in Scheme 4. The bromoketone **g13** can be cyclized into the aminothiazole **g21** by reaction with thiourea **g20,** in a solvent such as the mixture EtOH/Acetone, at an appropriate temperature. The functionalized aminothiazole **g14** can be obtained by methods such as Buchwald coupling reactions, known in the art of organic synthesis, mediated by palladium-complex catalysts such as $Pd(OAc)_2$, in the presence of a base such as $Cs_2CO_3$, in a solvent such as dioxane, at an appropriate temperature. Finally, the expected compound **g15** can be obtained using methods described above.

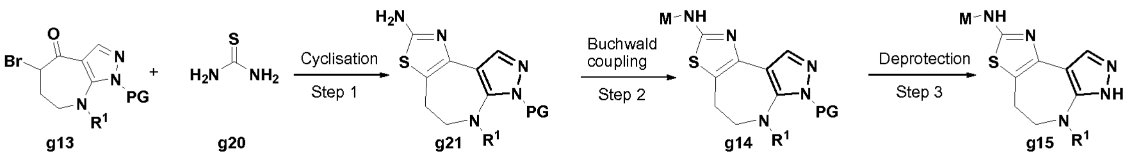


**Scheme 4**

In one embodiment of the present invention, compounds of Formula (I) may be prepared according to the synthetic sequences illustrated in Scheme 5. The expected compound **g24** can be obtained after two sequential deprotections. The intermediate compound **g23** can be treated with a solution of HCl in dioxane.

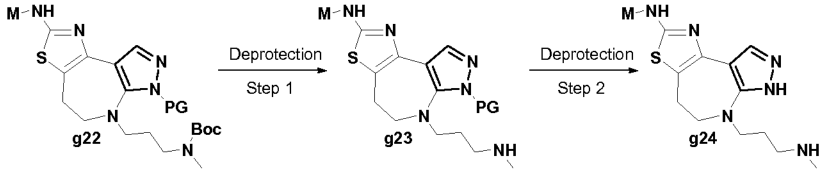


**Scheme 5**

In one embodiment of the present invention, compounds of Formula (I) may be prepared according to the synthetic sequences illustrated in Scheme 6. The ether **g25** can be demethylated in the presence of $BBr_3$ using standard methods well known from persons skilled in the art to yield the hydroxy compound **g26.**

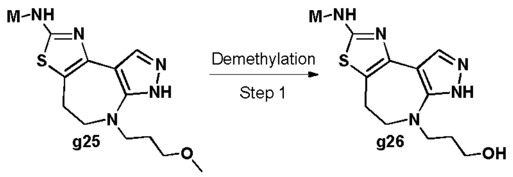


g25 g26

**Scheme 6**

In one embodiment of the present invention, compounds of Formula (I) may be prepared according to the synthetic sequences illustrated in Scheme 7. The intermediate compound **g27** can be functionalized into the morpholinopyridyl **g28** by methods such as Buchwald coupling reactions, known in the art of organic synthesis, mediated by palladium-complex catalysts such as $Pd(OAc)_2$ or $Pd_2(dba)_3$, in the presence of a base such as $Cs_2CO_3$, in a solvent such as dioxane, at an appropriate temperature. Finally, the expected compound **g29** can be obtained using methods described above.

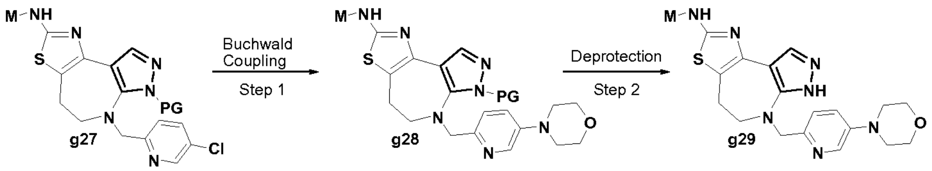


g27 g28 g29

**Scheme 7**

In one embodiment of the present invention, compounds of Formula (I) may be prepared according to the synthetic sequences illustrated in Scheme 8. The intermediate **g31** can be obtained after Boc deprotection using standard methods well known from persons skilled in the art. Subsequently, the piperazine **g31** can be functionalized by methods such as reductive amination, known in the art of organic synthesis to yield the substituted piperidine **g32.** Finally, the expected compound **g33** can be obtained using methods described above.

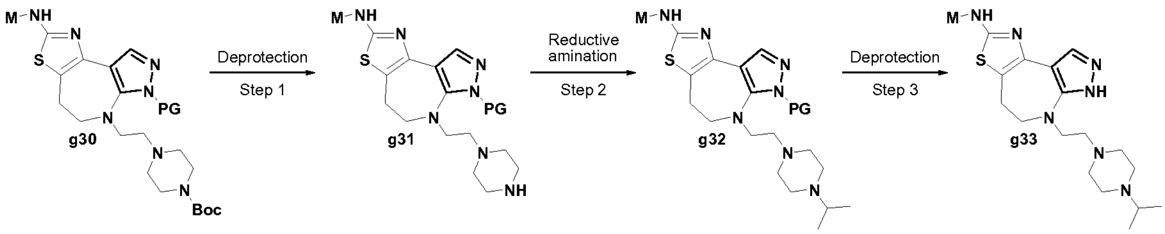


g30 g31 g32 g33

**Scheme 8**

In one embodiment of the present invention, compounds of Formula (I) may be prepared according to the synthetic sequences illustrated in Scheme 9. The intermediate **g35** can be obtained after TMS deprotection of derivative **g34** using standard methods well known from persons skilled in the art. Subsequently, the alcohol **g35** can be brominated in the presence of $CBr_4$ and $PPh_3$ to yield the corresponding bromo derivative **g36.** The functionalized piperidine **g37** could be obtained by nucleophilic substitution of **g36** using classical conditions known in the art of organic chemistry. Finally, the expected compound **g39** can be obtained in 2 steps from ketone **g37** using methods described above.

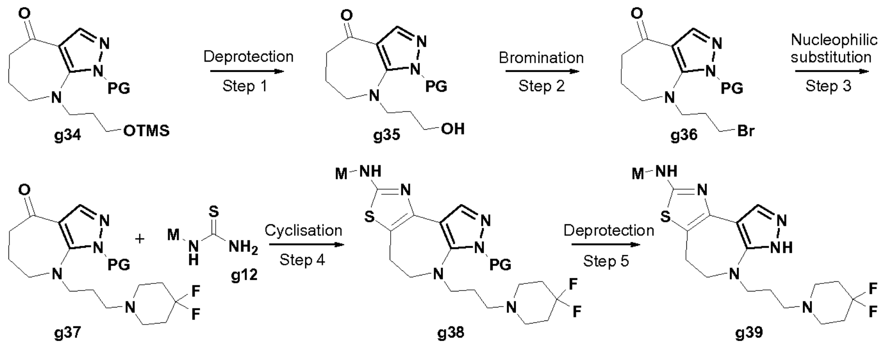


g34 g35 g36 g37 g12 g38 g39

**Scheme 9**

In one embodiment of the present invention, compounds of Formula (I) may be prepared according to the synthetic sequences illustrated in Scheme 10. The acid **g41** can be obtained after saponification of the ester **g40** using standard methods well known from persons skilled in the art. Subsequently, the oxadiazole **g42** can be synthesized from the derivative **g41** in the presence of hydroxyacetimidamide and a peptidic coupling agent such as HATU and a base such as $NEt_3$ in a solvent such as DMF. Finally, the expected compound **g44** can be obtained in 2 steps from ketone **g42** using methods described above.

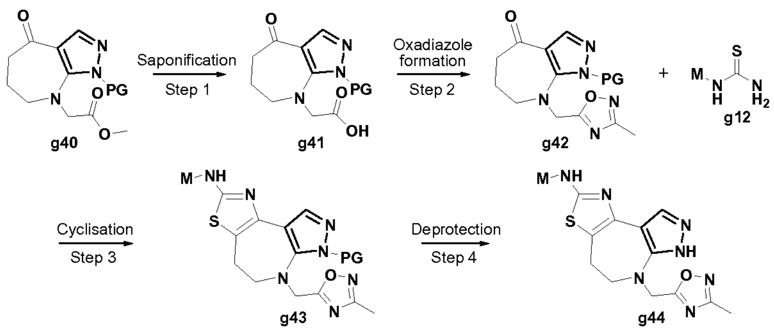


g40 g41 g42 g12 g43 g44

**Scheme 10**

In one embodiment of the present invention, compounds of Formula (I) may be prepared according to the synthetic sequences illustrated in Scheme 11. The amino derivative **g46** can be obtained after deprotection of the corresponding *N*-phthalimide protected compound **g45** using standard methods well known from persons skilled in the art. Subsequently, the dioxothiomorpholine **g48** can be synthesized from the derivative **g46** by cyclization with vinylsulfonylethene **g47** in a solvent such as dioxane. Finally, the expected compound **g50** can be obtained in 2 steps from ketone **g48** using methods described above.

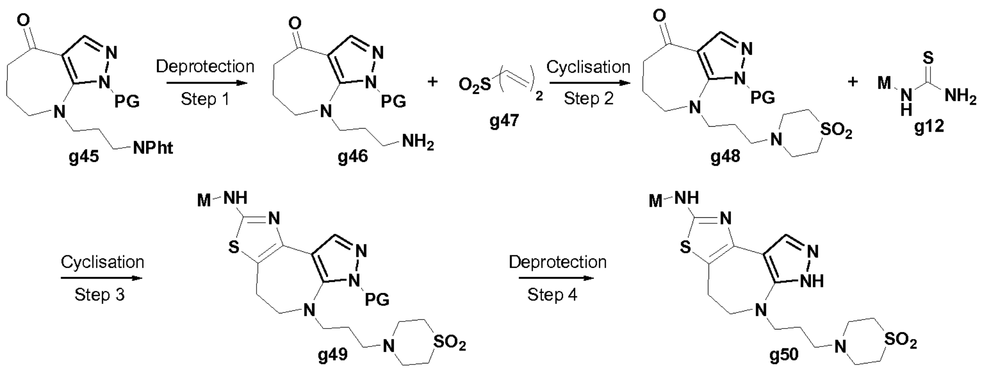


g45 g46 g47 g48 g12 g49 g50

**Scheme 11**

EXPERIMENTAL

Unless otherwise noted, all starting materials were obtained from commercial suppliers and used without further purification.

Specifically, the following abbreviations may be used in the examples and throughout the specification.

| | |
|---|---|
| ACN (Acetonitrile) | $MgSO_4$ (Magnesium sulfate) |
| AcOH (Acetic acid) | min (Minutes) |
| atm (Atmosphere) | mL (Milliliters) |
| $BBr_3$ (Boron tribromide) | mmol (Millimoles) |
| $CBr_4$ (Carbon tetrabromide) | Mp (Melting point) |
| $CHCl_3$ (Chloroform) | $NH_4Cl$ (Ammonium chloride) |
| $Cs_2CO_3$ (Cesium carbonate) | NaH (Sodium hydride) |
| $CuBr_2$ (Copper (II) bromide) | NaOH (Sodium hydroxide) |
| 1,2-DCE (1,2-Dichloroethane) | NaOtBu (Sodium tert-butoxide) |
| DCM (Dichloromethane) | $Na_2CO_3$ (Sodium carbonate) |
| DMF (Dimethylformamide) | $Na_2SO_4$ (Sodium sulfate) |
| EtOAc (Ethyl acetate) | $Na_2S_2O_3$ (Sodium thiosulfate) |
| EtOH (Ethanol) | $Pd(OAc)_2$ (Palladium(II)acetate) |
| $Et_3N$ (Triethylamine) | $Pd(OH)_2$ (Palladium(II) hydroxide) |
| h (Hour) | $Pd_2(dba)_3$ (Tris(dibenzylideneacetone)dipalladium(0)) |
| HATU (2-(1*H*-7-Azabenzotriazol-1-yl)-1,1,3, 3-tetramethyluronium hexafluorophosphate | Prep. HPLC (Preparative high pressure liquid chromatography) |
| HBr (Hydrobromic acid) | Prep. TLC (Preparative thin layer chromatography) |
| HCl (Hydrochloric acid) | rt (Room temperature) |
| $I_2$ (Diiodine) | RT (Retention Time) |
| KOtBu (Potassium *tert*-butoxide) | TFA (Trifluoroacetic acid) |
| $K_2CO_3$ (Potassium carbonate) | THF (Tetrahydrofuran) |
| LDA (Lithium diisopropylamide) | TLC (Thin layer chromatography) |
| M (Molar) | UPLC-MS (Ultra Performance Liquid Chromatography Mass Spectrometry) |
| MeOH (Methanol) | Xantphos (4,5-Bis(diphenylphosphino)-9,9-dimethylxanthene) |
| mg (Milligrams) | |

All references to brine refer to a saturated aqueous solution of NaCl. Unless otherwise indicated, all temperatures are expressed in °C (degrees Centigrade). All reactions are conducted under an inert atmosphere at room temperature unless otherwise noted.

Most of the reactions were monitored by thin-layer chromatography on 0.25mm Merck silica gel plates (60F-254), visualized with UV light. Flash column chromatography was performed on prepacked silica gel cartridges (15-40 μM, Merck).

Melting point determination was performed on a Electrothermal 1002D apparatus.

$^1$H-NMR spectra were recorded on a Bruker 300 MHz spectrometer and on a Jeol JNM-LA 400 MHz spectrometer. Chemical shifts are expressed in parts per million (ppm, δ units). Coupling constants are in units of hertz (Hz)

Splitting patterns describe apparent multiplicities and are designated as *s* (singlet), *d* (doublet), *t* (triplet), *q* (quadruplet), *m* (multiplet), *br* (broad).

EXAMPLES

**EXAMPLE 1: 3-((2-(5-Fluoropyrimidin-2-ylamino)-4,5-dihydropyrazolo[3,4-*b*] thiazolo[4,5-*d*]azepin-6(7*H*)-yl)methyl)benzonitrile (Compound number 1-1)**

*Ethyl 1-(4-methoxybenzyl)-1H-pyrazole-4-carboxylate*

According to Scheme 1, Step 1: A suspension of ethyl 1*H*-pyrazole-4-carboxylate (112 g, 799 mmol), 1-(chloromethyl)-4-methoxybenzene (119 mL, 879 mmol) and $K_2CO_3$ (166 g, 1,20 mol) in ACN (900 mL) was stirred at reflux for 4 h. At rt, the mixture was filtered and concentrated under reduced pressure. The resulting yellow oil was triturated in petroleum ether and the precipitate was isolated by filtration and dried under reduced pressure to afford the title compound (208 g, quantitative) as a white solid.
UPLC-MS (M1): RT = 1.01 min; MS *m/z* $ES^+$ = 261.

*1-(4-Methoxybenzyl)-1H-pyrazole-4-carboxylic acid*

According to Scheme 1, Step 2: A solution of NaOH (48.0 g, 1,21 mol) in water (180 mL) was added over a period of 1 h, at 80°C, to a solution of ethyl 1-(4-methoxybenzyl)-1*H*-pyrazole-4-carboxylate (208 g, 799 mmol) in THF/EtOH/$H_2O$ (1:1:0.2, 500 mL). The mixture was stirred at 80°C for 1 h. The mixture was diluted with cold $H_2O$ and was washed three times with EtOAc. The aqueous layer was acidified to pH 2 with HCl 12N. The resulting precipitate was collected, washed with $H_2O$ and diluted in EtOAc. The organic layer was dried over $MgSO_4$, filtered and concentrated under reduced pressure. The product was slurried in petroleum ether and dried under high vacuum to provide the title compound (93.6 g, 50%) as a white solid.
UPLC-MS (M1): RT = 0.77 min; MS *m/z* $ES^-$= 231.

*N-Methoxy-1-(4-methoxybenzyl)-N-methyl-1H-pyrazole-4-carboxamide*

According to Scheme 1, Step 3: A solution of 1-(4-methoxybenzyl)-1*H*-pyrazole-4-carboxylic acid (162 g, 698 mmol), oxalyl chloride (77.0 ml, 907 mmol) and DMF (2 mL) in DCM (811 mL) was stirred at rt for 2 h. After evaporation, the crude product was dissolved in DCM (322 mL) and the resulting solution was added at 0°C over a period of 30 min to a solution of *N,O*-dimethylhydroxylamine hydrochloride (102 g, 1,05 mol) in DCM (811 mL), followed by $Et_3N$ (252 mL, 1,81 mol). The mixture was stirred at rt for 2 h. The reaction was quenched with a saturated aqueous solution of $Na_2CO_3$ (300 mL) and the aqueous layer was extracted with DCM. The organic layer was dried over $MgSO_4$, filtered and concentrated under reduced pressure to afford the title compound (181 g, 94%) as a white solid.
UPLC-MS (M1): RT = 0.73 min; MS *m/z* $ES^+$ = 276.

*5-Bromo-N-methoxy-1-(4-methoxybenzyl)-N-methyl-1H-pyrazole-4-carboxamide*

According to Scheme 1, Step 4: In a flask equipped with a mechanical stirrer, at -78°C and under $N_2$ atm, a LDA solution (327 mL, 654 mmol) was added to a suspension of *N*-methoxy-1-(4-methoxybenzyl)-*N*-methyl-1*H*-pyrazole-4-carboxamide (90.0 g, 327 mmol) in anhydrous THF (1.5 L), over 55 min. The reaction mixture was stirred at - 78°C for 45 min. Then at -78°C, 1,2-dibromo-1,1,2,2-tetrachloroethane (117 g, 360 mmol) was added to the reaction mixture over 1 h and the mixture was allowed to warm to -40°C over 1 h. At -10°C, a saturated solution of $NH_4Cl$ (400 mL) was added over 30 min to the mixture followed by EtOAc (1 L) and brine (1 L). The aqueous layer was extracted twice with EtOAc (2 L). The organic layers were combined, dried over $MgSO_4$, filtered and concentrated under reduced pressure. The crude residue was diluted with EtOAc and filtered on a celite pad. The filtrate was concentrated under reduced pressure to afford the title compound (quantitative).
UPLC-MS (M1): RT = 0.86 min; MS *m/z* $ES^+$ = 354.

*5-(Benzylamino)-N-methoxy-1-(4-methoxybenzyl)-N-methyl-1H-pyrazole-4-carboxamide*

According to Scheme 1, Step 5: 5-Bromo-*N*-methoxy-1-(4-methoxybenzyl)-*N*-methyl-1*H*-pyrazole-4-carboxamide (160 g, 452 mmol) was dissolved in dioxane (700 mL), previously satured with $N_2$ atm. Then phenylmethanamine (290 g, 2.71 mol), $Cs_1CO_3$ (177 g, 542 mmol), Xantphos (15.7 g, 27.1 mmol) and $Pd(OAc)_2$ (10.1 g, 13.5 mmol) were added to the mixture and the reaction mixture was stirred at 110°C for 8 h. The hot crude solution was filtered on a celite

pad and washed with EtOAc (1 L). The filtrate was concentrated under reduced pressure.The crude residue was purified by flash column chromatography on silca gel using petroleum ether (100%) to cyclohexane/EtOAc (80/20) as eluent. The resulting product was diluted in EtOAc (2 L). The organic layer was washed two times with HC1 1N (1L), brine (1L), was dried over $MgSO_4$, filtered and concentrated under reduced pressure to afford the title compound (70 g, 41%). UPLC-MS (M1): RT = 1.04 min; MS *m/z* $ES^+$ = 381.

*5-(Allyl(benzyl)amino)-N-methoxy-1-(4-methoxybenzyl)-N-methyl-1H-pyrazole-4-carboxamide*

According to Scheme 1, Step 6: To a solution of 5-(benzylamino)-*N*-methoxy-1-(4-methoxybenzyl)-*N*-methyl-1*H*-pyrazole-4-carboxamide (66.1 g, 174 mmol) in anhydrous DMF (600 mL) and anhydrous THF (600 mL) at 0°C, was added portionwise 60% NaH (20.8 g, 521 mmol). The mixture was stirred for 30 min. Then 3-bromoprop-1-ene (44.1 g, 365 mmol) was added to the mixture and the reaction mixture was stirred for 30 min at rt. After cooling to 0°C, cold water (300 mL) was added slowly to the reaction mixture. Then the mixture was extracted with EtOAc (2 L). The organic layers were combined, washed with brine (1 L), dried over $MgSO_4$, filtered and concentrated under reduced pressure to afford the title compound (quantitative).
UPLC-MS (M1): RT = 1.18 min; MS *m/z* $ES^+$ = 421.

*1-(5-(Allyl(benzyl)amino)-1-(4-methoxybenzyl)-1H-pyrazol-4-yl)prop-2-en-1-one*

According to Scheme 1, Step 7: Under $N_2$, to a solution of 5-(allyl(benzyl)amino)-*N*-methoxy-1-(4-methoxybenzyl)-*N*-methyl-1*H*-pyrazole-4-carboxamide (80.0 g, 190 mmol) in anhydrous THF (1.5 L) was added dropwise over 40 min, at 0°C, vinylmagnesium bromide (533 mL, 533 mmol). After stirring the reaction mixture for 1 h, acetic anhydride (150 mL) was added followed by MeOH (150 mL). Then 25% of the reaction mixture was removed under reduced pressure and EtOAc (2 L) was added. The mixture was washed with an aqueous solution of $NH_4Cl$. The organic layer was dried over $MgSO_4$, filtered and concentrated under reduced pressure to afford the title compound (quantitative). The crude was used in the next step without any further purification.
UPLC-MS (M1): RT = 1.26 min; MS *m/z* $ES^+$ = 388.

*(Z)-8-Benzyl-1-(4-methoxybenzyl)-7,8-dihydropyrazolo[3,4-b]azepin-4(1H)-one*

According to Scheme 1, Step 8: Under $N_2$, to a solution of 1-(5-(allyl(benzyl)amino)-1-(4-methoxybenzyl)-1*H*-pyrazol-4-yl)prop-2-en-1-one crude (90.0 g, 232 mmol) in 1,2-dichloroethane (7.5 L) was added Grubbs catalyst 1st generation (3.82 g, 4.65 mmol) and the reaction mixture was stirred at reflux for 1 h. As the reaction was not complete, 0.01 eq of Grubbs reagent was added each 30 min over 4 h and the reaction mixture was stirred overnight under oxygen. Then the reaction mixture was poured on a flash column chromatography with silica gel and the product was purified using petroleum ether/EtOAc (100:0 to 50:50) as eluent to afford the title compound (31.7 g, 38%).
UPLC-MS (M1): RT = 1.05 min; MS *m/z* $ES^+$ = 360.

*1-(4-Methoxybenzyl)-5,6,7,8-tetrahydropyrazolo[3,4-b]azepin-4(1H)-one*

According to Scheme 1, Step 9: To a solution of (*Z*)-8-benzyl-1-(4-methoxybenzyl)-7,8-dihydropyrazolo[3,4-*b*]azepin-4(1*H*)-one (23.3 g, 64.8 mmol) in EtOH (365 mL) was added AcOH (365 mL). Then under $N_2$, $Pd(OH)_2$ (15.17 g, 13.0 mmol) was added and the mixture was stirred under $H_2$ with a vigourous agitation for 6 h. The reaction mixture was filtered on a celite pad, the pad was washed with DCM/EtOH (1:1, 300 mL) and then with EtOH (300 mL). The filtrate was concentrated under reduced pressure. The crude residue was purified by flash column chromatography on silica gel using DCM/MeOH (100:0 to 98.5:1.5) as eluent to afford the title compound (12.8 g, 73%).
UPLC-MS (M1): RT = 0.66 min; MS *m/z* $ES^+$ = 272; $^1$H-NMR (300MHz, DMSO-$d_6$) δ: 7.55 (1H, s), 7.15-7.05 (3H, m), 6.90 (2H, d), 5.00 (2H, s), 3.70 (3H, s), 3.35 (2H, m), 2.50 (2H, m), 1.90 (2H, m).

*3-((1-(4-Methoxybenzyl)-4-oxo-4,5,6,7-tetrahydropyrazolo[3,4-bjazepin-8(1H)-yl) methyl)benzonitrile*

According to Scheme 1, Step 10: To a solution of 1-(4-methoxybenzyl)-5,6,7,8-tetrahydropyrazolo[3,4-*b*]azepin-4(1*H*)-one (1.00 g, 3.69 mmol) in THF (20 mL) were added consecutively 18-crown-6 (1.46 g, 5.54 mmol), KO*t*Bu (827 mg, 7.38 mmol) and after stirring for 5 minutes, 3-(bromomethyl)benzonitrile (1.45 g, 7.38 mmol). The mixture was stirred at reflux overnight. The reaction was carefully quenched with water and the mixture was diluted with EtOAc. The separated aqueous layer was extracted with EtOAc. The organic layers were combined, dried over $Na_2SO_4$, filtered and concentrated under reduced pressure. The crude residue was purified by flash column chromatography on silica gel using petroleum ether/EtOAc (90:10 to 30:70) as eluent to afford the title compound (410 mg, 29%) as a beige solid.

UPLC-MS (M2): RT = 2.91 min; MS *m/z* $ES^+$ = 387.

*3-((5-Bromo-1-(4-methoxybenzyl)-4-oxo-4,5,6,7-tetrahydropyrazolo[3,4-b]azepin-8(1H)-yl)methyl)benzonitrile*

According to Scheme 1, Step 11: A solution of 3-((1-(4-methoxybenzyl)-4-oxo-4,5,6,7-tetrahydropyrazolo[3,4-*b*]azepin-8(1*H*)-yl)methyl)benzonitrile (410 mg, 1.06 mmol) and $CuBr_2$ (356 mg, 1.59 mmol) in MeOH (20 mL) was stirred at reflux for 4 h. After evaporation of MeOH, the residue was suspended in DCM and an aqueous solution of $Na_2CO_3$. The separated aqueous layer was extracted twice with DCM. The combined organic layers were dried over $Na_2SO_4$ and concentrated under reduced pressure to provide a brown oil. The crude oil was purified by flash column chromatography on silica gel using petroleum ether/EtOAc (90:10 to 50:50) as eluent to afford the title compound (210 mg, 43%) as a white viscous solid.
UPLC-MS (M2): RT = 3.07 min; MS *m/z* $ES^+$ = 465.

*3-((2-(5-Fluoropyrimidin-2-ylamino)-7-(4-methoxybenzyl)-4,5-dihydropyrazolo[3,4-b] thiazolo[4,5-d]azepin-6(7H)-yl)methyl)benzonitrile*

According to Scheme 1, Step 12: A solution of 3-((5-bromo-1-(4-methoxybenzyl)-4-oxo-4,5,6,7-tetrahydropyrazolo[3,4-*b*]azepin-8(1*H*)-yl)methyl)benzonitrile (210 mg, 0.45 mmol) and 1-(5-fluoropyrimidin-2-yl)thiourea (78 mg, 0.45 mmol) in EtOH (15 mL) and acetone (15 mL) was stirred at 50°C overnight. As the conversion was not complete, 1-(5-fluoropyrimidin-2-yl)thiourea (78.0 mg, 0.45 mmol) was added and the reaction mixture was stirred at 60°C for 3 h, at 70°C for 4 h, then at 60°C overnight. After cooling down to rt, the resulting precipitate was filtered to give the HBr salt of the title compound (180 mg). The resulting salt was partitioned between aqueous $Na_2CO_3$ and DCM and the two phases were separated. The aqueous layer was extracted twice with DCM and three times with EtOAc. The organic layers were combined, dried over $Na_2SO_4$, filtered and concentrated under reduced pressure to afford the title compound (100 mg, 41 %) as a white solid.
UPLC-MS (M2): RT = 3.27 min; MS *m/z* $ES^+$ = 539.

*3-((2-(5-Fluoropyrimidin-2-ylamino)-4,5-dihydropyrazolo[3,4-b]thiazolo[4,5-d] azepin-6(7H)-yl)maethyl)benzonitrile*

According to Scheme 1, Step 13: A solution of 3-((2-(5-fluoropyrimidin-2-ylamino)-7-(4-methoxybenzyl)-4,5-dihydropyrazolo[3,4-*b*]thiazolo[4,5-*d*]azepin-6(7*H*)-yl)methyl) benzonitrile (100 mg, 0.19 mmol) in TFA (3 mL) was stirred at 60°C for 2 h. Excess of TFA was evaporated and the residue was partitioned between aqueous $Na_2CO_3$ and DCM. The separated aqueous layer was extracted twice with DCM. The organic layers were combined, dried over $Na_2SO_4$, filtered and concentrated under reduced pressure. The crude solid was purified by flash column chromatography on silica gel using DCM/MeOH (100:0 to 97:3) as eluent to afford the title compound (20 mg, 25%) as a white solid.
Mp: 282-284°C; UPLC-MS (M2): RT = 2.93 min; MS *m/z* $ES^+$ = 419; $^1H$-NMR (400MHz, DMSO-$d_6$) $\delta$: 11.95 (1H, s), 11.69 (1H, s), 8.72 (2H, d), 7.84 (1H, s), 7.76-7.30 (3H, m), 7.57 (1H, t), 4.65 (2H, s), 3.05-3.03 (2H, m).

**REFERENCE EXAMPLE 2: *N*-(5-Fluoropyrimidin-2-yl)-6-(2-methoxy-l(*S*)-methyl-ethyl)-4,5,6,8 -tetrahydropyrazolo[3,4-*b*]thiazolo[4,5-*d*]azepin-2-amine (Compound number 1-131)**

*(S)-N-Methoxy-1-(4-methoxybenzyl)-5-(1-methoxypropan-2-ylamino)-N-methyl-1H-pyrazole-4-carboxamide*

According to Scheme 2, Step 1: 5-Bromo-*N*-methoxy-1-(4-methoxybenzyl)-*N*-methyl-1*H*-pyrazole-4-carboxamide (2.50 g, 7.06 mmol) was dissolved in dioxane (25 mL), previously satured with $N_2$ atm. Then (*S*)-1-methoxypropan-2-amine (2.96 mL, 28.2 mmol), $Cs_2CO_3$ (4.60 g, 14.1 mmol), Xantphos (0.41 g, 0.70 mmol) and $Pd_2(dba)_3$ (0.32 g, 0.35 mmol) were added to the mixture and the reaction mixture was stirred at 110°C overnight. The crude solution was filtered on a celite pad and washed with EtOAc (30 mL). The filtrate was concentrated under reduced pressure. The crude residue was purified by flash column chromatography on silica gel using cyclohexane/EtOAc (80/20) as eluent to afford the title compound (1.91 g, 74%) as a yellow oil.
UPLC-MS (M1): RT = 0.91 min; MS *m/z* $ES^+$ = 363.

*(S)-5-(Allyl(1-methoxypropan-2-yl)amino)-N-methoxy-1-(4-methoxybenzyl)-N-methyl-1H-pyrazole-4-carboxamide*

According to Scheme 2, Step 2: To a solution of (*S*)-*N*-methoxy-1-(4-methoxybenzyl)-5-(1-methoxypropan-2-ylamino)-*N*-methyl-1*H*-pyrazole-4-carboxamide (1.91 g, 5.27 mmol) in anhydrous DMF (20 mL) and anhydrous THF (20 mL) at 0°C, was added portionwise 60% NaH (0.63 g, 15.8 mmol). The mixture was stirred for 30 min. Then 3-bromoprop-1-ene (1.27 g, 10.5 mmol) was added to the mixture and the reaction mixture was stirred for 3.5 h at rt. After cooling to

0°C, cold water (20 mL) was added slowly to the reaction mixture. The mixture was then extracted with EtOAc (50 mL). The organic layers were combined, washed with brine, dried over $MgSO_4$, filtered and concentrated under reduced pressure. The crude residue was purified by flash column chromatography on silica gel using cyclohexane/EtOAc (80/20) as eluent to afford the title compound (1.47 g, 69%) as yellow oil.
UPLC-MS (M1): RT = 1.07 min; MS *m*/*z* $ES^+$ = 403.1.

*(S)-1-(5-(Allyl(1-methoxypropan-2-yl)amino)-1-(4-methoxybenzyl)-1H-pyrazol-4-yl) prop-2-en-1-one*

According to Scheme 2, Step 3: Under $N_2$, to a solution of (*S*)-5-(allyl(1-methoxypropan-2-yl)amino)-*N*-methoxy-1-(4-methoxybenzyl)-*N*-methyl-1*H*-pyrazole-4-carboxamide (1.37 g, 3.40 mmol) in anhydrous THF (25 mL) was added dropwise at 0°C, vinylmagnesium bromide (17.0 mL, 17.0 mmol). After stirring for 30 min, acetic anhydride (10 mL) was added followed by MeOH (10 mL). The reaction mixture was diluted with EtOAc and the organic layer was washed with aqueous $NH_4Cl$ solution then dried over $MgSO_4$, filtered and concentrated under reduced pressure to afford the title compound (quantitative). The crude was used in the next step without any further purification.
UPLC-MS (M1): RT = 1.17 min; MS *m*/*z* $ES^+$ = 370.1.

*(S,Z)-1-(4-Methoxybenzyl)-8-(1-methoxypropan-2-yl)-7,8-dihydropyrazolo[3,4-b] azepin-4(1H)-one*

According to Scheme 2, Step 4: Under $N_2$, to a solution of (*S*)-1-(5-(allyl(1-methoxypropan-2-yl)amino)-1-(4-methoxybenzyl)-1*H*-pyrazol-4-yl)prop-2-en-1-one crude (1.25 g, 3.4 mmol) in 1,2-dichloroethane (170 mL) was added Grubbs catalyst 2$^{nd}$ generation (0.14 g, 0.17 mmol). The reaction mixture was stirred at reflux for 1 h. As the reaction was not complete, 0.01 eq of Grubbs reagent was added twice in 1 h. Then the reaction mixture was poured on a flash column chromatography with silica gel and the product was purified using cyclohexane/EtOAc (100:0 to 60:40) as eluent to afford the title compound (0.84 g, 72%) as a brown oil.
UPLC-MS (M1): RT = 0.88 min; MS *m*/*z* $ES^+$ = 342.0.

*(S)-1-(4-Methoxybenzyl)-8-(1-methoxypropan-2-yl)-5,6,7,8-tetrahydropyrazolo[3,4-b] azepin-4(1H)-one*

According to Scheme 2, Step 5: Under $N_2$, to a solution of (*S*,*Z*)-1-(4-methoxybenzyl)-8-(1-methoxypropan-2-yl)-7,8-dihydropyrazolo[3,4-*b*]azepin-4(1*H*)-one (0.84 g, 2.46 mmol) in MeOH (15 mL) was added $Pd(OH)_2$ (0.035 g, 0.25 mmol) followed by ammonium formate (1.55 g, 24.6 mmol). The mixture was stirred at reflux for 1 h and then filtered on a celite pad, the pad was washed with DCM/MeOH (1:1, 30 mL). The reaction mixture was diluted with EtOAc and washed with saturated aqueous $NaHCO_3$ solution. The organic layer was dried over $MgSO_4$, filtered and concentrated under reduced pressure to afford the title compound (0.72 g, 85%). The crude was used in the next step without any further purification.
UPLC-MS (M1): RT = 0.90 min; MS *m*/*z* $ES^+$ = 344.3.

*N-(5-Fluoropyrimidin-2-yl)-7-(4-methoxybenzyl)-6-(2-methoxy-1(S)-methyl-ethyl)-4,5,6,7-tetrahydropyrazolo[3,4-b]thiazolo[4,5-d]azepin-2-amine*

According to Scheme 2, Step 6: To a solution of (*S*)-1-(4-methoxybenzyl)-8-(1-methoxypropan-2-yl)-5,6,7,8-tetrahydropyrazolo[3,4-*b*]azepin-4(1*H*)-one (0.31 g, 0.90 mmol) in pyridine (5 mL) were added consecutively 1-(5-fluoropyrimidin-2-yl)thiourea (0.23 g, 1.35 mmol) and $I_2$ (0.46 g, 1.80 mmol). The mixture was stirred at 110°C for 6 h. The mixture was then diluted with EtOAc and washed with water. The organic layers were combined, dried over $MgSO_4$, filtered and concentrated under reduced pressure. The crude residue was purified twice by flash column chromatography on silica gel using first DCM/MeOH (100:0 to 98:2) as eluent and then DCM/MeOH (99:1) as eluent to afford the title compound (90 mg, 20%) as a yellow solid.
UPLC-MS (M1): RT = 1.14 min; MS *m*/*z* $ES^+$ = 496.2.

*N-(5-Fluoropyrimidin-2-yl)-6-(2-methoxy-1(S)-methyl-ethyl)-4,5,6,8-tetrahydro pyrazolo[3,4-b]thiazolo[4,5-d]azepin-2-amiine*

According to Scheme 2, Step 7: A solution of *N*-(5-fluoropyrimidin-2-yl)-7-(4-methoxybenzyl)-6-(2-methoxy-1(*S*)-methyl-ethyl)-4,5,6,7-tetrahydropyrazolo[3,4-*b*]thiazolo[4,5-*d*]azepin-2-amine (100 mg, 0.19 mmol) in TFA (1.3 mL) was stirred at 60°C for 1 h. The mixture was directly purified on a SCX cartridge to afford a brown powder (60 mg) which was then purified by flash column chromatography on silica gel using DCM/MeOH (97:3) as eluent. The resulting solid was then suspended in MeOH and stirred at reflux for 1 h. After cooling down the powder was filtered off and dried to afford the title compound (35 mg, 51%) as a yellow solid.

Mp: 249-251°C; UPLC-MS (M1): RT = 0.85 min; MS *m/z* $ES^+$ = 376.2; $^1$H-NMR (300MHz, DMSO-$d_6$) δ: 11.80 (1H, s), 11.63 (1H, s), 8.69 (2H, s), 7.68 (1H, s), 4.24 (1H, s), 3.53 (1H, dd), 3.38 (1H, dd), 3.25 (5H, m), 2.95 (2H, d), 1.15 (3H, d).

**REFERENCE EXAMPLE 3: N-(5-Fluoropyrimidin-2-yl)-6-((5-morpholinopyridin-2-yl)methyl)-4,5,6,8-tetrahydropyrazolo[3,4-*b*]thiazolo[4,5-*d*]azepin-2-amine (Compound number 1-135)**

*8-((5-Chloropyridin-2-yl)methyl)-1-(4-methoxybenzyl)-5,6,7,8-tetrahydropyrazolo[3,4-b]azepin-4(1H)-one*

According to Scheme 3, Step 1: To a solution of 1-(4-methoxybenzyl)-5,6,7,8-tetrahydropyrazolo[3,4-*b*]azepin-4(1*H*)-one (0.50 g, 1.84 mmol) in THF (9.2 mL) were added consecutively 15-crown-5 (0.61 g, 2.76 mmol), NaO*t*Bu (0.35 g, 3.69 mmol) and after stirring for 10 min, 5-chloro-2-(chloromethyl)pyridine (0.33 g, 2.00 mmol). The mixture was stirred at reflux for 4 h. The reaction was quenched with water and the mixture was diluted with EtOAc. The separated aqueous layer was extracted with EtOAc. The organic layers were combined, dried over $Na_2SO_4$, filtered and concentrated under reduced pressure. The crude residue was purified by flash column chromatography on silica gel using cyclohexane/EtOAc (100:0 to 30:70) as eluent to afford the title compound (304 mg, 42%) as a yellow oil.
UPLC-MS (M1): RT = 0.98 min; MS *m/z* $ES^+$ = 397-399.

*6-((5-Chloropyridin-2-yl)methyl)-N-(5-fluoropyrimidin-2-yl)-7-(4-methoxybenzyl)-4,5,6,7-tetrahydropyrazolo[3,4-b]thiazolo[4,5-d]azepin-2-amine*

According to Scheme 3, Step 2: To a solution of 8-((5-chloropyridin-2-yl)methyl)-1-(4-methoxybenzyl)-5,6,7,8-tetrahydropyrazolo[3,4-*b*]azepin-4(1*H*)-one (0.24 g, 0.61 mmol) in pyridine (3 mL) were added consecutively 1-(5-fluoropyrimidin-2-yl)thiourea (0.10 g, 0.61 mmol) and $I_2$ (0.16 g, 0.61 mmol). The mixture was stirred at 80°C for 3 h. The mixture was then diluted with EtOAc and washed with saturated $Na_2S_2O_3$ solution and brine. The organic layer was then dried over $Na_2SO_4$, filtered and concentrated under reduced pressure. The crude residue was purified by flash column chromatography on silica gel using DCM/EtOAc (100:0 to 50:50) as eluent to afford the title compound (98 mg, 29%) as a brown solid.
UPLC-MS (M1): RT = 0.99 min; MS *m/z* $ES^+$ = 397-399.

*N(5-Fluoropyrimidin-2-yl)-7-(4-methoxybenzyl)-6-((5-morpholinopyridin-2-yl)methyl) -4,5,6,7-tetrahydropyrazolo[3,4-b] thiazolo[4,5-d]azepin-2-amine*

According to Scheme 7, Step 1: 6-((5-Chloropyridin-2-yl)methyl)-*N*-(5-fluoropyrimidin-2-yl)-7-(4-methoxybenzyl)-4,5,6,7-tetrahydropyrazolo[3,4-*b*]thiazolo[4,5-*d*]azepin-2-amine (98 mg, 0.18 mmol) was dissolved in toluene (1.2 mL), previously satured with $N_2$ atm. Then morpholine (31.0 mg, 0.36 mmol), *t*BuOK (4.60 g, 14.1 mmol), dicyclohexyl(2',4',6'-triisopropylbiphenyl-2-yl)phosphine (8.5 mg, 0.02 mmol) and $Pd_2(dba)_3$ (16.3 mg, 0.02 mmol) were added to the mixture. The reaction mixture was stirred at 120°C for 2 h. As the reaction was not complete, the same amount of reagents was added and the reaction mixture was stirred at 120°C for an additional 5 h. The crude solution was filtered on a celite pad and washed with EtOAc. The filtrate was washed with saturated $Na_2CO_3$ solution. The organic layer was then dried over $Na_2SO_4$, filtered and concentrated under reduced pressure. The crude residue was purified by flash column chromatography on silica gel using DCM/MeOH/1%$Et_3N$ (100:0 to 97:3) as eluent to afford the title compound (45 mg, 62%) as a brown solid.
UPLC-MS (M1): RT = 0.85 min; MS *m/z* $ES^+$ = 600.3

*N-(5-Fluoropyrimidin-2-yl)-6-((5-molpholinopyridin-2-yl)methyl)-4,5,6,8-tetrahydro pyrazolo[3,4-b]thiazolo[4,5-d]azepin-2-amine*

According to Scheme 7, Step 2: A solution of *N*-(5-fluoropyrimidin-2-yl)-7-(4-methoxybenzyl)-6-((5-morpholinopyridin-2-yl)methyl)-4,5,6,7-tetrahydropyrazolo[3,4-*b*]thiazolo[4,5-*d*]azepin-2-amine (45 mg, 47 μmol) in TFA (0.72 mL) was stirred at 60°C for 1 h. The reaction mixture was diluted with DCM and washed with saturated $Na_2CO_3$ solution. The aqueous layer was extracted with DCM and the combined organic layers were dried over $Na_2SO_4$, filtered and concentrated under reduced pressure. The crude residue was purified by flash column chromatography on silica gel using DCM/MeOH/1%$Et_3N$ (100:0 to 97:3). The residue was then purified on a SCX cartridge to afford the title compound (14 mg, 63%) as a yellow solid.
UPLC-MS (M1): RT = 0.66 min; MS *m/z* $ES^+$ = 480.2; $^1$H-NMR (300MHz, DMSO-$d_6$) δ: 11.86 (1H, bs), 8.69 (2H, s), 8.22 (1H, d), 7.70 (1H, s), 7.44-7.08 (2H, m), 4.56 (2H, s), 3.74 (4H, t), 3.13 (4H, t), 3.05-2.89 (2H, m), 2.62 (2H, d).
The hydrochloride salt form of Compound 1-135 was prepared according to the experimental procedure herebelow:

***N*-(5-Fluoropyrimidin-2-yl)-6-((5-morpholinopyridin-2-yl)methyl)-4,5,6,8-tetrahydropyrazolo [3,4-*b*]thiazolo [4,5-*d*]azepin-2-amine trihydrochloride salt (Compound number 1-135.3HCl)**

*N*-(5-Fluoropyrimidin-2-yl)-6-((5-morpholinopyridin-2-yl)methyl)-4,5,6,8-tetrahydropyrazolo[3,4-*b*]thiazolo[4,5-d]azepin-2-amine (60 mg, 125 μmol) was dissolved into 3 mL of HCl 4N in dioxane. The resulting solution was heated at 80°C for 3 h. The solvent was then removed and the resulting solid was washed with hot isopropanol. After drying under high vacuum, the title compound as trihydrochloride salt form was obtained (55 mg; brown light solid).
Mp: 240°C; UPLC-MS (M1): RT = 0.67 min; MS *m/z* $ES^+$ = 480.3; $^1H$-NMR (300MHz, DMSO-$d_6$) δ: 8.72 (s, 2H), 8.25 (d, 1H), 8.06 (dd, 1H), 7.81 (s, 1H), 7.74 (s, 1H), 4.81 (s, 2H), 3.80-3.70 (m, 5H), 3.50-3.40 (m, 3H), 3.33 (t, 5H), 3.19-3.05 (m, 2H).

**REFERENCE EXAMPLE 4: *N*-(5-Fluoropyrimidin-2-yl)-6-(2-morpholinoethyl)-4,5,6,8-tetrahydropyrazolo[3,4-*b*] thiazolo[4,5-*d*]azepin-2-amine (Compound number 1- 136)**

*1-(4-Methoxybenzyl)-8-(2-morpholinoethyl)-5,6,7,8-tetrahydropyrazolo[3,4-b]azepin-4(1H)-one*

According to Scheme 1, Step 10: To a solution of 1-(4-methoxybenzyl)-5,6,7,8-tetrahydropyrazolo[3,4-*b*]azepin-4(1*H*)-one (0.32 g, 1.19 mmol) in DMF (6.0 mL) was added 60% NaH (95 mg, 2.38 mmol). After 10 min, 4-(2-chloroethyl)morpholine (0.27 g, 1.78 mmol) was added and the mixture was stirred at rt overnight. Additional 60% NaH (20 mg) and 4-(2-chloroethyl)morpholine (1 eq) were added and the reaction mixture was stirred at 90°C for 4 h. The reaction was carefully quenched with water and the mixture was diluted with EtOAc. The organic layer was washed with water, dried over $Na_2SO_4$, filtered and concentrated under reduced pressure. The crude residue was purified by flash column chromatography on silica gel using DCM/MeOH/1%$Et_3N$ (100:0 to 97:3) as eluent to afford the title compound (304 mg, 42%) as an orange oil.
UPLC-MS (M1): RT = 0.47 min; MS *m/z* $ES^+$ = 385.5.

*5-Bromo-1-(4-methoxybenzyl)-8-(2-molpholinoethyl)-5,6,7,8-tetrahydropyrazolo[3,4-b]azepin-4(1H)-one*

According to Scheme 1, Step 11: A solution of 1-(4-methoxybenzyl)-8-(2-morpholinoethyl)-5,6,7,8-tetrahydropyrazolo[3,4-*b*]azepin-4(1*H*)-one (256 mg, 0.57 mmol) and pyridinium tribromide (199 mg, 0.62 mmol) in $CHCl_3$ (2.8 mL) was stirred at rt for 2 h. The reaction mixture was diluted with DCM. The organic layer was washed twice with water, dried over $Na_2SO_4$, filtered and concentrated under reduced pressure to afford the title compound (294 mg, 73%). The crude product was used in the next step without any further purification.
UPLC-MS (M2): RT = 0.62 min; MS *m/z* $ES^+$ = 463-465.

*N-(5-Fluoropyrimidin-2-yl)-7 -(4-methoxybenzyl)-6-(2-molpholinoethyl)-4,5,6,7-tetrahydropyrazolo[3,4-b]thiazolo[4,5-d] azepin-2-amine*

According to Scheme 1, Step 12: A solution of 5-bromo-1-(4-methoxybenzyl)-8-(2-morpholinoethyl)-5,6,7,8-tetrahydropyrazolo[3,4-*b*]azepin-4(1*H*)-one (294 mg, 0.54 mmol) and 1-(5-fluoropyrimidin-2-yl)thiourea (102 mg, 0.59 mmol) in EtOH (2.7 mL) and acetone (2.7 mL) was stirred at 90°C overnight. The reaction mixture was condensed under reduced pressure. The crude was dissolved in EtOAc and washed with aqueous $Na_2CO_3$. The organic layer was washed with brine, dried over $Na_2SO_4$, filtered and concentrated under reduced pressure. The crude residue was purified by flash column chromatography on silica gel using DCM/MeOH/1%$Et_3N$ (100:0 to 97:3) as eluent to afford the title compound (202 mg, 56%) as a brown solid.
UPLC-MS (M2): RT = 0.70 min; MS *m/z* $ES^+$ = 537.4.

*N-(5-Fluoropyrimidin-2-yl)-6-(2-morpholinoethyl)-4,5,6,8-tetrahydropyrazolo[3,4-b] thiazolo[4,5-d]azepin-2-amine*

According to Scheme 1, Step 13: To a solution of *N*-(5-fluoropyrimidin-2-yl)-7-(4-methoxybenzyl)-6-(2-morpholinoethyl)-4,5,6,7-tetrahydropyrazolo[3,4-*b*]thiazolo[4,5-d]azepin-2-amine (132 mg, 0.25 mmol) in TFA (1.9 mL) was added triisopropylsilane (195 mg, 1.23 mmol). The reaction mixture was stirred at 60°C for 1 h. The reaction mixture was concentrated under reduced pressure. The residue was then purified on a SCX cartridge. The solid was then slurried in EtOH at reflux for 1 h, filtered and dried under vaccum to afford the title compound (23 mg, 22%) as an off white solid
UPLC-MS (M1): RT = 0.57 min; MS *m/z* $ES^+$ = 417.2; $^1H$-NMR (300MHz, DMSO-$d_6$) δ: 11.82 (1H, bs), 11.63 (1H, bs), 8.69 (2H, d,), 7.66 (1H, s), 3.60-3.53 (4H, m), 3.50-3.42 (2H, m), 3.42-3.35 (2H, m), 3.02-2.97 (2H, dd,), 2.60-2.53 (2H, m), 2.44-2.40 (4H, m).

**REFERENCE EXAMPLE 5: *N*-(5-Fluoropyrimidin-2-yl)-6-((5-methyl-1,2,4-oxadiazol-3-yl) methyl)-4,5,6,8-tetrahydropyrazolo [3,4-*b*]thiazol [4,5-*d*]azepin-2-amine (Compound number 1-138)**

*1-(4-M ethoxybenzyl)-8-((5-methyl-1,2,4-oxadiazol-3-yl)methyl)-5,6,7,8-tetrahydro pyrazolo[3,4-b]azepin-4(1H)-one*

According to Scheme 3, Step 1: Under $N_2$, to a solution of 1-(4-methoxybenzyl)-5,6,7,8-tetrahydropyrazolo[3,4-*b*]azepin-4(1*H*)-one (0.50 g, 1.84 mmol) in THF (15 mL) were added consecutively 15-crown-5 (0.61 g, 2.76 mmol), NaO*t*Bu (0.35 g, 3.69 mmol) and after stirring for 10 min, 3-(chloromethyl)-5-methyl-1,2,4-oxadiazole (0.37 g, 2.76 mmol). The mixture was stirred at reflux for 2 h. The reaction was quenched with water and the mixture was diluted with EtOAc. The separated aqueous layer was extracted with EtOAc. The organic layers were combined, dried over $MgSO_4$, filtered and concentrated under reduced pressure. The crude residue was purified by flash column chromatography on silica gel using yclohexane/EtOAc (100:0 to 30:70) as eluent to afford the title compound (410 mg, 61%).
UPLC-MS (M1): RT = 0.82 min; MS *m*/*z* $ES^+$ = 368.3.

*N-(5-Fluoropyrimidin-2-yl)-7-(4-methoxybenzyl)-6-((5-methyl-1,2,4-oxadiazol-3-yl) methyl)-4,5,6,7-tetrahydropyrazolo[3,4-b]thiazo10[4,5-d]azepin-2-amine*

According to Scheme 3, Step 2: To a solution of 1-(4-methoxybenzyl)-8-((5-methyl-1,2,4-oxadiazol-3-yl)methyl)-5,6,7,8-tetrahydropyrazolo[3,4-*b*]azepin-4(1*H*-one (0.38 g, 1.03 mmol) in pyridine (8 mL) were added consecutively 1-(5-fluoropyrimidin-2-yl)thiourea (0.27 g, 1.55 mmol) and $I_2$ (0.53 g, 2.07 mmol). The mixture was stirred at 100°C for 1 h. One equivalent of both reagents was added and the reaction mixture was stirred at 100°C for an additional h. The mixture was then diluted with EtOAc and washed with water. The organic layers were combined, dried over $MgSO_4$, filtered and concentrated under reduced pressure. The crude residue was purified twice by flash column chromatography on silica gel using first DCM/MeOH (100:0 to 97:3) as eluent and then DCM/MeOH (99:1) as eluent to afford the title compound (285 mg, 53%).
UPLC-MS (M1): RT = 1.05 min; MS *m*/*z* $ES^+$ = 520.2.

*N-(5-Fluoropyrimidin-2-yl)-6-((5-methyl-1,2,4-oxadiazol-3-yl)methyl)-4,5,6,8-tetrahydropyrazolo[3,4-b]thiazolo[4,5-d]azepin-2-amine*

According to Scheme 3, Step 3: A solution of *N*-(5-fluoropyrimidin-2-yl)-7-(4-methoxybenzyl)-6-((5-methyl-1,2,4-oxadiazol-3-yl)methyl)-4,5,6,7-tetrahydropyrazolo[3,4-*b*]thiazolo[4,5-d]azepin-2-amine (270 mg, 0.52 mmol) in TFA (1.9 mL) was stirred at 60°C for 4 h. The reaction mixture was carefully quenched with water and diluted with EtOAc. The organic layer was dried over $MgSO_4$, filtered and concentrated under reduced pressure. The residue was then taken up in a DCM:MeOH (1:1) mixture (7 mL) and the formed precipitate was filtered off, rinsed and dried under to afford the title compound (77 mg, 37%) as a beige solid
Mp: 245°C; UPLC-MS (M1): RT = 0.80 min; MS *m*/*z* $ES^+$ = 400.2; $^1$H-NMR (300MHz, DMSO-$d_6$) $\delta$: 11.6 (1H, s), 8.70 (2H, s), 7.70 (1H, s), 4.65 (2H, s), 3.55-3.45 (2H, m), 3.10-3.00 (2H, m), 2.55 (3H, s).
The hydrochloride salt of Compound 1-138 was prepared according to the experimental procedure herebelow:

***N*-(5-Fluoropyrimidin-2-yl)-6-((5-methyl-1,2,4-oxadiazol-3-yl)methyl)-4,5,6,8-tetrahydropyrazolo[3,4-*b*]thiazolo[4,5-*d*]-azepin-2-amine (Compound number 1-138.HCl)**

*N*-(5-Fluoropyrimidin-2-yl)-6-((5-methyl-1,2,4-oxadiazol-3-yl)methyl)-4,5,6,8-tetrahydropyrazolo[3,4-*b*]thiazolo[4,5-d]azepin-2-amine (1.00 g, 2.50 mmol) was dissolved into 30 mL of HCl 4N in dioxane. The resulting solution was heated at 80°C for 3 h. The solvent was then removed and the resulting solid was washed with hot isopropanol. After drying under high vacuum, the title compound as hydrochloride salt form (1.2 eq HCl) was obtained as a beige powder (1.00 g).
Mp:> 320°C; UPLC-MS (M1): RT = 0.80 min; MS *m*/*z* $ES^+$ = 400.0; $^1$H-NMR (300MHz, DMSO-$d_6$) $\delta$: 8.70 (2H, s), 7.75 (1H, s), 4.65 (2H, s), 3.60-3.50 (2H, m), 3.10-3.00 (2H, m), 2.55 (3H, s).

**REFERENCE EXAMPLE 6: *N*-(5-Fluoro-4-methylpyrimidin-2-yl)-6-(2-methoxyethyl)-4,5,6,8-tetrahydropyrazolo[3,4-*b*]thiazolo[4,5-*d*]-azepin-2-amine (Compound number 1-129)**

*8-(4-Methoxybenzyl)-6-(2-methoxyethyl)-4,5,6,8-tetrahydropyrazolo[3,4-b]thiazolo [4,5-d]azepin-2-amine*

According to Scheme 4, Step 1: A solution of 5-bromo-2-(4-methoxybenzyl)-8-(2-methoxyethyl)-5,6,7,8-tetrahydropyrazolo[3,4-*b*]azepin-4(2*H*)-one (475 mg, 1.16 mmol, prepared according to Scheme 1) and thiourea (89.0 mg,

1.16 mmol) in EtOH (2.5 mL) and acetone (2.5 mL) was stirred at 90°C for 1.5 h. The reaction mixture was condensed under reduced pressure. The crude residue was purified by flash column chromatography on silica gel using DCM/MeOH (100:0 to 98:2) as eluent to afford the title compound (305 mg, 68%) as a green solid.
UPLC-MS (M1): RT = 0.67 min; MS *m/z* $ES^+$ = 386.5.

*N-(5-Fluoro-4-methylpyrimidin-2-yl)-8-(4-methoxybenzyl)-6-(2-methoxyethyl)-4,5,6,8-tetrahydropyrazolo[3,4-b]thiazolo[4,5-d]azepin-2-amine*

According to Scheme 4, Step 2: A solution of 8-(4-methoxybenzyl)-6-(2-methoxyethyl)-4,5,6,8-tetrahydropyrazolo[3,4-b]thiazolo[4,5-d]azepin-2-amine (200 mg, 0.39 mmol), 2-chloro-5-fluoro-4-methylpyrimidine (93.0 mg, 0.63 mmol), $Cs_2CO_3$ (339 mg, 1.04 mmol), Xantphos (45 mg, 80 $\mu$mol) and $Pd(OAc)_2$ (12 mg, 50 $\mu$mol) in dioxane (1.7 mL) was stirred at 120°C for 30 min. The crude solution was cooled down and diluted with 10 mL of water and 10 mL of EtOAc. The aqueous layer was extracted twice with EtOAc. The combined organic layers dried over $MgSO_4$, filtered and concentrated under reduced pressure. The crude residue was purified by flash column chromatography on silica gel using DCM/MeOH (100:0 to 98:2) as eluent to afford the title compound (138 mg, 54%) as a beige solid.
UPLC-MS (M1): RT = 1.16 min; MS *m/z* $ES^+$ = 496.2.

*N-(5-Fluoro-4-methylpyrimidin-2-yl)-6-(2-methoxyethyl)-4,5,6,8-tetrahydropyrazolo [3,4-b]thiazolo[4,5-d]azepin-2-amine*

According to Scheme 4, Step 3: A solution of *N*-(5-fluoro-4-methylpyrimidin-2-yl)-8-(4-methoxybenzyl)-6-(2-methoxyethyl)-4,5,6,8-tetrahydropyrazolo[3,4-*b*]thiazolo[4,5-*d*]azepin-2-amine (138 mg, 0.28 mmol) in DCM (0.5 mL) and TFA (2.2 mL) was stirred at 65°C for 2 h. After cooling down, the reaction mixture was concentrated under reduced pressure. The residue was purified on a SCX cartridge. The resulting solid was then washed with MeOH and DCM and dried to afford the title compound (30 mg, 28%) as a beige solid.
Mp: 261-263°C; UPLC-MS (M1): RT = 0.85 min; MS *m/z* $ES^+$ = 376.4; $^1$H-NMR (300MHz, DMSO-$d_6$) $\delta$: 11.82 (1H, s), 11.49 (1H, s), 8.52 (1H, d,), 7.67 (1H, s), 3.60-3.50 (4H, m), 3.43-3.35 (2H, m), 3.26 (3H, s), 3.05-2.94 (2H, m), 2.44 (3H, d,).

**REFERENCE EXAMPLE 7: *N*-(5-Fluoropyrimidin-2-yl)-6-(3-(methylamino)propyl)-4,5,6,8-tetrahydropyrazolo[3,4-*b*]thiazolo[4,5-*d*]azepin-2-amine (Compound number 1-130)**

*N-(5-Fluoropyrimidin-2-yl)-8-(4-methoxybenzyl)-6-(3-(methylamino)propyl)-4,5,6,8-tetrahydropyrazolo[3,4-b]thiazolo[4,5-d]azepin-2-amine*

According to Scheme 5, Step 1: A solution of *tert*-butyl 3-(2-(5-fluoropyrimidin-2-ylamino)-8-(4-methoxybenzyl)-4,5-dihydropyrazolo[3,4-*b*]thiazolo[4,5-*d*]azepin-6(8*H*)-yl)propyl(methyl)carbamate (140 mg, 0.17 mmol, prepared according to Scheme 3) in HCl 4N in dioxane (1.6 mL) and a few drops of MeOH was stirred at rt for 30 min. The reaction mixture was condensed under reduced pressure. The crude residue was taken up in DCM and neutralized with $Et_3N$. The resulting beige precipitate was filtered off and washed with DCM and water to afford the title compound (30 mg, 37%) as a beige solid.
UPLC-MS (M1): RT = 0.72 min; MS *m/z* $ES^+$ = 495.2.

*N-(5-Fluoropyrimidin-2-yl)-6-(3-(methylamino)propyl)-4,5,6,8-tetrahydropyrazolo [3,4-b]thiazolo[4,5-d]azepin-2-amine*

According to Scheme 5, Step 2: A solution of *N*-(5-fluoropyrimidin-2-yl)-8-(4-methoxybenzyl)-6-(3-(methylamino)propyl)-4,5,6,8-tetrahydropyrazolo[3,4-*b*]thiazolo[4,5-*d*]azepin-2-amine (30 mg, 60 $\mu$mol) in TFA (0.6 mL) was stirred at 60°C for 40 min. After cooling down, the reaction mixture was concentrated under reduced pressure. The residue was dissolved in MeOH and neutralized with $Et_3N$. The crude was then purified by preparative HPLC in basic mode to afford the title compound (6 mg, 28%) as a light yellow solid.
UPLC-MS (M1): RT = 0.61 min; MS *m/z* $ES^+$ = 375.0; $^1$H-NMR (300MHz, DMSO-$d_6$ + 0.1% TFA) $\delta$: 8.84 (2H, s), 8.15 (1H, s), 3.61-3.39 (4H, m), 3.22-3.07 (2H, m), 3.04-2.86 (2H, m), 2.62 (3H, s), 2.07-1.83 (2H, m).

**REFERENCE EXAMPLE 8: 3-(2-(5-Fluoropyrimidin-2-ylamino)-4,5-dihydropyrazolo[3,4-*b*] thiazolo[4,5-*d*]azepin-6(8*H*)-yl)propan-1-ol (Compound number 1-132)**

*3-(2-(5-Fluoropyrimidin-2-ylamino)-4,5-dihydropyrazolo[3,4-b]thiazolo[4,5-d]azepin-6(8H)-yl)propan-1-ol*

According to Scheme 6, step 1: To an ice-cooled solution of *N*-(5-fluoropyrimidin-2-yl)-6-(3-methoxypropyl)-4,5,6,8-tetrahydropyrazolo[3,4-*b*]thiazolo[4,5-*d*]azepin-2-amine (34 mg, 91 μmol, prepared according to Scheme 3) in DCM (2 mL) was added tribromoborane (0.27 mL, 0.27 mmol). The reaction mixture was stirred at 0°C for 10 min and 3 h at rt. The mixture was then filtered off. The resulting yellow solid was purified by flash column chromatography on silica gel using DCM/MeOH/ $Et_3N$ (97:3:0.3) as eluent to afford the title compound (10 mg, 30%) as a yellow solid.
UPLC-MS (M1): RT = 0.70 min; MS *m/z* $ES^+$ = 362.2; $^1$H-NMR (300MHz, DMSO-$d_6$ + 0.1% TFA) δ: 11.64 (1H, brs), 8.69 (2H, s), 7.67 (1H, s), 3.47-3.30 (6H, m), 3.02-2.99 (2H, m), 1.77-1.72 (2H, m).

**REFERENCE EXAMPLE 9: *N*-(5-Fluoropyrimidin-2-yl)-6-(2-(4-isopropylpiperazin-1-yl)ethyl)-4,5,6,8-tetrahydropyrazolo[3,4-*b*]thiazolo[4,5-*d*]azepin-2-amine (Compound number 1-141)**

*N-(5-Fluoropyrimidin-]-yl)-8-(4-methoxybenzyl)-6-(2-(piperazin-1-yl)ethyl)-4,5,6,8-tetrahydropyrazolo[3,4-b]thiazolo[4,5-d]azepin-2-amine*

According to Scheme 8, step 1: To a solution of *tert*-butyl 4-(2-(2-(5-fluoropyrimidin-2-ylamino)-8-(4-methoxybenzyl)-4,5-dihydropyrazolo[3,4-*b*]thiazolo[4,5-*d*]azepin-6(8*H*)-yl)ethyl)piperazine-1-carboxylate (48 mg, 76 μmol, prepared according to Scheme 1) in DCM (0.4 mL) and MeOH (0.4 mL) was added HCl 4N in dioxane (20 μL). The resulting solution was stirred at rt for 3 h. The reaction mixture was condensed under reduced pressure. The crude residue was taken up in MeOH, neutralized with $Et_3N$ and condensed under reduced pressure. The resulting beige solid (40 mg) was used as such in the next step.
UPLC-MS (M1): RT = 0.69 min; MS *m/z* $ES^+$ = 356.0.

*N-(5-Fluoropyrimidin-2-yl)-6-(2-(4-isopropylpiperazin-1-yl)ethyl)-8-(4-methoxybenzyl) -4,5,6,8-tetrahydropyrazolo[3,4-b]thiazo[4,5-d]azepin-2-amine*

According to Scheme 8, step 2: A solution of *N*-(5-fluoropyrimidin-2-yl)-8-(4-methoxybenzyl)-6-(2-(piperazin-1-yl)ethyl)-4,5,6,8-tetrahydropyrazolo[3,4-*b*]thiazolo[4,5-*d*]azepin-2-amine (40 mg, 75 μmol) and propan-2-one (2 mL) in DCM (0.7 mL) was stirred at rt for 15 min. $NaBH(OAc)_3$ (21 mg, 97 μmol) was then added and the reaction mixture was stirred at rt overnight. 37% conversion was observed by UPLC-MS. Another 2 mL of propan-2-one and 1.3 eq of $NaBH(OAc)_3$ were added and the mixture was stirred over the week end at rt. Then the reaction mixture was quenched with NaOH 1N. The organic layer was dried over $MgSO_4$, filtered and condensed under reduced pressure. The resulting light yellow solid (40 mg, 93%) was used as such in the next step.
UPLC-MS (M1): RT = 0.79 min; MS *m/z* $ES^+$ = 578.0.

*N-(5-Fluoropyrimidin-2-yl)-6-(2-(4-isopropylpiperazin-1-yl)ethyl)-4,5,6,8-tetrahydro pyrazolo[3,4-b]thiazolo[4,5-d] azepin-2-amine*

According to Scheme 8, step 3: A solution of *N*-(5-fluoropyrimidin-2-yl)-6-(2-(4-isopropylpiperazin-1-yl)ethyl)-8-(4-methoxybenzyl)-4,5,6,8-tetrahydropyrazolo[3,4-*b*]thiazolo[4,5-*d*]azepin-2-amine (40 mg, 69 μmol) and triisopropylsilane (55.0 mg, 346 μmol) in TFA (0.7 mL) was stirred at 60°C for 100 min. After cooling down, the reaction mixture was concentrated under reduced pressure. The residue was purified on a SCX cartridge; the resulting solid was then washed with MeOH and $Et_2O$ and dried to afford the title compound (11 mg, 35%) as a light yellow solid.
UPLC-MS (M1): RT = 0.59 min; MS *m/z* $ES^+$= 458.0; $^1$H-NMR (300MHz, DMSO-$d_6$ + 10% TFA) δ: 8.84 (2H, s), 8.07 (1H, s), 4.15-3.92 (2H, m), 3.87-3.33 (13H, m), 3.24-3.06 (2H, m), 1.32 (6H, d).

**REFERENCE EXAMPLE 10: 6-(3-(4,4-Difluoropiperidin-1-yl)propyl)-*N*-(5-fluoropyrimidin-2-yl)-4,5,6,8-tetrahydropyrazolo[3,4-*b*]thiazolo[4,5-*d*]azepin-2-amine (Compound number 1-148)**

*8-(3-Hydroxypropyl)-2-(4-methoxybenzyl)-5,6,7,8-tetrahydropyrazolo[3,4-b]azepin-4(2H)-one*

According to Scheme 9, step 1: To a solution of 2-(4-methoxybenzyl)-8-(3-(trimethylsilyloxy)propyl)-5,6,7,8-tetrahydropyrazolo[3,4-*b*]azepin-4(2*H*)-one (0.85 g, 2.12 mmol, prepared according to Scheme 3, step1) in dioxane (7.1 mL) was added HCl 4N in dioxane (0.53 mL, 2.12 mmol). The resulting solution was stirred at rt for 45 min. The reaction mixture was then concentrated under reduced pressure to afford the title compound (0.66 g, 95%) as a red oil used as such in the next step.
UPLC-MS (M1): RT = 0.67 min; MS *m/z* $ES^+$= 330.2.

*8-(3-Bromopropyl)-2-(4-methoxybenzyl)-5,6,7,8-tetrahydropyrazolo[3,4-b]azepin-4(2H)-one*

According to Scheme 9, step 2: To a mixture of 8-(3-hydroxypropyl)-2-(4-methoxybenzyl)-5,6,7,8-tetrahydropyrazolo[3,4-*b*]azepin-4(2*H*)-one (0.60 g, 1.82 mmol) and perbromomethane (1.21 g, 3.64 mmol) in THF (9.1 mL) was added trisphenylphosphine polymer-bounded (1.21 mL, 3.64 mmol). The resulting mixture was stirred at rt for 10 min and then filtered to remove the resin. The filtrate was concentrated under reduced pressure to give a red oil (0.73 g). Precipitation from cold 2-MeTHF afforded the title compound (0.71 g, 89 % yield) as a white powder.
UPLC-MS (M1): RT = 0.96 min; MS *m/z* $ES^+$= 392.2-394.2.

*8-(3-(4,4-Difluoropipeperidin-1-yl)propyl)-2-(4-methoxybenzyl)-5,6,7,8-tetrahydro pyrazolo[3,4-b]azepin-4((2H)-one*

According to Scheme 9, step 3: To a mixture of 4,4-difluoropiperidine hydrochloride (0.14 g, 0.89 mmol) and $N(Et)_2iPr$ (0.37 mL, 2.23 mmol) in 2-MeTHF (1.8 mL) was added 8-(3-bromopropyl)-2-(4-methoxybenzyl)-5,6,7,8-tetrahydropyrazolo[3,4-*b*] azepin-4(2*H*)-one (0.35 g, 0.89 mmol). The resulting mixture was heated at 80°C for 1.5 h. The reaction mixture was condensed under reduced pressure. The dark oily residue was then taken up in EtOAc and washed twice with a brine solution. Aqueous layer was extracted again and the combined organic layers were dried over $MgSO_4$, filtered and condensed under reduced pressure. The residue was purified by flash column chromatography on silica gel using DCM/MeOH (100:0 to 98:2) as eluent to afford the title compound (85 mg, 22%) as a reddish oil.
UPLC-MS (M1): RT = 0.60 min; MS *m/z* $ES^+$= 433.4.

*6-(3-(4,4-Difluoropiperidin-1-yl)propyl)-N-(5-fluoropyrimidin-2-yl)-8-(4-methoxy benzyl)-4,5,6,8-tetrahydropyrazolo[3,4-b]thiazolo[4,5-d]azepin-2-amine*

According to Scheme 9, step 4: To a solution of 8-(3-(4,4-difluoropiperidin-1-yl)propyl)-2-(4-methoxybenzyl)-5,6,7,8-tetrahydropyrazolo[3,4-*b*]azepin-4(2*H*)-one (194 mg, 0.45 mmol) and 1-(5-fluoropyrimidin-2-yl)thiourea (93.0 mg, 0.54 mmol) in pyridine (3 mL) was added under inert atmosphere $I_2$ (137 mg, 0.54 mmol). The resulting mixture was stirred at 80°C for 3 h. UPLC-MS monitoring showed a 65% conversion. 0.5 eq of both thiourea derivative and $I_2$ was added to the medium and the mixture was heated another 1 h at 80°C. The reaction mixture was quenched with a saturated aqueous $NaHCO_3$ solution (20 mL) and then extracted several times with EtOAc. The combined organic layers were dried over $MgSO_4$, filtered and concentrated under reduced pressure. The residue was purified by flash column chromatography on silica gel using DCM/MeOH (100:0 to 97:3) as eluent to give the title compound as an orange oil (100 mg). Precipitation from cold EtOH afforded the title compound (95 mg, 36%) as a pale pink powder.
UPLC-MS (M1): RT = 0.80 min; MS *m/z* $ES^+$= 585.4.

*6-(3-(4,4-Difluoropiperidin-1-yl)propyl)-N-(5-fluoropyrimidin-2-yl)-4,5,6,8-tetrahydro pyrazolo[3,4-b]thiazolo[4,5-d] azepin-2-amine*

According to Scheme 9, step 5: A solution of 6-(3-(4,4-difluoropiperidin-1-yl)propyl)-*N*-(5-fluoropyrimidin-2-yl)-8-(4-methoxybenzyl)-4,5,6,8-tetrahydropyrazolo[3,4-*b*]thiazolo[4,5-*d*]azepin-2-amine (92.0 mg, 0.16 mmol) and triisopropylsilane (0.1 mL, 0.63 mmol) in TFA (3.2 mL) was stirred at 65°C for 1 h. The reaction mixture was concentrated under reduced pressure. The dark residue was then taken up in DCM and washed twice with a brine solution. The aqueous layer was extracted again with EtOAc and the combined organic extracts were dried over $MgSO_4$, filtered and concentrated under reduced pressure. The crude was purified by flash column chromatography on silica gel using DCM/MeOH (100:0 to 95:5 (+1% $NEt_3$)) as eluent to give a yellow powder (28 mg). Trituration from warm iPrOH afforded the title compound (21 mg, 28%) as a light yellow powder.
Mp: 238-240°C; UPLC-MS (M1): RT = 0.67 min; MS *m/z* $ES^+$= 465.3; $^1$H-NMR (300MHz, DMSO-$d_6$) $\delta$: 11.80 (1H, d), 11.64 (1H, s), 8.69 (2H, s), 7.65 (1H, s), 3.40-3.25 (4H, m), 3.14-2.87 (2H, m), 2.65-2.40 (2H, m) 2.39 (4H, d), 1.94 (4H, dt), 1.77 (2H, t).

**REFERENCE EXAMPLE 11: *N*-(5-Fluoropyrimidin-2-yl)-6-((3-methyl-1,2,4-oxadiazol-5-yl) methyl)-4,5,6,8-tetrahydropyrazolo [3,4-*b*] thiazolo [4,5-*d*] azepin-2-amine (Compound number 1-150)**

*2-(2-(4-Methoxybenzyl)-4-oxo-4,5,6,7-tetrahydropyrazolo[3,4-b]azepin-8(2H)-yl)acetic acid*

According to Scheme 10, step 1: A solution of methyl 2-(2-(4-methoxybenzyl)-4-oxo-4,5,6,7-tetrahydropyrazolo[3,4-*b*]azepin-8(2*H*)-yl)acetate (200 mg, 0.58 mmol, prepared according to Scheme 3, step 1) and LiOH (56.0 mg, 2.33 mmol) in MeOH (1 mL), water (0.1 mL) and THF (1 mL) was stirred at rt for 2 h. The reaction mixture was concentrated under reduced pressure and the crude was taken up with EtOAc and acidified with HCl 1N. The organic layer was then

dried over $MgSO_4$, filtered and concentrated under reduced pressure to afford the title compound as an orange solid (150 mg, 78%) and was used as such in the next step.
UPLC-MS (M1): RT = 0.65 min; MS *m*/*z* $ES^+$= 330.

*2-(4-Methoxybenzyl)-8-((3-methyl-1,2,4-oxadiazol-5-yl)methyl)-5,6,7,8-tetrahydro pyrazolo[3,4-b]azepin-4(2H)-one*

According to Scheme 10, step 2: To a solution of 2-(2-(4-methoxybenzyl)-4-oxo-4,5,6,7-tetrahydropyrazolo[3,4-*b*]azepin-8(2*H*)-yl)acetic acid (450 mg, 1.37 mmol), HATU (623 mg, 1.64 mmol) and *N'*-hydroxyacetimidamide (202 mg, 2.73 mmol) in DMF (10 mL) was added $NEt_3$ (525 μL, 4.10 mmol). The resulting solution was stirred first 30 min at 60°C, then 2 h at 120°C. 15% conversion was observed. A saturated aqueous $Na_2CO_3$ solution was added to the reaction mixture and extraction was performed with EtOAc. The organic layer was dried over $MgSO_4$, filtered and concentrated under reduced pressure. The residue was purified by flash column chromatography on silica gel using Cyclohexane/EtOAc (100:0 to 30:70) as eluent to afford the title compound (70 mg, 14%).
UPLC-MS (M1): RT = 0.81 min; MS *m*/*z* $ES^+$= 368.3.

*N-(5-Fluoropyrimidin-2-yl)-8-(4-methoxybenzml)-6-((3-methyl-1,2,4-oxadiazol-5-yl) methyl)-4,5,6,8-tetrahydropyrazolo[3,4-b]thiazolo[4,5-d]azepin-2-amine*

According to Scheme 10, step 3: To a solution of 2-(4-methoxybenzyl)-8-((3-methyl-1,2,4-oxadiazol-5-yl)methyl)-5,6,7,8-tetrahydropyrazolo[3,4-*b*]azepin-4(2*H*)-one (70 mg.0, 0.19 mmol) and 1-(5-fluoropyrimidin-2-yl)thiourea (66.0 mg, 0.38 mmol) in pyridine (2 mL) was added $I_2$ (97.0 mg, 0.38 mmol) under an inert atmosphere. The resulting mixture was stirred at 100°C for 1 h. UPLC-MS monitoring showed a 50% conversion. 1 eq of both thiourea derivative and $I_2$ was added to the medium and the mixture was heated another 1 h at 100°C. The reaction mixture was quenched with water and then extracted several times with EtOAc. The combined organic layers were dried over $MgSO_4$, filtered and concentrated under reduced pressure. The residue was purified by flash column chromatography on silica gel using DCM/MeOH (100:0 to 97:3) as eluent to afford the title compound (55 mg, 56%).
UPLC-MS (M1): RT = 1.02 min; MS *m*/*z* $ES^+$= 520.2.

*N-(5-Fluoropyrimidin-2-yl)-6-((3-methyl-1,2,4-oxadiazol-5-yl)methyl)-4,5,6,8-tetrahydropyrazolo[3,4-b]thiazolo[4,5-d] azepin-2-amine*

According to Scheme 10, step 4: A solution of *N*-(5-fluoropyrimidin-2-yl)-8-(4-methoxybenzyl)-6-((3-methyl-1,2,4-oxadiazol-5-yl)methyl)-4,5,6,8-tetrahydropyrazolo [3,4-*b*]thiazolo[4,5-*d*]azepin-2-amine (55.0 mg, 0.11 mmol) in TFA (0.7 mL) was stirred at 60°C for 2 h. The reaction mixture was diluted with EtOAc and carefully quenched with water. The organic layer was then dried over $MgSO_4$, filtered and concentrated under reduced pressure. The crude was purified by flash column chromatography on silica gel using DCM/MeOH (100:0 to 94:6) as eluent to afford the title compound as a grey powder (18 mg, 43%).
UPLC-MS (M1): RT = 0.81 min; MS *m*/*z* $ES^+$= 400.1; $^1$H-NMR (300MHz, DMSO-$d_6$) δ: 11.91(1H, s), 11.67 (1H, s), 8.70 (2H, s), 7.69 (1H, s), 4.83 (2H, s), 3.65-3.45 (2H, m), 3.15-3.00 (2H, m), 2.30 (3H, s).

**REFERENCE EXAMPLE 12: 4-(3-(2-((5-Fluoropyrimidin-2-yl)amino)-4,5-dihydropyrazolo[3,4-*b*]thiazolo[4,5-*d*] azepin-6(8*H*)-yl)propyl)thiomorpholine 1,1-dioxide (Compound number 1-151)**

*8-(3-Aminopropyl)-1-(4-methoxybenzyl)-5,6,7,8-tetrahydropyrazolo[3,4-b]azepin-4(1H)-one*

According to Scheme 11, step 1: To a mixture of 2-(3-(1-(4-methoxybenzyl)-4-oxo-4,5,6,7-tetrahydropyrazolo[3,4-*b*]azepin-8(1*H*)-yl)propyl)isoindoline-1,3-dione (635 mg, 1.38 mmol) in EtOH (4.6 mL) was added hydrazine (67 μL, 1.38 mmol) and the resulting mixture was heated at 65°C for 1 h. An LC-MS monitoring of the reaction showed a full conversion of the SM with an extra addition of hydrazine on the carbonyl group. The crude mixture was poured onto water. Aqueous layer was acidified to pH=2 by the addition of a 6N HCl aq. solution and then heated up to 65°C for 2-3 min leading to the cleavage of the imino adduct. The aqueous layer was then neutralized to pH=6-7 by the addition of a 30% aq. NaOH solution and extracted several times with EtOAc. The combined organic layers were dried over $MgSO_4$, filtered and concentrated under reduced pressure to afford the title compound (0.36 g, 80%) as a colorless oil.
UPLC-MS (M1): RT = 0.50 min; MS *m*/*z* $ES^+$= 329.3.

*1-(4-Methoxybenzyl)-8-(3-dioxothiomorpholinopropyl)-5,6,7,8-tetrahydropyrazolo[3,4-b]azepin-4(1H)-one*

According to Scheme 11, step 2: A solution of vinylsulfonylethene (0.11 mL, 1.10 mmol) and 8-(3-aminopropyl)-1-(4-methoxybenzyl)-5,6,7,8-tetrahydropyrazolo[3,4-*b*]azepin-4(1*H*)-one (0.36 g, 1.10 mmol) in dry dioxane (3.7 mL) was heated at 110°C for 1.5 h. The reaction mixture was poured onto water and extracted three times with DCM. The combined organic layers were dried over $MgSO_4$, filtered and concentrated under reduced pressure. The crude was purified by flash column chromatography on silica gel using DCM/MeOH (100:0 to 95:5) as eluent to afford the title compound (0.34 g, 68%) as a light yellow oil.
UPLC-MS (M1): RT = 0.65 min; MS *m/z* $ES^+$= 447.5.

*N-(5-Fluoropyrimidin-2-yl)-7-(4-methoxybenzyl)-6-(3-dioxothiomorpholinopropyl)-4,5,6,7-tetrahydropyrazolo[3,4-b]thiazolo[4,5-d]azepin-2-amine*

According to Scheme 11, step 3: To a solution of 1-(4-methoxybenzyl)-8-(3-dioxothiomorpholinopropyl)-5,6,7,8-tetrahydropyrazolo[3,4-*b*]azepin-4(1*H*)-one (0.34 g, 0.76 mmol) and 1-(5-fluoropyrimidin-2-yl)thiourea (156 mg, 0.91 mmol) in pyridine (5.1 mL) was added $I_2$ (0.23 g, 0.91 mmol). The resulting solution was heated at 80°C for 1 h. UPLC-MS monitoring showed a 80% conversion rate. Then 0.5 eq of both $I_2$ and thiourea derivative was added and the mixture was stirred overnight at 80°C. The crude mixture was then poured onto a brine solution and extracted several times with EtOAc. The combined organic layers were dried over $MgSO_4$, filtered and concentrated under reduced pressure. The crude mixture was purified by flash column chromatography on silica gel using DCM/MeOH (100:0 to 98:2) as eluent. Trituration in isopropanol afforded the title compound (167 mg, 37%) as a light yellow powder.
UPLC-MS (M1): RT = 0.87 min; MS *m/z* $ES^+$= 599.3.

*4-(3-(2-((5-Fluoropyrimidin-2-yl)amino)-4,5-dihydropyrazolo[3,4-b]thiazolo[4,5-d] azepin-6(8H)-yl)propyl)thiomorpholine 1,1-dioxide*

According to Scheme 11, step 4: A solution of *N*-(5-fluoropyrimidin-2-yl)-7-(4-methoxybenzyl)-6-(3-dioxothiomorpholinopropyl)-4,5,6,7-tetrahydropyrazolo[3,4-*b*]thiazolo[4,5-*d*]azepin-2-amine (165 mg, 0.28 mmol) in TFA (5.5 mL) was heated at 65°C for 1 h. The reaction mixture was concentrated to dryness. The dark residue was taken up in DCM and neutralized with a saturated $NaHCO_3$ aqueous solution and washed twice with a brine solution. Aqueous layer was extracted again with EtOAc and the combined organic extracts were dried over $MgSO_4$, filtered and concentrated under reduced pressure. The crude was purified by flash column chromatography on silica gel using DCM/MeOH (100:0 to 95:5 (+1% $NEt_3$) as eluent. Trituration in warm isopropanol afforded the title compound as a light yellow powder. The target compound was then solubilized in a mixture of hot DMSO (0.5 mL) and water (50μL). The aliquot was then lyophilized for 2 days to afford the title compound (27 mg, 20% yield) as a beige powder.
Mp: 272-273°C; UPLC-MS (M1): RT = 0.63 min; MS *m/z* $ES^+$= 479.2; $^1$H-NMR (300MHz, DMSO-$d_6$) δ: 11.81 (1H, s), 11.62 (1H, s), 8.69 (2H, s), 7.87-7.44 (1H, m), 3.46-3.33 (4H, m), 3.08 (4H, t), 3.01 (2H, t), 2.87 (4H, t), 1.77 (2H, dd) +2H under water peak.

**REFERENCE EXAMPLE 13: 2-(4-Methoxybenzyl)-8-(3-morpholinopropyl)-5,6,7,8-tetrahydro pyrazolo[3,4-*b*] azepin-4(2*H*)-one (Compound number 1-140)**

*2-(4-Methoxybenzyl)-8-(3-morpholinopropyl)-5,6,7,8-tetrahydropyrazolo[3,4-b]azepin -4(2H)-one*

According to Scheme 3, step 1: To a mixture of 2-(4-methoxybenzyl)-5,6,7,8-tetrahydropyrazolo[3,4-*b*]azepin-4(2*H*)-one (12.0 g, 44.2 mmol) in dry THF (177 mL) were added under an inert atmosphere 1,4,7,10,13-pentaoxacyclopentadecane (13.1 mL, 66.3 mmol), sodium 2-methylpropan-2-olate (6.38 g, 66.3 mmol) and then 5 min later 4-(3-chloropropyl)morpholine (8.69 g, 53.1 mmol). The resulting reaction mixture was heated overnight at reflux. An LC-MS monitoring the reaction showed a 50% conversion rate of the starting material with no longer evolution. The crude reaction mixture was then quenched, adding 10 mL of water and the organic volatiles were removed under *vacuo*. The brown oily residue was then taken up in EtOAc and washed twice with a saturated $NH_4Cl$ solution. The aqueous layer was extracted again several times with EtOAc and combined organic extracts were dried over $MgSO_4$, filtered and concentrated under reduced pressure. The crude product was purified by flash column chromatography on silica gel using EtOAc/iPrOH (100:0 to 80:20) as eluent to give the title compound and 4 g of the starting material (azepine derivative). The reaction was repeated twice using the recovered starting material and following the same reaction procedure. Mixed batches (yellow oil) were then triturated with a mixture $Et_2O$/Petroleum Ether 2:1 until precipitation. A light yellow powder was filtered off, slurred twice with $Et_2O$ and dried under high vacuum pressure to afford the title compound (15 g, 90 %) as an off-white powder.
UPLC-MS (M1): RT =4.18 min; MS m/z $ES^+$= 399.4.

*N-(5-Fluoropyrimidin-2-yl)-8-(4-methoxybenzyl)-6-(3-morpholinopropyl)-4,5,6,8-tetrahydropyrazolo[3,4-b]thiazolo[4,5-d]azepin-2-amine*

According to Scheme 3, step 2: To a mixture of 2-(4-methoxybenzyl)-8-(3-morpholinopropyl)-5,6,7,8-tetrahydropyrazolo[3,4-*b*]azepin-4(2*H*)-one (4.00 g, 10.0 mmol) and 1-(5-fluoropyrimidin-2-yl)thiourea (2.38 g, 13.8 mmol)in pyridine (67 mL) was added $I_2$ (3.06 g, 12.05 mmol) under an inert atmosphere and the resulting mixture was left under stirring for 0.5 h at 80°C. The reaction mixture turned into a dark solution. An UPLC-MS monitoring of the reaction showed a 65% conversion rate of the starting material. 0.5 eq of both 1-(5-fluoropyrimidin-2-yl)thiourea and $I_2$ were added and the resulting mixture was heated to 80°C for another 0.5 h. The crude mixture was diluted with EtOAc and washed with a saturated $NaHCO_3$ aqueous solution. The aqueous layer was extracted again three times with EtOAc. The combined organic layers were dried over $MgSO_4$, filtered and concentrated under reduced pressure. The crude product was purified by flash column chromatography on silica gel using DCM/MeOH (100:0 to 95:5 (+1% $NEt_3$) as eluent. The recovered light yellow powder was then slurred twice in cold iPrOH, rinsed with $Et_2O$ and dried under vacuum pressure to afford the title compound (1.4 g, 25%) as a light yellow powder.
UPLC-MS (M1); RT =0.80min; MS m/z $ES^+$= 551.5.

*N-(5-Fluoropyrimidin-2-yl)-6-(3-morpholinopropyl)-4,5,6,8-tetrahydropyrazolo[3,4-b] thiazolo[4,5-d]azepin-2-amine*

According to Scheme 3, step 3: a mixture of *N*-(5-fluoropyrimidin-2-yl)-8-(4-methoxybenzyl)-6-(3-morpholinopropyl)-4,5,6,8-tetrahydropyrazolo[3,4-*b*]thiazolo[4,5-*d*]azepin-2-amine (240 mg, 436 μmol) and triisopropylsilane (1.21 mL, 5.90 mmol) in TFA (3.9 mL) was heated at 65°C for 3 h. The crude mixture was concentrated to dryness and the residue was taken up in 10 mL of EtOAc. The resulting organic mixture was quenched with water and neutralized with 10 mL of a saturated $NaHCO_3$ aqueous solution. The aqueous layer was then extracted several times with EtOAc. The combined organic layers were dried over $MgSO_4$, filtered and concentrated under reduced pressure. The crude product was purified by flash column chromatography on silica gel using DCM/MeOH (100:0 to 95:5 (+1% $NEt_3$) as eluent. Recrystallization in DMF (110-120°C) afforded the title compound (235 mg, 57%) as a light yellow powder.
Mp: 251-254°C;UPLC-MS (M1); RT= 4.27min; MS m/z $ES^+$= 431.3; $^1$H -NMR (300MHz; DMSO-$d_6$) δ: 11.82 (1H, s), 11.64 (1H, s), 8.69 (2H, d), 7.66 (1H, s), 3.58 (4H,s), 3.30 (2H, s), 3.01 (2H, t), 2.33 (6H, d), 1.79 (2H, d), 1.17 (1H, t).
The hydrochloride salt of Compound 1-140 was prepared according to the experimental procedure herebelow:

**N-(5-Fluoropyrimidin-2-yl)-6-(3-morpholinopropyl)-4,5,6,8-tetrahydropyrazolo [3,4-*b*]thiazolo[4,5-*d*]azepin-2-amine dihydrochloride dihydrate (Compound 1-140.2HCl.2$H_2O$)**

A mixture of *N*-(5-fluoropyrimidin-2-yl)-6-(3-morpholinopropyl)-4,5,6,8-tetrahydropyrazolo[3,4-*b*]thiazolo[4,5-*d*] azepin-2-amine (0.70 g, 1.63 mmol) and a 4N HCl solution in dioxane (5 mL, 1.63 mmol) was refluxed for 0.5 h. The precipitate was filtered off and rinsed with $Et_2O$. After a trituration from boiling iPrOH (85°C, 30 min), the solid was recovered, rinsed with $Et_2O$ and dried under high vacuum pressure to afford the title compound (58%) as a light grey powder.
Mp: 257-259°C; UPLC-MS (M1); RT= 0.6 min; MS m/z $ES^+$= 431.3; $^1$H-NMR (300MHz; DMSO-$d_6$) δ: 10.71 (1H, s), 8.71 (2H, d), 7.79 (1H, s), 3.94 (2H, s), 3.86-3.65 (2H, m), 3.56-3.27 (4H, m), 3.27-2.90 (4H, m), 2.07 (2H, d).
The compounds in the following Table have been synthezised according to the same methods as previous Examples 1 to 13, as denoted in the column denoted as "Exp. nr". The compounds denoted with the asterisk have been exemplified in the Examples.

**<u>Table 1:</u> Compounds prepared according to the Examples (Co.nr. 1-1 to 1-123 are compounds of the invention)**

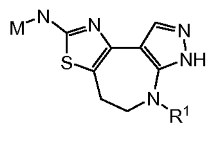

| Co.nr. | Exp nr. | M | $R^1$ |
|---|---|---|---|
| **1-1** | 1* | 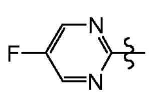 | 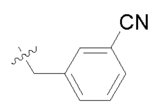 |

(continued)

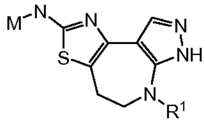

| Co.nr. | Exp nr. | M | $R^1$ |
|---|---|---|---|
| **1-2** | 1 | 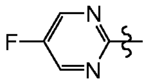 | 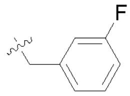 |
| **1-3** | 1 | 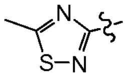 | 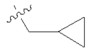 |
| **1-4** | 1 | 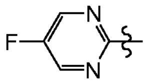 | 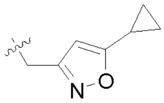 |
| **1-5** | 1 | 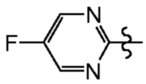 | 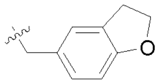 |
| **1-6** | 1 | 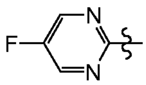 | 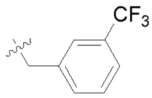 |
| **1-7** | 1 | 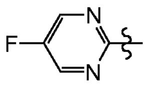 | 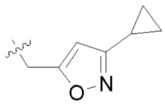 |
| **1-8** | 1 | 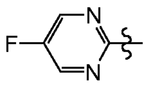 | 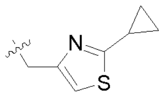 |
| **1-9** | 1 | 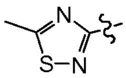 | 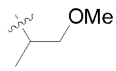 |
| **1-10** | 1 | 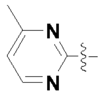 | 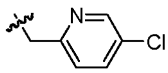 |
| **1-11** | 1 | 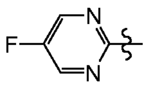 | 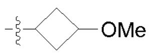 |
| **1-12** | 1 | 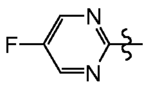 | 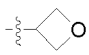 |
| **1-13** | 1 | 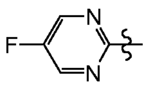 | 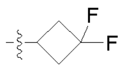 |
| **1-14** | 3 | 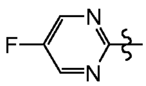 | 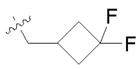 |

(continued)

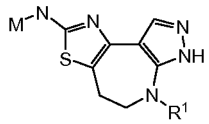

| Co.nr. | Exp nr. | M | R¹ |
|---|---|---|---|
| **1-15** | - | 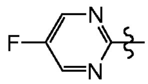 | 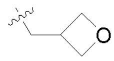 |
| **1-16** | - | 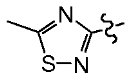 | 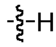 |
| **1-17** | - | 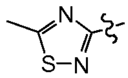 | 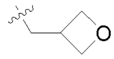 |
| **1-18** | - | 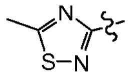 | 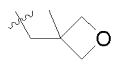 |
| **1-19** | - | 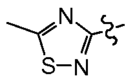 | 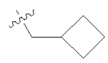 |
| **1-20** | - | 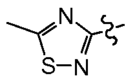 | 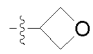 |
| **1-21** | - | 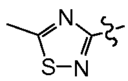 | 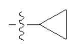 |
| **1-22** | - | 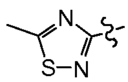 | 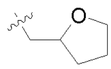 |
| **1-23** | - | 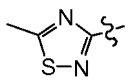 | 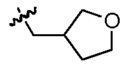 |
| **1-24** | - | 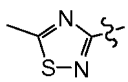 | 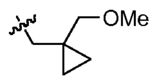 |
| **1-25** | - | 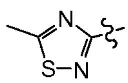 | 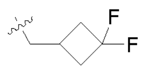 |
| **1-26** | - | 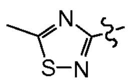 | 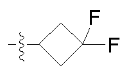 |
| **1-27** | - | 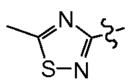 | 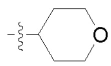 |
| **1-28** | - | 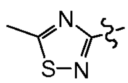 | 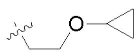 |
| **1-29** | - | 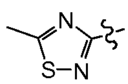 | 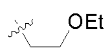 |

(continued)

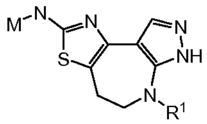

| Co.nr. | Exp nr. | M | R1 |
|---|---|---|---|
| **1-30** | - | 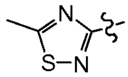 | 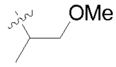 |
| **1-31** | - | 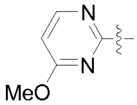 | 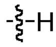 |
| **1-32** | - | 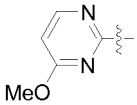 | 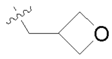 |
| **1-33** | - | 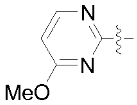 | 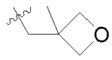 |
| **1-34** | - | 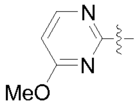 | 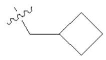 |
| **1-35** | - | 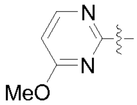 | 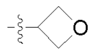 |
| **1-36** | - | 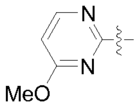 | 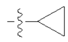 |
| **1-37** | - | 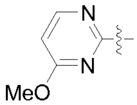 | 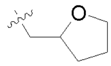 |
| **1-38** | - | 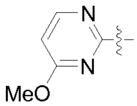 | 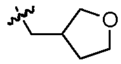 |
| **1-39** | - | 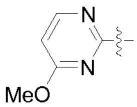 | 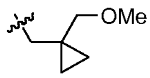 |
| **1-40** | - | 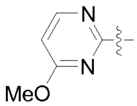 | 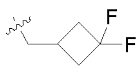 |
| **1-41** | - | 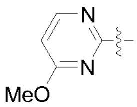 | 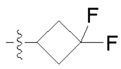 |

(continued)

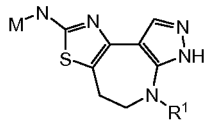

| Co.nr. | Exp nr. | M | $R^1$ |
|---|---|---|---|
| **1-42** | - | 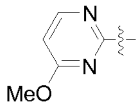 | 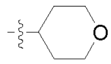 |
| **1-43** | - | 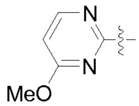 | 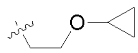 |
| **1-44** | - | 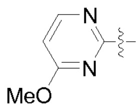 | 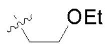 |
| **1-45** | - | 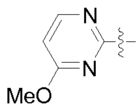 | 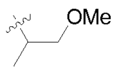 |
| **1-46** | - | 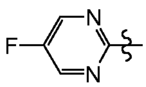 | 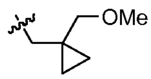 |
| **1-48** | - | 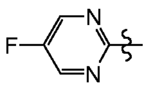 | 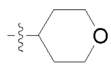 |
| **1-49** | - | 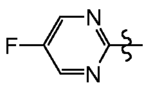 | 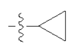 |
| **1-50** | - | 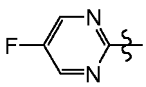 | 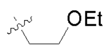 |
| **1-51** | - | 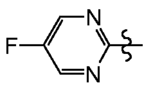 | 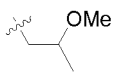 |
| **1-52** | **1** | 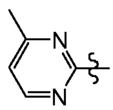 | 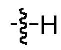 |
| **1-53** | - | 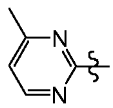 | 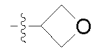 |
| **1-54** | - | 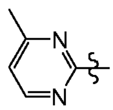 | 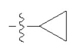 |

(continued)

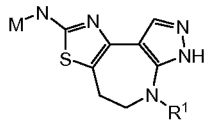

| Co.nr. | Exp nr. | M | R1 |
|---|---|---|---|
| **1-55** | - | 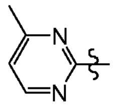 | 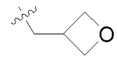 |
| **1-56** | - | 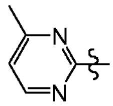 | 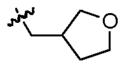 |
| **1-57** | - | 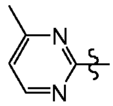 | 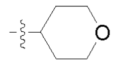 |
| **1-58** | - | 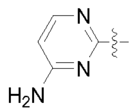 | 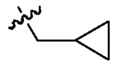 |
| **1-59** | - | 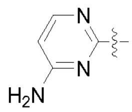 | 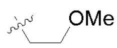 |
| **1-60** | - | 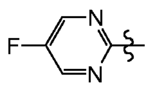 | 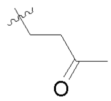 |
| **1-61** | - | 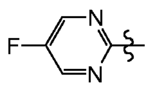 | 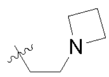 |
| **1-62** | - | 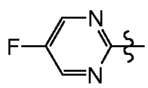 | 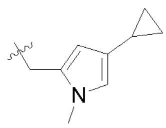 |
| **1-63** | - | 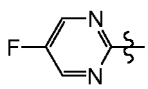 | 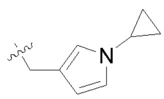 |
| **1-64** | - | 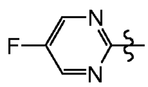 | 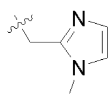 |
| **1-65** | - | 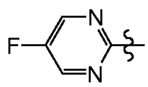 | 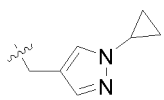 |
| **1-66** | - | 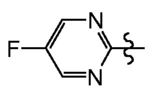 | 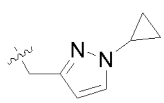 |

(continued)

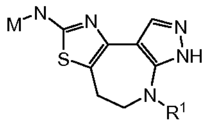

| Co.nr. | Exp nr. | M | R1 |
|---|---|---|---|
| **1-67** | - | 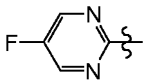 | 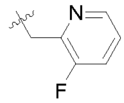 |
| **1-68** | - | 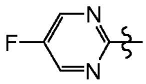 | 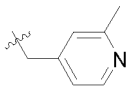 |
| **1-69** | - | 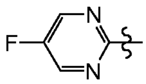 | 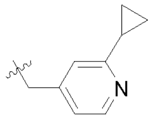 |
| **1-70** | - | 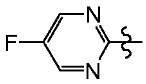 | 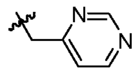 |
| **1-71** | - | 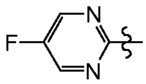 | 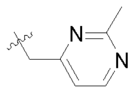 |
| **1-72** | - | 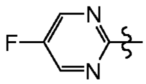 | 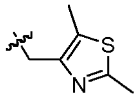 |
| **1-73** | - | 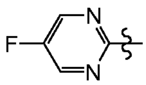 | 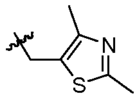 |
| **1-74** | - | 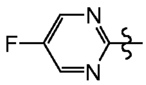 | 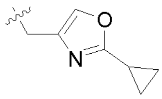 |
| **1-75** | - | 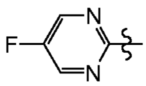 | 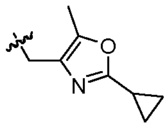 |
| **1-76** | - | 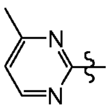 | 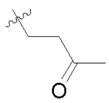 |
| **1-77** | - | 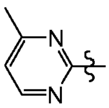 | 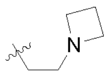 |
| **1-78** | - | 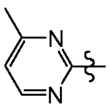 | 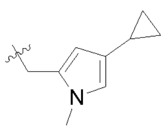 |

(continued)

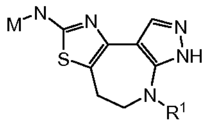

| Co.nr. | Exp nr. | M | R1 |
|---|---|---|---|
| **1-79** | - | 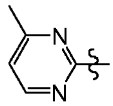 | 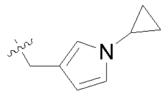 |
| **1-80** | - | 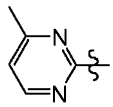 | 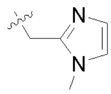 |
| **1-81** | - | 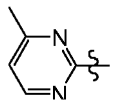 | 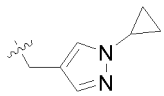 |
| **1-82** | - | 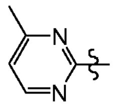 | 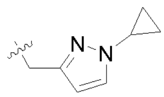 |
| **1-83** | - | 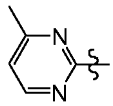 | 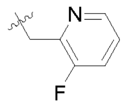 |
| **1-84** | - | 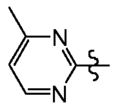 | 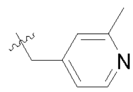 |
| **1-85** | - | 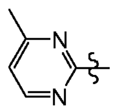 | 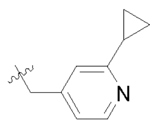 |
| **1-86** | - | 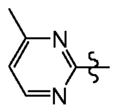 | 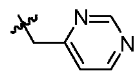 |
| **1-87** | - | 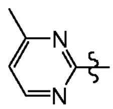 | 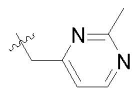 |
| **1-88** | - | 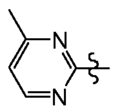 | 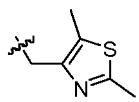 |
| **1-89** | - | 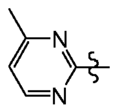 | 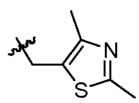 |
| **1-90** | - | 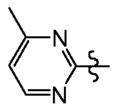 | 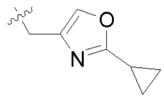 |

(continued)

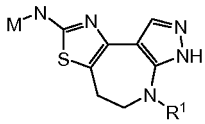

| Co.nr. | Exp nr. | M | R1 |
|---|---|---|---|
| **1-91** | - | 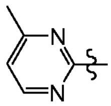 | 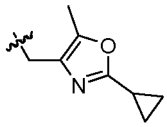 |
| **1-92** | - | 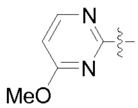 | 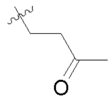 |
| **1-93** | - | 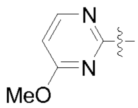 | 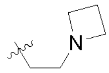 |
| **1-94** | - | 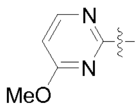 | 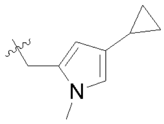 |
| **1-95** | - | 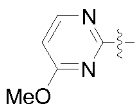 | 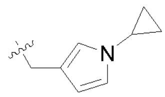 |
| **1-96** | - | 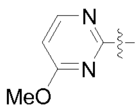 | 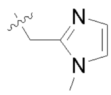 |
| **1-97** | - | 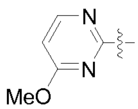 | 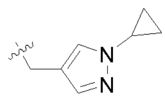 |
| **1-98** | - | 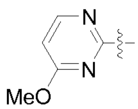 | 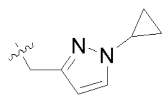 |
| **1-99** | - | 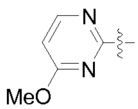 | 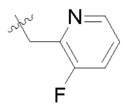 |
| **1-100** | - | 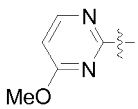 | 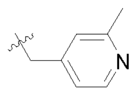 |
| **1-101** | - | 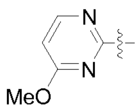 | 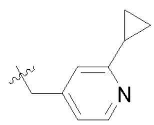 |

(continued)

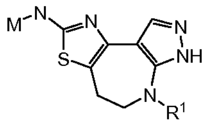

| Co.nr. | Exp nr. | M | R1 |
|---|---|---|---|
| 1-102 | - | 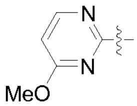 | 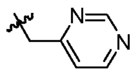 |
| 1-103 | - | 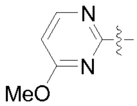 | 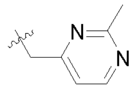 |
| 1-104 | - | 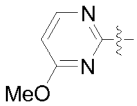 | 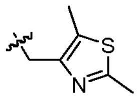 |
| 1-105 | - | 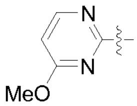 | 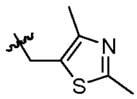 |
| 1-106 | - | 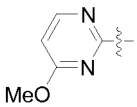 | 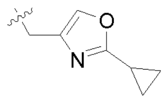 |
| 1-107 | - | 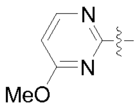 | 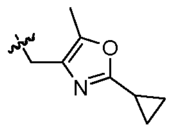 |
| 1-108 | - | 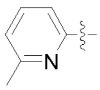 | 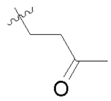 |
| 1-109 | - | 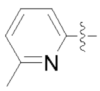 | 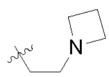 |
| 1-110 | - | 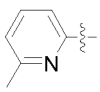 | 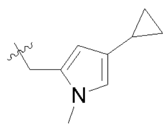 |
| 1-111 | - | 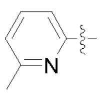 | 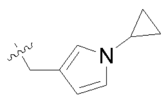 |
| 1-112 | - | 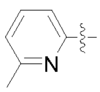 | 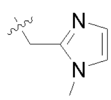 |
| 1-113 | - | 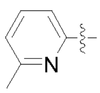 | 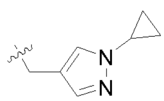 |

(continued)

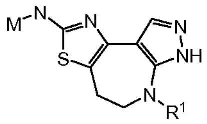

| Co.nr. | Exp nr. | M | R1 |
|---|---|---|---|
| **1-114** | - | 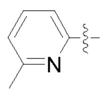 | 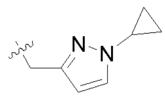 |
| **1-115** | - | 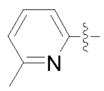 | 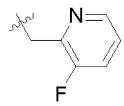 |
| **1-116** | - | 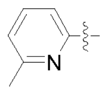 | 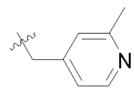 |
| **1-117** | - | 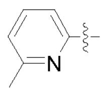 | 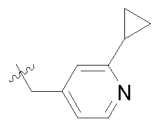 |
| **1-118** | - | 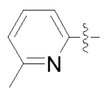 | 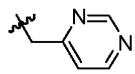 |
| **1-119** | - | 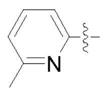 | 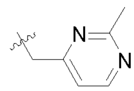 |
| **1-120** | - | 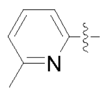 | 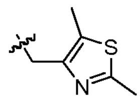 |
| **1-121** | - | 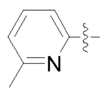 | 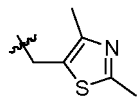 |
| **1-122** | - | 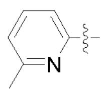 | 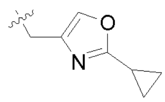 |
| **1-123** | - | 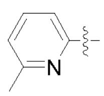 | 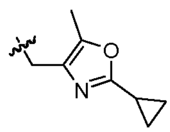 |
| **1-124** | **2** | 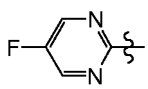 | 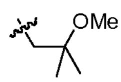 |
| **1-125** | **2** | 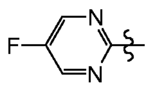 | 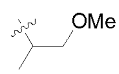 |

(continued)

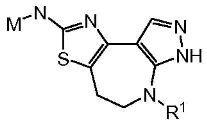

| Co.nr. | Exp nr. | M | $R^1$ |
|---|---|---|---|
| **1-126** | **2** | 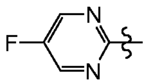 | 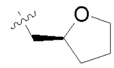 |
| **1-127** | **2** | 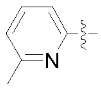 | 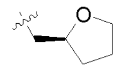 |
| **1-128** | **3** | 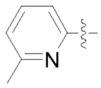 | 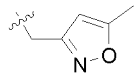 |
| **1-129** | **1** | 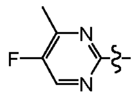 | 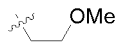 |
| **1-130** | **3** | 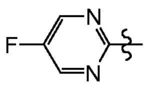 | 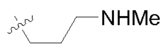 |
| **1-131** | **2*** | 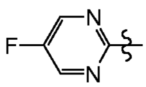 | 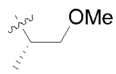 |
| **1-132** | **3** | 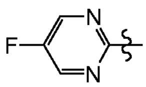 | 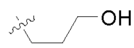 |
| **1-133** | **3** | 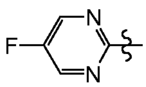 | 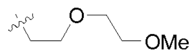 |
| **1-134** | **3** | 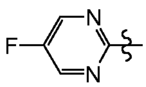 | 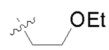 |
| **1-135** | **3*** | 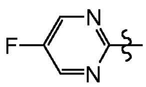 | 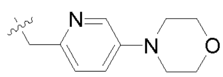 |
| **1-136** | **4*** | 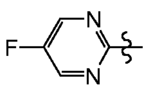 | 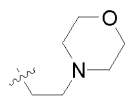 |
| **1-137** | **3** | 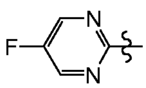 | 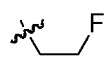 |
| **1-138** | **5*** | 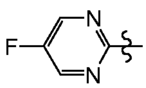 | 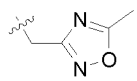 |
| **1-139** | **3** | 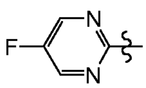 | 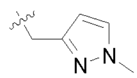 |

(continued)

| 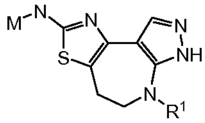 | | | |
|---|---|---|---|
| **Co.nr.** | **Exp nr.** | **M** | **$R^1$** |
| **1-140** | **3*** | 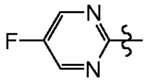 | 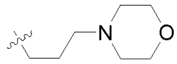 |
| **1-141** | **1** | 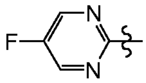 | 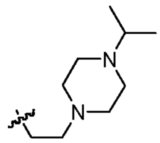 |
| **1-142** | **2** | 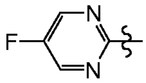 | 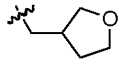 |
| **1-143** | **3** | 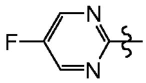 | 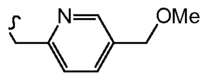 |
| **1-144** | **3** | 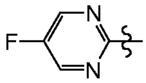 | 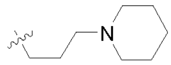 |
| **1-145** | **1** | 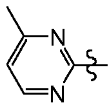 | 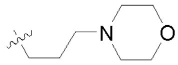 |
| **1-146** | **1** | 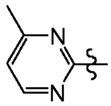 | 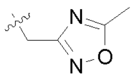 |
| **1-147** | **3** | 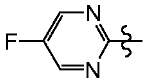 | 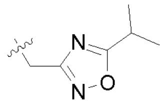 |
| **1-148** | **10*** | 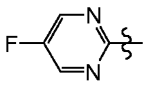 | 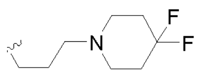 |
| **1-149** | **3** | 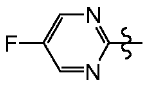 | 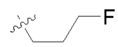 |
| **1-150** | **11*** | 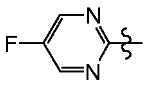 | 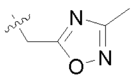 |
| **1-151** | **12*** | 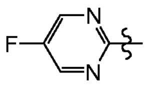 | 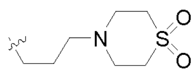 |
| **1-152** | **1** | 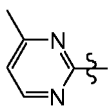 | 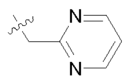 |

(continued)

| 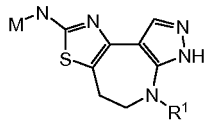 | | | |
|---|---|---|---|
| **Co.nr.** | **Exp nr.** | **M** | **$R^1$** |
| **1-153** | **3** | 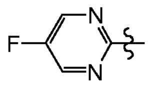 | 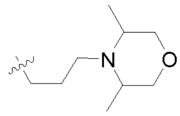 |

UPLC-MS method 1 (M1):

UPLC-MS were recorded on Waters ACQUITY UPLC with the following conditions: Reversed phase HPLC was carried out on BEH-$C_{18}$ cartridge (1.7 μm, 2.1 x 50 mm) from Waters, with a flow rate of 0.8 mL/min. The gradient conditions used are: 90 % A (water + 0.1 % of formic acid), 10% B (ACN + 0.1 % of formic acid) to 100 % B at 1.3 minutes, kept till 1.6 minutes and equilibrated to initial conditions at 1.7 minutes until 2.0 minutes. Injection volume 5 μL. ES MS detector was used, acquiring both in positive and negative ionization modes.

UPLC-MS method 2 (M2):

UPLC-MS were recorded on Perkin Elmer Series 200 autosampler and pump, Perkin Elmer PE785A UV detector, API150EX spectrometer (ES+/-). Agilent Poroshell 120 SB-$C_{18}$ column (4.6mm x 30mm, 2.7 micron).
Run Conditions:

Injection Volume 10 μl
HPLC grade water (containing 0.1% formic acid)
HPLC grade ACN(containing 0.1% formic acid)

| Total Time (min) | Flow Rate (ml/min) | % Aqueous | % ACN |
|---|---|---|---|
| 1.0 | 1.8 | 95 | 5 |
| 2.0 | 1.8 | 0 | 100 |
| 4.0 | 1.8 | 0 | 100 |
| 5.5 | 1.8 | 95 | 5 |

All mass spectra were taken under electrospray ionisation (ESI) methods.

LC-MS method 3 (M3):

UPLC-MS were recorded on Column 1 -EXTEND Zorbax SB-$C_{18}$, 1.8μm, 4.6 X 30mm Column and LC-MS analysis were recorded on a Waters Micromass ZQ 2996 system with the following conditions: reversed phase HPLC was carried out on an Zorbax SB-$C_{18}$ cartridge (1.8 μm, 4.6 x 30 mm) from Agilent, with a flow rate of 1.5 mL/min. The gradient conditions used are: 90 % A (water + 0.05 % of formic acid), 10% B (ACN + 0.05 % of formic acid) to 100 % B at 3.5 min, kept till 3.7 min and equilibrated to initial conditions at 3.8 min until 4.5 min. Injection volume 5-20 μL. ES MS detector was used, acquiring both in positive and negative ionization modes. Cone voltage was 30 V for both positive and negative ionization modes.

**Table 2: Physico-chemical data for some compounds (nd = not determined).**

| Co.Nr | Mp (°C) | [MH+] | RT (min) Method | 1H-NMR data |
|---|---|---|---|---|
| **1-1*** | 282-284 | 419.0 | 2.93 M2 | (400MHz, DMSO-$d_6$) δ: 11.95 (1H, s), 11.69 (1H,s), 8.72 (2H, d), 7.84 (1H,s), 7.76-7.30 (3H, m), 7.57 (1H, t), 4.65 (2H, s), 3.05-3.03 (2H, m) |
| **1-2** | 265-270 | 411.9 | 3.05 M2 | (400MHz, DMSO-$d_6$) δ: 11.86 (1H, s), 11.61 (1H,s), 8.64 (2H, d), 7.67 (1H,s), 7.34-7.29 (1H, m), 7.17-7.11 (2H, m), 7.03-6.99 (1H, m), 4.54 (2H, s), 2.95-2.90 (2H, m) |
| **1-3** | nd | 360.4 | 2.81 M2 | (400MHz, DMSO-$d_6$) δ: 7.61 (1H, s), 3.35 (2H, m), 3.15 (2H, d), 2.94 (2H, m) 2.67 (3H, s), 1.07 (1H, m), 0.38 (2H, m), 0.16 (2H, m) |
| **1-4** | nd | 425.0 | 2.91 M2 | (400MHz, DMSO-$d_6$) δ: 8.70-8.53 (2H, br m), 7.65 (1H, s), 6.05 (1H, s), 4.45 (2H, s), 3.21 (2H, m), 2.92 (2H, m), 2.03 (1H, m), 0.95 (2H, m), 0.78 (2H, m) |
| **1-7** | 266-268 | 425.1 | 2.91 M2 | (400MHz, DMSO-$d_6$) δ: 11.87-11.62 (1 H, brs), 8.61 (2H, s), 7.63 (1H, s), 6.01 (1H, s), 4.55 (2H, s), 3.30 (2H, m), 2.96 (2H, m), 1.88 (1H, m), 0.89 (2H, m), 0.65 (2H, m) |
| **1-8** | 240-245 | 441.1 | 2.95 M2 | (400MHz,$CD_3OD$) δ: 8.22 (2H, s), 7.60 (1H, s), 6.75 (1H, s), 4.40 (2H, s), 3.23-3.17 (2H, m), 2.85-2.79 (2H, m), 2.12-2.05 (1H, m), 0.95-0.90 (2H, m), 0.78-0.73 (2H, m) |
| **1-10** | 165-168 | 427 | 2.88 M2 | (400MHz, DMSO-$d_6$) δ: 11.36 (1H, br s), 8.46 (1H, d), 8.32 (1H, d), 7.76 (1H, dd), 7.61 (1H, s), 7.31 (1H, d), 6.75 (1H, s), 4.58 (2H, s), 3.35-3.33 (2H, m), 2.96-2.93 (2H, m), 2.32 (3H, s) |
| **1-14** | >280 | 408.2 | 0.97 M1 | (300MHz, DMSO-$d_6$) δ: 11.9 (1H, s), 11.65 (1H, s), 8.7 (2H, s), 7.7 (1H, s), 3.4-3.45 (2H, m), 3.3-3.38 (4H, m), 2.95-3.05 (2H, m), 2.55-2.65 (3H, m) |
| **1-52** | 229-232 | 300.2 | 0.62 M1 | (300 MHz, DMSO-$d_6$) δ: 11.34 (1H, s), 8.42 (1H, d), 7.54 (1H, s), 6.86 (1H, d), 3.27 (2H, d), 2.96 (2H, d), 2.41 (3H, s) |
| **1-124** | 282-285 | 390.2 | 0.89 M1 | (300MHz, DMSO-$d_6$) δ: 11.60 (1H, s), 8.70 (2H, s), 7.72 (1H, s), 3.50-3.40 (4H, m), 3.20 (3H, s), 3.05-3.00 (2H, m), 1.10 (6H, s) |
| **1-125** | 212-215 | 376.2 | 0.85 M1 | (300MHz, DMSO-$d_6$) δ: 11.60 (1H, s), 8.70 (2H, s), 7.72 (1H, s), 4.22-4.18 (2H, m), 3.60-3.50 (1H, m), 3.40-3.35 (2H, m), 3.22 (3H, s), 3.00-2.95 (2H, m), 1.15 (3H, d) |
| **1-126** | nd | 388.2 | 0.84 M1 | (300MHz, DMSO-$d_6$) δ: 11.60 (1H, s), 8.70 (2H, s), 7.72 (1H, s), 4.20-4.15 (1H, m), 3.80 (2H, q), 3.65 (1H, q), 3.55-3.40 (2H, m), 3.35 (1H, dd), 3.05-3.00 (2H, m), 1.90-1.75 (3H, m), 1.55-1.45 (1H, m) |
| **1-127** | 229-231 | 383.2 | 0.77 M1 | (300MHz, DMSO-$d_6$) δ: 11.20 (1H, s), 7.75 (1H, s), 7.60 (1H,dd), 6.85 (1H,d), 6.75 (1H, d), 4.20-4.15 (1H, m), 3.80 (1H, q), 3.65 (1H, q), 3.55-3.40 (3H, m), 3.35 (1H, dd), 3.05-3.00 (2H, m), 2.50 (3H,s), 1.90-1.75 (3H, m), 1.55-1.45 (1H, m) |
| **1-128** | 234-237 | 394.2 | 0.79 M1 | (300MHz, DMSO-$d_6$) δ: 11.95 (1H, s), 11.05 (1H, s), 7.70 (1H, s), 7.55 (1H, dd), 6.82 (1H, d), 6.72 (1H, d), 6.12 (1H, s), 4.55 (2H, s), 3.30-3.25 (2H, m), 3.05-3.00 (2H, m), 2.40 (3H, s), 2.35 (3H, s) |
| **1-129** | 261-263 | 376.4 | 0.85 M1 | (300 MHz, DMSO-$d_6$) δ: d 11.82 (1H, s), 11.49 (1H, s), 8.52 (1H, d), 7.67 (1H, s), 3.60-3.50 (4H, m), 3.43-3.35 (2H, m), 3.26 (3H, s), 3.05-2.94 (2H, m), 2.44 (3H, d) |
| **1-130** | nd | 375.0 | 0.61 M1 | (300MHz, DMSO-$d_6$+ 0.1% TFA-d) δ: 8.84 (2H, s), 8.15 (1H, s), 3.61-3.39 (4H, m), 3.22-3.07 (2H, m), 3.04-2.86 (2H, m), 2.62 (3H, s), 2.07-1.83 (2H, m) |

(continued)

| Co.Nr | Mp (°C) | [MH+] | RT (min) Method | $^1$H-NMR data |
|---|---|---|---|---|
| **1-131** | 249-251 | 376.2 | 0.85 M1 | (300 MHz, DMSO-$d_6$) δ: 11.80 (1H, s), 11.63 (1H, s), 8.69 (2H, s), 7.68 (1H, s), 4.24 (1H, s), 3.53 (1H, dd), 3.38 (1H, dd), 3.25 (5H, m), 2.95 (2H, d), 1.15 (3H, d) |
| **1-132** | nd | 362.2 | 0.70 M1 | (300MHz, DMSO-$d_6$) δ: 11.64 (1H, brs), 8.69 (2H, s), 7.67 (1H, s), 3.47-3.30 (6H, m), 3.02-2.99 (2H, m), 1.77-1.72 (2H, m) |
| **1-133** | >220 | 406.3 | 0.77 M1 | (300 MHz, DMSO-$d_6$) δ: 11.83 (1H, s), 11.63 (1H, s), 8.69 (2H, s), 7.67 (1H, s), 3.63 (2H, br t), 3.52 (4H, m), 3.47-3.36 (4H, m), 3.24 (3H, s), 3.00 (2H, br t) |
| **1-134** | 246 | 376.2 | 1.70 M3 | (300 MHz, DMSO-$d_6$) δ: 11.83 (1H, s), 11.64 (1H, s), 8.69 (2H, d), 7.67 (1H, s), 3.60 (2H, t), 3.53 (1H, d), 3.48-3.36 (4H, m), 3.00 (2H, dd), 1.11 (3H, t) |
| **1-135** | nd | 480.2 | 0.66 M1 | (300 MHz, DMSO-$d_6$) δ: 11.86 (1H, bs), 8.69 (2H, s), 8.22 (1H, d), 7.70 (1H, s), 7.44-7.08 (2H, m), 4.56 (2H, s), 3.74 (4H, t), 3.13 (4H, t), 3.05-2.89 (2H, m), 2.62 (2H, d) |
| **1-136** | nd | 417.3 | 0.57 M1 | (300 MHz, DMSO-$d_6$) δ: 11.82 (1H, bs), 11.63 (1H, bs), 8.69 (2H, d,), 7.66 (1H, s), 3.60-3.53 (4H, m), 3.50-3.42 (2H, m), 3.42-3.35 (2H, m), 3.02-2.97 (2H, dd,), 2.60-2.53 (2H, m), 2.44-2.40 (4H, m) |
| **1-137** | 283-286 | 350.2 | 0.81 M1 | (300 MHz, DMSO-$d_6$) δ: 11.90 (1H, s), 11.65 (1H, s), 8.70 (2H, s), 7.70 (1H, s), 4.74 (1H, m), 4.58 (1H, m), 3.77-3.58 (2H, m), 3.43 (2H, m), 3.02 (2H, m) |
| **1-138** | 245 | 400.2 | 0.80 M1 | (300MHz, DMSO-$d_6$) δ: 11.60 (1H, s), 8.70 (2H, s), 7.70 (1H, s), 4.65 (2H, s), 3.55-3.45 (2H, m), 3.10-3.10 (2H, m), 2.55 (3H, s) |
| **1-139** | 195-200 | 398.2 | 0.76 M1 | (300MHz, DMSO-$d_6$) δ: 8.69 (2H, s), 7.73 (1H, s), 7.59 (1H, d), 6.13 (1H, d), 4.45 (2H, s), 3.79 (3H, s), 3.25-3.22 (2H, m), 2.95-2.92 (2H, m) |
| **1-140** | 232 | 431.1 | 1.13 M3 | (300 MHz, DMSO-$d_6$) δ: 11.82 (1H, s), 11.64 (1H, s), 8.69 (2H, d), 7.66 (1H, s), 3.58 (4H,s), 3.30 (2H, s), 3.01 (2H, t), 2.33 (6H, d), 1.79 (2H, d), 1.17 (1H, t) |
| **1-141** | nd | 458.0 | 0.59 M1 | (300MHz, DMSO-$d_6$+ 10% TFA-d) δ: 8.84 (2H, s), 8.07 (1H, s), 4.15-3.92 (2H, m), 3.87-3.33 (13H, m), 3.24-3.06 (2H, m), 1.32 (6H, d) |
| **1-142** | 266-267 | 376.2 | 0.82 M1 | (300MHz, DMSO-$d_6$) δ: 11.82 (1H, brs), 11.64 (1H, brs), 8.69 (2H, s), 7.67 (1H, s), 3.39-3.30 (6H, m), 3.23 (3H, s), 3.02-2.99 (2H, m), 1.88-1.79 (2H, m) |
| **1-143** | 198-202 | 439.3 | 0.70 M1 | (300MHz, DMSO-$d_6$) δ: 8.70 (2H, s), 8.51 (1H, s), 7.84 (1H, d), 7.72 (1H, s), 7.51 (1H, d), 4.72 (2H, s), 4.46 (2H, s), 3.46-3.43 (2H, m), 3.31 (3H, s), 3.07-3.04 (2H, m) |
| **1-144** | 227 | 429.3 | 0.66 M1 | (300 MHz, DMSO-$d_6$) δ: 11.93-11.70 (1H, bs), 11.54 (1H, s), 8.69 (2H, s), 7.65 (1H, s), 3.00 (2H, t), 2.28 (5H, dd), 1.75 (2H, t), 1.49 (4H, q), 1.45-1.27 (2H, m) +5H under DMSO and water peaks. |
| **1-145** | 251 | 427.4 | 0.61 M1 | (300 MHz, DMSO-$d_6$) δ: 11.78 (1H, s), 11.57-10.91 (1H, m), 8.42 (1H, d), 7.65 (1H, s), 6.87 (1H, d), 3.57 (4H, t), 3.22-3.08 (1H, m), 3.08-2.90 (2H, m), 2.42 (3H, s), 2.34 (4H, q), 1.78 (2H, q) +5H under DMSO and water peaks. |
| **1-146** | nd | 396.2 | 0.77 M1 | (300MHz, DMSO-$d_6$) δ: 11.85 (1H, s), 11.40 (1H, s), 8.40 (1H, d), 7.70 (1H, s), 6.90 (1H, d), 4.65 (2H, s), 3.52-3.45 (2H, m), 3.10-3.0 (2H, m), 2.55 (3H, s), 2.40 (3H, s). |

(continued)

| Co.Nr | Mp (°C) | [MH+] | RT (min) Method | $^1$H-NMR data |
|---|---|---|---|---|
| **1-147** | >260 | 428.3 | 0.94 M1 | (300MHz, DMSO-$d_6$) δ: 11.60 (1H, s), 8.70 (2H, s), 7.70 (1H, s), 4.70 (2H, s), 3.55-3.45 (2H, m), 3.25 (1H, q), 3.10-3.00 (2H, m), 1.30 (6H, d). |
| **1-148** | 238-240 | 465.3 | 0.67 M1 | (300 MHz, DMSO-$d_6$) δ: 11.80 (1H, d), 11.64 (1H, s), 8.69 (2H, s), 7.65 (1H, s), 3.40-3.25 (4H, m), 3.14-2.87 (2H, m), 2.65-2.40 (2H, m) 2.39 (4H, d), 1.94 (4H, dt), 1.77 (2H, t). |
| **1-149** | >290 | 364.2 | 0.84 M1 | (300 MHz, DMSO-$d_6$) δ: 11.84 (1H, s), 11.64 (1H, s), 8.70 (2H, d), 7.68 (1H, s), 4.60 (1H, t), 4.43 (1H, d), 3.45 (2H, dd), 3.02 (2H, t), 2.01 (2H, dq). |
| **1-150** | nd | 400.1 | 0.81 M1 | (300 MHz, DMSO-$d_6$) δ: 11.91(1H,s), 11.67 (1H, s), 8.70 (2H, s), 7.69 (1H, s), 4.83 (2H, s), 3.65-3.45 (2H, m), 3.15-3.00 (2H, m), 2.30 (3H, s). |
| **1-151** | 272-273 | 479.2 | 0.63 M1 | (300 MHz, DMSO-$d_6$) δ: 11.81 (1H, s), 11.62 (1H, s), 8.69 (2H, s), 7.87-7.44 (1H, m), 3.46-3.33 (4H, m), 3.08 (4H, t), 3.01 (2H, t), 2.87 (4H, t), 1.77 (2H, dd) +2H under water peak. |
| **1-152** | nd | 392.2 | 0.70 M1 | (300 MHz, DMSO-$d_6$) δ: 11.38 (s, 1H), 8.72 (d, 2H), 8.43 (d, 1H), 7.66 (s, 1H), 7.33 (t, 1H), 6.88 (d, 1H), 4.79 (s, 2H), 3.65 (s, 2H), 3.07 (d, 2H), 2.43 (s, 3H). |
| **1-153** | 223 | 459.3 | 0.63 M1 | (300 MHz, DMSO-$d_6$) δ: 11.78 (1H, s), 11.63 (1H, s), 8.69 (2H, s), 7.66 (1H, s), 3.57 (2H, dd), 3.40-3.20 (4H, m), 3.07-2.90 (4H, m), 2.77-2.61 (2H, m), 1.68 (2H, t), 0.88 (6H, d). |

PHARMACOLOGY

The compounds provided in the present invention are positive allosteric modulators of $mGluR_4$. As such, these compounds do not appear to bind to the orthosteric glutamate recognition site, and do not activate the $mGluR_4$ by themselves. Instead, the response of $mGluR_4$ to a concentration of glutamate or $mGluR_4$ agonist is increased when compounds of Formula (I) to (III) are present. Compounds of Formula (I) to (III) are expected to have their effect at $mGluR_4$ by virtue of their ability to enhance the function of the receptor.

**$mGluR_4$ assay on HEK-expressing human $mGluR_4$**

The compounds of the present invention are positive allosteric modulators of $mGluR_4$ receptor. Their activity was examined on recombinant human $mGluR_{4a}$ receptors by detecting changes in intracellular $Ca^{2+}$ concentration, using the fluorescent $Ca^{2+}$-sensitive dye Fluo4-(AM) and a Fluorometric Imaging Plate Reader (FLIPR, Molecular Devices, Sunnyvale, CA).

**Transfection and Cell culture**

The cDNA encoding the human metabotropic glutamate receptor ($hmGluR_4$), (accession number NM_000841.1, NCBI Nucleotide database browser), was subcloned into an expression vector containing also the hygromycin resistance gene. In parallel, the cDNA encoding a G protein allowing redirection of the activation signal to intracellular calcium flux was subcloned into a different expression vector containing also the puromycin resistance gene. Transfection of both these vectors into HEK293 cells with PolyFect reagent (Qiagen) according to supplier's protocol, and hygromycin and puromycin treatment allowed selection of antibiotic resistant cells which had integrated stably one or more copies of the plasmids. Positive cellular clones expressing $hmGluR_4$ were identified in a functional assay measuring changes in calcium fluxes in response to glutamate or selective known $mGluR_4$ orthosteric agonists and antagonists.

HEK-293 cells expressing $hmGluR_4$ were maintained in media containing DMEM, dialyzed Fetal Calf Serum (10 %), Glutamax™ (2 mM), Penicillin (100 units/mL), Streptomycin (100 μg/mL), Geneticin (100 μg/mL) and Hygromycin-B (40 μg/mL) and puromycin (1 μg/mL) at 37°C/5%$CO_2$.

**Fluorescent cell based- $Ca^{2+}$ mobilization assay**

Human $mGluR_4$ HEK-293 cells were plated out 24 hours prior to $FLIPR^{384}$ assay in black-walled, clear-bottomed, poly-L-ornithine-coated 384-well plates at a density of 25,000 cells/well in a glutamine/glutamate free DMEM medium containing foetal bovine serum (10 %), penicillin (100 units/mL) and streptomycin (100 $\mu$g/mL) at 37°C/5 %$CO_2$.

On the day of the assay, the medium was aspirated and the cells were loaded with a 3 $\mu$M solution of Fluo4-AM (LuBioScience, Lucerne, Switzerland) in 0.03 % pluronic acid. After 1 hour at 37°C/ 5% $CO_2$, the non incorporated dye was removed by washing cell plate with the assay buffer and the cells were left in the dark at room temperature for six hours before testing. All assays were performed in a pH 7.4 buffered-solution containing 20 mM HEPES, 143 mM NaCl, 6 mM KCl, 1 mM $MgSO_4$, 1 mM $CaCl_2$, 0.125 mM sulfapyrazone and 0.1 % glucose.

After 10s of basal fluorescence recording, various concentrations of the compounds of the invention were added to the cells. Changes in fluorescence levels were first monitored for 180 s in order to detect any agonist activity of the compounds. Then the cells were stimulated by an $EC_{25}$ glutamate concentration for an additional 110 s in order to measure enhancing activities of the compounds of the invention. $EC_{25}$ glutamate concentration is the concentration giving 25% of the maximal glutamate response.

The concentration-response curves of representative compounds of the present invention were generated using the Prism GraphPad software (Graph Pad Inc, San Diego, USA). The curves were fitted to a four-parameter logistic equation:

$$(Y=Bottom + (Top-Bottom)/(1+10^{\wedge}((LogEC_{50}-X)*Hill\ Slope)$$

allowing the determination of $EC_{50}$ values.

The Table 3 below represents the mean $EC_{50}$ obtained from at least three independent experiments of selected molecules performed in duplicate.

**Table 3: Activity data for selected compounds** (Compound no. 1-124 to 1-153 are reference compounds)

| Compound no. | $Ca^{2+}$ Flux* | Compound no. | $Ca^{2+}$ Flux* |
|---|---|---|---|
| **1-1** | ++ | **1-135** | +++ |
| **1-2** | + | **1-136** | ++ |
| **1-3** | + | **1-137** | +++ |
| **1-4** | +++ | **1-138** | +++ |
| **1-7** | +++ | **1-139** | ++ |
| **1-8** | ++ | **1-140** | +++ |
| **1-10** | +++ | **1-141** | + |
| **1-14** | ++ | **1-142** | +++ |
| **1-52** | +++ | **1-143** | +++ |
| **1-124** | ++ | **1-144** | ++ |
| **1-125** | +++ | **1-145** | +++ |
| **1-126** | +++ | **1-146** | +++ |
| **1-127** | ++ | **1-147** | +++ |
| **1-128** | ++ | **1-148** | ++ |
| **1-129** | +++ | **1-149** | + |
| **1-130** | + | **1-150** | +++ |
| **1-131** | +++ | **1-151** | +++ |
| **1-132** | ++ | **1-152** | ++ |
| **1-133** | +++ | **1-153** | +++ |

(continued)

| Compound no. | $Ca^{2+}$ Flux* | Compound no. | $Ca^{2+}$ Flux* |
|---|---|---|---|
| 1-134 | +++ | | |
| ***Table legend:**<br>(+): 1 μM < $EC_{50}$ <10 μM<br>(++): 100 nM < $EC_{50}$ <1 μM<br>(+++): $EC_{50}$< 100 nM | | | |

The results shown in Table 3 demonstrate that the compounds described in the present invention are positive allosteric modulators of human $mGluR_4$ receptors. These compounds do not have activity by themselves but they rather increase the functional activity and/or maximal efficacy of glutamate or $mGluR_4$ agonist.

**Haloperidol-induced catalepsy model in the rat**

The haloperidol-induced catalepsy is a model of Parkinson's disease. It is used to assess potential anti-parkinsonian action of compound. In this model, haloperidol, a dopamine receptor antagonist, is administered to induce catalepsy, characterized by hypokinesia and rigidity. This state is described as an acute parkinsonian state. Anti-parkinsonian drugs show efficacy in this model by decreasing the catalepsy induced by haloperidol.

**Experimental design and administration procedure:**

One day before the test, Male Sprague-Dawley rats (Charles River, les Oncins, France) were placed in individual cages. The day of the experiment, rats were injected with a dopamine D2 receptor antagonist, haloperidol (1.5 mg/kg, i.p.) 30 minutes prior to oral administration of test compound (1, 3, 10 and 30 mg/kg) or vehicle. L-DOPA-benserazide (150 mg/kg) used as a positive control, was also orally administered 30 min post-haloperidol injection.

**Experimental procedure-Catalepsy test:**

Catalepsy was assessed 60 minutes after test compound or vehicle or MTEP treatments L-DOPA-benserazide using a grid test (e.g. 90 min post-haloperidol administration). Briefly, the rats were placed on a vertical wire grid with the head pointing toward the ceiling and all paws gripping the grid. Latency to movement of both forepaws to relocate the body was measured (in seconds) with a maximum latency "cut-off" time of 120-seconds. Brain and plasma were collected at the end of the experiment for compound exposure assessment.

**Unilateral 6-OHDA lesion treatments**

The effect of test compounds were assessed alone or in combination with L-DOPA in male Sprague-Dawley rats lesioned through medial forebrain bundle (Taconic).

Animals were orally administered with test compounds and then tested 55-65 min post dosing in the forelimb stepping test for akinesia and 65-70 minutes post-dosing in the cylinder test. L-DOPA (2, 6 or 20 mg/kg), used as positive control and in co-therapy were ip injected. Then forelimb akinesia and cylinder tests were carried out 30-45-minutes post-dosing. In co-therapy, rats received test compound 30 minutres prior to L-DOPA and they were tested as described above between 55 and 75 minutes post test compound dosing.

**Forelimb stepping test for akinesia**

Stepping movements made by the isolated ipsi- and contra-lateral forelimbs are assessed. The rat's weight is centered over the isolated limb with its head and forequarters oriented forward by the experimenter. The number of rat-initiated steps that shift weight to a new location are recorded for 30-s.

**Cylinder Test**

Measures spontaneous forelimb use while rats voluntarily explore a cylinder (d: 20-25cm; h: 30 cm) and scored for the number of either ipsi-lateral, contra-lateral (affected limb), or both paw contacts during exploratory movements

Preference scores are calculated for ipsi-, contra-, or both forelimb contacts during a 10-minutes interval for a

minimum of 20 events. For example, a zero score (lack of asymmetry) results from equal number of events for independent ipsi- versus contra-contacts, or simultaneous contacts of both paws.

Blood samples were taken immediately after testing.

## Marble Burying model of anxiety

Anxiety models in rodents are used as standard tests to demonstrate anxiolytic-like properties of novel compounds. Mice exhibit a tendency to bury harmless novel objects when encountered in a test cage. Marble burying behavior in mice is reduced by compounds which are efficacious anxiolytics in humans. Thus, marble burying in mice has been used as a model for the prediction of anxiolytic-like effects of compounds (Millan, M.J. et al.(2002) Neuropharmacology 42:677-684).

Male C57BL6/j mice (20-30 g), 7 weeks of age at the time of delivery were group housed in a temperature and humidity controlled facility on a 12 hour light /dark cycle for at least 5 days before use. Mice had access to food and water ad libitum except during marble burying experiments.

Assessment of marble burying: The effect of compounds on marble burying in mice was tested. On the day of the test, animals were marked on their tails and weighed in a separate preparation room 1 hour before drug administration. Test compound or vehicle was administered p.o. 60 minutes prior to the test session. Marble burying was tested in a separate experimental room. For the test, mice were placed individually into clear plastic cages (16 x 22 x 14 cm) with 5 cm of sawdust and 10 marbles evenly spaced against the walls of the cage. The mice were left undisturbed in the cages for 30 minutes. After removal of the mice from the test cages, the number of marbles buried was counted. A marble was considered buried if it was 2/3 or more covered.

Compound administration: Test compounds were dissolved in a solution of 100% PEG 400. Test compounds were administered by oral gavage (p.o.) in a volume of 10 mL/kg. Compound and vehicle-treated mice received the equivalent volume of vehicle solution p.o. in the absence of added compound.

Statistical analyses: Statistical analyses were performed using GraphPad PRISM version 4.01 statistical software (GraphPad, San Diego, CA, USA). Data were analyzed using one-way analysis of variance (ANOVA) followed by Bonferroni-corrected multiple comparisons, or t tests if only 2 groups were present. The significance level was set at $p<0.05$.

Thus, the positive allosteric modulators provided in the present invention are expected to increase the effectiveness of glutamate or $mGluR_4$ agonists at $mGluR_4$ receptor. Therefore, these positive allosteric modulators are expected to be useful for treatment of various neurological and psychiatric disorders associated with glutamate dysfunction described to be treated herein and others that can be treated by such positive allosteric modulators.

## FORMULATION EXAMPLES

Typical examples of recipes for the formulation of the invention are as follows:

1. Tablets

| | |
|---|---|
| Active ingredient | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

In this Example, active ingredient can be replaced by the same amount of any of the compounds according to the present invention, in particular by the same amount of any of the exemplified compounds.

2. Suspension

An aqueous suspension is prepared for oral administration so that each 1 milliliter contains 1 to 5 mg of one of the active compounds, 50 mg of sodium carboxymethyl cellulose, 1 mg of sodium benzoate, 500 mg of sorbitol and water ad 1 mL.

3. Injectable

A parenteral composition is prepared by stirring 1.5 % by weight of active ingredient of the invention in 10% by volume propylene glycol and water.

4. Ointment

| | |
|---|---|
| Active ingredient | 5 to 1000 mg |
| Stearyl alcohol | 3 g |
| Lanoline | 5 g |
| White petroleum | 15 g |
| Water | ad 100 g |

In this Example, active ingredient can be replaced with the same amount of any of the compounds according to the present invention, in particular by the same amount of any of the exemplified compounds.

Reasonable variations are not to be regarded as a departure from the scope of the invention. It will be obvious that the thus described invention may be varied in many ways by those skilled in the art.

## Claims

**1.** A compound, wherein said compound is a 2-amino-4,5,6,8-tetrahydropyrazolo[3,4-*b*]thiazolo[4,5-*d*]azepine derivative selected from:

3-((2-(5-Fluoropyrimidin-2-ylamino)-4,5-dihydropyrazolo[3,4-*b*]thiazolo[4,5-*d*]azepin-6(7*H*)-yl)methyl)benzonitrile
6-(3-Fluorobenzyl)-*N*-(5-fluoropyrimidin-2-yl)-4,5,6,7-tetrahydropyrazolo[3,4-*b*] thiazolo[4,5-*d*]azepin-2-amine
6-(Cyclopropylmethyl)-*N*-(5-methyl-1,2,4-thiadiazol-3-yl)-4,5,6,7-tetrahydropyrazolo [3,4-*b*]thiazolo[4,5-*d*]azepin-2-amine
6-((5-Cyclopropylisoxazol-3-yl)methyl)-*N*-(5-fluoropyrimidin-2-yl)-4,5,6,7-tetrahydro pyrazolo[3,4-*b*]thiazolo[4,5-*d*]azepin-2-amine

and a pharmaceutically acceptable acid or base addition salt thereof, a stereochemically isomeric form thereof and an N-oxide form thereof; or
wherein said compound is selected from:

6-((2,3-Dihydrobenzofuran-5-yl)methyl)-*N*-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-(3-(trifluoromethyl)benzyl)-4,5,6,7-tetrahydropyrazolo [3,4-*b*]thiazolo[4,5-*d*]azepin-2-amine
6-((3-Cyclopropylisoxazol-5-yl)methyl)-*N*-(5-fluoropyrimidin-2-yl)-4,5,6,7-tetrahydro pyrazolo[3,4-*b*]thiazolo[4,5-*d*]azepin-2-amine
6-((2-Cyclopropylthiazol-4-yl)methyl)-*N*-(5-fluoropyrimidin-2-yl)-4,5,6,7-tetrahydro pyrazolo[3,4-*b*]thiazolo[4,5-*d*]azepin-2-amine
6-(l-Methoxypropan-2-yl)-*N*-(5-methyl-l,2,4-thiadiazol-3-yl)-4,5,6,7-tetrahydro pyrazolo[3,4-*b*]thiazolo[4,5-*d*]azepin-2-amine
6-((5-Chloropyridin-2-yl)methyl)-*N*-(4-methylpyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-(3-methoxycyclobutyl)-4,5,6,7-tetrahydropyrazolo[3,4-*b*]thiazolo[4,5-*d*]azepin-2-amine
*N*-(5-Fluoropyrimidin-2-yl)-6-(oxetan-3-yl)-4,5,6,7-tetrahydropyrazolo[3,4-*b*]thiazolo [4,5-*d*]azepin-2-amine
6-(3,3-Difluorocyclobutyl)-*N*-(5-fluoropyrimidin-2-yl)-4,5,6,7-tetrahydropyrazolo[3,4-*b*]thiazolo[4,5-*d*]azepin-2-amine
6-((3,3-Difluorocyclobutyl)methyl)-*N*-(5-fluoropyrimidin-2-yl)-4,5,6,7-tetrahydro pyrazolo[3,4-*b*]thiazolo[4,5-*d*]azepin-2-amine

*N*-(5-Fluoropyrimidin-2-yl)-6-(oxetan-3-ylmethyl)-4,5,6,7-tetrahydropyrazolo[3,4-b] thiazolo[4,5-*d*]azepin-2-amine
*N*-(5-Methyl-1,2,4-thiadiazol-3-yl)-4,5,6,7-tetrahydropyrazolo[3,4-*b*]thiazolo[4,5-*d*] azepin-2-amine
*N*-(5-Methyl-1,2,4-thiadiazol-3-yl)-6-(oxetan-3-ylmethyl)-4,5,6,7-tetrahydropyrazolo [3,4-*b*]thiazolo[4,5-*d*] azepin-2-amine
*N*-(5-Methyl-1,2,4-thiadiazol-3-yl)-6-((3-methyloxetan-3-yl)methyl)-4,5,6,7-tetrahydro pyrazolo[3,4-*b*]thiazolo[4,5-*d*]azepin-2-amine
6-(Cyclobutylmethyl)-*N*-(5-methyl-1,2,4-thiadiazol-3-yl)-4,5,6,7-tetrahydropyrazolo [3,4-*b*]thiazolo[4,5-*d*] azepin-2-amine
*N*-(5-Methyl-1,2,4-thiadiazol-3-yl)-6-(oxetan-3-yl)-4,5,6,7-tetrahydropyrazolo[3,4-*b*] thiazolo[4,5-*d*]azepin-2-amine
6-Cyclopropyl-*N*-(5-methyl-1,2,4-thiadiazol-3-yl)-4,5,6,7-tetrahydropyrazolo [3,4-*b*] thiazolo[4,5-*d*]azepin-2-amine
*N*-(5-Methyl-1,2,4-thiadiazol-3-yl)-6-((tetrahydrofuran-2-yl)methyl)-4,5,6,7-tetrahydro pyrazolo[3,4-*b*]thiazolo[4,5-*d*]azepin-2-amine
*N*-(5-Methyl-1,2,4-thiadiazol-3-yl)-6-((tetrahydrofuran-3-yl)methyl)-4,5,6,7-tetrahydro pyrazolo[3,4-*b*]thiazolo[4,5-*d*]azepin-2-amine
6-((1-(Methoxymethyl)cyclopropyl)methyl)-*N*-(5-methyl-1,2,4-thiadiazol-3-yl)-4,5,6,7 -tetrahydropyrazolo[3,4-*b*]thiazolo[4,5-*d*]azepin-2-amine
6-((3,3-Difluorocyclobutyl)methyl)-*N*-(5-methyl-1,2,4-thiadiazol-3-yl)-4,5,6,7-tetrahydropyrazolo[3,4-*b*]thiazolo[4,5-*d*]azepin-2-amine
6-(3,3-Difluorocyclobutyl)-*N*-(5-methyl-1,2,4-thiadiazol-3-yl)-4,5,6,7-tetrahydro pyrazolo[3,4-*b*]thiazolo[4,5-*d*] azepin-2-amine
*N*-(5-Methyl-1,2,4-thiadiazol-3-yl)-6-(tetrahydro-2*H*-pyran-4-yl)-4,5,6,7-tetrahydro pyrazolo[3,4-*b*]thiazolo[4,5-*d*]azepin-2-amine
6-(2-Cyclopropoxyethyl)-*N*-(5-methyl-1,2,4-thiadiazol-3-yl)-4,5,6,7-tetrahydro pyrazolo[3,4-*b*]thiazolo[4,5-*d*] azepin-2-amine
6-(2-Ethoxyethyl)-*N*-(5-methyl-1,2,4-thiadiazol-3-yl)-4,5,6,7-tetrahydropyrazolo[3,4-*b*]thiazolo[4,5-*d*]azepin-2-amine
6-(1-Methoxypropan-2-yl)-*N*-(5-methyl-1,2,4-thiadiazol-3-yl)-4,5,6,7-tetrahydro pyrazolo[3,4-*b*]thiazolo[4,5-*d*] azepin-2-amine
*N*-(4-Methoxypyrimidin-2-yl)-4,5,6,7-tetrahydropyrazolo[3,4-*b*]thiazolo[4,5-*d*]azepin-2-amine
*N*-(4-Methoxypyrimidin-2-yl)-6-(oxetan-3-ylmethyl)-4,5,6,7-tetrahydropyrazolo[3,4-*b*] thiazolo[4,5-*d*]azepin-2-amine
*N*-(4-Methoxypyrimidin-2-yl)-6-((3-methyloxetan-3-yl)methyl)-4,5,6,7-tetrahydro pyrazolo[3,4-*b*]thiazolo[4,5-*d*] azepin-2-amine
6-(Cyclobutylmethyl)-*N*-(4-methoxypyrimidin-2-yl)-4,5,6,7-tetrahydropyrazolo[3,4-*b*] thiazolo[4,5-*d*]azepin-2-amine
*N*-(4-Methoxypyrimidin-2-yl)-6-(oxetan-3-yl)-4,5,6,7-tetrahydropyrazolo[3,4-*b*] thiazolo[4,5-*d*]azepin-2-amine
6-Cyclopropyl-*N*-(4-methoxypyrimidin-2-yl)-4,5,6,7-tetrahydropyrazolo[3,4-*b*] thiazolo[4,5-*d*]azepin-2-amine
*N*-(4-Methoxypyrimidin-2-yl)-6-((tetrahydrofuran-2-yl)methyl)-4,5,6,7-tetrahydro pyrazolo[3,4-*b*]thiazolo[4,5-*d*] azepin-2-amine
*N*-(4-Methoxypyrimidin-2-yl)-6-((tetrahydrofuran-3-yl)methyl)-4,5,6,7-tetrahydro pyrazolo[3,4-*b*]thiazolo[4,5-*d*] azepin-2-amine
6-((1-(Methoxymethyl)cyclopropyl)methyl)-*N*-(4-methoxypyrimidin-2-yl)-4,5,6,7-tetrahydropyrazolo[3,4-*b*]thiazolo[4,5-*d*]azepin-2-amine
6-((3,3-Difluorocyclobutyl)methyl)-*N*-(4-methoxypyrimidin-2-yl)-4,5,6,7-tetrahydro pyrazolo[3,4-*b*]thiazolo[4,5-*d*]azepin-2-amine
6-(3,3-Difluorocyclobutyl)-*N*-(4-methoxypyrimidin-2-yl)-4,5,6,7-tetrahydropyrazolo [3,4-*b*]thiazolo[4,5-*d*] azepin-2-amine
*N*-(4-Methoxypyrimidin-2-yl)-6-(tetrahydro-2*H*-pyran-4-yl)-4,5,6,7-tetrahydropyrazolo [3,4-*b*]thiazolo[4,5-*d*] azepin-2-amine
6-(2-Cyclopropoxyethyl)-*N*-(4-methoxypyrimidin-2-yl)-4,5,6,7-tetrahydropyrazolo [3,4-*b*]thiazolo[4,5-*d*]azepin-2-amine
6-(2-Ethoxyethyl)-*N*-(4-methoxypyrimidin-2-yl)-4,5,6,7-tetrahydropyrazolo[3,4-*b*] thiazolo[4,5-*d*]azepin-2-amine
6-(1-Methoxypropan-2-yl)-*N*-(4-methoxypyirmidin-2-yl)-4,5,6,7-tetrahydropyrazolo [3,4-*b*]thiazolo[4,5-*d*] azepin-2-amine

*N*-(5-Fluoropyrimidin-2-yl)-6-((1-(methoxymethyl)cyclopropyl)methyl)-4,5,6,7-tetrahydropyrazolo[3,4-*b*]thiazolo[4,5-*d*]azepin-2-amine
*N*-(5-Fluoropyrimidin-2-yl)-6-(tetrahydro-2*H*-pyran-4-yl)-4,5,6,7-tetrahydropyrazolo [3,4-*b*] thiazolo [4,5-*d*] azepin-2-amine
6-Cyclopropyl-*N*-(5-fluoropyrimidin-2-yl)-4,5,6,7-tetrahydropyrazolo[3,4-*b*]thiazolo [4,5-*d*]azepin-2-amine
6-(2-Ethoxyethyl)-*N*-(5-fluoropyrimidin-2-yl)-4,5,6,7-tetrahydropyrazolo[3,4-*b*] thiazolo[4,5-*d*]azepin-2-amine
*N*-(5-Fluoropyrimidin-2-yl)-6-(2-methoxypropyl)-4,5,6,7-tetrahydropyrazolo[3,4-*b*] thiazolo[4,5-*d*]azepin-2-amine
*N*-(4-Methylpyrimidin-2-yl)-4,5,6,7-tetrahydropyrazolo[3,4-*b*] thiazolo[4,5-*d*]azepin-2-amine
*N*-(4-Methylpyrimidin-2-yl)-6-(oxetan-3-yl)-4,5,6,7-tetrahydropyrazolo[3,4-*b*] thiazolo[4,5-*d*]azepin-2-amine
6-Cyclopropyl-*N*-(4-methylpyrimidin-2-yl)-4,5,6,7-tetrahydropyrazolo[3,4-*b*] thiazolo[4,5-*d*]azepin-2-amine
*N*-(4-Methylpyrimidin-2-yl)-6-(oxetan-3-ylmethyl)-4,5,6,7-tetrahydropyrazolo[3,4-*b*] thiazolo[4,5-*d*]azepin-2-amine
*N*-(4-Methylpyrimidin-2-yl)-6-((tetrahydrofuran-3-yl)methyl)-4,5,6,7-tetrahydro pyrazolo [3,4-*b*] thiazolo [4,5-*d*] azepin-2-amine
*N*-(4-Methylpyrimidin-2-yl)-6-(tetrahydro-2*H*-pyran-4-yl)-4,5,6,7-tetrahydropyrazolo [3,4-*b*]thiazolo[4,5-*d*] azepin-2-amine
$N^2$-(6-(Cyclopropylmethyl)-4,5,6,7-tetrahydropyrazolo [3,4-*b*]thiazolo[4,5-*d*] azepin-2-yl)pyrimidine-2,4-diamine
$N^2$-(6-(2-Methoxyethyl)-4,5,6,7-tetrahydropyrazolo [3,4-*b*] thiazolo[4,5-*d*] azepin-2-yl) pyrimidine-2,4-diamine
4-(2-(5-Fluoropyrimidin-2-ylamino)-4,5-dihydropyrazolo[3,4-*b*]thiazolo[4,5-*d*]azepin-6(7*H*)-yl)butan-2-one
6-(2-(Azetidin-1-yl)ethyl)-*N*-(5-fluoropyrimidin-2-yl)-4,5,6,7-tetrahydropyrazolo[3,4-*b*]thiazolo[4,5-*d*]azepin-2-amine
6-((4-Cyclopropyl-1-methyl-1*H*-pyrrol-2-yl)methyl)-*N*-(5-fluoropyrimidin-2-yl)-4,5,6,7-tetrahydropyrazolo[3,4-*b*] thiazolo[4,5-*d*]azepin-2-amine
6-((1-Cyclopropyl-1*H*-pyrrol-3-yl)methyl)-N-(5-fluoropyrimidin-2-yl)-4,5,6,7-tetrahydropyrazolo[3,4-*b*]thiazolo[4,5-*d*]azepin-2-amine
*N*-(5-Fluoropyrimidin-2-yl)-6-((1-methyl-1*H*-imidazol-2-yl)methyl)-4,5,6,7-tetrahydro pyrazolo [3,4-*b*] thiazolo [4,5-*d*] azepin-2-amine
6-((1-Cyclopropyl-1*H*-pyrazol-4-yl)methyl)-*N*-(5-fluoropyrimidin-2-yl)-4,5,6,7-tetrahydropyrazolo[3,4-*b*]thiazolo[4,5-*d*]azepin-2-amine
6-((1-Cyclopropyl-1*H*-pyrazol-3-yl)methyl)-*N*-(5-fluoropyrimidin-2-yl)-4,5,6,7-tetrahydropyrazolo[3,4-*b*]thiazolo[4,5-*d*]azepin-2-amine
6-((3-Fluoropyridin-2-yl)methyl)-*N*-(5-fluoropyrimidin-2-yl)-4,5,6,7-tetrahydro pyrazolo[3,4-*b*]thiazolo[4,5-*d*] azepin-2-amine
*N*-(5-Fluoropyrimidin-2-yl)-6-((2-methylpyridin-4-yl)methyl)-4,5,6,7-tetrahydro pyrazolo[3,4-*b*]thiazolo[4,5-*d*] azepin-2-amine
6-((2-Cyclopropylpyridin-4-yl)methyl)-N-(5-fluoropyrimidin-2-yl)-4,5,6,7-tetrahydro pyrazolo[3,4-*b*]thiazolo[4,5-*d*]azepin-2-amine
*N*-(5-Fluoropyrimidin-2-yl)-6-(pyrimidin-4-ylmethyl)-4,5,6,7-tetrahydropyrazolo[3,4-*b*]thiazolo[4,5-*d*]azepin-2-amine
*N*-(5-Fluoropyrimidin-2-yl)-6-((2-methylpyrimidin-4-yl)methyl)-4,5,6,7-tetrahydro pyrazolo[3,4-*b*]thiazolo[4,5-*d*] azepin-2-amine
6-((2,5-Dimethylthiazol-4-yl)methyl)-*N*-(5-fluoropyrimidin-2-yl)-4,5,6,7-tetrahydro pyrazolo[3,4-*b*]thiazolo[4,5-*d*]azepin-2-amine
6-((2,4-Dimethylthiazol-5-yl)methyl)-*N*-(5-fluoropyrimidin-2-yl)-4,5,6,7-tetrahydro pyrazolo[3,4-*b*]thiazolo[4,5-*d*]azepin-2-amine
6-((2-Cyclopropyloxazol-4-yl)methyl)-*N*-(5-fluoropyrimidin-2-yl)-4,5,6,7-tetrahydro pyrazolo[3,4-*b*]thiazolo[4,5-*d*]azepin-2-amine
6-((2-Cyclopropyl-5-methyloxazol-4-yl)methyl)-*N*-(5-fluoropyrimidin-2-yl)-4,5,6,7-tetrahydropyrazolo[3,4-*b*]thiazolo[4,5-*d*]azepin-2-amine
4-(2-(4-Methylpyrimidin-2-ylamino)-4,5-dihydropyrazolo[3,4-*b*]thiazolo[4,5-*d*]azepin-6(7*H*)-yl)butan-2-one
6-(2-(Azetidin-1-yl)ethyl)-*N*-(4-methylpyrimidin-2-yl)-4,5,6,7-tetrahydropyrazolo[3,4-*b*]thiazolo[4,5-*d*]azepin-2-amine
6-((4-Cyclopropyl-1-methyl-1*H*-pyrrol-2-yl)methyl)-*N*-(4-methylpyrimidin-2-yl)-4,5,6,7-tetrahydropyrazolo[3,4-*b*]thiazolo[4,5-d]azepin-2-amine
6-((1-Cyclopropyl-1*H*-pyrrol-3-yl)methyl)-*N*-(4-methylpyrimidin-2-yl)-4,5,6,7-tetrahydropyrazolo[3,4-*b*]thiazolo[4,5-*d*]azepin-2-amine

6-((1-Methyl-1H-imidazol-2-yl)methyl)-*N*-(4-methylpyrimidin-2-yl)-4,5,6,7-tetrahydro pyrazolo[3,4-*b*]thiazolo[4,5-*d*]azepin-2-amine
6-((1-Cyclopropyl-1*H*-pyrazol-4-yl)methyl)-*N*-(4-methylpyrimidin-2-yl)-4,5,6,7-tetrahydropyrazolo[3,4-b]thiazolo[4,5-*d*]azepin-2-amine
6-((1-Cyclopropyl-1*H*-pyrazol-3-yl)methyl)-*N*-(4-methylpyrimidin-2-yl)-4,5,6,7-tetrahydropyrazolo[3,4-*b*]thiazolo[4,5-*d*]azepin-2-amine
6-((3-Fluoropyridin-2-yl)methyl)-*N*-(4-methylpyrimidin-2-yl)-4,5,6,7-tetrahydro pyrazolo[3,4-*b*]thiazolo[4,5-*d*]azepin-2-amine
6-((2-Methylpyridin-4-yl)methyl)-*N*-(4-methylpyrimidin-2-yl)-4,5,6,7-tetrahydro pyrazolo[3,4-*b*]thiazolo[4,5-*d*]azepin-2-amine
6-((2-Cyclopropylpyridin-4-yl)methyl)-*N*-(4-methylpyrimidin-2-yl)-4,5,6,7-tetrahydro pyrazolo[3,4-*b*]thiazolo[4,5-*d*]azepin-2-amine
*N*-(4-Methylpyrimidin-2-yl)-6-(pyrimidin-4-ylmethyl)-4,5,6,7-tetrahydropyrazolo[3,4-*b*]thiazolo[4,5-*d*]azepin-2-amine
*N*-(4-Methylpyrimidin-2-yl)-6-((2-methylpyrimidin-4-yl)methyl)-4,5,6,7-tetrahydro pyrazolo[3,4-*b*]thiazolo[4,5-*d*]azepin-2-amine
6-((2,5-Dimethylthiazol-4-yl)methyl)-*N*-(4-methylpyrimidin-2-yl)-4,5,6,7-tetrahydro pyrazolo [3,4-*b*] thiazolo[4,5-*d*]azepin-2-amine
6-((2,4-Dimethylthiazol-5-yl)methyl)-*N*-(4-methylpyrimidin-2-yl)-4,5,6,7-tetrahydro pyrazolo [3,4-*b*] thiazolo [4,5-*d*] azepin-2-amine
6-((2-Cyclopropyloxazol-4-yl)methyl)-*N*-(4-methylpyrimidin-2-yl)-4,5,6,7-tetrahydro pyrazolo [3,4-*b*] thiazolo [4,5-*d*] azepin-2-amine
6-((2-Cyclopropyl-5-methyloxazol-4-yl)methyl)-*N*-(4-methylpyrimidin-2-yl)-4,5,6,7-tetrahydropyrazolo[3,4-*b*] thiazolo[4,5-*d*]azepin-2-amine
4-(2-(4-Methoxypyrimidin-2-ylamino)-4,5-dihydropyrazolo[3,4-*b*]thiazolo[4,5-*d*] azepin-6(7*H*)-yl)butan-2-one
6-(2-(Azetidin-1-yl)ethyl)-*N*-(4-methoxypyrimidin-2-yl)-4,5,6,7-tetrahydro pyrazolo[3,4-*b*]thiazolo[4,5-*d*]azepin-2-amine
6-((4-Cyclopropyl-1-methyl-1*H*-pyrrol-2-yl)methyl)-*N*-(4-methoxypyrimidin-2-yl)-4,5,6,7-tetrahydropyrazolo[3,4-*b*]thiazolo[4,5-*d*]azepin-2-amine
6-((1-Cyclopropyl-1*H*-pyrrol-3-yl)methyl)-*N*-(4-methoxypyrimidin-2-yl)-4,5,6,7-tetrahydropyrazolo[3,4-*b*]thiazolo[4,5-*d*]azepin-2-amine
*N*-(4-Methoxypyrimidin-2-yl)-6-((1-methyl-1*H*-imidazol-2-yl)methyl)-4,5,6,7-tetrahydropyrazolo[3,4-*b*]thiazolo[4,5-*d*]azepin-2-amine
6-((1-Cyclopropyl-1*H*-pyrazol-4-yl)methyl)-*N*-(4-methoxypyrimidin-2-yl)-4,5,6,7-tetrahydropyrazolo[3,4-*b*]thiazolo[4,5-*d*]azepin-2-amine
6-((1-Cyclopropyl-1*H*-pyrazol-3-yl)methyl)-*N*-(4-methoxypyrimidin-2-yl)-4,5,6,7-tetrahydropyrazolo[3,4-*b*]thiazolo[4,5-*d*]azepin-2-amine
6-((3-Fluoropyridin-2-yl)methyl)-*N*-(4-methoxypyrimidin-2-yl)-4,5,6,7-tetrahydro pyrazolo[3,4-*b*]thiazolo[4,5-*d*]azepin-2-amine
*N*-(4-Methoxypyrimidin-2-yl)-6-((2-methylpyridin-4-yl)methyl)-4,5,6,7-tetrahydro pyrazolo[3,4-*b*]thiazolo[4,5-*d*]azepin-2-amine
6-((2-Cyclopropylpyridin-4-yl)methyl)-*N*-(4-methoxypyrimidin-2-yl)-4,5,6,7-tetrahydropyrazolo[3,4-*b*]thiazolo[4,5-*d*]azepin-2-amine
*N*-(4-Methoxypyrimidin-2-yl)-6-(pyrimidin-4-ylmethyl)-4,5,6,7-tetrahydropyrazolo [3,4-*b*]thiazolo[4,5-*d*]azepin-2-amine
*N*-(4-Methoxypyrimidin-2-yl)-6-((2-methylpyrimidin-4-yl)methyl)-4,5,6,7-tetrahydro pyrazolo[3,4-*b*]thiazolo[4,5-*d*]azepin-2-amine
6-((2,5-Dimethylthiazol-4-yl)methyl)-*N*-(4-methoxypyrimidin-2-yl)-4,5,6,7-tetrahydro pyrazolo[3,4-*b*]thiazolo[4,5-*d*]azepin-2-amine
6-((2,4-Dimethylthiazol-5-yl)methyl)-*N*-(4-methoxypyrimidin-2-yl)-4,5,6,7-tetrahydro pyrazolo[3,4-*b*]thiazolo[4,5-*d*]azepin-2-amine
6-((2-Cyclopropyloxazol-4-yl)methyl)-*N*-(4-methoxypyrimidin-2-yl)-4,5,6,7-tetrahydropyrazolo[3,4-*b*]thiazolo[4,5-*d*]azepin-2-amine
6-((2-Cyclopropyl-5-methyloxazol-4-yl)methyl)-*N*-(4-methoxypyrimidin-2-yl)-4,5,6,7-tetrahydropyrazolo[3,4-*b*] thiazolo[4,5-*d*]azepin-2-amine
4-(2-(6-Methylpyridin-2-ylamino)-4,5-dihydropyrazolo[3,4-*b*]thiazolo[4,5-*d*]azepin-6(7*H*)-yl)butan-2-one
6-(2-(Azetidin-1-yl)ethyl)-*N*-(6-methylpyridin-2-yl)-4,5,6,7-tetrahydropyrazolo[3,4-*b*] thiazolo[4,5-*d*]azepin-2-amine

6-((4-Cyclopropyl-1-methyl-1*H*-pyrrol-2-yl)methyl)-*N*-(6-methylpyridin-2-yl)-4,5,6,7-tetrahydropyrazolo[3,4-*b*]thiazolo[4,5-*d*]azepin-2-amine
6-((1-Cyclopropyl-1*H*-pyrrol-3-yl)methyl)-*N*-(6-methylpyridin-2-yl)-4,5,6,7-tetrahydro pyrazolo[3,4-*b*]thiazolo[4,5-*d*]azepin-2-amine
6-((1-Methyl-1*H*-imidazol-2-yl)methyl)-*N*-(6-methylpyridin-2-yl)-4,5,6,7-tetrahydro pyrazolo[3,4-*b*]thiazolo[4,5-*d*]azepin-2-amine
6-((1-Cyclopropyl-1*H*-pyrazol-4-yl)methyl)-*N*-(6-methylpyridin-2-yl)-4,5,6,7-tetrahydropyrazolo[3,4-*b*]thiazolo[4,5-*d*]azepin-2-amine
6-((1-Cyclopropyl-1*H*-pyrazol-3-yl)methyl)-*N*-(6-methylpyridin-2-yl)-4,5,6,7-tetrahydropyrazolo[3,4-*b*]thiazolo[4,5-*d*]azepin-2-amine
6-((3-Fluoropyridin-2-yl)methyl)-*N*-(6-methylpyridin-2-yl)-4,5,6,7-tetrahydropyrazolo [3,4-*b*]thiazolo[4,5-*d*]azepin-2-amine
*N*-(6-Methylpyridin-2-yl)-6-((2-methylpyridin-4-yl)methyl)-4,5,6,7-tetrahydropyrazolo [3,4-*b*]thiazolo[4,5-*d*]azepin-2-amine
6-((2-Cyclopropylpyridin-4-yl)methyl)-*N*-(6-methylpyridin-2-yl)-4,5,6,7-tetrahydro pyrazolo[3,4-*b*]thiazolo[4,5-*d*]azepin-2-amine
*N*-(6-Methylpyridin-2-yl)-6-(pyrimidin-4-ylmethyl)-4,5,6,7-tetrahydropyrazolo[3,4-*b*] thiazolo[4,5-*d*]azepin-2-amine
*N*-(6-Methylpyridin-2-yl)-6-((2-methylpyrimidin-4-yl)methyl)-4,5,6,7-tetrahydro pyrazolo[3,4-*b*]thiazolo[4,5-*d*]azepin-2-amine
6-((2,5-Dimethylthiazol-4-yl)methyl)-*N*-(6-methylpyridin-2-yl)-4,5,6,7-tetrahydro pyrazolo[3,4-*b*]thiazolo[4,5-*d*]azepin-2-amine
6-((2,4-Dimethylthiazol-5-yl)methyl)-*N*-(6-methylpyridin-2-yl)-4,5,6,7-tetrahydro pyrazolo[3,4-*b*]thiazolo[4,5-*d*]azepin-2-amine
6-((2-Cyclopropyloxazol-4-yl)methyl)-*N*-(6-methylpyridin-2-yl)-4,5,6,7-tetrahydro pyrazolo[3,4-*b*]thiazolo[4,5-*d*]azepin-2-amine
6-((2-Cyclopropyl-5-methyloxazol-4-yl)methyl)-*N*-(6-methylpyridin-2-yl)-4,5,6,7-tetrahydropyrazolo[3,4-*b*]thiazolo[4,5-*d*]azepin-2-amine

and a pharmaceutically acceptable acid or base addition salt thereof, a stereochemically isomeric form thereof and an N-oxide form thereof.

**2.** A pharmaceutical composition comprising a therapeutically effective amount of a compound according to claim 1 and a pharmaceutically acceptable carrier and/or excipient.

**3.** A compound according to claim 1 or a composition according to claim 2 for use in treating or preventing central nervous system disorders selected from the group consisting of: addiction, tolerance or dependence; affective disorders, such as depression and anxiety; psychiatric disease such as psychotic disorders, attention-deficit/hyperactivity disorder and bipolar disorder; Parkinson's disease, memory impairment, Alzheimer's disease, dementia, delirium tremens, other forms of neurodegeneration, neurotoxicity, and ischemia, or for use in treating or preventing central nervous system disorders selected from the group consisting of: cognitive disorders such as delirium, substance-induced persisting delirium, dementia, dementia due to HIV disease, dementia due to Huntington's disease, dementia due to Parkinson's disease, Parkinsonian-ALS demential complex, dementia of the Alzheimer's type, substance-induced persisting dementia, and mild cognitive impairment, or for use in treating affective disorders selected from the group consisting of: anxiety, agoraphobia, generalized anxiety disorder (GAD), obsessive-compulsive disorder (OCD), panic disorder, posttraumatic stress disorder (PTSD), social phobia, other phobias, substance-induced anxiety disorder, and acute stress disorder, or for use in treating or preventing central nervous system disorders selected from the group consisting of: mood disorders, bipolar disorders (I & II), cyclothymic disorder, depression, dysthymic disorder, major depressive disorder, and substance-induced mood disorder, or for use in treating or preventing neurological disorders selected from the group consisting of: neurodegeneration, neurotoxicity or ischemia such as stroke, spinal cord injury, cerebral hypoxia, intracranial hematoma, Parkinson's disease, memory impairment, Alzheimer's disease, dementia, and delirium tremens, or for use in treating or preventing inflammatory central nervous system disorders selected from the group consisting of: multiple sclerosis forms such as benign multiple sclerosis, relapsing-remitting multiple sclerosis, secondary progressive multiple sclerosis, primary progressive multiple sclerosis, and progressive-relapsing multiple sclerosis, or for use in treating or preventing migraine, or for use in treating or preventing epilepsy and tremor, temporal lobe epilepsy, epilepsy secondary to another disease or injury such as chronic encephalitis, traumatic brain injury, stroke or ischemia, or for use in treating or preventing central nervous system disorders selected from the group consisting of psychotic disorders: schizophrenia, delusional disorder, schizoaffective disorder, schizophreniform disorder, substance-induced psychotic disorder, or for use in treating or preventing inflammation and/or neurodegeneration resulting from traumatic brain injury, stroke, ischemia, spinal cord injury, cerebral hypoxia or intracranial hematoma, or for use in treating or preventing sensory, motor or cognitive symptoms resulting from traumatic brain injury, stroke, ischemia, spinal cord injury, cerebral hypoxia or intracranial hematoma, or for use in treating medulloblastomas, or for use in treating or preventing inflammatory or neuropathic pain, or for use in treating, preventing, ameliorating, controlling or reducing the risk of various metabolic disorders associated with glutamate dysfunction, or for use in treating or preventing type 2 diabetes, or for use in treating or preventing diseases or disorders of the retina, retinal degeneration or macular degeneration, or for use in treating or preventing diseases or disorders of the gastrointestinal tract including gastro-esophageal reflux disease (GERD), lower esophageal sphincter diseases or disorders, diseases of gastrointestinal motility, colitis, Crohn's disease or irritable bowel syndrome (IBS).

**4.** A compound according to claim 1 or a composition according to claim 2 for use in treating or preventing central nervous system disorders selected from the group consisting of: Parkinson's disease and movement disorders such as bradykinesia, rigidity, dystonia, drug-induced parkinsonism, dyskinesia, tardive dyskinesia, L-DOPA-induced dyskinesia, dopamine agonist-induced dyskinesia, hyperkinetic movement disorders, Gilles de la Tourette syndrome, resting tremor, action tremor, akinesia, akinetic-rigid syndrome, akathisia, athetosis, asterixis, tics, postural instability, postencephalitic parkinsonism, muscle rigidity, chorea and choreaform movements, spasticity, myoclonus, hemiballismus, progressive supranuclear palsy, restless legs syndrome, and periodic limb movement disorder.

**5.** A compound according to claim 1 or a composition according to claim 2 in combination with an agent selected from the group consisting of: levodopa, levodopa with a selective extracerebral decarboxylase inhibitor, carbidopa, entacapone, a COMT inhibitor, a dopamine agonist, an anticholinergic, a cholinergic agonist, an NMDA receptor antagonist, an MAO-B inhibitor, an mGluR5 antagonist, an $A_{2A}$ antagonist, a butyrophenone neuroleptic agent, a diphenylbutylpiperidine neuroleptic agent, a heterocyclic dibenzazepine neuroleptic agent, an indolone neuroleptic agent, a phenothiazine neuroleptic agent or a thioxanthene neuroleptic agent, for use in treating or preventing a disorder as defined in claim 4.

**6.** A compound according to claim 1 in combination with levodopa or in combination with a dopamine agonist or in combination with an $A_{2A}$ antagonist for use in treating or preventing a condition as defined in claim 4.

**7.** Use of a compound according to claim 1 to prepare a tracer for imaging a metabotropic glutamate receptor, or as a taste agent, flavour agent, flavour enhancing agent or a food or beverage additive.

## Patentansprüche

**1.** Verbindung, wobei die genannte Verbindung ein 2-Amino-4,5,6,8-tetrahydropyrazolo[3,4-b]thiazolo[4,5-d]azepinderivat ist, ausgewählt aus:

3-((2-(5-Fluorpyrimidin-2-ylamino)-4,5-dihydropyrazolo[3,4-b]thiazolo[4,5-d]azepin-6(7H)-yl)methyl)benzonitril
6-(3-Fluorbenzyl)-N-(5-fluorpyrimidin-2-yl)-4,5,6,7-tetrahydropyrazolo[3,4-b]thiazolo[4,5-d]azepin-2-amin
6-(Cyclopropylmethyl)-N-(5-methyl-1,2,4-thiadiazol-3-yl)-4,5,6,7-tetrahydropyrazolo[3,4-b]thiazolo[4,5-d]azepin-2-amin
6-((5-Cyclopropylisoxazol-3-yl)methyl)-N-(5-fluorpyrimidin-2-yl)-4,5,6,7-tetrahydropyrazolo[3,4-b]thiazolo[4,5-d]azepin-2-amin

und ein pharmazeutisch akzeptables Säure- oder Basenadditionssalz davon, eine stereochemisch isomere Form davon und eine N-Oxidform davon; oder
wobei die genannte Verbindung ausgewählt ist aus:

6-((2,3-Dihydrobenzofuran-5-yl)methyl)-N-(5-fluorpyrimidin-2-yl)-4,5,6,7-tetrahydropyrazolo[3,4-b]thiazolo[4,5-d]azepin-2-amin
*N*-(5-Fluorpyrimidin-2-yl)-6-(3-(trifluormethyl)benzyl)-4,5,6,7-tetrahydropyrazolo [*3,4-b*]thiazolo[4,5-d]azepin-2-amin
6-((3-Cyclopropylisoxazol-5-yl)methyl)-N-(5-fluorpyrimidin-2-yl)-4,5,6,7-tetrahydropyrazolo[3,4-b]thiazolo[4,5-d]azepin-2-amin
6-((2-Cyclopropylthiazol-4-yl)methyl)-N-(5-fluorpyrimidin-2-yl)-4,5,6,7-tetrahydropyrazolo[3,4-b]thiazolo[4,5-d]

azepin-2-amin
6-(1-Methoxypropan-2-yl)-*N*-(5-methyl-1,2,4-thiadiazol-3-yl)-4,5,6,7-tetrahydropyrazolo[3,4-b]thiazolo[4,5-d]azepin-2-amin
6-((5-Chlorpyridin-2-yl)methyl)-*N*-(4-methylpyrimidin-2-yl)-4,5,6,7-tetrahydropyrazolo[3,4-b]thiazolo[4,5-d]azepin-2-amin
*N*-(5-Fluorpyrimidin-2-yl)-6-(3-methoxycyclobutyl)-4,5,6,7-tetrahydropyrazolo[3,4-*b*]thiazolo[4,5-d]azepin-2-amin
*N*-(5-Fluorpyrimidin-2-yl)-6-(oxetan-3-yl)-4,5,6,7-tetrahydropyrazolo[3,4-b]thiazolo [4,5-d]azepin-2-amin
6-(3,3-Difluorcyclobutyl)-*N*-(5-fluorpyrimidin-2-yl)-4,5,6,7-tetrahydropyrazolo[3,4-*b*]thiazolo[4,5-d]azepin-2-amin
6-((3,3-Difluorcyclobutyl)methyl)-*N*-(5-fluorpyrimidin-2-yl)-4,5,6,7-tetrahydropyrazolo[3,4-b]thiazolo[4,5-d]azepin-2-amin
*N*-(5-Fluorpyrimidin-2-yl)-6-(oxetan-3-ylmethyl)-4,5,6,7-tetrahydropyrazolo[3,4-b]thiazolo[4,5-d]azepin-2-amin
*N*-(5-Methyl-1,2,4-thiadiazol-3-yl)-4,5,6,7-tetrahydropyrazolo[3,4-b]thiazolo[4,5-d]azepin-2-amin
*N*-(5-Methyl-1,2,4-thiadiazol-3-yl)-6-(oxetan-3-ylmethyl)-4,5,6,7-tetrahydropyrazolo[*3,4-b*]thiazolo[4,5-d]azepin-2-amin
*N*-(5-Methyl-1,2,4-thiadiazol-3-yl)-6-((3-methyloxetan-3-yl)methyl)-4,5,6,7-tetrahydropyrazolo[3,4-b]thiazolo[4,5-d]azepin-2-amin
6-(Cyclobutylmethyl)-*N*-(5-methyl-1,2,4-thiadiazol-3-yl)-4,5,6,7-tetrahydropyrazolo[*3,4-b*]thiazolo[4,5-d]azepin-2-amin
*N*-(5-Methyl-1,2,4-thiadiazol-3-yl)-6-(oxetan-3-yl)-4,5,6,7-tetrahydropyrazolo[3,4-b]thiazolo[4,5-d]azepin-2-amin
6-Cyclopropyl-*N*-(5-methyl-1,2,4-thiadiazol-3-yl)-4,5,6,7-tetrahydropyrazolo[3,4-b]thiazolo[4,5-d]azepin-2-amin
*N*-(5-Methyl-1,2,4-thiadiazol-3-yl)-6-((tetrahydrofuran-2-yl)methyl)-4,5,6,7-tetrahydropyrazolo[3,4-b]thiazolo[4,5-d]azepin-2-amin
*N*-(5-Methyl-1,2,4-thiadiazol-3-yl)-6-((tetrahydrofuran-3-yl)methyl)-4,5,6,7-tetrahydropyrazolo[3,4-b]thiazolo[4,5-d]azepin-2-amin
6-((1-(Methoxymethyl)cyclopropyl)methyl)-*N*-(5-methyl-1,2,4-thiadiazol-3-yl)-4,5,6,7-tetrahydropyrazolo[3,4-b]thiazolo[*4,5-d*]azepin-2-amin
6-((3,3-Difluorcyclobutyl)methyl)-*N*-(5-methyl-1,2,4-thiadiazol-3-yl)-4,5,6,7-tetrahydropyrazolo[3,4-b]thiazolo[*4,5-d*]azepin-2-amin
6-(3,3-Difluorcyclobutyl)-*N*-(5-methyl-1,2,4-thiadiazol-3-yl)-4,5,6,7-tetrahydropyrazolo[3,4-b]thiazolo[4,5-d]azepin-2-amin
*N*-(5-Methyl-1,2,4-thiadiazol-3-yl)-6-(tetrahydro-2H-pyran-4-yl)-4,5,6,7-tetrahydropyrazolo[3,4-b]thiazolo[4,5-d]azepin-2-amin
6-(2-Cyclopropoxyethyl)-*N*-(5-methyl-1,2,4-thiadiazol-3-yl)-4,5,6,7-tetrahydropyrazolo[3,4-b]thiazolo[4,5-d]azepin-2-amin
6-(2-Ethoxyethyl)-*N*-(5-methyl-1,2,4-thiadiazol-3-yl)-4,5,6,7-tetrahydropyrazolo[3,4-*b*]thiazolo[4,5-d]azepin-2-amin
6-(1-Methoxypropan-2-yl)-*N*-(5-methyl-1,2,4-thiadiazol-3-yl)-4,5,6,7-tetrahydropyrazolo[3,4-b]thiazolo[4,5-d]azepin-2-amin
N-(4-Methoxypyrimidin-2-yl)-4,5,6,7-tetrahydropyrazolo[3,4-b]thiazolo[4,5-d]azepin-2-amin
*N*-(4-Methoxypyrimidin-2-yl)-6-(oxetan-3-ylmethyl)-4,5,6,7-tetrahydropyrazolo[3,4-b]thiazolo[4,5-d]azepin-2-amin
*N*-(4-Methoxypyrimidin-2-yl)-6-((3-methyloxetan-3-yl)methyl)-4,5,6,7-tetrahydropyrazolo[3,4-b]thiazolo[4,5-d]azepin-2-amin
6-(Cyclobutylmethyl)-*N*-(4-methoxypyrimidin-2-yl)-4,5,6,7-tetrahydropyrazolo[3,4-b]thiazolo[4,5-d]azepin-2-amin
*N*-(4-Methoxypyrimidin-2-yl)-6-(oxetan-3-yl)-4,5,6,7-tetrahydropyrazolo[3,4-b]thiazolo[4,5-d]azepin-2-amin
6-Cyclopropyl-N-(4-methoxypyrimidin-2-yl)-4,5,6,7-tetrahydropyrazolo[3,4-b]thiazolo[4,5-d]azepin-2-amin
*N*-(4-Methoxypyrimidin-2-yl)-6-((tetrahydrofuran-2-yl)methyl)-4,5,6,7-tetrahydropyrazolo[3,4-b]thiazolo[4,5-d]azepin-2-amin
*N*-(4-Methoxypyrimidin-2-yl)-6-((tetrahydrofuran-3-yl)methyl)-4,5,6,7-tetrahydropyrazolo[3,4-b]thiazolo[4,5-d]azepin-2-amin
6-((1-(Methoxymethyl)cyclopropyl)methyl)-N-(4-methoxypyrimidin-2-yl)-4,5,6,7-tetrahydropyrazolo[3,4-b]thiazolo[*4,5-d*]azepin-2-amin
6-((3,3-Difluorcyclobutyl)methyl)-N-(4-methoxypyrimidin-2-yl)-4,5,6,7-tetrahydropyrazolo[3,4-b]thiazolo[4,5-d]

azepin-2-amin
6-(3,3-Difluorcyclobutyl)-N-(4-methoxypyrimidin-2-yl)-4,5,6,7-tetrahydropyrazolo[*3,4-b*]thiazolo[4,5-d]azepin-2-amin
*N*-(4-Methoxypyrimidin-2-yl)-6-(tetrahydro-2H-pyran-4-yl)-4,5,6,7-tetrahydropyrazolo[*3,4-b*]thiazolo[4,5-d]azepin-2-amin
6-(2-Cyclopropoxyethyl)-*N*-(4-methoxypyrimidin-2-yl)-4,5,6,7-tetrahydropyrazolo[*3,4-b*]thiazolo[4,5-d]azepin-2-amin
6-(2-Ethoxyethyl)-*N*-(4-methoxypyrimidin-2-yl)-4,5,6,7-tetrahydropyrazolo[3,4-b]thiazolo[4,5-d]azepin-2-amin
6-(1-Methoxypropan-2-yl)-N-(4-methoxypyrimidin-2-yl)-4,5,6,7-tetrahydropyrazolo[*3,4-b*]thiazolo[4,5-d]azepin-2-amin
N-(5-Fluorpyrimidin-2-yl)-6-((1-(methoxymethyl)cyclopropyl)methyl)-4,5,6,7-tetrahydropyrazolo[3,4-b]thiazolo[4,5-d]azepin-2-amin
*N*-(5-Fluorpyrimidin-2-yl)-6-(tetrahydro-2H-pyran-4-yl)-4,5,6,7-tetrahydropyrazolo[*3,4-b*]thiazolo[4,5-d]azepin-2-amin
6-Cyclopropyl-*N*-(5-fluorpyrimidin-2-yl)-4,5,6,7-tetrahydropyrazolo[3,4-b]thiazolo[4,5-d]azepin-2-amin
6-(2-Ethoxyethyl)-N-(5-fluorpyrimidin-2-yl)-4,5,6,7-tetrahydropyrazolo[3,4-b]thiazolo[4,5-d]azepin-2-amin
*N*-(5-Fluorpyrimidin-2-yl)-6-(2-methoxypropyl)-4,5,6,7-tetrahydropyrazolo[3,4-b]thiazolo[4,5-d]azepin-2-amin
*N*-(4-Methylpyrimidin-2-yl)-4,5,6,7-tetrahydropyrazolo[3,4-b]thiazolo[4,5-d]azepin-2-amin
*N*-(4-Methylpyrimidin-2-yl)-6-(oxetan-3-yl)-4,5,6,7-tetrahydropyrazolo[3,4-b]thiazolo[4,5-d]azepin-2-amin
6-Cyclopropyl-N-(4-methylpyrimidin-2-yl)-4,5,6,7-tetrahydropyrazolo[3,4-b]thiazolo[4,5-d]azepin-2-amin
*N*-(4-Methylpyrimidin-2-yl)-6-(oxetan-3-ylmethyl)-4,5,6,7-tetrahydropyrazolo[3,4-b]thiazolo[4,5-d]azepin-2-amin
*N*-(4-Methylpyrimidin-2-yl)-6-((tetrahydrofuran-3-yl)methyl)-4,5,6,7-tetrahydropyrazolo[3,4-b]thiazolo[4,5-d]azepin-2-amin
*N*-(4-Methylpyrimidin-2-yl)-6-(tetrahydro-2H-pyran-4-yl)-4,5,6,7-tetrahydropyrazolo[*3,4-b*]thiazolo[4,5-d]azepin-2-amin
$N^2$-(6-(Cyclopropylmethyl)-4,5,6,7-tetrahydropyrazolo[3,4-b]thiazolo[*4,5-d*]azepin-2-yl)pyrimidin-2,4-diamin
$N^2$-(6-(2-Methoxyethyl)-4,5,6,7-tetrahydropyrazolo[3,4-b]thiazolo[*4,5-d*] azepin-2-yl)pyrimidin-2,4-diamin
4-(2-(5-Fluorpyrimidin-2-ylamino)-4,5-dihydropyrazolo[3,4-b]thiazolo[4,5-d]azepin-6(7H)-yl)butan-2-on
6-(2-(Azetidin-1-yl)ethyl)-N-(5-fluorpyrimidin-2-yl)-4,5,6,7-tetrahydropyrazolo[3,4-*b*]thiazolo[4,5-d]azepin-2-amin
6-((4-Cyclopropyl-1-methyl-1H-pyrrol-2-yl)methyl)-*N*-(5-fluorpyrimidin-2-yl)-4,5,6,7-tetrahydropyrazolo[3,4-b]thiazolo[4,5-d]azepin-2-amin
6-((1-Cyclopropyl-1H-pyrrol-3-yl)methyl)-*N*-(5-fluorpyrimidin-2-yl)-4,5,6,7-tetrahydropyrazolo[3,4-b]thiazolo[*4,5-d*]azepin-2-amin
*N*-(5-Fluorpyrimidin-2-yl)-6-((1-methyl-1H-imidazol-2-yl)methyl)-4,5,6,7-tetrahydropyrazolo[3,4-b]thiazolo[4,5-d]azepin-2-amin
6-((1-Cyclopropyl-1H-pyrazol-4-yl)methyl)-*N*-(5-fluorpyrimidin-2-yl)-4,5,6,7-tetrahydropyrazolo[3,4-b]thiazolo[*4,5-d*]azepin-2-amin
6-((1-Cyclopropyl-1H-pyrazol-3-yl)methyl)-*N*-(5-fluorpyrimidin-2-yl)-4,5,6,7-tetrahydropyrazolo[3,4-b]thiazolo[*4,5-d*] azepin-2-amin
6-((3-Fluorpyridin-2-yl)methyl)-*N*-(5-fluorpyrimidin-2-yl)-4,5,6,7-tetrahydropyrazolo[3,4-b]thiazolo[4,5-d]azepin-2-amin
*N*-(5-Fluorpyrimidin-2-yl)-6-((2-methylpyridin-4-yl)methyl)-4,5,6,7-tetrahydropyrazolo[3,4-b]thiazolo[4,5-d]azepin-2-amin
6-((2-Cyclopropylpyridin-4-yl)methyl)-*N*-(5-fluorpyrimidin-2-yl)-4,5,6,7-tetrahydropyrazolo[3,4-b]thiazolo[4,5-d]azepin-2-amin
*N*-(5-Fluorpyrimidin-2-yl)-6-(pyrimidin-4-ylmethyl)-4,5,6,7-tetrahydropyrazolo[3,4-*b*]thiazolo[4,5-d]azepin-2-amin
*N*-(5-Fluorpyrimidin-2-yl)-6-((2-methylpyrimidin-4-yl)methyl)-4,5,6,7-tetrahydropyrazolo[3,4-b]thiazolo[4,5-d]azepin-2-amin
6-((2,5-Dimethylthiazol-4-yl)methyl)-*N*-(5-fluorpyrimidin-2-yl)-4,5,6,7-tetrahydropyrazolo[3,4-b]thiazolo[4,5-d]azepin-2-amin
6-((2,4-Dimethylthiazol-5-yl)methyl)-*N*-(5-fluorpyrimidin-2-yl)-4,5,6,7-tetrahydropyrazolo[3,4-b]thiazolo[4,5-d]azepin-2-amin
6-((2-Cyclopropyloxazol-4-yl)methyl)-*N*-(5-fluorpyrimidin-2-yl)-4,5,6,7-tetrahydropyrazolo[3,4-b]thiazolo[4,5-d]azepin-2-amin
6-((2-Cyclopropyl-5-methyloxazol-4-yl)methyl)-*N*-(5-fluorpyrimidin-2-yl)-4,5,6,7-tetrahydropyrazolo[3,4-b]thiazolo[*4,5-d]*azepin-2-amin
4-(2-(4-Methylpyrimidin-2-ylamino)-4,5-dihydropyrazolo[3,4-b]thiazolo[*4,5-d]*azepin-6(7H)-yl)butan-2-on
6-(2-(Azetidin-1-yl)ethyl)-*N*-(4-methylpyrimidin-2-yl)-4,5,6,7-tetrahydropyrazolo[3,4-*b*]thiazolo[4,5-d]azepin-2-amin
6-((4-Cyclopropyl-1-methyl-1H-pyrrol-2-yl)methyl)-*N*-(4-methylpyrimidin-2-yl)-4,5,6,7-tetrahydropyrazolo[3,4-b]thiazolo[*4,5-d]*azepin-2-amin
6-((1-Cyclopropyl-1H-pyrrol-3-yl)methyl)-*N*-(4-methylpyrimidin-2-yl)-4,5,6,7-tetrahydropyrazolo[3,4-b]thiazolo[4,5-d]azepin-2-amin
6-((1-Methyl-1H-imidazol-2-yl)methyl)-N-(4-methylpyrimidin-2-yl)-4,5,6,7-tetrahydropyrazolo[3,4-b]thiazolo[4,5-d]azepin-2-amin
6-((1-Cyclopropyl-1H-pyrazol-4-yl)methyl)-*N*-(4-methylpyrimidin-2-yl)-4,5,6,7-tetrahydropyrazolo[3,4-b]thiazolo[*4,5-d]*azepin-2-amin
6-((1-Cyclopropyl-1H-pyrazol-3-yl)methyl)-*N*-(4-methylpyrimidin-2-yl)-4,5,6,7-tetrahydropyrazolo[3,4-b]thiazolo[*4,5-d]*azepin-2-amin
6-((3-Fluorpyridin-2-yl)methyl)-*N*-(4-methylpyrimidin-2-yl)-4,5,6,7-tetrahydropyrazolo[3,4-b]thiazolo[4,5-d]azepin-2-amin
6-((2-Methylpyridin-4-yl)methyl)-*N*-(4-methylpyrimidin-2-yl)-4,5,6,7-tetrahydropyrazolo[3,4-b]thiazolo[4,5-d]azepin-2-amin
6-((2-Cyclopropylpyridin-4-yl)methyl)-*N*-(4-methylpyrimidin-2-yl)-4,5,6,7-tetrahydropyrazolo[3,4-b]thiazolo[4,5-d]azepin-2-amin
*N*-(4-Methylpyrimidin-2-yl)-6-(pyrimidin-4-ylmethyl)-4,5,6,7-tetrahydropyrazolo[3,4-b]thiazolo[4,5-d]azepin-2-amin
*N*-(4-Methylpyrimidin-2-yl)-6-((2-methylpyrimidin-4-yl)methyl)-4,5,6,7-tetrahydropyrazolo[3,4-b]thiazolo[4,5-d]azepin-2-amin
6-((2,5-Dimethylthiazol-4-yl)methyl)-*N*-(4-methylpyrimidin-2-yl)-4,5,6,7-tetrahydropyrazolo[3,4-b]thiazolo[4,5-d]azepin-2-amin
6-((2,4-Dimethylthiazol-5-yl)methyl)-*N*-(4-methylpyrimidin-2-yl)-4,5,6,7-tetrahydropyrazolo[3,4-b]thiazolo[4,5-d]azepin-2-amin
6-((2-Cyclopropyloxazol-4-yl)methyl)-*N*-(4-methylpyrimidin-2-yl)-4,5,6,7-tetrahydropyrazolo[3,4-b]thiazolo[4,5-d]azepin-2-amin
6-((2-Cyclopropyl-5-methyloxazol-4-yl)methyl)-*N*-(4-methylpyrimidin-2-yl)-4,5,6,7-tetrahydropyrazolo[3,4-b]thiazolo[4,5-d]azepin-2-amin
4-(2-(4-Methoxypyrimidin-2-ylamino)-4,5-dihydropyrazolo[3,4-b]thiazolo[*4,5-d*]azepin-6(7H)-yl)butan-2-on
6-(2-(Azetidin-1-yl)ethyl)-*N*-(4-methoxypyrimidin-2-yl)-4,5,6,7-tetrahydropyrazolo[3,4-b]thiazolo[4,5-d]azepin-2-amin
6-((4-Cyclopropyl-1-methyl-1H-pyrrol-2-yl)methyl)-*N*-(4-methoxypyrimidin-2-yl)-4,5,6,7-tetrahydropyrazolo[3,4-b]thiazolo[*4,5-d]*azepin-2-amin
6-((1-Cyclopropyl-1H-pyrrol-3-yl)methyl)-*N*-(4-methoxypyrimidin-2-yl)-4,5,6,7-tetrahydropyrazolo[3,4-b]thiazolo[*4,5-d]*azepin-2-amin
*N*-(4-Methoxypyrimidin-2-yl)-6-((1-methyl-1H-imidazol-2-yl)methyl)-4,5,6,7-tetrahydropyrazolo[3,4-b]thiazolo[*4,5-d]*azepin-2-amin
6-((1-Cyclopropyl-1H-pyrazol-4-yl)methyl)-*N*-(4-methoxypyrimidin-2-yl)-4,5,6,7-tetrahydropyrazolo[3,4-b]thiazolo[*4,5-d]*azepin-2-amin
6-((1-Cyclopropyl-1H-pyrazol-3-yl)methyl)-*N*-(4-methoxypyrimidin-2-yl)-4,5,6,7-tetrahydropyrazolo[3,4-b]thiazolo[*4,5-d]*azepin-2-amin
6-((3-Fluorpyridin-2-yl)methyl)-*N*-(4-methoxypyrimidin-2-yl)-4,5,6,7-tetrahydropyrazolo[3,4-b]thiazolo[4,5-d]azepin-2-amin
*N*-(4-Methoxypyrimidin-2-yl)-6-((2-methylpyridin-4-yl)methyl)-4,5,6,7-tetrahydropyrazolo[3,4-b]thiazolo[4,5-d]azepin-2-amin
6-((2-Cyclopropylpyridin-4-yl)methyl)-*N*-(4-methoxypyrimidin-2-yl)-4,5,6,7-tetrahydropyrazolo[3,4-b]thiazolo[*4,5-d]*azepin-2-amin
*N*-(4-Methoxypyrimidin-2-yl)-6-(pyrimidin-4-ylmethyl)-4,5,6,7-tetrahydropyrazolo*[3,4-b*]thiazolo[4,5-d]azepin-2-amin
*N*-(4-Methoxypyrimidin-2-yl)-6-((2-methylpyrimidin-4-yl)methyl)-4,5,6,7-tetrahydropyrazolo[3,4-b]thiazolo[4,5-d]azepin-2-amin
6-((2,5-Dimethylthiazol-4-yl)methyl)-*N*-(4-methoxypyrimidin-2-yl)-4,5,6,7-tetrahydropyrazolo[3,4-b]thiazolo[4,5-d]azepin-2-amin
6-((2,4-Dimethylthiazol-5-yl)methyl)-*N*-(4-methoxypyrimidin-2-yl)-4,5,6,7-tetrahydropyrazolo[3,4-b]thiazolo[4,5-d]azepin-2-amin
6-((2-Cyclopropyloxazol-4-yl)methyl)-*N*-(4-methoxypyrimidin-2-yl)-4,5,6,7-tetrahydropyrazolo[3,4-b]thiazolo[*4,5-d]*azepin-2-amin
6-((2-Cyclopropyl-5-methyloxazol-4-yl)methyl)-*N*-(4-methoxypyrimidin-2-yl)-4,5,6,7-tetrahydropyrazolo[3,4-b]thiazolo[*4,5-d]*azepin-2-amin
4-(2-(6-Methylpyridin-2-ylamino)-4,5-dihydropyrazolo[3,4-b]thiazolo[*4,5-d]*azepin-6(7H)-yl)butan-2-on
6-(2-(Azetidin-1-yl)ethyl)-*N*-(6-methylpyridin-2-yl)-4,5,6,7-tetrahydropyrazolo[3,4-b]thiazolo[4,5-d]azepin-2-amin
6-((4-Cyclopropyl-1-methyl-1H-pyrrol-2-yl)methyl)-*N*-(6-methylpyridin-2-yl)-4,5,6,7-tetrahydropyrazolo[3,4-b]thiazolo[*4,5-d]*azepin-2-amin
6-((1-Cyclopropyl-1H-pyrrol-3-yl)methyl)-*N*-(6-methylpyridin-2-yl)-4,5,6,7-tetrahydropyrazolo[3,4-b]thiazolo[4,5-d]azepin-2-amin
6-((1-Methyl-1H-imidazol-2-yl)methyl)-N-(6-methylpyridin-2-yl)-4,5,6,7-tetrahydropyrazolo[3,4-b]thiazolo[4,5-d]azepin-2-amin
6-((1-Cyclopropyl-1H-pyrazol-4-yl)methyl)-*N*-(6-methylpyridin-2-yl)-4,5,6,7-tetrahydropyrazolo[3,4-b]thiazolo[*4,5-d]*azepin-2-amin
6-((1-Cyclopropyl-1H-pyrazol-3-yl)methyl)-*N*-(6-methylpyridin-2-yl)-4,5,6,7-tetrahydropyrazolo[3,4-b]thiazolo[*4,5-d]*azepin-2-amin
6-((3-Fluorpyridin-2-yl)methyl)-*N*-(6-methylpyridin-2-yl)-4,5,6,7-tetrahydropyrazolo*[3,4-b*]thiazolo[4,5-d]azepin-2-amin
*N*-(6-Methylpyridin-2-yl)-6-((2-methylpyridin-4-yl)methyl)-4,5,6,7-tetrahydropyrazolo*[3,4-b*]thiazolo[4,5-d]azepin-2-amin
6-((2-Cyclopropylpyridin-4-yl)methyl)-*N*-(6-methylpyridin-2-yl)-4,5,6,7-tetrahydropyrazolo[3,4-b]thiazolo[4,5-d]azepin-2-amin
*N*-(6-Methylpyridin-2-yl)-6-(pyrimidin-4-ylmethyl)-4,5,6,7-tetrahydropyrazolo[3,4-b]thiazolo[4,5-d]azepin-2-amin
*N*-(6-Methylpyridin-2-yl)-6-((2-methylpyrimidin-4-yl)methyl)-4,5,6,7-tetrahydropyrazolo[3,4-b]thiazolo[4,5-d]azepin-2-amin
6-((2,5-Dimethylthiazol-4-yl)methyl)-*N*-(6-methylpyridin-2-yl)-4,5,6,7-tetrahydropyrazolo[3,4-b]thiazolo[4,5-d]azepin-2-amin
6-((2,4-Dimethylthiazol-5-yl)methyl)-*N*-(6-methylpyridin-2-yl)-4,5,6,7-tetrahydropyrazolo[3,4-b]thiazolo[4,5-d]azepin-2-amin
6-((2-Cyclopropyloxazol-4-yl)methyl)-*N*-(6-methylpyridin-2-yl)-4,5,6,7-tetrahydropyrazolo[3,4-b]thiazolo[4,5-d]azepin-2-amin
6-((2-Cyclopropyl-5-methyloxazol-4-yl)methyl)-*N*-(6-methylpyridin-2-yl)-4,5,6,7-tetrahydropyrazolo[3,4-b]thiazolo[*4,5-d]*azepin-2-amin

und ein pharmazeutisch akzeptables Säure- oder Basenadditionssalz davon, eine stereochemisch isomere Form davon und eine *N*-Oxidform davon.

**2.** Pharmazeutische Zusammensetzung, die eine therapeutisch wirksame Menge einer Verbindung nach Anspruch 1 und einen pharmazeutisch akzeptablen Träger und/oder Hilfsstoff umfasst.

**3.** Verbindung nach Anspruch 1 oder Zusammensetzung nach Anspruch 2 zur Verwendung bei der Behandlung oder Verhütung von Störungen des zentralen Nervensystems, ausgewählt aus der Gruppe bestehend aus: Sucht, Toleranz oder Abhängigkeit; affektiven Störungen wie Depression und Angst; psychiatrischen Krankheiten wie psychotischen Störungen, Aufmerksamkeitsdefizit/Hyperaktivitätsstörung und bipolarer Störung; Parkinson-Krankheit, Gedächtnisbeeinträchtigung, Alzheimer-Krankheit, Demenz, Delirium tremens, anderen Formen von Neurodegeneration, Neurotoxizität und Ischämie, oder zur Verwendung bei der Behandlung oder Verhütung von Störungen des zentralen Nervensystems, ausgewählt aus der Gruppe bestehend aus: kognitiven Störungen wie Delirium, substanzinduziertem persistentem Delirium, Demenz, Demenz aufgrund der HIV-Krankheit, Demenz aufgrund der Huntington-Krankheit, Demenz aufgrund der Parkinson-Krankheit, Parkinson-ALS-Demenz-Komplex, Demenz des Alzheimer-Typs, substanzinduzierter persistenter Demenz und milder kognitiver Beeinträchtigung, oder zur Verwendung bei der Behandlung von affektiven Störungen, ausgewählt aus der Gruppe bestehend aus: Angst, Agoraphobie, generalisierter Angststörung (GAD), Zwangsstörung (OCD), Panikstörung, posttraumatischer Belastungsstörung (PTSD), sozialer Phobie, anderen Phobien, substanzinduzierter Angststörung und akuter Belastungsstörung, oder zur Verwendung bei der Behandlung oder Verhütung von Störungen des zentralen Nervensystems, ausgewählt aus der Gruppe bestehend aus: Gemütsstörungen, bipolaren Störungen (I & II), Zyklothymostörungen, Depression,

dysthymer Störung, schwerer depressiver Störung und substanzinduzierter Gemütsstörung, oder zur Verwendung bei der Behandlung oder Verhütung von neurologischen Störungen, ausgewählt aus der Gruppe bestehend aus: Neurodegeneration, Neurotoxizität oder Ischämie wie Schlaganfall, Rückenmarksverletzung, Gehirnhypoxie, intrakranialem Hämatom, Parkinson-Krankheit, Gedächtnisbeeinträchtigung, Alzheimer-Krankheit, Demenz und Delirium tremens, oder zur Verwendung bei der Behandlung oder Verhütung von Entzündungsstörungen des zentralen Nervensystems, ausgewählt aus der Gruppe bestehend aus: Formen von multipler Sklerose wie benigne multiple Sklerose, schubförmig-wiederkehrende multiple Sklerose, sekundäre progressive multiple Sklerose, primäre progressive multiple Sklerose und progressive rezidivierende multiple Sklerose, oder zur Verwendung bei der Behandlung oder Verhütung von Migräne, oder zur Verwendung bei der Behandlung oder Verhütung von Epilepsie und Tremor, Temporallappen-Epilepsie, zu einer anderen Krankheit oder Verletzung sekundäre Epilepsie, wie chronische Encephalitis, traumatische Hirnverletzung, Schlaganfall oder Ischämie, oder zur Verwendung bei der Behandlung oder Verhütung von Störungen des zentralen Nervensystems, ausgewählt aus der Gruppe bestehend aus psychotischen Störungen: Schizophrenie, wahnhafte Störung, schizoaffektive Störung, schizophrenieforme Störung, substanzinduzierte psychotische Störung, oder zur Verwendung bei der Behandlung oder Verhütung von Entzündung und/oder Neurodegeneration aufgrund einer traumatischen Hirnverletzung, eines Schlaganfalls, von Ischämie, Rückenmarksverletzung, Gehirnhypoxie oder intrakranialem Hämatom, oder zur Verwendung bei der Behandlung oder Verhütung von sensorischen, motorischen oder kognitiven Symptomen aufgrund einer traumatischen Hirnverletzung, eines Schlaganfalls, von Ischämie, Rückenmarksverletzung, Gehirnhypoxie oder intrakranialem Hämatom, oder zur Verwendung bei der Behandlung von Medulloblastomen, oder zur Verwendung bei der Behandlung oder Verhütung von entzündlichen oder neuropathischen Schmerzen, oder zur Verwendung bei der Behandlung, Verhütung, Linderung, Kontrollierung oder Reduzierung des Risikos von verschiedenen metabolischen Störungen, die mit Glutamatdysfunktion assoziiert sind, oder zur Verwendung bei der Behandlung oder Verhütung von Diabetes Typ II, oder zur Verwendung bei der Behandlung oder Verhütung von Krankheiten oder Störungen der Netzhaut, Netzhautdegeneration oder Makuladegeneration, oder zur Verwendung bei der Behandlung oder Verhütung von Krankheiten oder Störungen des Magen-Darm-Trakts, einschließlich Speiseröhrenkrebs-Refluxkrankheit (GERD), Krankheiten oder Störungen des niedrigen Speiseröhrenschließmuskels, Krankheiten der gastrointestinalen Motilität, Colitis, Crohn-Krankheit oder Reizdarmsyndrom (IBS).

**4.** Verbindung nach Anspruch 1 oder Zusammensetzung nach Anspruch 2 zur Verwendung bei der Behandlung oder Verhütung von Störungen des zentralen Nervensystems, ausgewählt aus der Gruppe bestehend aus: Parkinson-Krankheit und Bewegungsstörungen wie Bradykinesie, Rigidität, Dystonie, arzneimittelinduzierter Parkinsonismus, Dyskinesie, tardive Dyskinesie, L-DOPA-induzierte Dyskinesie, Dopaminagonist-induzierte Dyskinesie, hyperkinetische Bewegungsstörungen, Gilles de la Tourette Syndrom, Ruhetremor, Aktionstremor, Akinesie, akinetisch-rigides Syndrom, Akathisie, Athetose, Asterixis, Tickstörung, posturale Instabilität, postencephalitischer Parkinsonismus, Muskelstarrheit, Chorea und choreatiforme Bewegungen, Spastizität, Myokloni, Hämiballismus, progressive supranukleäre Blickparese, Restless-Legs-Syndrom und Störung durch periodische Bewegungen der Gliedmaßen.

**5.** Verbindung nach Anspruch 1 oder Zusammensetzung nach Anspruch 2 in Kombination mit einem Mittel, ausgewählt aus der Gruppe bestehend aus: Levodopa, Levodopa mit einem selektiven extrazerebralen Decarboxylaseinhibitor, Carbidopa, Entacapon, einem COMT-Inhibitor, einem Dopaminagonisten, einem Anticholinergikum, einem cholinergen Agonisten, einem NMDA-Rezeptorantagonisten, einem MAO-B-Inhibitor, einem mGluR5-Antagonisten, einem $A_{2A}$-Antagonisten, einem Butyrophenon-Neuroleptikum, einem Diphenylbutylpiperidin-Neuroleptikum, einem heterocyclischen Dibenzazepin-Neuroleptikum, einem Indolon-Neuroleptikum, einem Phenothiazin-Neuroleptikum oder einem Thioxanthen-Neuroleptikum, zur Verwendung bei der Behandlung oder Verhütung einer Störung wie in Anspruch 4 definiert.

**6.** Verbindung nach Anspruch 1 in Kombination mit Levodopa oder in Kombination mit einem Dopaminagonisten oder in Kombination mit einem $A_{2A}$-Antagonisten zur Verwendung bei der Behandlung oder Verhütung eines Zustands gemäß Definition in Anspruch 4.

**7.** Verwendung einer Verbindung nach Anspruch 1 zur Herstellung eines Tracers zum Abbilden eines metabotropen Glutamatrezeptors oder als Geschmacksmittel, Aromamittel, geschmacksverbesserndes Mittel oder als Lebensmittel- oder Getränkezusatz.

**Revendications**

**1.** Composé, dans lequel ledit composé est un dérivé de 2-amino-4,5,6,8-tétrahydropyrazolo[3,4-*b*]thiazolo[4,5-*d*]azépine choisi parmi :

le 3-((2-(5-fluoropyrimidin-2-ylamino)-4,5-dihydropyrazolo[3,4-*b*]thiazolo[4,5-*d*]azépin-6(7*H*)-yl)méthyl)benzonitrile
la 6-(3-fluorobenzyl)-*N*-(5-fluoropyrimidin-2-yl)-4,5,6,7-tétrahydropyrazolo[3,4-b]thiazolo[4,5-*d*]azépin-2-amine
la 6-(cyclopropylméthyl)-*N*-(5-méthyl-1,2,4-thiadiazol-3-yl)-4,5,6,7-tétrahydropyrazolo[3,4-*b*]thiazolo[4,5-*d*]azépin-2-amine
la 6-((5-cyclopropylisoxazol-3-yl)méthyl)-*N*-(5-fluoropyrimidin-2-yl)-4,5,6,7-tétrahydropyrazolo[3,4-*b*]thiazolo[4,5-*d*]azépin-2-amine

et un sel d'addition d'acide ou de base pharmaceutiquement acceptable de celui-ci, une forme stéréochimiquement isomérique de celui-ci et une forme N-oxyde de celui-ci ; ou
dans lequel ledit composé est choisi parmi :

la 6-((2,3-dihydrobenzofuran-5-yl)méthyl)-*N*-(5-fluoropyrimidin-2-yl)-4,5,6,7-tétrahydropyrazolo[3,4-*b*]thiazolo[4,5-*d*]azépin-2-amine
la *N*-(5-fluoropyrimidin-2-yl)-6-(3-(trifluorométhyl)benzyl)-4,5,6,7-tétrahydropyrazolo[3,4-*b*]thiazolo[4,5-*d*]azépin-2-amine
la 6-((3-cyclopropylisoxazol-5-yl)méthyl)-*N*-(5-fluoropyrimidin-2-yl)-4,5,6,7-tétrahydropyrazolo[3,4-*b*]thiazolo[4,5-*d*]azépin-2-amine
la 6-((2-cyclopropylthiazol-4-yl)méthyl)-*N*-(5-fluoropyrimidin-2-yl)-4,5,6,7-tétrahydropyrazolo[3,4-*b*]thiazolo[4,5-*d*]azépin-2-amine
la 6-(1-méthoxypropan-2-yl)-*N*-(5-méthyl-1,2,4-thiadiazol-3-yl)-4,5,6,7-tétrahydropyrazolo[3,4-*b*]thiazolo[4,5-*d*]azépin-2-amine
la 6-((5-chloropyridin-2-yl)méthyl)-*N*-(4-méthylpyrimidin-2-yl)-4,5,6,7-tétrahydropyrazolo[3,4-*b*]thiazolo[4,5-*d*]azépin-2-amine
la *N*-(5-fluoropyrimidin-2-yl)-6-(3-méthoxycyclobutyl)-4,5,6,7-tétrahydropyrazolo[3,4-b]thiazolo[4,5-*d*]azépin-2-amine
la *N*-(5-fluoropyrimidin-2-yl)-6-(oxétan-3-yl)-4,5,6,7-tétrahydropyrazolo[3,4-b]thiazolo[4,5-*d*]azépin-2-amine
la 6-(3,3-difluorocyclobutyl)-*N*-(5-fluoropyrimidin-2-yl)-4,5,6,7-tétrahydropyrazolo[3,4-b]thiazolo[4,5-*d*]azépin-2-amine
la 6-((3,3-difluorocyclobutyl)méthyl)-*N*-(5-fluoropyrimidin-2-yl)-4,5,6,7-tétrahydropyrazolo[3,4-*b*]thiazolo[4,5-*d*]azépin-2-amine
la *N*-(5-fluoropyrimidin-2-yl)-6-(oxétan-3-ylméthyl)-4,5,6,7-tétrahydropyrazolo[3,4-b]thiazolo[4,5-*d*]azépin-2-amine
la *N*-(5-méthyl-1,2,4-thiadiazol-3-yl)-4,5,6,7-tétrahydropyrazolo[3,4-b]thiazolo[4,5-*d*]azépin-2-amine
la *N*-(5-méthyl-1,2,4-thiadiazol-3-yl)-6-(oxétan-3-ylméthyl)-4,5,6,7-tétrahydropyrazolo[3,4-*b*]thiazolo[4,5-*d*]azépin-2-amine
la *N*-(5-méthyl-1,2,4-thiadiazol-3-yl)-6-((3-méthyloxétan-3-yl)méthyl)-4,5,6,7-tétrahydropyrazolo[3,4-*b*]thiazolo[4,5-*d*]azépin-2-amine
la 6-(cyclobutylméthyl)-*N*-(5-méthyl-1,2,4-thiadiazol-3-yl)-4,5,6,7-tétrahydropyrazolo[3,4-*b*]thiazolo[4,5-*d*] azépin-2-amine
la *N*-(5-méthyl-1,2,4-thiadiazol-3-yl)-6-(oxétan-3-yl)-4,5,6,7-tétrahydropyrazolo[3,4-b]thiazolo[4,5-*d*]azépin-2-amine
la 6-cyclopropyl-*N*-(5-méthyl-1,2,4-thiadiazol-3-yl)-4,5,6,7-tétrahydropyrazolo[3,4-b]thiazolo[4,5-*d*]azépin-2-amine
la *N*-(5-méthyl-1,2,4-thiadiazol-3-yl)-6-((tétrahydrofuran-2-yl)méthyl)-4,5,6,7-tétrahydropyrazolo[3,4-*b*]thiazolo[4,5-*d*]azépin-2-amine
la N-(5-méthyl-1,2,4-thiadiazol-3-yl)-6-((tétrahydrofuran-3-yl)méthyl)-4,5,6,7-tétrahydropyrazolo[3,4-*b*]thiazolo[4,5-*d*]azépin-2-amine
la 6-((1-(méthoxyméthyl)cyclopropyl)méthyl)-*N*-(5-méthyl-1,2,4-thiadiazol-3-yl)-4,5,6,7-tétrahydropyrazolo[3,4-*b*]thiazolo[4,5-*d*] azépin-2-amine
la 6-((3,3-difluorocyclobutyl)méthyl)-*N*-(5-méthyl-1,2,4-thiadiazol-3-yl)-4,5,6,7-tétrahydropyrazolo[3,4-*b*]thiazolo[4,5-*d*]azépin-2-amine
la 6-(3,3-difluorocyclobutyl)-*N*-(5-méthyl-1,2,4-thiadiazol-3-yl)-4,5,6,7-tétrahydropyrazolo[3,4-*b*]thiazolo[4,5-*d*]azépin-2-amine
la *N*-(5-méthyl-1,2,4-thiadiazol-3-yl)-6-(tétrahydro-2*H*-pyran-4-yl)-4,5,6,7-tétrahydropyrazolo[3,4-*b*]thiazolo[4,5-*d*]azépin-2-amine

la 6-(2-cyclopropoxyéthyl)-*N*-(5-méthyl-1,2,4-thiadiazol-3-yl)-4,5,6,7-tétrahydropyrazolo[3,4-*b*]thiazolo[4,5-*d*]azépin-2-amine
la 6-(2-éthoxyéthyl)-*N*-(5-méthyl-1,2,4-thiadiazol-3-yl)-4,5,6,7-tétrahydropyrazolo[3,4-b]thiazolo[4,5-*d*]azépin-2-amine
la 6-(1-méthoxypropan-2-yl)-*N*-(5-méthyl-1,2,4-thiadiazol-3-yl)-4,5,6,7-tétrahydropyrazolo[3,4-*b*]thiazolo[4,5-*d*]azépin-2-amine
la *N*-(4-méthoxypyrimidin-2-yl)-4,5,6,7-tétrahydropyrazolo[3,4-*b*]thiazolo[4,5-*d*]azépin-2-amine
la *N*-(4-méthoxypyrimidin-2-yl)-6-(oxétan-3-ylméthyl)-4,5,6,7-tétrahydropyrazolo[3,4-b]thiazolo[4,5-*d*]azépin-2-amine
la *N*-(4-méthoxypyrimidin-2-yl)-6-((3-méthyloxétan-3-yl)méthyl)-4,5,6,7-tétrahydropyrazolo[3,4-*b*]thiazolo[4,5-*d*]azépin-2-amine
la 6-(cyclobutylméthyl)-*N*-(4-méthoxypyrimidin-2-yl)-4,5,6,7-tétrahydropyrazolo[3,4-b]thiazolo[4,5-*d*]azépin-2-amine
la *N*-(4-méthoxypyrimidin-2-yl)-6-(oxétan-3-yl)-4,5,6,7-tétrahydropyrazolo[3,4-b]thiazolo[4,5-*d*]azépin-2-amine
la 6-cyclopropyl-*N*-(4-méthoxypyrimidin-2-yl)-4,5,6,7-tétrahydropyrazolo[3,4-b]thiazolo[4,5-*d*]azépin-2-amine
la *N*-(4-méthoxypyrimidin-2-yl)-6-((tétrahydrofuran-2-yl)méthyl)-4,5,6,7-tétrahydropyrazolo[3,4-*b*]thiazolo[4,5-*d*]azépin-2-amine
la *N*-(4-méthoxypyrimidin-2-yl)-6-((tétrahydrofuran-3-yl)méthyl)-4,5,6,7-tétrahydropyrazolo[3,4-*b*]thiazolo[4,5-*d*]azépin-2-amine
la 6-((1-(méthoxyméthyl)cyclopropyl)méthyl)-*N*-(4-méthoxypyrimidin-2-yl)-4,5,6,7-tétrahydropyrazolo[3,4-*b*]thiazolo[4,5-*d*]azépin-2-amine
la 6-((3,3-difluorocyclobutyl)méthyl)-*N*-(4-méthoxypyrimidin-2-yl)-4,5,6,7-tétrahydropyrazolo[3,4-*b*]thiazolo[4,5-*d*]azépin-2-amine
la 6-(3,3-difluorocyclobutyl)-*N*-(4-méthoxypyrimidin-2-yl)-4,5,6,7-tétrahydropyrazolo[3,4-*b*]thiazolo[4,5-*d*]azépin-2-amine
la *N*-(4-méthoxypyrimidin-2-yl)-6-(tétrahydro-2*H*-pyran-4-yl)-4,5,6,7-tétrahydropyrazolo[3,4-*b*]thiazolo[4,5-*d*]azépin-2-amine
la 6-(2-cyclopropoxyéthyl)-*N*-(4-méthoxypyrimidin-2-yl)-4,5,6,7-tétrahydropyrazolo[3,4-*b*]thiazolo[4,5-*d*]azépin-2-amine
la 6-(2-éthoxyéthyl)-*N*-(4-méthoxypyrimidin-2-yl)-4,5,6,7-tétrahydropyrazolo[3,4-b]thiazolo[4,5-*d*]azépin-2-amine
la 6-(1-méthoxypropan-2-yl)-*N*-(4-méthoxypyrimidin-2-yl)-4,5,6,7-tétrahydropyrazolo[3,4-*b*]thiazolo[4,5-*d*]azépin-2-amine
la *N*-(5-fluoropyrimidin-2-yl)-6-((1-(méthoxyméthyl)cyclopropyl)méthyl)-4,5,6,7-tétrahydropyrazolo[3,4-*b*]thiazolo[4,5-*d*]azépin-2-amine
la *N*-(5-fluoropyrimidin-2-yl)-6-(tétrahydro-2*H*-pyran-4-yl)-4,5,6,7-tétrahydropyrazolo[3,4-*b*]thiazolo[4,5-*d*]azépin-2-amine
la 6-cyclopropyl-*N*-(5-fluoropyrimidin-2-yl)-4,5,6,7-tétrahydropyrazolo[3,4-*b*] thiazolo[4,5-*d*]azépin-2-amine
la 6-(2-éthoxyéthyl)-*N*-(5-fluoropyrimidin-2-yl)-4,5,6,7-tétrahydropyrazolo[3,4-b]thiazolo[4,5-*d*]azépin-2-amine
la *N*-(5-fluoropyrimidin-2-yl)-6-(2-méthoxypropyl)-4,5,6,7-tétrahydropyrazolo[3,4-b]thiazolo[4,5-*d*]azépin-2-amine
la *N*-(4-méthylpyrimidin-2-yl)-4,5,6,7-tétrahydropyrazolo[3,4-*b*]thiazolo[4,5-*d*]azépin-2-amine
la *N*-(4-méthylpyrimidin-2-yl)-6-(oxétan-3-yl)-4,5,6,7-tétrahydropyrazolo[3,4-b]thiazolo[4,5-*d*]azépin-2-amine
la 6-cyclopropyl-*N*-(4-méthylpyrimidin-2-yl)-4,5,6,7-tétrahydropyrazolo[3,4-b]thiazolo[4,5-*d*]azépin-2-amine
la *N*-(4-méthylpyrimidin-2-yl)-6-(oxétan-3-ylméthyl)-4,5,6,7-tétrahydropyrazolo[3,4-b]thiazolo[4,5-*d*]azépin-2-amine
la *N*-(4-méthylpyrimidin-2-yl)-6-((tétrahydrofuran-3-yl)méthyl)-4,5,6,7-tétrahydropyrazolo[3,4-*b*]thiazolo[4,5-*d*]azépin-2-amine
la *N*-(4-méthylpyrimidin-2-yl)-6-(tétrahydro-2*H*-pyran-4-yl)-4,5,6,7-tétrahydropyrazolo[3,4-*b*]thiazolo[4,5-*d*]azépin-2-amine
la $N^2$-(6-(cyclopropylméthyl)-4,5,6,7-tétrahydropyrazolo[3,4-*b*]thiazolo[4,5-*d*]azépin-2-yl)pyrimidine-2,4-diamine
la $N^2$-(6-(2-méthoxyéthyl)-4,5,6,7-tétrahydropyrazolo[3,4-*b*]thiazolo[4,5-*d*]azépin-2-yl)pyrimidine-2,4-diamine
la 4-(2-(5-fluoropyrimidin-2-ylamino)-4,5-dihydropyrazolo[3,4-*b*]thiazolo[4,5-*d*]azépin-6(7*H*)-yl)butan-2-one
la 6-(2-(azétidin-1-yl)éthyl)-*N*-(5-fluoropyrimidin-2-yl)-4,5,6,7-tétrahydropyrazolo[3,4-b]thiazolo[4,5-*d*]azépin-2-amine
la 6-((4-cyclopropyl-1-méthyl-1*H*-pyrrol-2-yl)méthyl)-*N*-(5-fluoropyrimidin-2-yl)-4,5,6,7-tétrahydropyrazolo[3,4-*b*]thiazolo[4,5-*d*]azépin-2-amine

la 6-((1-cyclopropyl-1*H*pyrrol-3-yl)méthyl)-*N*-(5-fluoropyrimidin-2-yl)-4,5,6,7-tétrahydropyrazolo[3,4-*b*]thiazolo[4,5-*d*]azépin-2-amine

la *N*-(5-fluoropyrimidin-2-yl)-6-((1-méthyl-1*H*-imidazol-2-yl)méthyl)-4,5,6,7-tétrahydropyrazolo[3,4-*b*]thiazolo[4,5-*d*]azépin-2-amine

la 6-((1-cyclopropyl-1*H*-pyrazol-4-yl)méthyl)-*N*-(5-fluoropyrimidin-2-yl)-4,5,6,7-tétrahydropyrazolo[3,4-*b*]thiazolo[4,5-*d*]azépin-2-amine

la 6-((1-cyclopropyl-1*H*-pyrazol-3-yl)méthyl)-*N*-(5-fluoropyrimidin-2-yl)-4,5,6,7-tétrahydropyrazolo[3,4-*b*]thiazolo[4,5-*d*]azépin-2-amine

la 6-((3-fluoropyridin-2-yl)méthyl)-*N*-(5-fluoropyrimidin-2-yl)-4,5,6,7-tétrahydropyrazolo[3,4-*b*]thiazolo[4,5-*d*]azépin-2-amine

la *N*-(5-fluoropyrimidin-2-yl)-6-((2-méthylpyridin-4-yl)méthyl)-4,5,6,7-tétrahydropyrazolo[3,4-*b*]thiazolo[4,5-*d*]azépin-2-amine

la 6-((2-cyclopropylpyridin-4-yl)méthyl)-*N*-(5-fluoropyrimidin-2-yl)-4,5,6,7-tétrahydropyrazolo[3,4-*b*]thiazolo[4,5-*d*]azépin-2-amine

la *N*-(5-fluoropyrimidin-2-yl)-6-(pyrimidin-4-ylméthyl)-4,5,6,7-tétrahydropyrazolo[3,4-b]thiazolo[4,5-*d*]azépin-2-amine

la *N*-(5-fluoropyrimidin-2-yl)-6-((2-méthylpyrimidin-4-yl)méthyl)-4,5,6,7-tétrahydropyrazolo[3,4-*b*]thiazolo[4,5-*d*]azépin-2-amine

la 6-((2,5-diméthylthiazol-4-yl)méthyl)-*N*-(5-fluoropyrimidin-2-yl)-4,5,6,7-tétrahydropyrazolo[3,4-*b*]thiazolo[4,5-*d*]azépin-2-amine

la 6-((2,4-diméthylthiazol-5-yl)méthyl)-*N*-(5-fluoropyrimidin-2-yl)-4,5,6,7-tétrahydropyrazolo[3,4-*b*]thiazolo[4,5-*d*]azépin-2-amine

la 6-((2-cyclopropyloxazol-4-yl)méthyl)-*N*-(5-fluoropyrimidin-2-yl)-4,5,6,7-tétrahydropyrazolo[3,4-*b*]thiazolo[4,5-*d*]azépin-2-amine

la 6-((2-cyclopropyl-5-méthyloxazol-4-yl)méthyl)-*N*-(5-fluoropyrimidin-2-yl)-4,5,6,7-tétrahydropyrazolo[3,4-*b*]thiazolo[4,5-*d*]azépin-2-amine

le 4-(2-(4-méthylpyrimidin-2-ylamino)-4,5-dihydropyrazolo[3,4-b]thiazolo[4,5-*d*] azépin-6(7*H*)-yl)butan-2-one

la 6-(2-(azétidin-1-yl)éthyl)-*N*-(4-méthylpyrimidin-2-yl)-4,5,6,7-tétrahydropyrazolo[3,4-b]thiazolo[4,5-*d*]azépin-2-amine

la 6-((4-cyclopropyl-1-méthyl-1*H*-pyrrol-2-yl)méthyl)-*N*-(4-méthylpyrimidin-2-yl)-4,5,6,7-tétrahydropyrazolo[3,4-*b*]thiazolo[4,5-*d*]azépin-2-amine

la 6-((1-cyclopropyl-1*H*-pyrrol-3-yl)méthyl)-*N*-(4-méthylpyrimidin-2-yl)-4,5,6,7-tétrahydropyrazolo[3,4-*b*]thiazolo[4,5-*d*]azépin-2-amine

la 6-((1-méthyl-1*H*-imidazol-2-yl)méthyl)-*N*-(4-méthylpyrimidin-2-yl)-4,5,6,7-tétrahydropyrazolo[3,4-*b*]thiazolo[4,5-*d*]azépin-2-amine

la 6-((1-cyclopropyl-1*H*-pyrazol-4-yl)méthyl)-*N*-(4-méthylpyrimidin-2-yl)-4,5,6,7-tétrahydropyrazolo[3,4-*b*]thiazolo[4,5-*d*]azépin-2-amine

la 6-((1-cyclopropyl-1*H*-pyrazol-3-yl)méthyl)-*N*-(4-méthylpyrimidin-2-yl)-4,5,6,7-tétrahydropyrazolo[3,4-*b*]thiazolo[4,5-*d*]azépin-2-amine

la 6-((3-fluoropyridin-2-yl)méthyl)-*N*-(4-méthylpyrimidin-2-yl)-4,5,6,7-tétrahydropyrazolo[3,4-*b*]thiazolo[4,5-*d*]azépin-2-amine

la 6-((2-méthylpyridin-4-yl)méthyl)-*N*-(4-méthylpyrimidin-2-yl)-4,5,6,7-tétrahydropyrazolo[3,4-*b*]thiazolo[4,5-*d*]azépin-2-amine

la 6-((2-cyclopropylpyridin-4-yl)méthyl)-*N*-(4-méthylpyrimidin-2-yl)-4,5,6,7-tétrahydropyrazolo[3,4-*b*]thiazolo[4,5-*d*]azépin-2-amine

la *N*-(4-méthylpyrimidin-2-yl)-6-(pyrimidin-4-ylméthyl)-4,5,6,7-tétrahydropyrazolo[3,4-b]thiazolo[4,5-*d*]azépin-2-amine

la *N*-(4-méthylpyrimidin-2-yl)-6-((2-méthylpyrimidin-4-yl)méthyl)-4,5,6,7-tétrahydropyrazolo[3,4-*b*]thiazolo[4,5-*d*]azépin-2-amine

la 6-((2,5-diméthylthiazol-4-yl)méthyl)-*N*-(4-méthylpyrimidin-2-yl)-4,5,6,7-tétrahydropyrazolo[3,4-*b*]thiazolo[4,5-*d*]azépin-2-amine

la 6-((2,4-diméthylthiazol-5-yl)méthyl)-*N*-(4-méthylpyrimidin-2-yl)-4,5,6,7-tétrahydropyrazolo[3,4-*b*]thiazolo[4,5-*d*]azépin-2-amine

la 6-((2-cyclopropyloxazol-4-yl)méthyl)-*N*-(4-méthylpyrimidin-2-yl)-4,5,6,7-tétrahydropyrazolo[3,4-*b*]thiazolo[4,5-*d*]azépin-2-amine

la 6-((2-cyclopropyl-5-méthyloxazol-4-yl)méthyl)-*N*-(4-méthylpyrimidin-2-yl)-4,5,6,7-tétrahydropyrazolo[3,4-*b*]thiazolo[4,5-*d*]azépin-2-amine

le 4-(2-(4-méthoxypyrimidin-2-ylamino)-4,5-dihydropyrazolo[3,4-*b*]thiazolo[4,5-*d*]azépin-6(7*H*)-yl)butan-2-one

la 6-(2-(azétidin-1-yl)éthyl)-*N*-(4-méthoxypyrimidin-2-yl)-4,5,6,7-tétrahydropyrazolo [3,4-*b*]thiazolo[4,5-*d*]azépin-2-amine

la 6-((4-cyclopropyl-1-méthyl-1*H*-pyrrol-2-yl)méthyl)-*N*-(4-méthoxypyrimidin-2-yl)-4,5,6,7-tétrahydropyrazolo[3,4-*b*]thiazolo[4,5-*d*]azépin-2-amine

la 6-((1-cyclopropyl-1*H*-pyrrol-3-yl)méthyl)-*N*-(4-méthoxypyrimidin-2-yl)-4,5,6,7-tétrahydropyrazolo[3,4-*b*]thiazolo[4,5-*d*]azépin-2-amine

la *N*-(4-méthoxypyrimidin-2-yl)-6-((1-méthyl-1*H*-imidazol-2-yl)méthyl)-4,5,6,7-tétrahydropyrazolo[3,4-*b*]thiazolo[4,5-*d*]azépin-2-amine

la 6-((1-cyclopropyl-1*H*-pyrazol-4-yl)méthyl)-*N*-(4-méthoxypyrimidin-2-yl)-4,5,6,7-tétrahydropyrazolo[3,4-*b*] thiazolo[4,5-*d*]azépin-2-amine

la 6-((1-cyclopropyl-1*H*-pyrazol-3-yl)méthyl)-*N*-(4-méthoxypyrimidin-2-yl)-4,5,6,7-tétrahydropyrazolo[3,4-*b*] thiazolo[4,5-*d*]azépin-2-amine

la 6-((3-fluoropyridin-2-yl)méthyl)-*N*-(4-méthoxypyrimidin-2-yl)-4,5,6,7-tétrahydropyrazolo[3,4-*b*]thiazolo[4,5-*d*] azépin-2-amine

la *N*-(4-méthoxypyrimidin-2-yl)-6-((2-méthylpyridin-4-yl)méthyl)-4,5,6,7-tétrahydropyrazolo[3,4-*b*]thiazolo[4,5-*d*]azépin-2-amine

la 6-((2-cyclopropylpyridin-4-yl)méthyl)-*N*-(4-méthoxypyrimidin-2-yl)-4,5,6,7-tétrahydropyrazolo[3,4-*b*]thiazolo[4,5-*d*]azépin-2-amine

la *N*-(4-méthoxypyrimidin-2-yl)-6-(pyrimidin-4-ylméthyl)-4,5,6,7-tétrahydropyrazolo[3,4-*b*]thiazolo[4,5-*d*]azépin-2-amine

la *N*-(4-méthoxypyrimidin-2-yl)-6-((2-méthylpyrimidin-4-yl)méthyl)-4,5,6,7-tétrahydropyrazolo[3,4-*b*]thiazolo[4,5-*d*]azépin-2-amine

la 6-((2,5-diméthylthiazol-4-yl)méthyl)-*N*-(4-méthoxypyrimidin-2-yl)-4,5,6,7-tétrahydropyrazolo[3,4-*b*]thiazolo[4,5-*d*]azépin-2-amine

la 6-((2,4-diméthylthiazol-5-yl)méthyl)-*N*-(4-méthoxypyrimidin-2-yl)-4,5,6,7-tétrahydropyrazolo[3,4-*b*]thiazolo[4,5-*d*]azépin-2-amine

la 6-((2-cyclopropyloxazol-4-yl)méthyl)-*N*-(4-méthoxypyrimidin-2-yl)-4,5,6,7-tétrahydropyrazolo[3,4-*b*]thiazolo[4,5-*d*]azépin-2-amine

la 6-((2-cyclopropyl-5-méthyloxazol-4-yl)méthyl)-*N*-(4-méthoxypyrimidin-2-yl)-4,5,6,7-tétrahydropyrazolo[3,4-*b*]thiazolo[4,5-*d*]azépin-2-amine

le 4-(2-(6-méthylpyridin-2-ylamino)-4,5-dihydropyrazolo[3,4-*b*]thiazolo[4,5-*d*]azépin-6(7*H*)-yl)butan-2-one

la 6-(2-(azétidin-1-yl)éthyl)-*N*-(6-méthylpyridin-2-yl)-4,5,6,7-tétrahydropyrazolo[3,4-b]thiazolo[4,5-*d*]azépin-2-amine

la 6-((4-cyclopropyl-1-méthyl-1H-pyrrol-2-yl)méthyl)-*N*-(6-méthylpyridin-2-yl)-4,5,6,7-tétrahydropyrazolo[3,4-*b*] thiazolo[4,5-*d*]azépin-2-amine

la 6-((1-cyclopropyl-1*H*-pyrrol-3-yl)méthyl)-*N*-(6-méthylpyridin-2-yl)-4,5,6,7-tétrahydropyrazolo[3,4-*b*]thiazolo[4,5-*d*]azépin-2-amine

la 6-((1-méthyl-1*H*-imidazol-2-yl)méthyl)-*N*-(6-méthylpyridin-2-yl)-4,5,6,7-tétrahydropyrazolo[3,4-*b*]thiazolo[4,5-*d*]azépin-2-amine

la 6-((1-cyclopropyl-1H-pyrazol-4-yl)méthyl)-*N*-(6-méthylpyridin-2-yl)-4,5,6,7-tétrahydropyrazolo[3,4-*b*]thiazolo[4,5-*d*]azépin-2-amine

la 6-((1-cyclopropyl-1*H*-pyrazol-3-yl)méthyl)-*N*-(6-méthylpyridin-2-yl)-4,5,6,7-tétrahydropyrazolo[3,4-*b*]thiazolo[4,5-*d*]azépin-2-amine

la 6-((3-fluoropyridin-2-yl)méthyl)-*N*-(6-méthylpyridin-2-yl)-4,5,6,7-tétrahydropyrazolo[3,4-*b*]thiazolo[4,5-*d*]azépin-2-amine

la *N*-(6-méthylpyridin-2-yl)-6-((2-méthylpyridin-4-yl)méthyl)-4,5,6,7-tétrahydropyrazolo[3,4-*b*]thiazolo[4,5-*d*] azépin-2-amine

la 6-((2-cyclopropylpyridin-4-yl)méthyl)-*N*-(6-méthylpyridin-2-yl)-4,5,6,7-tétrahydropyrazolo[3,4-*b*]thiazolo[4,5-*d*]azépin-2-amine

la *N*-(6-méthylpyridin-2-yl)-6-(pyrimidin-4-ylméthyl)-4,5,6,7-tétrahydropyrazolo[3,4-b]thiazolo[4,5-*d*]azépin-2-amine

la *N*-(6-méthylpyridin-2-yl)-6-((2-méthylpyrimidin-4-yl)méthyl)-4,5,6,7-tétrahydropyrazolo[3,4-*b*]thiazolo[4,5-*d*] azépin-2-amine

la 6-((2,5-diméthylthiazol-4-yl)méthyl)-*N*-(6-méthylpyridin-2-yl)-4,5,6,7-tétrahydropyrazolo[3,4-*b*]thiazolo[4,5-*d*]azépin-2-amine

la 6-((2,4-diméthylthiazol-5-yl)méthyl)-*N*-(6-méthylpyridin-2-yl)-4,5,6,7-tétrahydropyrazolo[3,4-*b*]thiazolo[4,5-*d*]azépin-2-amine

la 6-((2-cyclopropyloxazol-4-yl)méthyl)-*N*-(6-méthylpyridin-2-yl)-4,5,6,7-tétrahydropyrazolo[3,4-*b*]thiazolo[4,5-*d*]azépin-2-amine
la 6-((2-cyclopropyl-5-méthyloxazol-4-yl)méthyl)-*N*-(6-méthylpyridin-2-yl)-4,5,6,7-tétrahydropyrazolo[3,4-*b*] thiazolo[4,5-*d*]azépin-2-amine

et un sel d'addition d'acide ou de base pharmaceutiquement acceptable de celui-ci, une forme stéréochimiquement isomérique de celui-ci et une forme *N*-oxyde de celui-ci.

**2.** Composition pharmaceutique comprenant une quantité thérapeutiquement efficace d'un composé selon la revendication 1 et un vecteur et/ou excipient pharmaceutiquement acceptable.

**3.** Composé selon la revendication 1 ou composition selon la revendication 2, à utiliser dans le traitement ou la prévention de troubles du système nerveux central choisis dans le groupe consistant en : l'addiction, la tolérance ou la dépendance ; les troubles affectifs, tels que la dépression et l'anxiété ; une maladie psychiatrique telle que les troubles psychotiques, un trouble de déficit de l'attention/d'hyperactivité et un trouble bipolaire ; la Maladie de Parkinson, une altération de la mémoire, la maladie d'Alzheimer, la démence, le delirium tremens, d'autres formes de neurodégénérescence, la neurotoxicité, et l'ischémie, ou à utiliser dans le traitement ou la prévention de troubles du système nerveux central choisis dans le groupe consistant en : les troubles cognitifs tels que le delirium, le delirium persistant induit par substance, la démence, la démence due à la maladie du VIH, la démence due à la maladie de Huntington, la démence due à la maladie de Parkinson, le complexe de démence parkinsonien-ALS, la démence du type Alzheimer, la démence persistante induite par substance, et le trouble cognitif léger, ou à utiliser dans le traitement de troubles affectifs choisis dans le groupe consistant en : l'anxiété, l'agoraphobie, le trouble d'anxiété généralisé (TAG), le trouble obsessionnel compulsif (TOC), le trouble de panique, le trouble de stress post-traumatique (TSPT), la phobie sociale, d'autres phobies, le trouble d'anxiété induit par substance, et le trouble de stress aigu, ou à utiliser dans le traitement ou la prévention de troubles du système nerveux central choisis dans le groupe consistant en : les troubles de l'humeur, les troubles bipolaires (I & II), le trouble cyclothymique, la dépression, le trouble dysthymique, le trouble dépressif majeur, et le trouble de l'humeur induit par substance, ou à utiliser dans le traitement ou la prévention de troubles neurologiques choisis dans le groupe consistant en : la neurodégénérescence, la neurotoxicité ou l'ischémie telle qu'un accident vasculaire cérébral, une lésion de la moelle épinière, une hypoxie cérébrale, un hématome intracrânien, la maladie de Parkinson, une altération de la mémoire, la maladie d'Alzheimer, la démence, et le delirium tremens, ou à utiliser dans le traitement ou la prévention de troubles inflammatoires du système nerveux central choisis dans le groupe consistant en : les formes de sclérose en plaques telle que la sclérose en plaques bénigne, la sclérose en plaques rémanente-rémittente, la sclérose en plaques progressive secondaire, la sclérose en plaques progressive primaire, et la sclérose en plaques progressive rémittente, ou à utiliser dans le traitement ou la prévention de la migraine, ou à utiliser dans le traitement ou la prévention de l'épilepsie et du tremblement, d'une épilepsie temporale, d'une épilepsie consécutive à une autre maladie ou lésion telle qu'une encéphalite chronique, une lésion du cerveau traumatique, un accident vasculaire cérébral ou une ischémie, ou à utiliser dans le traitement ou la prévention de troubles du système nerveux central choisis dans le groupe consistant en les troubles psychotiques : la schizophrénie, un trouble délirant, un trouble schizoaffectif, un trouble schizophréniforme, un trouble psychotique induit par substance, ou à utiliser dans le traitement ou la prévention d'une inflammation et/ou d'une neurodégénérescence résultant d'une lésion du cerveau traumatique, d'un accident vasculaire cérébral, d'une ischémie, d'une lésion de la moelle épinière, d'une hypoxie cérébrale ou d'un hématome intracrânien, ou à utiliser dans le traitement ou la prévention de symptômes sensoriels, moteurs ou cognitifs résultant d'une lésion du cerveau traumatique, d'un accident vasculaire cérébral, d'une ischémie, d'une lésion de la moelle épinière, d'une hypoxie cérébrale ou d'un hématome intracrânien, ou à utiliser dans le traitement de médulloblastomes, ou à utiliser dans le traitement ou la prévention d'une douleur inflammatoire ou neuropathique, ou à utiliser dans le traitement, la prévention, l'amélioration, le contrôle ou la réduction du risque de divers troubles métaboliques associés à un dysfonctionnement du glutamate, ou à utiliser dans le traitement ou la prévention du diabète de type 2, ou à utiliser dans le traitement ou la prévention de maladies ou troubles de la rétine, de la dégénérescence rétinienne ou de la dégénérescence maculaire, ou à utiliser dans le traitement ou la prévention de maladies ou troubles du tractus gastro-intestinal dont la maladie du reflux gastro-oesophagien (MRGO), les maladies ou troubles du sphincter oesophagien inférieur, des maladies de motilité gastro-intestinale, la colite, la maladie de Crohn ou le syndrome du côlon irritable (SCI).

**4.** Composé selon la revendication 1 ou composition selon la revendication 2, à utiliser dans le traitement ou la prévention de troubles du système nerveux central choisis dans le groupe consistant en : la maladie de Parkinson et les troubles du mouvement tels que la bradykinésie, la rigidité, la dystonie, le parkinsonisme induit par drogue ou médicament, la dyskinésie, la dyskinésie tardive, la dyskinésie induite par L-DOPA, la dyskinésie induite par agoniste de la dopamine, les troubles du mouvement hyperkinétique, le syndrome de Gilles de la Tourette, le

tremblement de repos, le tremblement d'intention, l'akinésie, le syndrome akinéto-rigide, l'akathisie, l'athétose, l'astérixis, les tics, l'instabilité posturale, le parkinsonisme post-encéphalitique, la rigidité musculaire, la chorée et les mouvements choréiformes, la spasticité, le myoclonus, l'hémiballisme, la paralysie pseudo-bulbaire progressive, le syndrome des jambes sans repos, et le trouble du mouvement involontaire des membres.

**5.** Composé selon la revendication 1 ou composition selon la revendication 2, en combinaison avec un agent choisi dans le groupe consistant en : le lévodopa, le lévodopa avec un inhibiteur de la décarboxylase extracérébral sélectif, le carbidopa, l'entacapone, un inhibiteur de la COMT, un agoniste de la dopamine, un anti-cholinergique, un agoniste cholinergique, un antagoniste de récepteur NMDA, un inhibiteur de la MAO-B, un antagoniste de mGluR5, un antagoniste d'$A_{2A}$, un agent neuroleptique butyrophénone, un agent neuroleptique diphénylbutylpipéridine, un agent neuroleptique dibenzazépine hétérocyclique, un agent neuroleptique indolone, un agent neuroleptique phénothiazine ou un agent neuroleptique thioxanthène, à utiliser dans le traitement ou la prévention d'un trouble tel que défini dans la revendication 4.

**6.** Composé selon la revendication 1, en combinaison avec du lévodopa ou en combinaison avec un agoniste de la dopamine ou en combinaison avec un antagoniste d'$A_{2A}$ à utiliser dans le traitement ou la prévention d'une affection telle que définie dans la revendication 4.

**7.** Utilisation d'un composé selon la revendication 1, pour préparer un traceur en vue de réaliser une image d'un récepteur métabotrope du glutamate, ou en tant qu'agent de goût, agent d'arôme, agent exhausteur d'arôme ou additif pour aliment ou boisson.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005007096 A
- WO 2009010454 A
- WO 2009010455 A
- WO 2010079238 A
- WO 2012009001 A

### Non-patent literature cited in the description


- **NAKANISHI S. et al.** *Brain Res. Rev.,* 1998, vol. 26 (2-3), 230-235
- **SCHOEPP D.D. et al.** *Neuropharmacology,* 1999, vol. 38 (10), 1431-1476
- **BRADLEY et al.** *Journal of Comparative Neurology,* 1999, vol. 407, 33-46
- **CORTI et al.** *Neuroscience,* 2002, vol. 110, 403-420
- **MILLAN et al.** *Journal of Biological Chemistry,* 2002, vol. 277, 47796-47803
- **VALENTI et al.** *Journal of Neuroscience,* 2003, vol. 23, 7218-7226
- **SCHOEPP et al.** *Neuropharmacology,* 1999, vol. 38, 1431-1476
- **VALENTI et al.** *J. Neurosci.,* 2003, vol. 23, 7218-7226
- **BRUNO et al.** *J. Neurosci.,* 2000, vol. 20, 6413-6420
- **MARINO et al.** *Curr. Topics Med. Chem.,* 2005, vol. 5, 885-895
- **KONIECZNY et al.** *Neuroscience,* 2007, vol. 145, 611-620
- **LOPEZ et al.** *J. Neuroscience,* 2007, vol. 27, 6701-6711
- **CONN et al.** *Nature Review Neuroscience,* 2005, vol. 6, 787-798
- **MARINO et al.** *Amino Acids,* 2002, vol. 23, 185-191
- **KNOFLACH F. et al.** *Proc. Natl. Acad. Sci. USA,* 2001, vol. 98, 13402-13407
- **JOHNSON M.P. et al.** *Neuropharmacology,* 2002, vol. 43, 799-808
- **O'BRIEN J.A. et al.** *Mol. Pharmacol.,* 2003, vol. 64, 731-740
- **JOHNSON M.P. et al.** *J. Med. Chem.,* 2003, vol. 46, 3189-3192
- **MARINO M.J. et al.** *Proc. Natl. Acad. Sci. USA,* 2003, vol. 100, 13668-13673
- **MITSUKAWA K. et al.** 2005. *Proc. Natl. Acad. Sci. USA,* vol. 102 (51), 18712-18717
- **WILSON J. et al.** *Neuropharmacology,* 2005, vol. 49, 278
- **MUTEL V.** *Expert Opin. Ther. Patents,* 2002, vol. 12, 1-8
- **KEW J.N.** *Pharmacol. Ther.,* 2004, vol. 104 (3), 233-244
- **JOHNSON M.P. et al.** *Biochem. Soc. Trans.,* 2004, vol. 32, 881-887
- **RITZEN A. ; MATHIESEN, J.M. ; THOMSEN C.** *Basic Clin. Pharmacol. Toxicol.,* 2005, vol. 97, 202-213
- **MAJ et al.** *Neuropharmacology,* 2003, vol. 45, 895-906
- **MATHIESEN et al.** *British Journal of Pharmacology,* 2003, vol. 138, 1026-1030
- **MARINO et al.** *Proc. Nat. Acad. Sci. USA,* 2003, vol. 100, 13668-13673
- **VALENTI et al.** *J. Pharmacol. Exp. Ther.,* 2005, vol. 313, 1296-1304
- **VERNON et al.** *Eur. J. Neurosci.,* 2005, vol. 22, 1799-1806
- **BATTAGLIA et al.** *J. Neurosci.,* 2006, vol. 26, 7222-7229
- **STACHOWICZ et al.** *Eur. J. Pharmacol.,* 2004, vol. 498, 153-156
- **TATARCZYNSKA et al.** *Pol. J. Pharmacol.,* 2002, vol. 54 (6), 707-710
- **STACHOWICZ et al.** *Neuropharmacology,* 2009, vol. 57 (3), 227-234
- **UEHARA et al.** *Diabetes,* 2004, vol. 53, 998-1006
- **BESONG et al.** *Journal of Neuroscience,* 2002, vol. 22, 5403-5411
- **STOVER J.F. et al.** *Eur. J. Clin. Invest.,* 1997, vol. 27, 1038-1043
- **SRINIVASAN R. et al.** *Brain,* 2005, vol. 128, 1016-1025
- **FRIGO M. et al.** *Current Med. Chem.,* 2012, vol. 19, 1295-1299
- **FALLARINO et al.** *Nature Medicine,* 2010, vol. 16, 897-902
- **GOUDET C. et al.** *Brain Res. Rev.,* 2009, vol. 60, 43-56
- *Pain,* 2008, vol. 137, 112-24

- **WANG H. et al.** *NeuroReport,* 2011, vol. 22, 244-248
- **PALUCHA-PONIEWIERA et al.** *Neuropharmacology,* 2008, vol. 55, 517-24
- **WIERONSKA et al.** *Psychopharmacology,* 2012, vol. 220 (3), 481-494
- **MONASTYRSKAIA et al.** *Br. J Pharmacol.,* 1999, vol. 128, 1027-1034
- **TOYONO et al.** *Arch. Histol. Cytol.,* 2002, vol. 65, 91-96
- **PAGE et al.** *Gastroenterology,* 2005, vol. 128, 402-10
- **YOUNG et al.** *Neuropharmacol,* 2008, vol. 54, 965-975
- **NISWENDER et al.** *Bioorganic & Medicinal Chemistry Letters,* 2008, vol. 18 (20), 5626-5630
- **WILLIAMS et al.** *Bioorganic & Medicinal Chemistry Letters,* 2009, vol. 19, 962-966
- **ENGERS et al.** *Journal of Medicinal Chemistry,* 2009, vol. 52 (14), 4115-4118
- **NISWENDER et al.** *Molecular Pharmacology,* 2008, vol. 74 (5), 1345-1358
- **EAST STEPHEN P. et al.** *Expert Opin. Ther. Patents,* 2010, vol. 20 (3), 441-445
- **WILLIAMS R. et al.** Re-exploration of the PHCCC scaffold. *ACS Chemical Neuroscience,* 2010, vol. 1 (6), 411-419
- **ROBICHAUD A. et al.** *ACS Chem. Neuroscience,* 2011, vol. 2, 433-49
- **HONG S.-P et al.** *J. Med. Chem.,* 2011, vol. 54 (14), 5070-5081
- Remington's Pharmaceutical Sciences. Mack Publishing company, 1985
- **STAHL ; WERMUTH.** Handbook of Pharmaceutical Salts: Properties, Selection, and Use. Wiley-VCH, 2002
- **REMINGTON.** the Science and Practice of Pharmacy. Mack Publishing Co, 1995
- **GREEN T.W. ; WUTS P.G.M.** Protecting Groups in Organic Synthesis. John Wiley & Sons, 1991
- **ELIEL E.L. ; WILEN S.H. ; MANDER L.N.** Stereochemistiy of Organic Compounds. Wiley-Interscience, 1984
- **KATRIZKY A.R. ; REES C.W.** Comprehensive Heterocyclic Chemistry. Pergamon Press, 1984
- **MILLAN, M.J. et al.** *Neuropharmacology,* 2002, vol. 42, 677-684